# EUROPEAN PATENT APPLICATION

(11) **EP 4 365 175 A1**
(43) Date of publication of application: **08.05.2024**
(21) Application number: 22833709.3
(22) Date of filing: 01.07.2022
(51) Int. Cl.: C07D 491/147, A61K 31/5025, A61K 31/5377, A61P 35/00

(54) **NOVEL BENZOPYRAN DERIVATIVE AND USE THEREOF**

(30) Priority: 02.07.2021 KR 20210086949
(71) Applicant: Spark Biopharma, Inc., Seoul 08791 (KR)
(72) Inventor: AHN, Sung Oh, Seoul 08791 (KR); PARK, Byung Sun, Seoul 08791 (KR); LIM, Tae Il, Seoul 08791 (KR); SEO, Ji Soo, Seoul 08791 (KR); KIM, Hyeon Ji, Seoul 08791 (KR); JUNG, Na Jin, Seoul 08791 (KR); KIM, Hyo Young, Seoul 08791 (KR); GIL, Ki Cheol, Seoul 08791 (KR); LEE, Hyeon Ju, Seoul 08791 (KR)
(74) Representative: Schön, Christoph
(86) International application number: PCT/KR2022/009541
(87) International publication number: WO 2023/277656

(57) **Abstract**

The present invention relates to a novel benzopyran derivative useful for preparing a drug for treating cancer, and more specifically, to a novel benzopyran derivative which is a micromolecule having a macrophage inhibitory effect and is useful for preparing a drug for cancer treatment. The drug particularly inhibits the differentiation of immune macrophages to stimulate the immune system, and thus is useful for treating immune-related cancers.

## Description

### TECHNICAL FIELD

The present invention relates to a novel benzopyran derivative useful in the manufacture of medicine for treating cancer diseases. More specifically, the present invention relates to a novel benzopyran derivative that is a low-molecular-weight compound having a macrophage inhibitory effect and is useful in the manufacture of medicine for anti-cancer treatment.

The above medicine is specifically useful in the treatment of immune-related cancer diseases by means of stimulating the immune system by inhibiting the differentiation of immune macrophages.

### BACKGROUND ART

Unlike conventional anticancer drugs that attack the cancer itself, immunotherapy drugs are therapeutic drugs that induce immune cells to selectively attack only cancer cells by stimulating the body's immune system. Among the various immune functions of the human body, tumor immune surveillance is a system in which cancer cells are recognized by T cells among the host's immune cells during the development and growth stages of cancer cells, and various cytokines secreted by T cells mobilize immune cells around the tumor and kill cancer cells. Accordingly, cancer cells go through a process of tumor immunoediting to avoid attack by T cells, and it is known that the cancer cells themselves induce immune tolerance to evade the immune surveillance function of the human immune system, thereby enabling cancer cells' growth and metastasis (References: Nature Reviews Clinical Oncology 16 (2019) 151-167; Cancer Immunotherapy, Immune Suppression and Tumor Growth, 2013, Chapter 7).

Therefore, it can be said that cancer immunotherapy drugs are drugs developed with a mechanism to restore or strengthen the tumor recognition or destruction ability of the immune system in order to overcome the immunosuppression or immune evasion mechanism acquired by cancer cells. Recently, representative cancer immunotherapy drugs include immune checkpoint inhibitors (CTLA4 inhibitors, PD-1 inhibitors, PD-L1 inhibitors), immune cell therapy, anti-cancer vaccine, immune virus therapy and the like.

The tumor microenvironment (TME) is a complex tumor cell environment that develops with cancer cells and allows them to grow in the course of the tumor transforming into a malignant tumor. Recent studies on the tumor microenvironment have revealed that among various immune cells, there are cells that have a function of actively supporting or promoting cancer growth, unlike the classic host defense mechanism function. As such, research is being actively conducted to develop treatments that inhibit cancer cell growth by regulating the activity of these cells. Immune cells present in the tumor microenvironment are largely divided into myeloid cells and lymphocyctes. These immune cells originate from hematopoietic stem cells. The most abundant hematopoietic stem cells in the human body are mainly present in bone marrow and lymphoid tissue, and myeloid cells go through several differentiation stages and ultimately differentiate into mast cells, macrophages, dendritic cells and granulocytes (neutrophil, eosinophil, and basophil). Lymphocytes differentiate into B cells, T cells, NK cells and the like. Depending on their function, these cells do tumor promoting (pro-tumoral), or tumor killing or suppressing (anti-tumoral) actions on cancer cells, respectively.

Tumor-associated macrophages (TAMs) are cells that are terminally differentiated from myeloid cells, and migrate to the tumor microenvironment through the blood and differentiate. These macrophages are polarized into M1 macrophages and M2 macrophages under various physiological and pathological conditions. M1 macrophages are activated by stimuli such as external pathogens and interferon gamma, secrete IL-12 to cause an inflammatory response, and are known to suppress cancer in the tumor microenvironment. M2 macrophages are known to have anti-inflammatory effects when activated by IL-4, IL-10, IL-13 and the like, and also promote cancer growth by secreting IL-10, TGF-b, VGEF and the like. In addition, M2 macrophages weaken the cytotoxicity of T cells through immune function, promote the proliferation of Th2 cells instead of Th1 cells, and activate regulatory T cells (Treg) to cause immune tolerance. As for non-immune actions, they directly promote angiogenesis, support the invasion and metastatic ability of tumor cells, and induce tumor growth by suppressing cell death caused by anticancer drugs. In addition, for these reasons, it has been reported that the presence of tumor-associated macrophages (TAM) is clinically associated with poor prognosis in various types of cancer (References: European Jounal of Cancer 42 (2006) 717-727; Clinics 66 (2011) 1879-1886; Cancer Immunotherapy, Immune Suppression and Tumor Growth, 2013, Chapter 27; Med Biol Sci Eng 2 (2019) 1-5).

Recently, various studies have been attempted to induce selective re-polarization from M2 macrophages to M1 macrophages as a study to achieve therapeutic effects for cancer diseases by controlling the polarization of macrophages, but relatively little research has been published on small molecule drugs that regulate the polarization for developing to M2 macrophages (References: Korean Patent Application Publication No. 10-2020-0120624, Method and Composition for Macrophage Polarization; ACS Cent. Sci. 6 (2020) 1208-1222; International Journal of Oncology 50 (2017) 545-554; Inflammation, 43 (2020), 95-108; Cells 9 (2020) 1-24).

Therefore, the present invention is intended to develop a therapeutic agent that inhibits cancer growth by blocking polarization of M2-like tumor-associated macrophages (TAM) using benzopyran derivative small molecule compounds.

### [Prior Art Documents]

### [Patent Documents]

Korean Patent Application Publication No. 10-2020-0120624
Korean Patent Publication No. 10-0799246
Korean Patent Application Publication No. 10-2012-0060957
Korean Patent Application Publication No. 10-2016-0017934

### [Non-patent Documents]

Nature Reviews Clinical Oncology 16 (2019) 151-167
Cancer Immunotherapy, Immune Suppression and Tumor Growth, 2013, Chapter 7
European Jounal of Cancer 42 (2006) 717-727
Clinics 66 (2011) 1879-1886
Cancer Immunotherapy, Immune Suppression and Tumor Growth, 2013, Chapter 27
Med Biol Sci Eng 2 (2019) 1-5
ACS Cent. Sci. 6 (2020) 1208-1222
International Journal of Oncology 50 (2017) 545-554
Inflammation, 43 (2020), 95-108
Cells 9 (2020) 1-24
Chem. Commun., 2011, 47, 12754-12761
ACS Comb. Sci. 2012, 14, 124-134
J. Am. Chem. Soc. 2014, 136, 14629-14638

### DISCLOSURE OF INVENTION

### TECHNICAL PROBLEM

The technical problem to be solved by the present invention is the provision of a novel benzopyran derivative that can enhance immune activity in tumor by suppressing the polarization of tumor-associated macrophages (TAM) in the tumor microenvironment (TME), or a pharmaceutically acceptable salt or isomer thereof.

In addition, another technical problem to be solved by the present invention is the provision of a pharmaceutical composition comprising the benzopyran derivate, or a pharmaceutically acceptable salt or isomer thereof as an active ingredient, together with a pharmaceutically acceptable carrier.

### SOLUTION TO PROBLEM

According to one aspect of the present invention, there is provided a compound of the following Formula 1, or a pharmaceutically acceptable salt or isomer thereof: 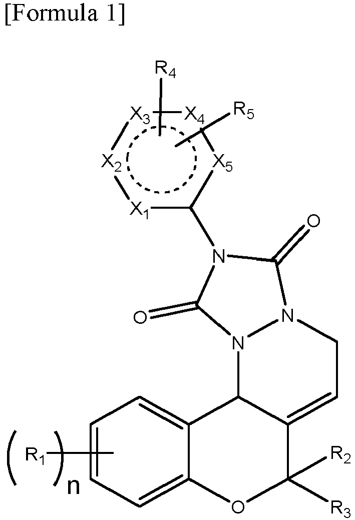 wherein
X₁, X₂, X₃, X₄ and X₅ are each independently carbon or nitrogen;
R₁ is halogen, hydroxy, nitro, amino, cyano, alkyl, cycloalkyl, alkylamino, dialkylamino, alkylcarbonyl, haloalkyl, haloalkoxy, hydroxyalkyl, hydroxyalkoxy, alkylsulfonyl, alkylsulfonylamino, aminosulfonyloxy, 3- to 10-membered heterocycloalkyl, 5- to 10-membered heteroaryl, 3- to 10-membered heterocycloalkyl-oxy, 3- to 10-membered heterocyclyl-alkoxy, or any one of the following formulas (i) to (vii), wherein the heterocycloalkyl or heteroaryl may be substituted with one or more substituents selected from the group consisting of hydroxy, carboxy, alkyl, alkylsulfonyl, alkylcarbonyl, oxo, hydroxyalkyl and halophenyl; 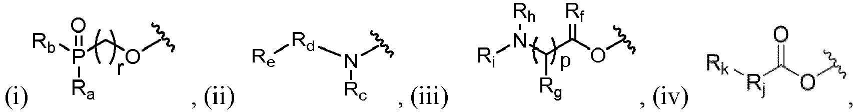 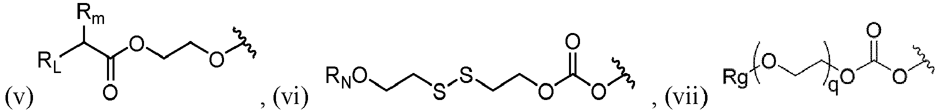
Ra and Rb are each independently hydroxy or alkoxy;
Rc is hydrogen or alkyl;
Rd is a direct bond, carbonyl or sulfonyl;
Re is hydrogen, alkyl, haloalkyl, alkoxy, amino or 3- to 10-membered heterocycloalkyl;
Rf is O or S;
Rg is hydrogen, alkyl, aminocarbonylalkyl or guanidinoalkyl;
Rh is hydrogen, alkyl, dialkylaminoalkyl, or may be fused with Ri to form 3- to 12-membered heterocycloalkyl, wherein the heterocycloalkyl may be substituted with one or more substituents selected from the group consisting of alkyl, oxo, aminoalkyl, dialkylaminoalkylcarbonyl, alkoxycarbonyl, acetylcarbonyl, 3- to 10-membered heterocycloalkyl, 5- to 10-membered heteroaryl, 3- to 10-membered heterocycloalkylcarbonyl, 3- to 10-membered heterocycloalkyl-alkyl, 5- to 10-membered heteroarylalkyl, acetylcarbonylheterocycloalkyl, alkoxyalkoxyalkoxyalkylcarbonyl and alkylcarbonyl substituted with amino;
Ri is hydrogen, alkyl, aminoalkyl, carboxyalkyl, alkylaminoalkyl, dialkylaminoalkyl, alkylsulfonyl, alkylcarbonyl, 3- to 10-membered heterocycloalkyl, alkyl substituted with alkoxycarbonyl, guanidinoalkyl substituted with carboxy, acetylamino or alkoxycarbonylalkyl;
Rj is a direct bond, alkylene, arylene-alkyleneoxy or alkylene substituted with alkylcarbonylamino;
Rk is alkyl, alkoxycarbonylalkyl, alkoxy, trialkylamino, nitro, alkyl substituted with amino, aminocarbonylalkyl, 3- to 10-membered heterocycloalkyl or 5- to 10-membered heteroaryl;
RL is amino or dialkylamino;
Rm is hydrogen or alkyl;
RN is hydrogen or carboxyalkylcarbonyl;
R₂ and R₃ are each independently alkyl or haloalkyl;
R₄ is hydrogen, halogen, alkyl, alkylcarbonyl, haloalkyl, carboxy, hydroxyalkyl, 3- to 10-membered heterocycloalkylcarbonyl, or 3- to 10-membered heterocycloalkylcarbonyl substituted with alkyl or alkoxycarbonyl;
R₅ is hydrogen, halogen or alkyl;
n is an integer of 1 to 3;
r and p are an integer of 0 to 3; and
q is an integer of 1 to 4;
provided that when R₁ is hydroxy and n is 1, i) R₂ and R₃ are not alkyl at the same time, ii) at least one of X₁, X₂, X₃, X₄ and X₅ is nitrogen, or iii) R₄ is hydroxyalkyl, 3- to 10-membered heterocycloalkylcarbonyl, or 3- to 10-membered heterocycloalkylcarbonyl substituted with alkyl or alkoxycarbonyl; and
when R₁ is halogen or alkylamino, R₄ and R₅ are not hydrogen at the same time; and
wherein the heterocycloalkyl and heteroaryl have one or more heteroatoms selected from the group consisting of N, O and S.

The present invention is described in detail hereinafter.

The following terms in the present invention have the meanings described below unless otherwise indicated. Any undefined terms have meanings understood in the art.

In the present invention, the term "halo" or "halogen," either alone or in combination with additional terms (for example, haloalkyl), refers to fluorine (F), chlorine (Cl), bromine (Br) or iodine (I).

In the present invention, the term "hydroxy" group refers to -OH.

In the present invention, the term "nitro" group refers to -NO₂.

In the present invention, the term "amino", alone or in combination, may refer to a primary, secondary or tertiary amino group bonded through a nitrogen atom.

In the present invention, the term "cyano" group refers to -CN.

In the present invention, the term "carboxy" group refers to -COOH.

In the present invention, the term "carbonyl" group refers to -C(=O)-.

In the present invention, the term "sulfonyl" group refers to -S(=O)₂-.

In the present invention, the term "oxo" group refers to =O (i.e., oxygen with a double bond).

In the present invention, the term "guanidino" group refers to -NH-C(=NH)-NH₂.

In the present invention, the term "acetyl" group refers to -C(=O)-CH₃.

In the present invention, the term "alkyl," either alone or in combination with additional terms (for example, haloalkyl), refers to a radical of a linear or branched chain, saturated aliphatic hydrocarbon group having, for example, 1 to 7 carbon atoms or 1 to 5 carbon atoms. For example, the alkyl may include methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl and the like, but is not limited thereto.

In the present invention, the term "alkoxy" refers to alkyloxy (-O-alkyl group) having, for example, 1 to 7 carbon atoms or 1 to 5 carbon atoms.

In the present invention, the term "alkylene" refers to a divalent straight or branched chain radical of a saturated aliphatic hydrocarbon group having, for example, 1 to 7 carbon atoms or 1 to 5 carbon atoms.

In the present invention, the term "cycloalkyl" refers to a radical of a cyclic, saturated aliphatic hydrocarbon group having, for example, 3 to 10 carbon atoms or 3 to 8 carbon atoms. Typical examples of cycloalkyl group may include include cyclopropyl, cyclobutyl, cyclopentyl and cyclohexyl, but is not limited thereto.

In the present invention, the term "aryl" refers to an aromatic hydrocarbon group having, for example 6 to 10 carbon atoms. For example, the aryl may include phenyl and naphthyl, but is not limited thereto.

In the present invention, the term "arylene" refers to a divalent aromatic hydrocarbon group having, for example 6 to 10 carbon atoms.

In the present invention, the term "heterocycloalkyl" refers to a cyclic, for example, 3- to 10-membered or 4- to 8-membered saturated aliphatic hydrocarbon including one or more heteroatoms selected from N, O and S as a ring member.

In the present invention, the term "heteroaryl" refers to, for example 5- to 10-membered or 5- to 8-membered aromatic hydrocarbons which form a single or fused ring-which may be fused with benzo or cycloalkyl-including one or more heteroatoms selected from N, O and S as a ring member. Examples of heteroaryl include, but are not limited to, pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl , isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, furazanyl, benzofurazanyl, benzothiophenyl, benzothiazolyl, benzoxazolyl, quinazolinyl, quinoxalinyl, naphthyridinyl or furopyridinyl.

According to one embodiment of the present invention, in the above Formula 1,
X₁, X₂, X₃, X₄ and X₅ are each independently -CH=, =CH-, -N= or =N-; or -C= or =C-when it is substituted with R₄ or R₅;
R₁ is halogen, hydroxy, nitro, amino, cyano, C₁-C₇ alkyl, C₃-C₁₀ cycloalkyl, C₁-C₇ alkylamino, di(C₁-C₇ alkyl)amino, C₁-C₇ alkylcarbonyl, halo-C₁-C₇ alkyl, halo-C₁-C₇ alkoxy, hydroxy-C₁-C₇ alkyl, hydroxy-C₁-C₇ alkoxy, C₁-C₇ alkylsulfonyl, C₁-C₇ alkylsulfonylamino, aminosulfonyloxy, 3- to 10-membered heterocycloalkyl, 5- to 10-membered heteroaryl, 3- to 10-membered heterocycloalkyl-oxy, 3- to 10-membered heterocyclyl-alkoxy, or any one of the following formulas (i) to (vii), wherein the heterocycloalkyl or heteroaryl may be substituted with 1 to 4 substituents selected from the group consisting of hydroxy, carboxy, C₁-C₇ alkyl, C₁-C₇ alkylsulfonyl, C₁-C₇ alkylcarbonyl, oxo, hydroxy-C₁-C₇ alkyl and halophenyl; 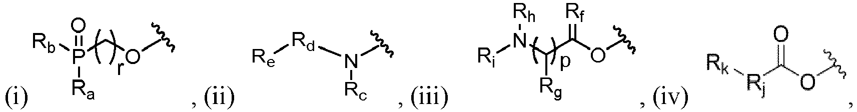 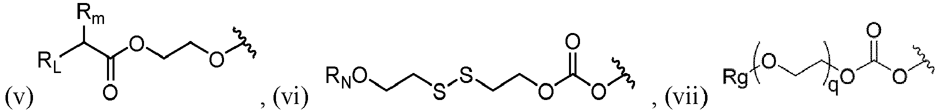
Ra and Rb are each independently hydroxy or C₁-C₇ alkoxy;
Rc is hydrogen or C₁-C₇ alkyl;
Rd is a direct bond, carbonyl or sulfonyl;
Re is hydrogen, C₁-C₇ alkyl, halo-C₁-C₇ alkyl, C₁-C₇ alkoxy, amino or 3- to 10-membered heterocycloalkyl;
Rf is O or S;
Rg is hydrogen, C₁-C₇ alkyl, aminocarbonyl-C₁-C₇ alkyl or guanidino-C₁-C₇ alkyl;
Rh is hydrogen, C₁-C₇ alkyl, di(C₁-C₇ alkyl)amino-C₁-C₇ alkyl, or may be fused with Ri to form 3- to 12-membered heterocycloalkyl, wherein the heterocycloalkyl may be substituted with 1 to 4 substituents selected from the group consisting of C₁-C₇ alkyl, oxo, amino-C₁-C₇ alkyl, di(C₁-C₇ alkyl)amino-C₁-C₇ alkylcarbonyl, C₁-C₇ alkoxycarbonyl, acetylcarbonyl, 3- to 10-membered heterocycloalkyl, 5- to 10-membered heteroaryl, 3- to 10-membered heterocycloalkylcarbonyl, 3- to 10-membered heterocycloalkyl-C₁-C₇ alkyl, 5- to 10-membered heteroaryl-C₁-C₇ alkyl, acetylcarbonyl-3- to 10-membered heterocycloalkyl, C₁-C₇ alkoxy-C₁-C₇ alkoxy-C₁-C₇ alkoxy-C₁-C₇ alkylcarbonyl and C₁-C₇ alkylcarbonyl substituted with amino;
Ri is hydrogen, C₁-C₇ alkyl, amino-C₁-C₇ alkyl, carboxy-C₁-C₇ alkyl, C₁-C₇ alkylamino-C₁-C₇ alkyl, di(C₁-C₇ alkyl)amino-C₁-C₇ alkyl, C₁-C₇ alkylsulfonyl, C₁-C₇ alkylcarbonyl, 3- to 10-membered heterocycloalkyl, C₁-C₇ alkyl substituted with C₁-C₇ alkoxycarbonyl, guanidino-C₁-C₇ alkyl substituted with carboxy, acetylamino or C₁-C₇ alkoxycarbonyl-C₁-C₇ alkyl;
Rj is a direct bond, C₁-C₇ alkylene, C₆-C₁₀ arylene-C₁-C₇ alkyleneoxy or C₁-C₇ alkylene substituted with C₁-C₇ alkylcarbonylamino;
Rk is C₁-C₇ alkyl, C₁-C₇ alkoxycarbonyl-C₁-C₇ alkyl, C₁-C₇ alkoxy, tri(C₁-C₇ alkyl)amino, nitro, C₁-C₇ alkyl substituted with amino, aminocarbonyl-C₁-C₇ alkyl, 3- to 10-membered heterocycloalkyl or 5- to 10-membered heteroaryl;
RL is amino or di(C₁-C₇ alkyl)amino;
Rm is hydrogen or C₁-C₇ alkyl;
RN is hydrogen or carboxy-C₁-C₇ alkylcarbonyl;
R₂ and R₃ are each independently C₁-C₇ alkyl or halo-C₁-C₇ alkyl;
R₄ is hydrogen, halogen, C₁-C₇ alkyl, C₁-C₇ alkylcarbonyl, halo-C₁-C₇ alkyl, carboxy, hydroxy-C₁-C₇ alkyl, 3- to 10-membered heterocycloalkylcarbonyl, or 3- to 10-membered heterocycloalkylcarbonyl substituted with C₁-C₇ alkyl or C₁-C₇ alkoxycarbonyl;
R₅ is hydrogen, halogen or C₁-C₇ alkyl;
n is an integer of 1 to 3;
r and p are an integer of 0 to 3; and
q is an integer of 1 to 4;
provided that when Ri is hydroxy and n is 1, i) R₂ and R₃ are not C₁-C₇ alkyl at the same time, ii) at least one of X₁, X₂, X₃, X₄ and X₅ is -N= or =N-, or iii) R₄ is hydroxy-C₁-C₇ alkyl, 3-to 10-membered heterocycloalkylcarbonyl, or 3- to 10-membered heterocycloalkylcarbonyl substituted with C₁-C₇ alkyl or C₁-C₇ alkoxycarbonyl; and
when R₁ is halogen or C₁-C₇ alkylamino, R₄ and R₅ are not hydrogen at the same time; and
wherein the heterocycloalkyl and heteroaryl have 1 to 4 heteroatoms selected from the group consisting of N, O and S.

According to one embodiment of the present invention, the compound of Formula 1 is a compound of the following Formula 2: 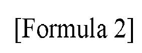 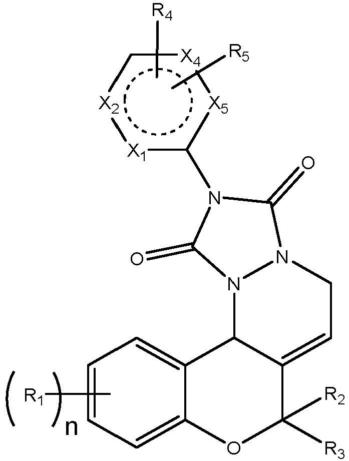 wherein X₁, X₂, X₄, X₅, R₁, R₂, R₃, R₄, R₅ and n are the same as defined in Formula 1.

According to one embodiment of the present invention, the compound of Formula 1 is a compound of the following Formula 3: 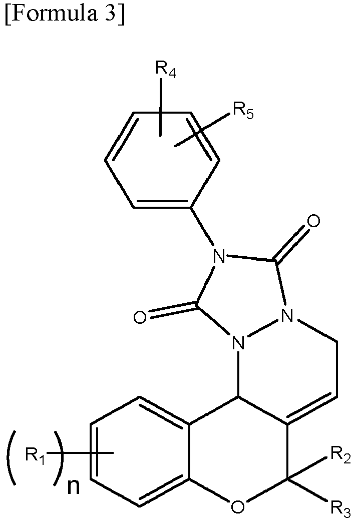 wherein R₁, R₂, R₃, R₄, R₅ and n are the same as defined in Formula 1.

According to one embodiment of the present invention, in the above Formula 1,
R₁ is halogen, hydroxy, nitro, amino, cyano, C₁-C₅ alkyl, C₃-C₈ cycloalkyl, C₁-C₅ alkylamino, di(C₁-C₅ alkyl)amino, C₁-C₅ alkylcarbonyl, halo-C₁-C₅ alkyl, halo-C₁-C₅ alkoxy, hydroxy-C₁-C₅ alkyl, hydroxy-C₁-C₅ alkoxy, C₁-C₅ alkylsulfonyl, C₁-C₅ alkylsulfonylamino, aminosulfonyloxy, 4- to 8-membered heterocycloalkyl, 5- to 8-membered heteroaryl, 4- to 8-membered heterocycloalkyl-oxy, 4-to 8-membered heterocyclyl-alkoxy, or any one of the following formulas (i) to (vii); wherein the heterocycloalkyl may be substituted with 1 to 4 substituents selected from the group consisting of hydroxy, carboxy, C₁-C₅ alkyl, C₁-C₅ alkylsulfonyl, C₁-C₅ alkylcarbonyl, oxo, hydroxy-C₁-C₅ alkyl and halophenyl; and the heteroaryl may be substituted with 1 to 3 substituents selected from the group consisting of hydroxy, C₁-C₅ alkyl, C₁-C₅ alkylsulfonyl, C₁-C₅ alkylcarbonyl and hydroxy-C₁-C₅ alkyl; 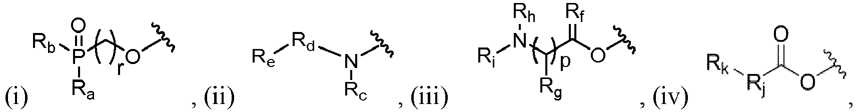 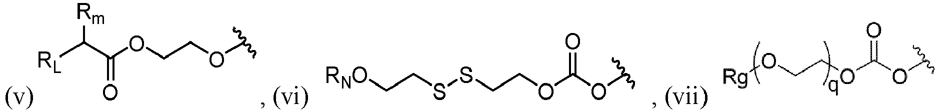
Ra and Rb are each independently hydroxy or C₁-C₅ alkoxy;
Rc is hydrogen or C₁-C₅ alkyl;
Rd is a direct bond, carbonyl or sulfonyl;
Re is hydrogen, C₁-C₅ alkyl, halo-C₁-C₅ alkyl, C₁-C₅ alkoxy, amino or 3- to 10-membered heterocycloalkyl;
Rf is O or S;
Rg is hydrogen, C₁-C₅ alkyl, aminocarbonyl-C₁-C₅ alkyl or guanidino-C₁-C₅ alkyl;
Rh is hydrogen, C₁-C₅ alkyl, di(C₁-C₅ alkyl)amino-C₁-C₅ alkyl, or may be fused with Ri to form 4- to 8-membered heterocycloalkyl, wherein the heterocycloalkyl may be substituted with one or more substituents selected from the group consisting of C₁-C₅ alkyl, oxo, amino-C₁-C₅ alkyl, di(C₁-C₅ alkyl)amino-C₁-C₅ alkylcarbonyl, C₁-C₅ alkoxycarbonyl, acetylcarbonyl, 4- to 8-membered heterocycloalkyl, 5- to 8-membered heteroaryl, 4-8 membered heterocycloalkylcarbonyl, 4- to 8-membered heterocycloalkyl-C₁-C₅ alkyl, 5- to 8-membered heteroaryl-C₁-C₅ alkyl, acetylcarbonyl-4- to 8-membered heterocycloalkyl, C₁-C₅ alkoxy-C₁-C₅ alkoxy-C₁-C₅ alkoxy-C₁-C₅ alkylcarbonyl and C₁-C₅ alkylcarbonyl substituted with amino;
Ri is hydrogen, C₁-C₅ alkyl, amino-C₁-C₅ alkyl, carboxy-C₁-C₅ alkyl, C₁-C₅ alkylamino-C₁-C₅ alkyl, di(C₁-C₅ alkyl)amino-C₁-C₅ alkyl, C₁-C₅ alkylsulfonyl, C₁-C₅ alkylcarbonyl, 3- to 10-membered heterocycloalkyl, C₁-C₅ alkyl substituted with C₁-C₅ alkoxycarbonyl, guanidino-C₁-C₅ alkyl substituted with carboxy, acetylamino or C₁-C₅ alkoxycarbonyl-C₁-C₅ alkyl;
Rj is a direct bond, C₁-C₅ alkylene, C₆-C₁₀ arylene-C₁-C₅ alkyleneoxy, C₁-C₅ alkylene substituted with C₁-C₅ alkylcarbonylamino;
Rk is C₁-C₅ alkyl, C₁-C₅ alkoxycarbonyl-C₁-C₅ alkyl, C₁-C₅ alkoxy, tri(C₁-C₅ alkyl)amino, nitro, C₁-C₅ alkyl substituted with amino, aminocarbonyl-C₁-C₅ alkyl, 4- to 8-membered heterocycloalkyl or 5- to 8-membered heteroaryl;
RL is amino or di(C₁-C₅ alkyl)amino;
Rm is hydrogen or C₁-C₅ alkyl; and
RN is hydrogen or carboxy-C₁-C₅ alkylcarbonyl.

According to one embodiment of the present invention, in the above Formula 1, R₂ and R₃ are each independently C₁-C₅ alkyl or halo-C₁-C₅ alkyl.

According to one embodiment of the present invention, in the above Formula 1,
R₄ is hydrogen, halogen, C₁-C₅ alkyl, C₁-C₅ alkylcarbonyl, halo-C₁-C₅ alkyl, carboxy, hydroxy-C₁-C₅ alkyl, 4- to 8-membered heterocycloalkylcarbonyl, or 4- to 8-membered heterocycloalkylcarbonyl substituted with C₁-C₅ alkyl or C₁-C₅ alkoxycarbonyl; and
R₅ is hydrogen, halogen or C₁-C₅ alkyl.

Representative examples of the compound of Formula 1 according to the present invention may include the following compounds, but are not limited thereto:
1) 7,7-bis(fluoromethyl)-10-hydroxy-2-phenyl-5,12b-dihydro-1*H*,7*H*-chromeno[4,3-*c*][1,2,4]triazolo[1,2-*a*]pyridazine-1,3(2*H*)-dione;
2) 2-(4-acetylphenyl)-7,7-bis(fluoromethyl)-10-hydroxy-5,12b-dihydro-1*H*,7*H-*chromeno[4,3-*c*][1,2,4]triazolo[1,2-*a*]pyridazine-1,3(2*H*)-dione;
3) *N*-(2-(4-acetylphenyl)-7,7-dimethyl-1,3-dioxo-2,3,5,12b-tetrahydroxy-1*H*,7*H-*chromeno[4,3-*c*][1,2,4]triazolo[1,2-*a*]pyridazin-10-yl)acetamide;
4) 2-(4-acetylphenyl)-7,7-dimethyl-10-(methylsulfonyl)-5,12b-dihydro-1*H*,7*H-*chromeno[4,3-*c*][1,2,4]triazolo[1,2-*a*]pyridazine-1,3(2*H*)-dione;
5) *N*-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)methanesulfonamide;
6) tert-butyl (2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)carbamate;
7) 2-(4-acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione;
8) 2-(4-acetylphenyl)-7,7-dimethyl-10-morpholino-5,12b-dihydro-1*H*,7*H*-chromeno[4,3-*c*][1,2,4]triazolo[1,2-*a*]pyridazine-1,3(2*H*)-dione;
9) *N*-(2-(4-acetylphenyl)-7,7-dimethyl-1,3-dioxo-2,3,5,12b-tetrahydro-1*H*,7*H-*chromeno[4,3-*c*][1,2,4]triazolo[1,2*-*a]pyridazin-10-yl)sulfuric diamide;
10) 2-(4-acetylphenyl)-9,11-dibromo-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
11) 2-(4-acetylphenyl)-9,11-dichloro-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
12) 2-(6-fluoropyridin-2-yl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
13) 2-(4-acetylphenyl)-7,7-dimethyl-10-nitro-5,12b-dihydro-1*H*,7*H*-chromeno[4,3-c][1,2,4]triazolo[1,2-*a*]pyridazine-1,3(2*H*)-dione;
14) 2-(4-acetylphenyl)-10-(hydroxymethyl)-7,7-dimethyl-5,12b-dihydro-1*H*,7*H-*chromeno[4,3-*c*][1,2,4]triazolo[1,2-*a*]pyridazine-1,3(2*H*)-dione;
15) 1-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)urea;
16) 2-(4-acetylphenyl)-10-(ethylamino)-7,7-dimethyl-5,12b-dihydro-1*H*,7H-chromeno[4,3-*c*][1,2,4]triazolo[1,2-*a*]pyridazine-1,3(2*H*)-dione;
17) 2-(4-acetylphenyl)-7,7-dimethyl-10-((2,2,2-trifluoroethyl)amino)-5,12b-dihydro-1*H*,7*H*-chromeno[4,3-*c*][1,2,4]triazolo[1,2-*a*]pyridazine-1,3(2*H*)-dione;
18) 2-(4-acetylphenyl)-7,7-dimethyl-10-(piperazin-1-yl)-5,12b-dihydro-1*H*,7*H-*chromeno[4,3-*c*][1,2,4]triazolo[1,2-*a*]pyRidazine-1,3(2*H*)-dione hydrochloride;
19) 2-(4-acetylphenyl)-10-(4-acetylpiperazin-1-yl)-7,7-dimethyl-5,12b-dihydro-1*H*,7*H-*chromeno[4,3-*c*][1,2,4]triazolo[1,2-*a*]pyridazine-1,3(2*H*)-dione;
20) 2-(5-acetylpyridin-2-yl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
21) 10-acetyl-2-(4-acetylphenyl)-7,7-dimethyl-5,12b-dihydro-1*H*,7*H*-chromeno[4,3-*c*][1,2,4]triazolo[1,2-*a*]pyridazine-1,3(2*H*)-dione;
22) 11-acetyl-2-(4-acetylphenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
23) 2-(6-acetylpyridin-3-yl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
24) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazine-10-carbonitrile;
25) 2-(4-acetylphenyl)-10-hydroxy-7,7-dimethyl-11-(trifluoromethoxy)-5,12b-dihydro-1*H*,7*H*-chromeno[4,3-*c*][1,2,4]triazolo[1,2-*a*]pyridazine-1,3(2*H*)-dione;
26) 2-(4-acetylphenyl)-11-cyclopropyl-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
27) 2-(4-acetylphenyl)-9,11-difluoro-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
28) 2-(4-acetylphenyl)-10-amino-9,11-dibromo-7,7-dimethyl-5,12b-dihydro-1*H*,7*H-*chromeno[4,3-*c*][1,2,4]triazolo[1,2-*a*]pyridazine-1,3(2*H*)-dione;
29) 2-(4-acetylphenyl)-10-(isopropylamino)-7,7-dimethyl-5,12b-dihydro-1*H*,7*H-*chromeno[4,3-*c*][1,2,4]triazolo[1,2-*a*]pyridazine-1,3(2*H*)-dione;
30) 2-(4-acetylphenyl)-10-(tert-butylamino)-7,7-dimethyl-5,12b-dihydro-1*H*,7*H-*chromeno[4,3-*c*][1,2,4]triazolo[1,2-*a*]pyridazine-1,3(2*H*)-dione;
31) 2-(4-acetylphenyl)-10-amino-9,11-dichloro-7,7-dimethyl-5,12b-dihydro-1*H*,7*H-*chromeno[4,3-*c*][1,2,4]triazolo[1,2-*a*]pyridazine-1,3(2*H*)-dione;
32) 2-(4-acetylphenyl)-7,7-dimethyl-10-(methylamino)-5,12b-dihydro-1*H*,7*H-*chromeno[4,3-*c*][1,2,4]triazolo[1,2-*a*]pyridazine-1,3(2*H*)-dione;
33) 2-(4-acetylphenyl)-10-(diethylamino)-7,7-dimethyl-5,12b-dihydro-1*H*,7*H-*chromeno[4,3-*c*][1,2,4]triazolo[1,2-*a*]pyridazine-1,3(2*H*)-dione;
34) 2-(4-acetylphenyl)-7,7-dimethyl-10-(pyrrolidin-1-yL)-5,12b-dihydro-1*H*,7*H-*chromeno[4,3-*c*][1,2,4]triazolo[1,2-*a*]pyridazine-1,3(2*H*)-dione;
35) 2-(4-fluorophenyl)-7,7-dimethyl-10-(oxetan-3-ylamino)-5,12b-dihydro-1*H*,7*H-*chromeno[4,3-*c*][1,2,4]triazolo[1,2-*a*]pyridazine-1,3(2*H*)-dione;
36) 2-(4-acetylphenyl)-9,11-dichloro-7,7-dimethyl-10-(methylamino)-5,12b-dihydro-1*H*,7*H*-chromeno[4,3-*c*][1,2,4]triazolo[1,2-*a*]pyridazine-1,3(2*H*)-dione;
37) 2-(4-acetylphenyl)-9,11-dichloro-10-(isopropylamino)-7,7-dimethyl-5,12b-dihydro-1*H*,7*H*-chromeno[4,3-*c*][1,2,4]triazolo[1,2-*a*]pyridazine-1,3(2*H*)-dione;
38) 2-(4-acetylphenyl)-10-(1,3-dimethyl-1H-pyrazol-5-yl)-7,7-dimethyl-5,12b-dihydro-1*H*,7*H*-chromeno[4,3-*c*][1,2,4]triazolo[1,2-*a*]pyridazine-1,3(2*H*)-dione;
39) 2-(4-acetylphenyl)-10-(2,5-dimethylthiazol-4-yl)-7,7-dimethyl-5,12b-dihydro-1H,7H-chromeno[4,3-*c*][1,2,4]triazolo[1,2-*a*]pyridazine-1,3(2*H*)-dione;
40) 2-(4-acetylphenyl)-10-(1-ethyl-3,5-dimethyl-1*H*-pyrazol-4-yl)-7,7-dimethyl-5,12b-dihydro-1*H*,7*H*-chromeno[4,3-*c*][1,2,4]triazolo[1,2-*a*]pyridazine-1,3(2*H*)-dione;
41) 2-(4-acetylphenyl)-7,7-dimethyl-10-(3-methylisoxazol-4-yl)-5,12b-dihydro-1*H*,7*H-*chromeno[4,3-*c*][1,2,4]triazolo[1,2-*a*]pyridazine-1,3(2*H*)-dione;
42) 2-(4-acetylphenyl)-7,7-dimethyl-10-(pyridin-3-yl)-5,12b-dihydro-1*H*,7*H-*chromeno[4,3-*c*][1,2,4]triazolo[1,2-*a*]pyridazine-1,3(2*H*)-dione;
43) 2-(4-acetylphenyl)-7,7-dimethyl-10-(pyridin-4-yl)-5,12b-dihydro-1*H*,7*H-*chromeno[4,3-*c*][1,2,4]triazolo[1,2-*a*]pyridazine-1,3(2*H*)-dione;
44) 4-(7,7-dimethyl-1,3-dioxo-10-(pyrrolidin-1-yl)-5,12b-dihydro-1*H*,7*H*-chromeno[4,3-*c*][1,2,4]triazolo[1,2-*a*]pyridazin-2(3*H*)-yl)benzoic acid;
45) 2-(4-acetylphenyl)-10-(isopropyl(methyl)amino)-7,7-dimethyl-5,12b-dihydro-1*H*,7*H-*chromeno[4,3-*c*][1,2,4]triazolo[1,2-*a*]pyridazine-1,3(2*H*)-dione;
46) 2-(4-acetylphenyl)-10-(ethyl(isopropyl)amino)-7,7-dimethyl-5,12b-dihydro-1*H*,7*H-*chromeno[4,3-*c*][1,2,4]triazolo[1,2-*a*]pyridazine-1,3(2*H*)-dione;
47) 2-(4-acetylphenyl)-10-(diisopropylamino)-7,7-dimethyl-5,12b-dihydro-1*H*,1*H-*chromeno[4,3-*c*][1,2,4]triazolo[1,2-*a*]pyridazine-1,3(2*H*)-dione;
48) 2-(4-(tert-butyl)phenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione;
49) 7,7-dimethyl-10-(4-methylpyrerazin-1-yl)-2-(4-(trifluoromethyl)phenyl)-5,12b-dihydro-1*H*,7*H*-chromeno[4,3-*c*][1,2,4]triazolo[1,2-*a*]pyridazine-1,3(2*H*)-dione;
50) 2-(4-fluorophenyl)-7,7-dimethyl-10-(4-methylpiperazin-1-yl)-5,12b-dihydro-1*H*,7*H-*chromeno[4,3-*c*][1,2,4]triazolo[1,2-*a*]pyridazine-1,3(2*H*)-dione;
51) 2-(4-acetylphenyl)-7,7-dimethyl-10-(4-methylpiperazin-1-yl)-5,12b-dihydro-1*H*,7*H-*chromeno[4,3-*c*][1,2,4]triazolo[1,2-*a*]pyridazine-1,3(2*H*)-dione;
52) 2-(4-acetylphenyl)-7,7-dimethyl-10-(4-(methylsulfonyl)piperazin-1-yl)-5,12b-dihydro-1*H*,7*H*-chromeno[4,3-*c*][1,2,4]triazolo[1,2-*a*]pyridazine-1,3(2*H*)-dione;
53) 10-hydroxy-7,7-dimethyl-2-(4-(morpholine-4-carbonyl)phenyl)-5,12b-dihydro-1*H*,7*H*-chromeno[4,3-*c*][1,2,4]triazolo[1,2-*a*]pyridazine-1,3(2*H*)-dione;
54) tert-butyl 4-(4-(10-hydroxy-7,7-dimethyl-1,3-dioxo-5,12b-dihydro-1*H*,7*H-*chromeno[4,3-*c*][1,2,4]triazolo[1,2-*a*]pyridazin-2(3*H*)-yl)benzoyl)piperazine-1-carboxylate;
55) 10-hydroxy-7,7-dimethyl-2-(4-(piperazine-1-carbonyl)phenyl)-5,12b-dihydro-1*H*,7*H-*chromeno[4,3-*c*][1,2,4]triazolo[1,2-*a*]pyridazine-1,3(2*H*)-dione;
56) 10-hydroxy-7,7-dimethyl-2-(4-(4-methylpiperazine-1-carbonyl)phenyl)-5,12b-dihydro-1*H*,7*H*-chromeno[4,3-*c*][1,2,4]triazolo[1,2-*a*]pyridazine-1,3(2*H*)-dione;
57) 7,7-dimethyl-2-(4-(morpholine-4-carbonyl)phenyl)-10-(pyrrolidin-1-yl)-5,12b-dihydro-1*H*,7*H*-chromeno[4,3-*c*][1,2,4]triazolo[1,2-*a*]pyridazine-1,3(2*H*)-dione;
58) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl pivalate;
59) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 3-methylbutanoate;
60) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl acetate;
61) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl ethyl carbonate;
62) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate;
63) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl morpholine-4-carboxylate;
64) (2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxytetrahydro-2*H*-pyran-2-carboxylic acid;
65) sodium (2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxy tetrahydro-2*H*-pyran-2-carboxylate;
66) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-methylpiperazine-1-carboxylate hydrochloride;
67) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl piperazine-1-carboxylate hydrochloride;
68) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(2-oxopropanoyl)piperazine-1-carboxylate;
69) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
70) disodium 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl phosphate;
71) 1,3-dihydroxy-2-(hydroxymethyl)propane-2-aminium 2-(4-acetylphenyl)-7,7-dimethyl-1,3-dioxo-2,3,5,12b-tetrahydro-1*H*,7*H*-chromeno[4,3-c][1,2,4]triazolo[1,2-a]pyridazin-10-yl phosphate;
72) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylglycinate hydrochloride;
73) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-methoxyethyl) carbonate;
74) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl pyrrolidine-1-carboxylate;
75) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2,5,8,11-tetraoxatridecan-13-yl) carbonate;
76) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl diethylcarbamate;
77) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-glutaminate 2,2,2-trifluoroacetic acid;
78) (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-arginine 2,2,2-trifluoroacetic acid;
79) 1-(2-(4-acetylphenyl)-7,7-dimethyl-1,3-dioxo-2,3,5,12b-tetrahydro-1*H*,7*H-*chromeno[4,3-c][1,2,4]triazolo[1,2-a]pyridazin-10-yl) 2-methyl (2*S*)-pyrrolidine-1,2-dicarboxylate;
80) methyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-leucinate;
81) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-leucinate 2,2,2-trifluoroacetic acid;
82) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-prolyl)piperazine-1-carboxylate hydrochloride;
83) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(2-(methylamino)ethyl)carbamate 2,2,2-trifluoroacetic acid;
84) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-aminoethyl)(ethyl)carbamate hydrochloride;
85) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(pyrrolidin-3-yl)carbamate hydrochloride;
86) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl bis(3-(dimethylamino)propyl)carbamate dihydrochloride;
87) *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N*-methyl-*L*-alanine;
88) sodium *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N*-methyl-*L*-alaninate;
89) *N-*(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N*-methylglycine;
90) sodium *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N*-methylglycinate;
91) 3-((((2-(4-acetylphenyl)-7,7-dimethyl-1,3-dioxo-2,3,5,12b-tetrahydro-1*H*,*7H-*chromeno[4,3-*c*][1,2,4]triazolo[1,2-*a*]pyridazin-10-yl)oxy)carbonyl)(methyl)amino)propanoic acid;
92) sodium 3-((((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)(methyl)amino)propanoate;
93) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(aminomethyl)piperidine-1-carboxylate trifluoroacetate;
94) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(piperidin-4-yl)piperazine-1-carboxylate dihydrochloride;
95) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(pyridin-2-ylmethyl)piperazine-1-carboxylate dihydrochloride;
96) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(2-(2-(2-methoxyethoxy)ethoxy)acetyl)piperazine-1-carboxylate;
97) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-valyl)piperazine-1-carboxylate formate;
98) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-leucyl)piperazine-1-carboxylate hydrochloride;
99) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(dimethylglycyl)piperazine-1-carboxylate hydrochloride;
100) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethylpiperazine-1-carboxylate hydrochloride;
101) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethyl-4-prolylpiperazine-1-carboxylate hydrochloride;
102) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-ylheptanoate;
103) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,2-dimethylbutanoate;
104) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl ethyl succinate;
105) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl diisopropylcarbamate;
106) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl isopropyl carbonate;
107) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-((2-hydroxyethyl)disulfanyl)ethyl) carbonate;
108) 4-(2-((2-((((2-(4-acetylphenyl)-7,7-dimethyl-1,3-dioxo-2,3,5,12b-tetrahydro-1*H*,7*H-*chromeno[4,3-c][1,2,4]triazolo[1,2-*a*]pyridazin-10-yl)oxy)carbonyl)oxy)ethyl)disulfanyl)ethoxy)-4-oxobutanoic acid;
109) sodium 4-(2-((2-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)oxy)ethyl)disulfanyl)ethoxy)-4-oxobutanoate;
110) 2-(4-acetylphenyl)-10-(2-hydroxyethoxy)-7,7-dimethyl-5,12b-dihydro-1*H*,7*H-*chromeno[4,3-*c*][1,2,4]triazolo[1,2-*a*]pyridazine-1,3(2*H*)-dione;
111) 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-a]pyridazin-10-yl)oxy)ethyl *L*-leucinate hydrochloride;
112) 2-(4-acetylphenyl)-7,7-dimethyl-10-(2-(piperidin-1-yl)ethoxy)-5,12b-dihydro-1*H*,7*H*-chromeno[4,3-*c*][1,2,4]triazolo[1,2-*a*]pyridazine-1,3(2*H*)-dione hydrochloride;
113) 2-(4-acetylphenyl)-7,7-dimethyl-10-(2-morpholinoethoxy)-5,12b-dihydro-1*H*,7*H-*chromeno[4,3-c][1,2,4]triazolo[l,2-a]pyridazine-1,3(2*H*)-dione formate;
114) (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)methyl)phosphonic acid;
115) disodium (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)methyl)phosphonate;
116) 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)ethyl dimethylglycinate hydrochloride;
117) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(1-(2-oxopropanoyl)piperidin-4-yl)piperazine-1-carboxylate hydrochloride;
118) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,2-dimethylpiperazine-1-carboxylate hydrochloride;
119) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(dimethylglycyl)-2,2-dimethylpiperazine-1-carboxylate hydrochloride;
120) 2-(4-acetylphenyl)-10-(3-(4-(3-chlorophenyl)piperazin-1-yl)propoxy)-7,7-dimethyl-5,12b-dihydro-1*H*,7*H*-chromeno[4,3-c][1,2,4]triazolo[1,2-a]pyridazine-1,3(2*H*)-dione dihydrochloride;
121) 2-(4-acetylphenyl)-7,7-dimethyl-10-(((2*S*,3*R*,4*S*,5*S*,6*R*)-3,4,5-trihydroxy-6-(hydroxymethyl)tetrahydro-2*H*-pyran-2-yl)oxy)-5,12b-dihydro-1*H*,7*H*-chromeno[4,3-c][1,2,4]triazolo[l,2-a]pyridazine-1,3(2*H*)-dione;
122) 2-(4-acetylphenyl)-7,7-dimethyl-10-(((2*S*,3*R*,4*S*,5*R*,6*R*)-3,4,5-trihydroxy-6-(hydroxymethyl)tetrahydro-2H-pyran-2-yl)oxy)-5,12b-dihydro-1*H*,7*H*-chromeno[4,3-*c*][1,2,4]triazolo[1,2-*a*]pyridazine-1,3(2*H*)-dione;
123) 2-((2-(4-acetylphenyl)-7,7-dimethyl-1,3-dioxo-2,3,5,12b-tetrahydro-1*H*,7*H-*chromeno[4,3-c][1,2,4]triazolo[1,2-a]pyridazin-10-yl)oxy)-*N,N,N*-trimethyl-2-oxoethan-1-aminium iodide;
124) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (4-nitrobenzyl) carbonate;
125) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl piperidine-1-carboxylate;
126) *O*-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl) dimethylcarbamothioate;
127) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2-oxoimidazolidine-1-carboxylate;
128) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (3-(dimethylamino)-2,2-dimethylpropyl)carbamate formate;
129) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-(dimethylamino)ethyl)(methyl)carbamate formate;
130) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(piperidin-3-yl)carbamate hydrochloride;
131) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(methylsulfonyl)carbamate;
132) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-valinate 2,2,2-trifluoroacetic acid;
133) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-prolinate 2,2,2-trifluoroacetic acid;
134) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl acetyl-*L*-glutaminate;
135) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-argininate 2,2,2-trifluoroacetic acid;
136) methyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-valinate;
137) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl sulfamate;
138) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl picolinate hydrochloride;
139) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl isonicotinate hydrochloride;
140) 10-hydroxy-2-(4-(1-hydroxyethyl)phenyl)-7,7-dimethyl-5,12b-dihydro-1*H*,7*H-*chromeno[4,3-*c*][1,2,4]triazolo[1,2-*a*]pyridazine-1,3(2*H*)-dione;
141) 2-(4-acetylphenyl)-10-(azetidin-1-yl)-7,7-dimethyl-5,12b-dihydro-1*H*,*7H-*chromeno[4,3-*c*][1,2,4]triazolo[1,2-*a*]pyridazine-1,3(2*H*)-dione;
142) 7,7-dimethyl-1,3-dioxo-2-(4-(trifluoromethyl)phenyl)-2,3,5,12b-tetrahydro-1*H*,7*H-*chromeno[4,3-*c*][1,2,4]triazolo[1,2-*a*]pyridazin-10-yl dihydrogen phosphate;
143) 2-(4-fluorophenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
144) 2-(4-(tert-butyl)phenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
145) 7,7-dimethyl-1,3-dioxo-2-(4-(trifluoromethyl)phenyl)-2,3,5,12b-tetrahydro-1*H*,7*H-*chromeno[4,3-*c*][1,2,4]triazolo[1,2-*a*]pyridazin-10-yl dimethylcarbamate;
146) 2-(4-fluorophenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate; and
147) 2-(4-(tert-butyl)phenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate.

The above-listed names of the compounds are described in accordance with the nomenclature method provided by CambridgeSoft's CS ChemDraw Ultra (version: 19.0.1.28) software.

The compound of Formula 1 according to the present invention may have an asymmetric carbon center and an asymmetric axis or an asymmetric plane, so it may exist as stereoisomers such as E- or Z-isomers, R- or S-isomers, and racemates, and all of these isomers and mixtures are included in the scope of the invention. The compound of Formula 1 according to the present invention may be a racemate, and this racemate may be separated by a conventional separation method-for example, using a chiral column chromatography device in the normal phase or reverse phase, using a corresponding developing solvent, preferably a mixture of hexane, ethyl acetate, dichloromethane and methanol in the normal phase, and a mixed solution of water and acetonitrile in reverse phase.

In addition, the compound of Formula 1 according to the present invention may form pharmaceutically acceptable salts. Representative acids that can be used in the preparation of such pharmaceutically acceptable salts include, but are not limited to, the following. Examples include acids that form non-toxic acid addition salts containing anions, for example, inorganic acids such as hydrochloric acid, sulfuric acid, nitric acid, phosphoric acid, hydrobromic acid, hydroiodic acid; organic carbonic acids such as tartaric acid, formic acid, citric acid, acetic acid, trichloroacetic acid or trifluoroacetic acid, gluconic acid, benzoic acid, lactic acid, fumaric acid, maleic acid; and sulfonic acids such as methanesulfonic acid, benzenesulfonic acid, p-toluenesulfonic acid or naphthalsulfonic acid. In addition, salts with alkali metals such as sodium and potassium are included. Furthermore, salts with other acids or bases such as aromatic amidine derivatives or lactam derivatives that are known and used in the technical field may be also included. They may be manufactured by commonly known processes.

Compounds of the present invention having the structure of Formula 1 can be prepared according to the method described in the Examples below, but are not limited thereto.

According to another aspect of the present invention, there is provided a pharmaceutical composition comprising a therapeutically effective amount of the compound of Formula 1, or a pharmaceutically acceptable salt or isomer thereof as an active ingredient, together with a pharmaceutically acceptable carrier.

The compound of Formula 1 according to the present invention has the activity to treat cancer diseases. The compound of formula 1 according to the present invention is a therapeutic agent for cancer diseases, and is useful in the treatment of cancer diseases, specifically cancer disease selected from the group consisting of colon cancer, skin cancer, melanoma, glioblastoma, bone cancer, liver cancer, stomach cancer, pancreas cancer, colon cancer, rectal cancer, blood cancer, bladder cancer, kidney cancer, biliary tract cancer, cervical cancer, uterine cancer, ovarian cancer, breast cancer, lung cancer, non-small cell lung cancer, esophageal cancer, small intestine cancer, endocrine cancer, thyroid cancer and parathyroid cancer.

A pharmaceutical composition according to the present invention may be prepared by mixing a therapeutically effective amount of a compound of Formula 1, or a pharmaceutically acceptable salt or isomer thereof as an active ingredient, with a pharmaceutically acceptable carrier, binder, stabilizer and/or diluent. In addition, when the pharmaceutical composition according to the present invention is prepared in an injection liquid form, a pharmaceutically acceptable buffer, dissolution adjuvant and/or isotonic agent may be mixed with the compound of Formula 1, or a pharmaceutically acceptable salt or isomer thereof.

The pharmaceutical composition according to the prevent invention may be prepared in a delivery form of a pharmaceutical composition comprising one or more dosage units of pharmaceutical agent by using a preparation technique known or available to a skilled artisan, and a suitable pharmaceutical excipient. In a method of the present invention, the composition may be administered via suitable delivery route, for example, such as oral or parenteral, percutaneous, rectal, topical or ocular administration, or by inhalation. The pharmaceutical formulation may be in a form of tablet, capsule, sachet, sugar-coated pill, powder, granule, lozenge, powder for reconstitution, liquid preparation or suppository. For example, the composition may be formulated in a form for intravenous injection, spray, topical or oral administration.

In case of preparing a formulation in oral dosage form, any conventional pharmaceutical carriers may be used. For example, water, glycols, oils, alcohols and the like may be used as a carrier in case of oral liquid formulations such as suspensions, syrups, elixirs and solutions; and starches, sugars, kaolin, lubricants, binders, disintegrating agents and the like may be used as a carrier in case of solid formulations such as powders, pills, capsules and tablets. Because of the easiness of administration, tablets and capsules are the most convenient dose forms, and tablets and pills are preferably prepared as enteric coating formulations.

In case of parenteral formulations, sterilized water is used usually and other ingredient(s) such as a dissolution adjuvant may also be comprised. Injection formulations, for example, sterilized aqueous- or oil-based suspension for injection may be prepared according to known techniques by using appropriate dispersing agent, wetting agent or suspending agent. The solvents useful for this purpose include water, ringer solution and isotonic NaCl solution, and sterilized, immobilized oils are also used as a solvent or a suspending medium conventionally. Any non-irritant immobilized oils including mono- and di-glycerides may be used for this purpose, and fatty acids such as an oleic acid may be used for an injection formulation.

In case of percutaneous formulations, a penetration-enhancing agent and/or a suitable wetting agent may be used as a carrier, optionally in combination with suitable non-irritant additive(s) to the skin. As such additives, those helpful in enhancing the administration through the skin and/or preparing the desired composition may be selected. The percutaneous formulation may be administered in various ways, for example, such as a transdermal patch, a spot-on treatment or an ointment.

The administration time and dosage of the pharmaceutical composition according to the present invention may be suitably determined according to the patient's disease, condition, age, body weight and administration form. In case of adults, the pharmaceutical composition may be administered in an amount of 0.1-2,000 mg, preferably 1-200 mg per day, in a single dose or multiple doses, but not limited thereto.

### EFFECTS OF INVENTION

The compound of Formula 1, or a pharmaceutically acceptable salt or isomer thereof according to the present invention has an inhibitory effect against M2 macrophage polarization. Specifically, the compound of Formula 1, or a pharmaceutically acceptable salt or isomer thereof according to the present invention shows excellent anti-cancer effects in a syngenic mouse tumor model in which melanoma B16F10 cells are transplanted into C57BL/6 mice, and can efficiently treat cancer diseases such as skin cancer, various solid cancers, and blood cancers through monotherapy or combined therapy with conventional immunosuppressants.

### MODE FOR THE INVENTION

Hereinafter, the present invention is explained in more detail with the following examples and experimental examples. However, it must be understood that the protection scope of the present disclosure is not limited to the examples and experimental examples.

The abbreviations used in the following examples are defined as follows.

**[Table 1]**

| **Abbreviation** | **Full Name** |
|---|---|
| CDCl₃ | Chloroform |
| D₂O | Deuterium oxide |
| DMSO-D₆ | Dimethyl sulfoxide |
| MeOH | Methanol |
| MPLC | Medium pressure liquid chromatography |
| Xphos | 2-Dicyclohexylphosphino-2',4',6'-triisopropylbiphenyl |

### Intermediate 1: Synthesis of 4-(4-acetylphenyl)-1,2,4-triazolidine-3,5-dione

### (a) Synthesis of methyl 2-((4-acetylphenyl)carbamoyl)hydrazine-1-carboxylate

*N*-aminocarbamate (5.59 g, 62.05 mmol) was dissolved in tetrahydrofuran (400 mL), and 1-(4-isocyanatophenyl)ethan-1-one (10 g, 62.05 mmol) was added thereto and stirred at room temperature for 15 hours. When the reaction was completed, the obtained solid was filtered, washed with hexane (200 mL) and dried to obtain methyl 2-((4-acetylphenyl)carbamoyl)hydrazine-1-carboxylate (15.4 g, 98%).

### (b) Synthesis of 4-(4-acetylphenyl)-1,2,4-triazolidine-3,5-dione

Methyl 2-((4-acetylphenyl)carbamoyl)hydrazine-1-carboxylate (15 g, 59.7 mmol) and potassium carbonate (37.13 g, 268.67 mmol) were added to water (600 mL) and stirred under reflux for 15 hours. When the reaction was completed, the reaction mixture was cooled to room temperature, undissolved impurities were removed by filtration, and concentrated hydrochloric acid was added dropwise to the filtrate (pH <2) to precipitate a solid. The obtained solid was filtered, triturated with an ethyl acetate/hexane mixed solvent, filtered and dried to obtain 4-(4-acetylphenyl)-1,2,4-triazolidine-3,5-dione (11 g, 84%).

### Intermediate 2: Synthesis of 4-(4-(trifluoromethyl)phenyl)-1,2,4-triazolidine-3,5-dione

### (a) Synthesis of methyl 2-((4-(trifluoromethyl)phenyl)carbamoyl)hydrazine-1-carboxylate

Except that 1-isocyanato-4-(trifluoromethyl)benzene was used instead of 1-(4-isocyanatophenyl)ethan-1-one, the same synthesis method as in Intermediate 1 (a) above was carried out to obtain methyl 2-((4-(trifluoromethyl)phenyl)carbamoyl)hydrazine-1-carboxylate (96%).

### (b) Synthesis of 4-(4-(trifluoromethyl)phenyl)-1,2,4-triazolidine-3,5-dione

Except that methyl 2-((4-(trifluoromethyl)phenyl)carbamoyl)hydrazine-1-carboxylate was used instead of methyl 2-((4-acetylphenyl)carbamoyl)hydrazine-1-carboxylate, the same synthesis method as in Intermediate 1 (b) above was carried out to obtain 4-(4-(trifluoromethyl)phenyl)-1,2,4-triazolidine-3,5-dione (94%).

### Intermediate 3: Synthesis of 4-(4-fluorophenyl)-1,2,4-triazolidine-3,5-dione

### (a) Synthesis of methyl 2-((4-fluorophenyl)carbamoyl)hydrazine-1-carboxylate

Except that 1-fluoro-4-isocyanatobenzene was used instead of 1-(4-isocyanatophenyl)ethan-1-one, the same synthesis method as in Intermediate 1 (a) above was carried out to obtain methyl 2-((4-fluorophenyl)carbamoyl)hydrazine-1-carboxylate (98%).

### (b) Synthesis of 4-(4-fluorophenyl)-1,2,4-triazolidine-3,5-dione

Except that methyl 2-((4-fluorophenyl)carbamoyl)hydrazine-1-carboxylate was used instead of methyl 2-((4-acetylphenyl)carbamoyl)hydrazine-1-carboxylate, the same synthesis method as in Intermediate 1 (b) above was carried out to obtain 4-(4-fluorophenyl)-1,2,4-triazolidine-3,5-dione (93%).

### Intermediate 4: Synthesis of 4-(4-(tert-butyl)phenyl)-1,2,4-triazolidine-3,5-dione

### (a) Synthesis of methyl 2-((4-(tert-butyl)phenyl)carbamoyl)hydrazine-1-carboxylate

Except that 1-(tert-butyl)-4-isocyanatobenzene was used instead of 1-(4-isocyanato)ethan-1-one, the same synthesis method as in Intermediate 1 (a) above was carried out to obtain methyl 2-((4-(tert-butyl)phenyl)carbamoyl)hydrazine-1-carboxylate (98%).

### (b) Synthesis of 4-(4-(tert-butyl)phenyl)-1,2,4-triazolidine-3,5-dione

Except that methyl 2-((4-(tert-butyl)phenyl)carbamoyl)hydrazine-1-carboxylate was used instead of methyl 2-((4-acetylphenyl)carbamoyl)hydrazine-1-carboxylate, the same synthesis method as in Intermediate 1 (b) above was carried out to obtain 4-(4-(tert-butyl)phenyl)-1,2,4-triazolidine-3,5-dione (95%).

### Intermediate 5: Synthesis of 4-(4-bromophenyl)-1,2,4-triazolidine-3,5-dione

### (a) Synthesis of methyl 2-((4-bromophenyl)carbamoyl)hydrazine-1-carboxylate

Except that 1-bromo-4-isocyanatobenzene was used instead of 1-(4-isocyanato)ethan-1-one, the same synthesis method as in Intermediate 1 (a) above was carried out to obtain 2-((4-bromophenyl)carbamoyl)hydrazine-1-carboxylate (55%).

### (b) Synthesis of 4-(4-bromophenyl)-1,2,4-triazolidine-3,5-dione

Except that the compound methyl 2-((4-bromophenyl)carbamoyl)hydrazine-1-carboxylate was used instead of the compound methyl 2-((4-acetylphenyl)carbamoyl)hydrazine-1-carboxylate, the same synthesis method as in Intermediate 1 (b) above was carried out to obtain 4-(4-bromophenyl)-1,2,4-triazolidine-3,5-dione (42%).

### Intermediate 6: Synthesis of 4-(3,5-dioxo-1,2,4-triazolidin-4-yl)benzoic acid

### (a) Synthesis of 4-(2-(methoxycarbonyl)hydrazine-1-carboxamido)benzoic acid

Except that 4-isocyanatobenzoic acid was used instead of 1-(4-isocyanato)ethan-1-one, the same synthesis method as in Intermediate 1 (a) above was carried out to obtain 4-(2-(methoxycarbonyl)hydrazine-1-carboxamido)benzoic acid (77%).

### (b) Synthesis of 4-(3,5-dioxo-1,2,4-triazolidin-4-yl)benzoic acid

Except that 4-(2-(methoxycarbonyl)hydrazine-1-carboxamido)benzoic acid was used instead of methyl 2-((4-acetylphenyl)carbamoyl)hydrazine-1-carboxylate, the same synthesis method as in Intermediate 1 (b) above was carried out to obtain 4-(3,5-dioxo-1,2,4-triazolidin-4-yl)benzoic acid (57%).

### Intermediate 7: Synthesis of ((2,2-dimethyl-3-vinyl-2H-chromen-7-yl)oxy)triisopropylsilane

### (a) Synthesis of 7-hydroxy-2,2-dimethylchroman-4-one

1-(2,4-Dihydroxyphenyl)ethanone (500 mg, 3.29 mmol) was dissolved in ethanol (32.9 ml), and pyrrolidine (0.549 ml, 6.57 mmol) and acetone (2.413 ml, 32.9 mmol) were added thereto. The reaction mixture was stirred under reflux for 3 days. When the reaction was completed, the solvent was removed through distillation under reduced pressure, and the residue was diluted in dichloromethane and washed with 1N aqueous hydrochloric acid solution and aqueous sodium chloride solution. The separated organic layer was dried over sodium sulfate, filtered, concentrated by distillation under reduced pressure, and separated by MPLC (ethyl acetate: hexane = 1:5, v/v) to obtain 7-hydroxy-2,2-dimethylchroman-4-one. (516 mg, 82%).

### (b) Synthesis of 3-bromo-7-hydroxy-2,2-dimethylchroman-4-one

7-Hydroxy-2,2-dimethylchroman-4-one (515 mg, 2.68 mmol) obtained in Intermediate 7 (a) above was dissolved in dichloromethane (5 mL), and copper (II) bromide (1.26 g, 5.63 mmol) was added thereto and stirred under reflux for 4 hours. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (ethyl acetate: hexane = 1: 7, v/v) to obtain 3-bromo-7-hydroxy-2,2-dimethylchroman-4-one (655 mg, 90%).

### (c) Synthesis of 3-bromo-2,2-dimethyl-7-((triisopropylsilyl)oxy)chroman-4-one

3-Bromo-7-hydroxy-2,2-dimethylchroman-4-one (650 mg, 2.4 mmol) obtained in Intermediate 7 (b) above and imidazole (245 mg, 3.6 mmol) were added to dichloromethane (10 mL) and cooled to 0°C, and triisopropylsilyl chloride (616 µl, 2.88 mmol) was added dropwise thereto and stirred at room temperature for 12 hours. When the reaction was completed, the reaction mixture was diluted in dichloromethane and washed with an aqueous ammonium chloride solution and an aqueous sodium chloride solution. The separated organic layer was dried over sodium sulfate, filtered, concentrated by distillation under reduced pressure, and separated by MPLC (ethyl acetate: hexane = 1:30, v/v) to obtain 3-bromo-2,2-dimethyl-7-((triisopropylsilyl)oxy)chroman-4-one (931 mg, 91%).

### (d) Synthesis of ((3-bromo-2,2-dimethyl-2H-chromen-7-yl)oxy)triisopropylsilane

3-Bromo-2,2-dimethyl-7-((triisopropylsilyl)oxy)chroman-4-one (500 mg, 1.17 mmol) obtained in Intermediate 7 (c) above was dissolved in ethanol (6 mL), and sodium borohydride (44 mg, 1.17 mmol) was added thereto, and heated and stirred at 40°C for 1 hour. When the reaction was completed, the reaction mixture was diluted in water, neutralized with an aqueous ammonium chloride solution, and extracted with ethyl acetate. The separated organic layer was washed with aqueous sodium chloride solution, dried over sodium sulfate, filtered, and concentrated by distillation under reduced pressure. The residue was dissolved in toluene (6 mL), p-toluenesulfonic acid (20 mg, 0.117 mmol) was added thereto, and heated and stirred at 80°C for 4 hours. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (ethyl acetate: hexane = 1:30, v/v) to obtain ((3-bromo-2,2-dimethyl-2*H*-chromen-7-yl)oxy)triisopropylsilane (320 mg, 66.5%).

### (e) Synthesis of ((2,2-dimethyl-3-vinyl-2H-chromen-7-yl)oxy)triisopropylsilane

((3-Bromo-2,2-dimethyl-2*H*-chromen-7-yl)oxy)triisopropylsilane (175 mg, 0.43 mmol) obtained in Intermediate 7 (d) above, tetrakis(triphenylphosphine)palladium(0) (24 mg, 0.022 mmol) and sodium carbonate (113 mg, 1.06 mmol) were suspended in a mixed solvent of ethanol/toluene/water (1:2:2.5 mL), and vinylboronic acid dibutyl ester (113 µl, 0.51 mmol) was added thereto, and heated and stirred at 70°C for 12 hours. When the reaction was completed, the reaction mixture was diluted in ethyl acetate and washed with aqueous sodium chloride solution. The separated organic layer was dried over sodium sulfate, filtered, and distilled under reduced pressure to quantitatively obtain ((2,2-dimethyl-3-vinyl-2*H*-chromen-7-yl)oxy)triisopropylsilane, which was used in the next reaction without purification.

### Intermediate 8: Synthesis of 1-(2,2-dimethyl-7-((triisopropylsilyl)oxy)-3-vinyl-2H-chromen-6-yl)ethan-1-one

### (a) Synthesis of 1-(3-bromo-7-hydroxy-2,2-dimethyl-2H-chromen-6-yl)ethan-1-one

((3-Bromo-2,2-dimethyl-2H-chromen-7-yl)oxy)triisopropylsilane (447 mg, 1.09 mmol) obtained in Intermediate 7 (d) above was added to dichloromethane (10 mL) and cooled to 0°C. Aluminum chloride (290 mg, 2.17 mmol) and acetyl chloride (1.16 mL, 16.3 mmol) were added thereto and stirred at 0°C for 15 minutes. When the reaction was completed, the reaction mixture was diluted in dichloromethane and washed with water and aqueous sodium chloride solution. The separated organic layer was dried over sodium sulfate, filtered, and distilled under reduced pressure. The obtained residue was dissolved in MeOH (10 mL), and 1N aqueous sodium hydroxide solution (2.17 mL, 2.17 mmol) was added thereto, stirred at room temperature for 15 minutes, diluted in ethyl acetate, and washed with 1N aqueous hydrochloric acid solution, water and aqueous sodium chloride solution. The separated organic layer was dried over sodium sulfate, filtered, concentrated by distillation under reduced pressure, and separated by MPLC (ethyl acetate: hexane = 1: 5, v/v) to obtain 1-(3-bromo-7-hydroxy-2,2-dimethyl-2*H*-chromen-6-yl)ethan-1-one (162 mg, 50%).

### (b) Synthesis of 1-(3-bromo-2,2-dimethyl-7-((triisopropylsilyl)oxy)-2H-chromen-6-yl)ethan-1-one

Except that 1-(3-bromo-7-hydroxy-2,2-dimethyl-2*H*-chromen-6-ylethane-1-one was used instead of 3-bromo-7-hydroxy-2,2-dimethylchroman-4-one, the same synthesis method as in Intermediate 7 (c) above was carried out to obtain 1-(3-bromo-2,2-dimethyl-7-((triisopropylsilyl)oxy)-2*H*-chromen-6-yl)ethan-1-one (80 mg, 60%).

### (c) Synthesis of 1-(2,2-dimethyl-7-((triisopropylsilyl)oxy)-3-vinyl-2H-chromen-6-yl)ethan-1-one

Except that 1-(3-bromo-2,2-dimethyl-7-((triisopropylsilyl)oxy)-2*H*-chromen-6-yl)ethan-1-one was used instead of ((3-bromo-2,2-dimethyl-2*H*-chromen-7-yl)oxy)triisopropylsilane, the same synthesis method as in Intermediate 7 (e) above was carried out to quantitatively obtain 1-(2,2-dimethyl-7-((triisopropylsilyl)oxy)-3-vinyl-2*H*-chromen-6-yl)ethan-1-one. The obtained compound was used in the next reaction without purification.

### Intermediate 9: Synthesis of N-(2,2-dimethyl-3-vinyl-2H-chromen-7-yl)acetamide

### (a) Synthesis of 7-bromo-2,2-dimethylchroman-4-one

Except that 1-(4-bromo-2-hydroxyphenyl)ethan-1-one was used instead of 1-(2,4-dihydroxyphenyl)ethenone, the same synthesis method as in Intermediate 7 (a) above was carried out to obtain 7-bromo-2,2-dimethylchroman-4-one (950 mg, 85%).

### (b) Synthesis of tert-butyl (2,2-dimethyl-4-oxochroman-7-yl)carbamate

7-Bromo-2,2-dimethylchroman-4-one (500 mg, 1.96 mmol) obtained in Intermediate 9 (a) above was dissolved in 1,4-dioxane (10 mL), and *O*-tert-butyl-carbamate (344 mg, 2.94 mmol), palladium(II) acetate (44 mg, 0.196 mmol), Xphos (187, mg, 0.392 mmol) and cesium carbonate (958 mg, 2.94 mmol) were added thereto and stirred under reflux for 1 hour. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (ethyl acetate: hexane = 1: 5, v/v) to obtain tert-butyl (2,2-dimethyl-4-oxochroman-7-yl)carbamate (560 mg, 98%).

### (c) Synthesis of 7-amino-3-bromo-2,2-dimethylchroman-4-one

Except that tert-butyl (2,2-dimethyl-4-oxochroman-7-yl)carbamate was used instead of 7-hydroxy-2,2-dimethylchroman-4-one, the same synthesis method as in Intermediate 7 (b) above was carried out to obtain 7-amino-3-bromo-2,2-dimethylchroman-4-one (320 mg, 54%).

### (d) Synthesis of N-(3-bromo-2,2-dimethyl-4-oxochroman-7-yl)acetamide

7-Amino-3-bromo-2,2-dimethylchroman-4-one (120 mg, 0.44 mmol) obtained in Intermediate 9 (c) above and 1-(3-dimethylaminopropyl)-ethylcarbodiimide hydrochloride (128 mg, 0.67 mmol) were added to pyridine (2 mL), and acetic acid (25 µl, 0.44 mmol) was added thereto. After the reaction mixture was stirred at room temperature for 12 hours, acetic acid (25 µl, 0.44 mmol) was further added and stirred for additional 4 hours. When the reaction was completed, the reaction mixture was concentrated by distillation under reduced pressure, and the residue was separated by reverse phase chromatography (acetonitrile containing 0.1% formic acid: water containing 0.1% formic acid = 5:95-100:0) to obtain N-(3-bromo-2,2-dimethyl-4-oxochroman-7-yl)acetamide (126 mg, 91%).

### (e) Synthesis of N-(3-bromo-2,2-dimethyl-2H-chromen-7-yl)acetamide

Except that *N*-(3-bromo-2,2-dimethyl-4-oxochroman-7-yl)acetamide was used instead of 3-bromo-2,2-dimethyl-7-((triisopropylsilyl)oxy)chroman-4-one, the same synthesis method as in Intermediate 7 (d) above was carried out to obtain *N*-(3-bromo-2,2-dimethyl-2H-chromen-7-yl)acetamide (78 mg, 72%).

### (f) Synthesis of N-(2,2-dimethyl-3-vinyl-2H-chromen-7-yl)acetamide

Except that *N*-(3-bromo-2,2-dimethyl-2*H*-chromen-7-yl)acetamide was used instead of ((3-bromo-2,2-dimethyl-2*H*-chromen-7-yl)oxy)triisopropylsilane, the same synthesis method as in Intermediate 7 (e) above was carried out to quantitatively obtain *N*-(2,2-dimethyl-3-vinyl-2*H-*chromen-7-yl)acetamide. The obtained compound was used in the next reaction without purification.

### Intermediate 10: Synthesis of 2,2-dimethyl-7-(methylsulfonyl)-3-vinyl-2H-chromene

### (a) Synthesis of 2,2-dimethyl-7-(methylsulfonyl)chroman-4-one

7-Bromo-2,2-dimethylchroman-4-one (500 mg, 1.96 mmol) obtained in Intermediate 9 (a) above was dissolved in dimethyl sulfoxide (10 mL), and methanesulfonic acid (400 mg, 3.92 mmol, Na salt), *L*-proline (45 mg, 0.39 mmol), copper(I) iodide (37 mg, 0.2 mmol) and potassium carbonate (542 mg, 3.92 mmol) were added thereto, and heated and stirred at 130°C for 2 hours. When the reaction was completed, the reaction mixture was added to water and extracted with ethyl acetate. The separated organic layer was washed with aqueous sodium chloride solution, dried over sodium sulfate, filtered, concentrated by distillation under reduced pressure, and separated by column chromatography (ethyl acetate: hexane = 1:10, v/v) to obtain 2,2-dimethyl-7-(methylsulfonyl)chroman-4-one (160 mg, 32%).

### (b) Synthesis of 3-bromo-2,2-dimethyl-7-(methylsulfonyl)chroman-4-one

Except that 2,2-dimethyl-7-(methylsulfonyl)chroman-4-one was used instead of 7-hydroxy-2,2-dimethylchroman-4-one, the same synthesis method as in Intermediate 7 (b) above was carried out to obtain 3-bromo-2,2-dimethyl-7-(methylsulfonyl)chroman-4-one (84 mg, 67%).

### (c) Synthesis of 3-bromo-2,2-dimethyl-7-(methylsulfonyl)-2H-chromene

Except that 3-bromo-2,2-dimethyl-7-(methylsulfonyl)chroman-4-one was used instead of 3-bromo-2,2-dimethyl-7-((triisopropylsilyl)oxy)chroman-4-one, the same synthesis method as in Intermediate 7 (d) above was carried out to obtain 3-bromo-2,2-dimethyl-7-(methylsulfonyl)-2*H-*chromene (53 mg, 65%).

### (d) Synthesis of 2,2-dimethyl-7-(methylsulfonyl)-3-vinyl-2H-chromene

Except that 3-bromo-2,2-dimethyl-7-(methylsulfonyl)-2*H*-chromene was used instead of ((3-bromo-2,2-dimethyl-2*H*-chromen-7-yl)oxy)triisopropylsilane, the same synthesis method as in Intermediate 7 (e) above was carried out to quantitatively obtain 2,2-dimethyl-7-(methylsulfonyl)-3-vinyl-2*H*-chromene. The obtained compound was used in the next reaction without purification.

### Intermediate 11: Synthesis of N-(2,2-dimethyl-3-vinyl-2H-chromen-7-yl)methanesulfonamide

### (a) Synthesis of N-(3-bromo-2,2-dimethyl-4-oxochroman-7-yl)methanesulfonamide

7-Amino-3-bromo-2,2-dimethylchroman-4-one (200 mg, 0.74 mmol) obtained in Intermediate 9 (c) above was dissolved in dichloromethane (10 mL) and cooled to 0°C. Pyridine (299 µl, 3.7 mmol) and methanesulfonyl chloride (288 µl, 3.7 mmol) were added dropwise to the reaction mixture, raised to room temperature and stirred for 12 hours. When the reaction is completed, the solvent is removed by distillation under reduced pressure, and the residue is separated by MPLC (ethyl acetate: hexane: 1: 5, v/v) to obtain *N-*(3-bromo-2,2-dimethyl-4-oxochroman-7-yl)methanesulfonamide (230 mg, 89%).

### (b) Synthesis of N-(3-bromo-2,2-dimethyl-2H-chromen-7-yl)methanesulfonamide

Except that *N*-(3-bromo-2,2-dimethyl-4-oxochroman-7-yl)methanesulfonamide was used instead of 7-hydroxy-2,2-dimethylchroman-4-one, the same synthesis method as in Intermediate 7 (b) above was carried out to obtain *N*-(3-bromo-2,2-dimethyl-2*H*-chromen-7-yl)methanesulfonamide (170 mg, 70%).

### (c) Synthesis of N-(2,2-dimethyl-3-vinyl-2H-chromen-7-yl)methanesulfonamide

Except that *N*-(3-bromo-2,2-dimethyl-2*H*-chromen-7-yl)methanesulfonamide was used instead of ((3-bromo-2,2-dimethyl-2*H*-chromen-7-yl)oxy)triisopropylsilane, the same synthesis method as in Intermediate 7 (e) above was carried out to quantitatively obtain *N-*(2,2-dimethyl-3-vinyl-2*H*-chromen-7-yl)methanesulfonamide. The obtained compound was used in the next reaction without purification.

### Intermediate 12: Synthesis of tert-butyl (2,2-dimethyl-3-vinyl-2H-chromen-7-yl)carbamate

### (a) Synthesis of tert-butyl (2,2-dimethyl-2H-chromen-7-yl)carbamate

Except that tert-butyl (2,2-dimethyl-4-oxochroman-7-yl)carbamate was used instead of 3-bromo-2,2-dimethyl-7-((triisopropylsilyl)oxy)chroman-4-one, the same synthesis method as in Intermediate 7 (d) above was carried out to obtain tert-butyl (2,2-dimethyl-2*H*-chromen-7-yl)carbamate (240 mg, 67%).

### (b) Synthesis of tert-butyl (3-bromo-2,2-dimethyl-2H-chromen-7-yl)carbamate

Except that tert-butyl (2,2-dimethyl-2H-chromen-7-yl) carbamate was used instead of 7-hydroxy-2,2-dimethylchroman-4-one, the same synthesis method as in Intermediate 7 (b) above was carried out to obtain tert-butyl (3-bromo-2,2-dimethyl-2*H*-chromen-7-yl)carbamate (100 mg, 78%).

### (c) Synthesis of tert-butyl (2,2-dimethyl-3-vinyl-2H-chromen-7-yl)carbamate

Except that tert-butyl (3-bromo-2,2-dimethyl-2*H*-chromen-7-yl)carbamate was used instead of ((3-bromo-2,2-dimethyl-2*H*-chromen-7-yl)oxy)triisopropylsilane, the same synthesis method as in Intermediate 7 (e) above was carried out to quantitatively obtain tert-butyl (2,2-dimethyl-3-vinyl-2*H*-chromen-7-yl)carbamate. The obtained compound was used in the next reaction without purification.

### Intermediate 13: Synthesis of 4-(2,2-dimethyl-3-vinyl-2H-chromen-7-yl)morpholine

### (a) Synthesis of 1-(2-hydroxy-4-morpholinophenyl)-3-methylbut-2-en-1-one

Except that morpholine was used instead of *O*-tert-butyl-carbamate, the same synthesis method as in Intermediate 9 (b) above was carried out to obtain 1-(2-hydroxy-4-morpholinophenyl)-3-methylbut-2-en-1-one (200 mg, 56%).

### (b) Synthesis of 2,2-dimethyl-7-morpholinochroman-4-one

1-(2-Hydroxy-4-morpholinophenyl)-3-methylbut-2-en-1-one (150 mg, 0.57 mmol) obtained in Intermediate 13 (a) above and triethylamine (173 mg, 1.71 mmol) were dissolved in 1,2-dichloroethane, and sodium tert-butoxide (55 mg, 0.57 mmol) was added thereto, and heated and stirred at 70°C for 12 hours. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (ethyl acetate: hexane = 1:3, v/v) to obtain 2,2-dimethyl-7-morpholinochroman-4-one (149 mg, 99%).

### (c) Synthesis of 3-bromo-2,2-dimethyl-7-morpholinochroman-4-one

Except that 2,2-dimethyl-7-morpholinochroman-4-one was used instead of 7-hydroxy-2,2-dimethylchroman-4-one, the same synthesis method as in Intermediate 7 (b) above was carried out to obtain 3-bromo-2,2-dimethyl-7-morpholinochroman-4-one (105 mg, 87%).

### (d) Synthesis of 4-(3-bromo-2,2-dimethyl-2H-chromen-7-yl)morpholine

Except that 3-bromo-2,2-dimethyl-7-morpholinochroman-4-one was used instead of 3-bromo-2,2-dimethyl-7-((triisopropylsilyl)oxy)chroman-4-one, the same synthesis method as in Intermediate 7 (d) above was carried out to obtain 4-(3-bromo-2,2-dimethyl-2*H*-chromen-7-yl)morpholine (55 mg, 58%).

### (e) Synthesis of 4-(2,2-dimethyl-3-vinyl-2H-chromen-7-yl)morpholine

Except that 4-(3-bromo-2,2-dimethyl-2*H*-chromen-7-yl)morpholine was used instead of ((3-bromo-2,2-dimethyl-2*H*-chromen-7-yl)oxy)triisopropylsilane, the same synthesis method as in Intermediate 7 (c) above was carried out to quantitatively obtain 4-(2,2-dimethyl-3-vinyl-2H-chromen-7-yl)morpholine. The obtained compound was used in the next reaction without purification.

### Intermediate 14: Synthesis of 7-(methoxymethyl)-2,2-dimethyl-3-vinyl-2H-chromene

### (a) Synthesis of 2,2,7-trimethylchroman-4-one

Except that 1-(2-hydroxy-4-methylphenyl)ethan-1-one was used instead of 1-(2,4-dihydroxyphenyl)ethenone, the same synthesis method as in Intermediate 7 (a) above was carried out to obtain 2,2,7-trimethylchroman-4-one (340 mg, 95%).

### (b) Synthesis of 3-bromo-2,2,7-trimethylchroman-4-one

Except that 2,2,7-trimethylchroman-4-one was used instead of 7-hydroxy-2,2-dimethylchroman-4-one, the same synthesis method as in Intermediate 7 (b) above was carried out to obtain 3-bromo-2,2,7-trimethylchroman-4-one (220 mg, 75%).

### (c) Synthesis of 3-bromo-2,2,7-trimethyl-2H-chromene

Except that 3-bromo-2,2,7-trimethylchroman-4-one was used instead of 3-bromo-2,2-dimethyl-7-((triisopropylsilyl)oxy)chroman-4-one, the same synthesis method as in Intermediate 7 (d) above was carried out to obtain 3-bromo-2,2,7-trimethyl-2*H*-chromene (100 mg, 58%).

### (d) Synthesis of 3-bromo-7-(bromomethyl)-2,2-dimethyl-2H-chromene

3-Bromo-2,2,7-trimethyl-2*H*-chromene (50 mg, 0.2 mmol) obtained in Intermediate 14 (c) above was dissolved in dichloromethane (2 mL), and *N*-bromosuccinimide (35 mg, 0.2 mmol) and benzoyl peroxide (4.8 mg, 0.02 mmol) were added thereto and stirred under reflux for 1 hour. When the reaction was completed, the reaction mixture was concentrated by distillation under reduced pressure and separated by MPLC (ethyl acetate: hexane = 1: 4, v/v) to quantitatively obtain 3-bromo-7-(bromomethyl)-2,2-dimethyl-2*H*-chromene.

### (e) Synthesis of 3-bromo-7-(methoxymethyl)-2,2-dimethyl-2H-chromene

3-Bromo-7-(bromomethyl)-2,2-dimethyl-2*H*-chromene (45 mg, 0.14 mmol) obtained in Intermediate 14 (d) above was dissolved in methanol (5 mL), and 1N aqueous sodium hydroxide solution (0.68 mL, 0.68 mmol) was added thereto and stirred at room temperature for 2 hours. Then, sodium methoxide (7.3 mg, 0.14 mmol) was added thereto and stirred for additional 1 hour at room temperature. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (ethyl acetate: hexane = 1:5, v/v) to obtain 3-bromo-7-(methoxymethyl)-2,2-dimethyl-2H-chromene (22 mg, 57%).

### (f) Synthesis of 7-(methoxymethyl)-2,2-dimethyl-3-vinyl-2H-chromene

Except that 3-bromo-7-(methoxymethyl)-2,2-dimethyl-2*H*-chromene was used instead of ((3-bromo-2,2-dimethyl-2*H*-chromen-7-yl)oxy)triisopropylsilane, the same synthesis method as in Intermediate 7 (e) above was carried out to quantitatively obtain 7-(methoxymethyl)-2,2-dimethyl-3-vinyl-2*H*-chromene. The obtained compound was used in the next reaction without purification

### Intermediate 15: Synthesis of 7-(((4-methoxyphenyl)diphenylmethoxy)methyl)-2,2-dimethyl-3-vinyl-2H-chromene

### (a) Synthesis of (3-bromo-2,2-dimethyl-2H-chromen-7-yl)methanol

3-Bromo-7-(bromomethyl)-2,2-dimethyl-2*H*-chromene (120 mg, 0.36 mmol) obtained in Intermediate 14 (d) above was suspended in 1,4-dioxane (4 mL), *N*-methyl morpholine-*N*-oxide (169 mg, 1.45 mmol) was added thereto, and heated and stirred at 50°C for 30 minutes. When the reaction was completed, the reaction mixture was added to water and extracted with ethyl acetate. The separated organic layer was washed with aqueous sodium chloride solution, dried over sodium sulfate, filtered, and distilled under reduced pressure. The obtained residue was dissolved in ethanol (4 mL), and sodium borohydride (21 mg, 0.54 mmol) was added thereto and stirred at room temperature for 1 hour. When the reaction was completed, the reaction mixture was concentrated by distillation under reduced pressure and separated by reverse-phase chromatography (acetonitrile containing 0.1% formic acid: water containing 0.1% formic acid = 5:95-100:0) to obtain (3-bromo-2,2-dimethyl-2*H*-chromen-7-yl)methanol (69 mg, 71%).

### (b) Synthesis of 3-bromo-7-(((4-methoxyphenyl)diphenylmethoxy)methyl)-2,2-dimethyl-2H-chromene

(3-Bromo-2,2-dimethyl-2*H*-chromen-7-yl)methanol (72 mg, 0.27 mmol) obtained in Intermediate 15 (a) above was dissolved in pyridine (1.5 mL), 4-methoxytrityl chloride (165 mg, 0.54 mmol) was added thereto, and heated and stirred at 70°C for 1 hour. When the reaction was completed, the reaction mixture was added to water and extracted with ethyl acetate. The separated organic layer was washed with aqueous sodium chloride solution, dried over sodium sulfate, filtered, concentrated by distillation under reduced pressure and separated by reverse-phase chromatography (acetonitrile containing 0.1% formic acid: water containing 0.1% formic acid = 5:95-100:0) to obtain 3-bromo-7-(((4-methoxyphenyl)diphenylmethoxy)methyl)-2,2-dimethyl-2*H*-chromene (115 mg, 79%).

### (c) Synthesis of 7-(((4-methoxyphenyl)diphenylmethoxy)methyl)-2,2-dimethyl-3-vinyl-2H-chromene

Except that 3-bromo-7-(((4-methoxyphenyl)diphenylmethoxy)methyl)-2,2-dimethyl-2*H-*chromene was used instead of ((3-bromo-2,2-dimethyl-2*H*-chromen-7-yl)oxy)triisopropylsilane, the same synthesis method as in Intermediate 7 (e) above was carried out to quantitatively obtain 7-(((4-methoxyphenyl)diphenylmethoxy)methyl)-2,2-dimethyl-3-vinyl-2*H*-chromene. The obtained compound was used in the next reaction without purification.

### Intermediate 16: Synthesis of tert-butyl 4-(2,2-dimethyl-3-vinyl-2H-chromen-7-yl)piperazine-1-carboxylate

### (a) Synthesis of 7-bromo-2,2-dimethyl-2H-chromene

Except that 7-bromo-2,2-dimethylchroman-4-one was used instead of 3-bromo-2,2-dimethyl-7-((triisopropylsilyl)oxy)chroman-4-one, the same synthesis method as in Intermediate 7 (d) above was carried out to obtain 7-bromo-2,2-dimethyl-2*H*-chromene (300 mg, 67%).

### (b) Synthesis of tert-butyl 4-(2,2-dimethyl-2H-chromen-7 -yl)piperazine-1-carboxylate

Except that 7-bromo-2,2-dimethyl-2*H*-chromene and tert-butyl piperazine-1-carboxylate were used instead of 7-bromo-2,2-dimethylchroman-4-one and *O*-tert-butyl-carbamate, respectively, the same synthesis method as in Intermediate 9 (b) above was carried out to obtain tert-butyl 4-(2,2-dimethyl-2*H*-chromen-7-yl)piperazine-1-carboxylate (210 mg, 74%).

### (c) Synthesis of tert-butyl 4-(3-bromo-2,2-dimethyl-2H-chromen-7-yl)piperazine-1-carboxylate

Except that tert-butyl 4-(2,2-dimethyl-2*H*-chromen-7-yl)piperazine-1-carboxylate was used instead of 7-hydroxy-2,2-dimethylchroman-4-one, the same synthesis method as in Intermediate 7 (b) above was carried out to obtain tert-butyl 4-(3-bromo-2,2-dimethyl-2*H-*chromen-7-yl)piperazine-1-carboxylate (82 mg, 78%).

### (d) Synthesis of tert-butyl 4-(2,2-dimethyl-3-vinyl-2H-chromen-7-yl)piperazine-1-carboxylate

Except tha tert-butyl 4-(3-bromo-2,2-dimethyl-2*H*-chromen-7-yl)piperazine-1-carboxylate was used instead of ((3-bromo-2,2-dimethyl-2H-chromen-7-yl)oxy)triisopropylsilane, the same synthesis method as in Intermediate 7 (e) above was carried out to quantitatively obtain tert-butyl 4-(2,2-dimethyl-3-vinyl-2*H*-chromen-7-yl)piperazine-1-carboxylate. The obtained compound was used in the next reaction without purification.

### Intermediate 17: Synthesis of tert-butyl (2,2-dimethyl-3-vinylchromen-7-yl)(ethyl)carbamate

### (a) Synthesis of tert-butyl ethyl(3-formyl-2,2-dimethylchromen-7-yl)carbamate

Except that 7-bromo-2,2-dimethyl-2*H*-chromen-3-carbaldehyde and tert-butyl ethyl carbamate were used instead of 7-bromo-2,2-dimethylchroman-4-one and *O*-tert-butyl-carbamate, respectively, the same synthesis method as in Intermediate 9 (b) above was carried out to obtain tert-butyl (2,2-dimethyl-3-vinylchromen-7-yl)(ethyl)carbamate (900 mg, 85%).

### (b) Synthesis of tert-butyl (2,2-dimethyl-3-vinylchromen-7-yl)(ethyl)carbamate

Methyltriphenylphosphonium bromide (2.14 g, 6.00 mmol) was dissolved in tetrahydrofuran and cooled to 0°C. Potassium tert-butoxide (673 mg, 6.00 mmol) was added to the reaction mixture and stirred for 1 hour at 0°C under nitrogen atmosphere. Then, tert-butyl ethyl(3-formyl-2,2-dimethylchromen-7-yl)carbamate (800 mg, 2.4 mmol) dissolved in tetrahydrofuran (5 mL) was added dropwise thereto at 0°C. The reaction mixture was raised to room temperature and stirred for 2 hours. When the reaction was completed, the reaction mixture was placed in ice water and extracted with ethyl acetate. The separated organic layer was dried over sodium sulfate, filtered and concentrated by distillation under reduced pressure. The obtained residue was used in the next reaction without purification.

### Intermediate 18: Synthesis of ((6,8-difluoro-2,2-dimethyl-3-vinyl-2H-chromen-7-yl)oxy)triisopropylsilane

### (a) Synthesis of 2,4-difluorobenzene-1,3-diol

1,3-Difluoro-2,4-dimethoxybenzene (645 mg, 3.7 mmol) was dissolved in dichloromethane (5 mL), and boron tribromide (11.11 mL, 11.11 mmol, 1M/hexane) was added dropwise thereto and stirred at room temperature for 19 hours. When the reaction was completed, water was added and extracted with diethyl ether. The separated organic layer was washed with aqueous sodium chloride solution, dried over sodium sulfate, filtered, concentrated by distillation under reduced pressure, and separated by MPLC (ethyl acetate: hexane = 1:3, v/v) to obtain 2,4-difluorobenzene-1,3-diol (463 mg, 86%).

### (b) Synthesis of 6,8-difluoro-7-hydroxy-2,2-dimethylchroman-4-one

2,4-Difluorobenzene-1,3-diol (463 mg, 3.17 mmol) obtained in Intermediate 18 (a) above, zinc chloride (432 mg, 3.17 mmol) and 3,3-dimethylacrylic acid (317 mg, 3.17 mmol) were added to phosphoryl chloride (2.6 mL), and heated and stirred at 50°C for 4 hours. When the reaction was completed, the reaction mixture was cooled to room temperature, placed in ice water, and extracted with ethyl acetate. The separated organic layer was washed with aqueous sodium chloride solution, dried over sodium sulfate, filtered, concentrated by distillation under reduced pressure, and separated by MPLC (ethyl acetate: hexane = 1:3, v/v) to obtain 6,8-difluoro-7-hydroxy-2,2-dimethylchroman-4-one (234 mg, 32.4%).

### (c) Synthesis of 3-bromo-6,8-difluoro-7-hydroxy-2,2-dimethylchroman-4-one

Except that 6,8-difluoro-7-hydroxy-2,2-dimethylchroman-4-one was used instead of 7-hydroxy-2,2-dimethylchroman-4-one, the same synthesis method as in Intermediate 7 (b) above was carried out to obtain 3-bromo-6,8-difluoro-7-hydroxy-2,2-dimethylchroman-4-one (299 mg, 90%).

### (d) Synthesis of 3-bromo-6,8-difluoro-2,2-dimethyl-7-((triisopropylsilyl)oxy)chroman-4-one

Except that 3-bromo-6,8-difluoro-7-hydroxy-2,2-dimethylchroman-4-one was used instead of 3-bromo-7-hydroxy-2,2-dimethylchroman-4-one, the same synthesis method as in Intermediate 7 (c) above was carried out to obtain 3-bromo-6,8-difluoro-2,2-dimethyl-7-((triisopropylsilyl)oxy)chroman-4-one (245 mg, 61%).

### (e) Synthesis of ((6,8-difluoro-2,2-dimethyl-2H-chromen-7-yl)oxy)triisopropylsilane

Except that 3-bromo-6,8-difluoro-2,2-dimethyl-7-((triisopropylsilyl)oxy)chroman-4-one oxy)chroman-4-one was used instead of 3-bromo-2,2-dimethyl-7-((triisopropylsilyl)oxy)chroman-4-one, the same synthesis method as in Intermediate 7 (d) above was carried out to obtain ((6,8-difluoro-2,2-dimethyl-2*H*-chromen-7-yl)oxy)triisopropylsilane (127 mg, 51%).

### (f) Synthesis of 3-bromo-6,8-difluoro-2,2-dimethyl-7-((triisopropylsilyl)oxy)chroman-4-ol

((6,8-Difluoro-2,2-dimethyl-2*H*-chromen-7-yl)oxy)triisopropylsilane (125 mg, 0.34 mmol) obtained in Intermediate 18 (e) above was dissolved in a mixed solvent of dimethyl sulfoxide/water (5:1, 3 mL), and *N-*bromosuccinimide (154 mg, 0.86 mmol) was added thereto and stirred at room temperature for 4 hours. When the reaction is completed, the reaction mixture is separated by reverse-phase chromatography (acetonitrile containing 0.1% formic acid: water containing 0.1% formic acid = 5:95-100:0) to obtain 3-bromo-6,8-difluoro-2,2-dimethyl-7-((triisopropylsilyl)oxy)chroman-4-ol (158 mg).

### (g) Synthesis of ((3-bromo-6,8-difluoro-2,2-dimethyl-2H-chromen-7-yl)oxy)triisopropylsilane

3-Bromo-6,8-difluoro-2,2-dimethyl-7-((triisopropylsilyl)oxy)chroman-4-ol (135 mg, 0.29 mmol) obtained in Intermediate 18 (f) above was dissolved in toluene (3 mL), and p-toluenesulfonic acid (8.6 mg, 0.05 mmol) was added thereto, and heated and stirred at 80°C for 2 hours. When the reaction was completed, the reaction mixture was diluted with ethyl acetate and then washed with aqueous sodium bicarbonate solution and aqueous sodium chloride solution. The separated organic layer was dried over sodium sulfate, filtered, concentrated by distillation under reduced pressure, and separated by MPLC (hexane : dichloromethane = 3 : 1, v/v) to obtain ((3-bromo-6,8-difluoro-2,2-dimethyl-2*H*-chromen-7-yl)oxy)triisopropylsilane (105 mg, 81%).

### (h) Synthesis of ((6,8-difluoro-2,2-dimethyl-3-vinyl-2H-chromen-7-yl)oxy)triisopropylsilane

Except that ((3-bromo-6,8-difluoro-2,2-dimethyl-2*H*-chromen-7-yl)oxy)triisopropylsilane was used instead of ((3-bromo-2,2-dimethyl-2*H*-chromen-7-yl)oxy)triisopropylsilane, the same synthesis method as in Intermediate 7 (e) above was carried out to quantitatively obtain ((6,8-difluoro-2,2-dimethyl-3-vinyl-2*H*-chromen-7-yl)oxy)triisopropylsilane. The obtained compound was used in the next reaction without purification.

### Intermediate 19: Synthesis of 2,2-dimethyl-N-(2,2,2-trifluoroethyl)-3-vinyl-2H-chromen-7-amine

### (a) Synthesis of 2,2-dimethyl-N-(2,2,2-trifluoroethyl)-2H-chromen-7-amine

7-Bromo-2,2-dimethyl-2*H*-chromene (500 mg, 2.09 mmol) obtained in Intermediate 16 (a) above, allylpalladium chloride dimer (153 mg, 0.42 mmol), [3,6-dimethoxy-2',4',6'-tris(1-methylethyl) [1,1'-biphenyl]-2-yl]bis(1,1-dimethylethyl)phosphine (51 mg, 0.105 mmol) and phenol (0.2 mL, 2.3 mmol) were dissolved in 1,4-dioxane (21 mL), and potassium tert-butoxide (2.2 mL, 2.2 mmol, 1M/tetrahydrofuran) and 2,2,2 trifluoroethylamine (0.33 mL, 4.18 mmol) were added thereto, and stirred under reflux under nitrogen atmosphere for 1 hour. When the reaction was completed, the reaction mixture was diluted in ethyl acetate and washed with water and aqueous sodium chloride solution. The separated organic layer was dried over sodium sulfate, filtered, concentrated by distillation under reduced pressure, and separated by MPLC (dichloromethane : hexane = 2 : 3, v/v) to obtain 2,2-dimethyl-*N*-(2,2,2-trifluoroethyl)-2*H-*chromen-7-amine (423 mg, 79%).

### (b) Synthesis of 3-bromo-2,2-dimethyl-N-(2,2,2-trifluoroethyl)-2H-chromen-7-amine

Except that 2,2-dimethyl-*N-*(2,2,2-trifluoroethyl)-2*H*-chromen-7-amine was used instead of 7-hydroxy-2,2-dimethylchroman-4-one, the same synthesis method as in Intermediate 1 (a) above was carried out to obtain 3-bromo-2,2-dimethyl-*N*-(2,2,2-trifluoroethyl)-2*H*-chromen-7-amine (200 mg, 86%).

### (c) Synthesis of 2,2-dimethyl-N-(2,2,2-trifluoroethyl)-3-vinyl-2H-chromen-7-amine

Except that 3-bromo-2,2-dimethyl-N (2,2,2-trifluoroethyl)-2*H*-chromen-7-amine was used instead of ((3-bromo-2,2-dimethyl-2*H*-chromen-7-yl)oxy)triisopropylsilane, the same synthesis method as in Intermediate 7 (e) above was carried out to quantitatively obtain 2,2-dimethyl-N (2,2,2-trifluoroethyl)-3-vinyl-2*H*-chromen-7-amine. The obtained compound was used in the next reaction without purification.

### Intermediate 20: Synthesis of 1-(2,2-dimethyl-3-vinyl-2H-chromen-7-yl)ethan-1-one

### (a) Synthesis of 2,2-dimethyl-4-oxochroman-7-carbonitrile

7-Bromo-2,2-dimethylchroman-4-one (170 mg, 0.64 mmol) obtained in Intermediate 9 (a) above, zinc cyanide (149 mg, 1.27 mmol), tris(dibenzylidine acetone)dipalladium(0) (29 mg, 0.032 mmol) and 2-dicyclohexylphosphino-2',6'-dimethoxy-1,1'-biphenyl (26 mg, 0.064 mmol) were added to a mixed solvent of *N*,*N*-dimethylformamide/water (10:1, 7 mL) and reacted using a microwave (150W, 120°C, 20 minutes) under nitrogen atmosphere. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (hexane : ethyl acetate = 3 : 1, v/v) to obtain 2,2-dimethyl-4-oxochroman-7-carbonitrile (105 mg, 72%).

### (b) Synthesis of 2,2-dimethyl-2H-chromen-7-carbonitrile

Except that 2,2-dimethyl-4-oxochroman-7-carbonitrile was used instead of 3-bromo-2,2-dimethyl-7-((triisopropylsilyl)oxy)chroman-4-one, the same synthesis method as in Intermediate 7 (d) above was carried out to obtain 2,2-dimethyl-2*H*-chromen-7-carbonitrile (54 mg, 66%).

### (c) Synthesis of 1-(2,2-dimethyl-2H-chromen-7-yl)ethan-1-one

2,2-Dimethyl-2*H*-chromen-7-carbonitrile (200 mg, 1.08 mmol) obtained in Intermediate 20 (b) above was dissolved in tetrahydrofuran (1 mL) and then cooled to 0°C. Methylmagnesium bromide (1.8 mL, 5.4 mmol, 3M/diethyl ether) was added dropwise thereto under nitrogen atmosphere and stirred at room temperature for 30 minutes. When the reaction was completed, the reaction mixture was cooled to 0°C, aqueous ammonium chloride solution was added dropwise thereto, stirred for 1 hour, and extracted with ethyl acetate. The separated organic layer was dried over sodium sulfate, filtered, concentrated by distillation under reduced pressure, and separated by MPLC (100% dichloromethane) to obtain 1-(2,2-dimethyl-2*H*-chromen-7-yl)ethan-1-one (110 mg, 50%).

### (d) Synthesis of 1-(3-bromo-4-hydroxy-2,2-dimethylchroman-7-yl)ethan-1-one

Except that 1-(2,2-dimethyl-2*H*-chromen-7-yl)ethan-1-one was used instead of ((6,8-difluoro-2,2-dimethyl-2*H*-chromen-7-yl)oxy)triisopropylsilane, the same synthesis method as in Intermediate 18 (f) above was carried out to obtain 1-(3-bromo-4-hydroxy-2,2-dimethylchroman-7-yl)ethan-1-one (58 mg, 58%).

### (e) Synthesis of 1-(3-bromo-2,2-dimethyl-2H-chromen-7-yl)ethan-1-one

Except that 1-(3-bromo-4-hydroxy-2,2-dimethylchroman-7-yl)ethan-1-one was used instead of 3-bromo-6,8-difluoro-2,2-dimethyl-7-((triisopropylsilyl)oxy)chroman-4-ol, the same synthesis method as in Intermediate 18 (g) above was carried out to obtain 1-(3-bromo-2,2-dimethyl-2*H*-chromen-7-yl)ethan-1-one (35 mg, 85%).

### (f) Synthesis of 1-(2,2-dimethyl-3-vinyl-2H-chromen-7-yl)ethan-1-one

Except that 1-(3-bromo-2,2-dimethyl-2*H*-chromen-7-yl)ethan-1-one was used instead of ((3-bromo-2,2-dimethyl-2*H*-chromen-7-yl)oxy)triisopropylsilane, the same synthesis method as in Intermediate 7 (e) above was carried out to quantitatively obtain 1-(2,2-dimethyl-3-vinyl-2*H-*chromen-7-yl)ethan-1-one. The obtained compound was used in the next reaction without purification.

### Intermediate 21: Synthesis of tert-butyl (2,2-dimethyl-3-vinyl-2H-chromen-7-yl)(methyl)carbamate

### (a) Synthesis of tert-butyl (3-formyl-2,2-dimethyl-2H-chromen-7-yl)(methyl)carbamate

Except that 7-bromo-2,2-dimethyl-2H-chromen-3-carbaldehyde and tert-butyl methylcarbamate were used instead of 7-bromo-2,2-dimethylchroman-4-one and *O*-tert-butyl-carbamate, respectively, the same synthesis method as in Intermediate 9 (b) above was carried out to obtain tert-butyl (3-formyl-2,2-dimethyl-2*H*-chromen-7-yl)(methyl)carbamate (500 mg, 88%).

### (b) Synthesis of tert-butyl (2,2-dimethyl-3-vinyl-2H-chromen-7-yl)(methyl)carbamate

Except that tert-butyl (3-formyl-2,2-dimethyl-2*H*-chromen-7-yl)(methyl)carbamate was used instead of tert-butyl ethyl(3-formyl-2,2-dimethylchromen-7-yl)carbamate, the same synthesis method as in Intermediate 17 (b) above was carried out to quantitatively obtain tert-butyl (2,2-dimethyl-3-vinyl-2*H*-chromen-7-yl)(methyl)carbamate. The obtained compound was used in the next reaction without purification.

### Intermediate 22: Synthesis of N,N-diethyl-2,2-dimethyl-3-vinyl-2H-chromen-7-amine

### (a) Synthesis of 7-(diethylamino)-2,2-dimethyl-2H-chromen-3-carbaldehyde

Except that 7-bromo-2,2-dimethyl-2*H*-chromen-3-carbaldehyde and diethylamine were used instead of 7-bromo-2,2-dimethylchroman-4-one and *O*-tert-butyl-carbamate, respectively, the same synthesis method as in Intermediate 9 (b) above was carried out to obtain 7-(diethylamino)-2,2-dimethyl-2*H*-chromen-3-carbaldehyde (500 mg, 88%).

### (b) Synthesis of N,N-diethyl-2,2-dimethyl-3-vinyl-2H-chromen-7-amine

Except that 7-(diethylamino)-2,2-dimethyl-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain *N*,*N-*diethyl-2,2-dimethyl-3-vinyl-2*H*-chromen-7-amine. The obtained compound was used in the next reaction without purification.

### Intermediate 23: Synthesis of 1-(2,2-dimethyl-3-vinyl-2H-chromen-7-yl)azetidine

### (a) Synthesis of 1-(2,2-dimethyl-2H-chromen-7-yl)azetidine

Except that 7-bromo-2,2-dimethyl-2*H*-chromene and azetidine hydrochloride were used instead of 7-bromo-2,2-dimethylchroman-4-one and *O*-tert-butyl-carbamate, respectively, the same synthesis method as in Intermediate 9 (b) above was carried out to obtain 1-(2,2-dimethyl-2*H*-chromen-7-yl)azetidine (520 mg, 84%).

### (b) Synthesis of 1-(3-bromo-2,2-dimethyl-2H-chromen-7-yl)azetidine

Except that 1-(2,2-dimethyl-2*H*-chromen-7-yl)azetidine was used instead of 7-hydroxy-2,2-dimethylchroman-4-one, the same synthesis method as in Intermediate 7 (b) above was carried out to obtain 1-(3-bromo-2,2-dimethyl-2*H*-chromen-7-yl)azetidine (580 mg, 90%).

### (c) Synthesis of 1-(2,2-dimethyl-3-vinyl-2H-chromen-7-yl)azetidine

Except that 1-(3-bromo-2,2-dimethyl-2*H*-chromen-7-yl)azetidine was used instead of ((3-bromo-2,2-dimethyl-2H-chromen-7-yl)oxy)triisopropylsilane, the same synthesis method as in Intermediate 7 (e) above was carried out to quantitatively obtain 1-(2,2-dimethyl-3-vinyl-2*H-*chromen-7-yl)azetidine. The obtained compound was used in the next reaction without purification.

### Intermediate 24: Synthesis of 1-(2,2-dimethyl-3-vinyl-2H-chromen-7-yl)pyrrolidine

### (a) Synthesis of 2,2-dimethyl-7-(pyrrolidin-1-yl)-2H-chromen-3-carbaldehyde

Except that 7-bromo-2,2-dimethyl-2*H*-chromen-3-carbaldehyde and piperidine were used instead of 7-bromo-2,2-dimethylchroman-4-one and *O*-tert-butyl-carbamate, respectively, the same synthesis method as in Intermediate 9 (b) above was carried out to obtain 2,2-dimethyl-7-(pyrrolidin-1-yl)-2*H*-chromen-3-carbaldehyde (550 mg, 94%).

### (b) Synthesis of 1-(2,2-dimethyl-3-vinyl-2H-chromen-7-yl)pyrrolidine

Except that 2,2-dimethyl-7-(pyrrolidin-1-yl)-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 1-(2,2-dimethyl-3-vinyl-2*H*-chromen-7-yl)pyrrolidine. The obtained compound was used in the next reaction without purification.

### Intermediate 25: Synthesis of ((2,2-dimethyl-6-(trifluoromethoxy)-3-vinyl-2H-chromen-7-yl)oxy)triisopropylsilane

### (a) Synthesis of 7-hydroxy-2,2-dimethyl-6-(trifluoromethoxy)chroman-4-one

Except that 4-(trifluoromethoxy)benzene-1,3-diol was used instead of 2,4-difluorobenzene-1,3-diol, the same synthesis method as in Intermediate 18 (g) above was carried out to obtain 7-hydroxy-2,2-dimethyl-6-(trifluoromethoxy)chroman-4-one (700 mg, 84%).

### (b) Synthesis of 3-bromo-7-hydroxy-2,2-dimethyl-6-(trifluoromethoxy)chroman-4-one

Except that 7-hydroxy-2,2-dimethyl-6-(trifluoromethoxy)chroman-4-one was used instead of 7-hydroxy-2,2-dimethylchroman-4-one, the same synthesis method as in Intermediate 7 (b) above was carried out to obtain 3-bromo-7-hydroxy-2,2-dimethyl-6-(trifluoromethoxy)chroman-4-one (720 mg, 92%).

### (c) Synthesis of 3-bromo-2,2-dimethyl-6-(trifluoromethoxy)-7-((triisopropylsilyl)oxy)chroman-4-one

Except that 3-bromo-7-hydroxy-2,2-dimethyl-6-(trifluoromethoxy)chroman-4-one was used instead of 3-bromo-7-hydroxy-2,2-dimethylchroman-4-one, the same synthesis method as in Intermediate 7 (c) above was carried out to obtain 3-bromo-2,2-dimethyl-6-(trifluoromethoxy)-7-((triisopropylsilyl)oxy)chroman-4-one (500 mg, 75%).

### (d) Synthesis of ((3-bromo-2,2-dimethyl-6-(trifluoromethoxy)-2H-chromen-7-yl)oxy)triisopropylsilane

Except that 3-bromo-2,2-dimethyl-6-(trifluoromethoxy)-7-((triisopropylsilyl)oxy)chroman-4-one was used instead of 3-bromo-2,2-dimethyl-7-((triisopropylsilyl)oxy)chroman-4-one, the same synthesis method as in Intermediate 7 (d) above was carried out to obtain ((3-bromo-2,2-dimethyl-6-(trifluoromethoxy)-2*H*-chromen-7-yl)oxy)triisopropylsilane (210 mg, 54%).

### (e) Synthesis of ((2,2-dimethyl-6-(trifluoromethoxy)-3-vinyl-2H-chromen-7-yl)oxy)triisopropylsilane

Except that ((3-bromo-2,2-dimethyl-6-(trifluoromethoxy)-2*H*-chromen-7-yl)oxy)triisopropylsilane was used instead of ((3-bromo-2,2-dimethyl-2*H*-chromen-7-yl)oxy)triisopropylsilane, the same synthesis method as in Intermediate 7 (e) above was carried out to quantitatively obtain ((2,2-dimethyl-6-(trifluoromethoxy)-3-vinyl-2*H*-chromen-7-yl)oxy)triisopropylsilane. The obtained compound was used in the next reaction without purification.

### Intermediate 26: Synthesis of 2,2-dimethyl-3-vinyl-2H-chromen-7-carbonitrile

### (a) Synthesis of 3-formyl-2,2-dimethyl-2H-chromen-7-carbonitrile

Except that 7-bromo-2,2-dimethyl-2*H*-chromen-3-carbaldehyde was used instead of 7-bromo-2,2-dimethylchroman-4-one, the same synthesis method as in Intermediate 20 (a) above was carried out to obtain 3-formyl-2,2-dimethyl-2*H*-chromen-7-carbonitrile (320 mg, 65%).

### (b) Synthesis of 2,2-dimethyl-3-vinyl-2H-chromen-7-carbonitrile

Except that 3-formyl-2,2-dimethyl-2*H*-chromen-7-carbonitrile was used instead of tert-butyl ethyl(3-formyl-2,2-dimethylchromen-7-yl)carbamate, the same synthesis method as in Intermediate 17 (b) above was carried out to quantitatively obtain 2,2-dimethyl-3-vinyl-2*H-*chromen-7-carbonitrile. The obtained compound was used in the next reaction without purification.

### Intermediate 27: Synthesis of 2,2-dimethyl-7-nitro-3-vinyl-2H-chromene

### (a) Synthesis of (E)-2-(3-hydroxy-3-methylbut-1-en-1-yl)-5-nitrophenol

2-Bromo-5-nitrophenol (500 mg, 2.3 mmol) was dissolved in *N,N*-dimethylacetamide (11 mL), and 2-methylbut-3-en-2-ol ( 395 mg, 4.6 mmol), triphenylphosphine (241 mg, 0.92 mmol), palladium(II) acetate (103 mg, 0.46 mmol) and triethylamine (696 mg, 6.88 mmol) were added thereto and stirred under reflux at 130°C for 4 hours. When the reaction was completed, the reaction mixture was diluted in ethyl acetate and washed with aqueous sodium chloride solution. The separated organic layer was dried over sodium sulfate, filtered, concentrated by distillation under reduced pressure, and separated by reverse-phase chromatography (acetonitrile containing 0.1% formic acid : water containing 0.1% formic acid = 5 : 95-90 : 10) to obtain (E)-2-(3-hydroxy-3-methylbut-1-en-1-yl)-5-nitrophenol (288 mg, 56%).

### (b) Synthesis of 2,2-dimethyl-7-nitro-2H-chromene

(*E*)-2-(3-hydroxy-3-methylbut-1-en-1-yl)-5-nitrophenol (288 mg, 1.29 mmol) obtained in Intermediate 27 (a) above was dissolved in *N*,*N-*dimethylformamide (2.4 mL), and silica gel (1,550 mg, 25.8 mmol) was added thereto, and heated and stirred at 140°C for 18 hours. When the reaction was completed, the reaction mixture was concentrated by distillation under reduced pressure, and the residue was filtered and washed with ethyl acetate. The filtrate was separated into layers of ethyl acetate and water, and the water layer was extracted with ethyl acetate. The separated organic layer was washed with aqueous sodium chloride solution, dried over sodium sulfate, filtered, concentrated by distillation under reduced pressure, and separated by MPLC (ethyl acetate : hexane = 10 : 90, v/v) to obtain 2,2-dimethyl-7-nitro-2*H*-chromene (190 mg, 72%).

### (c) Synthesis of 3-bromo-2,2-dimethyl-7-nitrochroman-4-ol

Except that 2,2-dimethyl-7-nitro-2*H*-chromene was used instead of ((6,8-difluoro-2,2-dimethyl-2*H*-chromen-7-yl)oxy)triisopropylsilane, the same synthesis method as in Intermediate 18 (f) above was carried out to obtain 3-bromo-2,2-dimethyl-7-nitrochroman-4-ol (120 mg, 69%).

### (d) Synthesis of 3-bromo-2,2-dimethyl-7-nitro-2H-chromene

Except that 3-bromo-2,2-dimethyl-7-nitrochroman-4-ol was used instead of 3-bromo-6,8-difluoro-2,2-dimethyl-7-((triisopropylsilyl)oxy)chroman-4-ol, the same synthesis method as in Intermediate 18 (g) above was carried out to obtain 3-bromo-2,2-dimethyl-7-nitro-2*H*-chromene (60 mg, 58%).

### (e) Synthesis of 2,2-dimethyl-7-nitro-3-vinyl-2H-chromene

Except that 3-bromo-2,2-dimethyl-7-nitro-2*H*-chromene was used instead of ((3-bromo-2,2-dimethyl-2*H*-chromen-7-yl)oxy)triisopropylsilane, the same synthesis method as in Intermediate 7 (e) above was carried out to quantitatively obtain 2,2-dimethyl-7-nitro-3-vinyl-2*H-*chromene. The obtained compound was used in the next reaction without purification.

### Intermediate 28: Synthesis of 1-(2,2-dimethyl-3-vinyl-2H-chromen-7-yl)-4-methylpiperazine

### (a) Synthesis of 2,2-dimethyl-7-(4-methylpiperazin-1-yl)-2H-chromen-3-carbaldehyde

Except that 7-bromo-2,2-dimethyl-2*H*-chromen-3-carbaldehyde and N-methyl piperazine were used instead of 7-bromo-2,2-dimethylchroman-4-one and *O*-tert-butyl-carbamate, respectively, the same synthesis method as in Intermediate 9 (b) above was carried out to obtain 2,2-dimethyl-7-(4-methylpiperazin-1-yl)-2*H*-chromen-3-carbaldehyde (520 mg, 84%).

### (b) Synthesis of 1-(2,2-dimethyl-3-vinyl-2H-chromen-7-yl)-4-methylpiperazine

Except that 2,2-dimethyl-7-(4-methylpiperazin-1-yl)-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 1-(2,2-dimethyl-3-vinyl-2*H*-chromen-7-yl)-4-methylpiperazine. The obtained compound was used in the next reaction without purification.

### Intermediate 29: Synthesis of tert-butyl 4-(2,2-dimethyl-3-vinyl-2H-chromen-7-yl)piperazine-1-carboxylate

### (a) Synthesis of tert-butyl 4-(3-formyl-2,2-dimethyl-2H-chromen-7-yl)piperazine-1-carboxylate

Except that 7-bromo-2,2-dimethyl-2*H*-chromen-3-carbaldehyde and *N-*tert-butoxycarbonyl piperazine were used instead of 7-bromo-2,2-dimethylchroman-4-one and *O*-tert-butyl-carbamate, respectively, the same synthesis method as in Intermediate 9 (b) above was carried out to obtain tert-butyl 4-(3-formyl-2,2-dimethyl-2*H*-chromen-7-yl)piperazine-1-carboxylate (500 mg, 90%).

### (b) Synthesis of tert-butyl 4-(2,2-dimethyl-3-vinyl-2H-chromen-7-yl)piperazine-1-carboxylate

Except that tert-butyl 4-(3-formyl-2,2-dimethyl-2*H*-chromen-7-yl)piperazine-1-carboxylate was used instead of tert-butyl ethyl(3-formyl-2,2-dimethylchromen-7-yl)carbamate, the same synthesis method as in Intermediate 17 (b) above was carried out to quantitatively obtain tert-butyl 4-(2,2-dimethyl-3-vinyl-2*H*-chromen-7-yl)piperazine-1-carboxylate. The obtained compound was used in the next reaction without purification.

### Intermediate 30: Synthesis of ((6-cyclopropyl-2,2-dimethyl-3-vinyl-2H-chromen-7-yl)oxy)triisopropylsilane

### (a) Synthesis of 6-bromo-7-hydroxy-2,2-dimethylchroman-4-one

Except that 4-bromobenzene-1,3-diol was used instead of 2,4-difluorobenzene-1,3-diol, the same synthesis method as in Intermediate 18 (g) above was carried out to obtain 6-bromo-7-hydroxy-2,2-dimethylchroman-4-one (800 mg, 80%).

### (b) Synthesis of 6-bromo-7-((4-methoxybenzyl)oxy)-2,2-dimethylchroman-4-one

6-Bromo-7-hydroxy-2,2-dimethylchroman-4-one (180 mg, 0.66 mmol) obtained in Intermediate 30 (a) above and potassium carbonate (184 mg, 1.33 mmol) were dissolved in *N*,*N-*dimethylformamide (7 mL), and *p*-methoxybenzyl chloride (90 µl, 0.66 mmol) was added dropwise thereto and stirred at room temperature for 12 hours. To the reaction mixure *p-*methoxybenzyl chloride (90 µl, 0.66 mmol) and potassium carbonate (184 mg, 1.33 mmol) were added and stirred for 2 hours. Then, the reaction temperature was raised to 40°C and stirred for additional 4 hours. When the reaction was completed, the reaction mixture was diluted in ethyl acetate and washed with water and aqueous sodium chloride solution. The separated organic layer was dried over sodium sulfate, filtered, concentrated by distillation under reduced pressure, and separated by reverse-phase chromatography (acetonitrile containing 0.1% formic acid : water containing 0.1% formic acid = 5 : 95-100 : 0) to obtain 6-bromo-7-((4-methoxybenzyl)oxy)-2,2-dimethylchroman-4-one (240 mg, 92%).

### (c) Synthesis of 6-cyclopropyl-7-((4-methoxybenzyl)oxy)-2,2-dimethylchroman-4-one

Except that 6-bromo-7-((4-methoxybenzyl)oxy)-2,2-dimethylchroman-4-one was used instead of ((3-bromo-2,2-dimethyl-2*H*-chromen-7-yl)oxy)triisopropylsilane, the same synthesis method as in Intermediate 7 (e) above was carried out to obtain 6-cyclopropyl-7-((4-methoxybenzyl)oxy)-2,2-dimethylchroman-4-one (200 mg, 85%).

### (d) Synthesis of 3-bromo-6-cyclopropyl-7-hydroxy-2,2-dimethylchroman-4-one

Except that 6-cyclopropyl-7-((4-methoxybenzyl)oxy)-2,2-dimethylchroman-4-one was used instead of 7-hydroxy-2,2-dimethylchroman-4-one, the same synthesis method as in Intermediate 7 (b) above was carried out to obtain 3-bromo-6-cyclopropyl-7-hydroxy-2,2-dimethylchroman-4-one (160 mg, 78%).

### (e) Synthesis of 3-bromo-6-cyclopropyl-2,2-dimethyl-7-((triisopropylsilyl)oxy)chroman-4-one

Except that 3-bromo-6-cyclopropyl-7-hydroxy-2,2-dimethylchroman-4-one was used instead of 3-bromo-7-hydroxy-2,2-dimethylchroman-4-one, the same synthesis method as in Intermediate 7 (c) above was carried out to obtain 3-bromo-6-cyclopropyl-2,2-dimethyl-7-((triisopropylsilyl)oxy)chroman-4-one (140 mg, 80%).

### (f) Synthesis of ((3-bromo-6-cyclopropyl-2,2-dimethyl-2H-chromen-7-yl)oxy)triisopropylsilane

Except that 3-bromo-6-cyclopropyl-2,2-dimethyl-7-((triisopropylsilyl)oxy)chroman-4-one was used instead of 3-bromo-2,2-dimethyl-7-((triisopropylsilyl)oxy)chroman-4-one, the same synthesis method as in Intermediate 7 (d) above was carried out to obtain ((3-bromo-6-cyclopropyl-2,2-dimethyl-2*H*-chromen-7-yl)oxy)triisopropylsilane (55 mg, 52%).

### (g) Synthesis of ((6-cyclopropyl-2,2-dimethyl-3-vinyl-2H-chromen-7-yl)oxy)triisopropylsilane

Except that ((3-bromo-6-cyclopropyl-2,2-dimethyl-2*H*-chromen-7-yl)oxy)triisopropylsilane was used instead of ((3-bromo-2,2-dimethyl-2*H*-chromen-7-yl)oxy)triisopropylsilane, the same synthesis method as in Intermediate 7 (e) above was carried out to quantitatively obtain ((6-cyclopropyl-2,2-dimethyl-3-vinyl-2*H*-chromen-7-yl)oxy)triisopropylsilane. The obtained compound was used in the next reaction without purification.

### Intermediate 31: Synthesis of ((2,2-bis(fluoromethyl)-3-vinyl-2H-chromen-7-yl)oxy)triisopropylsilane

### (a) Synthesis of 1-(2-hydroxy-4-((tetrahydro-2H-pyran-2-yl)oxy)phenyl)ethan-1-one

1-(2,4-Dihydroxyphenyl)ethanone (2.51g, 16.47 mmol) and pyridinium p-toluenesulfonic acid (170 mg, 0.68 mmol) were dissolved in dichloromethane (25 mL), and 3,4-dihydro-2H-pyran (3.46 g, 41.18 mmol) was added thereto and stirred at room temperature for 3 hours. When the reaction was completed, the reaction mixture was diluted in dichloromethane and washed with aqueous sodium chloride solution. The separated organic layer was dried over sodium sulfate, filtered, concentrated by distillation under reduced pressure, and separated by MPLC (hexane : ethyl acetate = 10 : 1, v/v) to obtain 1-(2-hydroxy-4-((tetrahydro-2*H*-pyran-2-yl)oxy)phenyl)ethan-1-one (3.65 g, 94%).

### (b) Synthesis of 4-fluoro-3-(fluoromethyl)-3-hydroxy-1-(2-hydroxy-4-((tetrahydro-2H-pyran-2-yl)oxy)phenyl)butan-1 -one

After cooling anhydrous tetrahydrofuran (15 mL) to 0°C, lithium diasopropylamide (15 mL, 30 mmol, 2M/tetrahydrofuran) was added thereto under nitrogen atmosphere, and 1-(2-hydroxy-4-((tetrahydro-2*H*-pyran-2-yl)oxy)phenyl)ethan-1-one (3.02 g, 12.78 mmol) dissolved in anhydrous tetrahydrofuran (7 mL) was added dropwise thereto over 30 minutes. The reaction mixture was cooled to -40°C, and difluoroacetone (1.57 g, 16.71 mmol) diluted in anhydrous tetrahydrofuran (7 mL) was added dropwise thereto over 1 hour and stirred at -40°C for additional 40 minutes. When the reaction was completed, the temperature of the reaction mixture was adjusted to 0°C, and aqueous ammonium chloride solution was added dropwise thereto. The reaction mixture was diluted in ethyl acetate, washed with aqueous ammonium chloride solution and aqueous sodium chloride solution, dried over sodium sulfate, filtered, and concentrated by distillation under reduced pressure to quantitatively obtain 4-fluoro-3-(fluoromethyl)-3-hydroxy-1-(2-hydroxy-4-((tetrahydro-2*H*-pyran-2-yl)oxy)phenyl)butan-1-one. The obtained compound was used in the next reaction without purification.

### (c) Synthesis of 2,2-bis(fluoromethyl)-7-((tetrahydro-2H-pyran-2-yl)oxy)chroman-4-one

4-Fluoro-3-(fluoromethyl)-3-hydroxy-1-(2-hydroxy-4-((tetrahydro-2*H*-pyran-2-yl) oxy)phenyl)butan-1-one (4.22 g, 12.78 mmol) obtained in Intermediate 31 (b) above was added to pyridine (30 mL) and cooled to 0°C. While maintaining the reaction mixture at 0°C, trifluoroacetic anhydride (5.55 g, 26.42 mmol) was added dropwise thereto for 2 hours, and ethanol (20 mL) and 1,8-diazabicyclo[5,4,0]undec-7-ene (12.24 g, 80.4 mmol) were then added sequentially. The reaction mixture was raised to 50°C, and heated and stirred for 1 hour. When the reaction was completed, the reaction mixture was diluted in ethyl acetate and washed with an aqueous ammonium chloride solution, an aqueous sodium bicarbonate solution and an aqueous sodium chloride solution. The separated organic layer was dried over sodium sulfate, filtered, concentrated by distillation under reduced pressure, and separated by MPLC (hexane : ethyl acetate = 10 : 1, v/v) to obtain 2,2-bis(fluoromethyl)-7-((tetrahydro-2*H*-pyran-2-yl)oxy)chroman-4-one (1.08 g, 27%).

### (d) Synthesis of 2,2-bis(fluoromethyl)-7-hydroxychroman-4-one

2,2-Bis(fluoromethyl)-7-((tetrahydro-2*H*-pyran-2-yl)oxy)chroman-4-one (1.08 g, 3.45 mmol) obtained in Intermediate 31 (c) above was dissolved in tetrahydrofuran (10 mL), and p-toluenesulfonic acid (134 mg, 0.7 mmol) and methanol (10 mL) were added thereto, and heated and stirred at 50°C for 2 hours. When the reaction was completed, the reaction mixture was concentrated by distillation under reduced pressure and separated by MPLC (hexane : ethyl acetate = 2 : 1, v/v) to obtain 2,2-bis(fluoromethyl)-7-hydroxychroman-4-one (626 mg, 80%).

### (e) Synthesis of 3-bromo-2,2-bis(fluoromethyl)-7-hydroxychroman-4-one

Except that 2,2-bis(fluoromethyl)-7-hydroxychroman-4-one was used instead of 7-hydroxy-2,2-dimethylchroman-4-one, the same synthesis method as in Intermediate 7 (b) above was carried out to quantitatively obtain 3-bromo-2,2-bis(fluoromethyl)-7-hydroxychroman-4-one. The obtained compound was used in the next reaction without purification.

### (f) Synthesis of 3-bromo-2,2-bis(fluoromethyl)-7-((triisopropylsilyl)oxy)chroman-4-one

Except that 3-bromo-2,2-bis(fluoromethyl)-7-hydroxychroman-4-one was used instead of 3-bromo-7-hydroxy-2,2-dimethylchroman-4-one, the same synthesis method as in Intermediate 7 (c) above was carried out to obtain 3-bromo-2,2-bis(fluoromethyl)-7-((triisopropylsilyl)oxy)chroman-4-one (1.17 g, 92%).

### (g) Synthesis of 3-bromo-2,2-bis(fluoromethyl)-7-((triisopropylsilyl)oxy)chroman-4-ol

3-Bromo-2,2-bis(fluoromethyl)-7-((triisopropylsilyl)oxy)chroman-4-one (1.17 g, 2.52 mmol) obtained in Intermediate 31 (f) above was dissolved in ethanol (12 mL) and cooled to 0°C, and sodium borohydride (48 mg, 1.28 mmol) was then added thereto. After the reaction mixture was stirred at 0°C for 15 minutes, sodium borohydride (46 mg, 1.22 mmol) was added in portions three times and stirred at 0°C for additional 5 minutes. When the reaction was completed, the temperature of the reaction mixture was adjusted to 0°C, aqueous ammonium chloride solution was added dropwise thereto, and extraction was performed several times with ethyl acetate. The separated organic layer was washed with aqueous sodium chloride solution, dried over sodium sulfate, filtered, and concentrated by distillation under reduced pressure to quantitatively obtain 3-bromo-2,2-bis(fluoromethyl)-7-((triisopropylsilyl)oxy)chroman-4-ol. The obtained compound was used in the next reaction without purification.

### (h) Synthesis of ((3-bromo-2,2-bis(fluoromethyl)-2H-chromen-7-yl)oxy)triisopropylsilane

Except that 3-bromo-2,2-bis(fluoromethyl)-7-((triisopropylsilyl)oxy)chroman-4-ol was used instead of 3-bromo-6,8-difluoro-2,2-dimethyl-7-((triisopropylsilyl)oxy)chroman-4-ol, the same synthesis method as in Intermediate 18 (g) above was carried out to obtain ((3-bromo-2,2-bis(fluoromethyl)-2*H*-chromen-7-yl)oxy)triisopropylsilane (506 mg, 1.13 45%).

### (i) Synthesis of ((2,2-bis(fluoromethyl)-3-vinyl-2H-chromen-7-yl)oxy)triisopropylsilane

Except that ((3-bromo-2,2-bis(fluoromethyl)-2*H*-chromen-7-yl)oxy)triisopropylsilane was used instead of ((3-bromo-2,2-dimethyl-2*H*-chromen-7-yl)oxy)triisopropylsilane, the same synthesis method as in Intermediate 7 (e) above was carried out to quantitatively obtain ((2,2-bis(fluoromethyl)-3-vinyl-2*H*-chromen-7-yl)oxy)triisopropylsilane. The obtained compound was used in the next reaction without purification.

### Intermediate 32: Synthesis of N,N-diisopropyl-2,2-dimethyl-3-vinyl-2H-chromen-7-amine

### (a) Synthesis of 7-(diisopropylamino)-2,2-dimethyl-2H-chromen-3-carbaldehyde

Except that 7-bromo-2,2-dimethyl-2*H*-chromen-3-carbaldehyde and diisopropylamine were used instead of 7-bromo-2,2-dimethylchroman-4-one and *O*-tert-butyl-carbamate, respectively, the same synthesis method as in Intermediate 9 (b) above was carried out to obtain 7-(diisopropylamino)-2,2-dimethyl-2*H*-chromen-3-carbaldehyde (620 mg, 93%).

### (b) Synthesis of N,N-diisopropyl-2,2-dimethyl-3-vinyl-2H-chromen-7-amine

Except that 7-(diisopropylamino)-2,2-dimethyl-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain *N*,*N-*diisopropyl-2,2-dimethyl-3-vinyl-2*H*-chromen-7-amine. The obtained compound was used in the next reaction without purification.

### Intermediate 33: Synthesis of N-isopropyl-N,2,2-trimethyl-3-vinyl-2H-chromen-7-amine

### (a) Synthesis of 7-(isopropyl(methyl)amino)-2,2-dimethyl-2H-chromen-3-carbaldehyde

Except that 7-bromo-2,2-dimethyl-2*H*-chromen-3-carbaldehyde and *N-*methylisopropylamine were used instead of 7-bromo-2,2-dimethylchroman-4-one and *O*-tert-butyl-carbamate, respectively, the same synthesis method as in Intermediate 9 (b) above was carried out to obtain 7-(isopropyl(methyl)amino)-2,2-dimethyl-2*H*-chromen-3-carbaldehyde (430 mg, 85%).

### (b) Synthesis of N-isopropyl-N,2,2-trimethyl-3-vinyl-2H-chromen-7-amine

Except that 7-(isopropyl(methyl)amino)-2,2-dimethyl-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain *N*-isopropyl-*N*,2,2-trimethyl-3-vinyl-2*H*-chromen-7-amine. The obtained compound was used in the next reaction without purification.

### Intermediate 34: Synthesis of tert-butyl (2,2-dimethyl-3-vinyl-2H-chromen-7-yl)(ethyl)carbamate

### (a) Synthesis of tert-butyl ethyl(3-formyl-2,2-dimethyl-2H-chromen-7 -yl)carbamate

Except that 7-bromo-2,2-dimethyl-2*H*-chromen-3-carbaldehyde and tert-butyl ethylcarbamate were used instead of 7-bromo-2,2-dimethylchroman-4-one and *O*-tert-butyl-carbamate, respectively, the same synthesis method as in Intermediate 9 (b) above was carried out to obtain tert-butyl ethyl(3-formyl-2,2-dimethyl-2*H*-chromen-7-yl)carbamate (520 mg, 88%).

### (b) Synthesis of tert-butyl (2,2-dimethyl-3-vinyl-2H-chromen-7-yl)(ethyl)carbamate

Except that tert-butyl ethyl(3-formyl-2,2-dimethyl-2*H*-chromen-7-yl)carbamate was used instead of tert-butyl ethyl(3-formyl-2,2-dimethylchromen-7-yl)carbamate, the same synthesis method as in Intermediate 17 (b) above was carried out to quantitatively obtain tert-butyl (2,2-dimethyl-3-vinyl-2*H*-chromen-7-yl)(ethyl)carbamate. The obtained compound was used in the next reaction without purification.

### Intermediate 35: Synthesis of 5-(2,2-dimethyl-3-vinyl-2H-chromen-7-yl)-1,3-dimethyl-1H-pyrazole

### (a) Synthesis of 7-(1,3-dimethyl-1H-pyrazol-5-yl)-2,2-dimethyl-2H-chromen-3-carbaldehyde

Except that 7-bromo-2,2-dimethyl-2*H*-chromen-3-carbaldehyde and (1,3-dimethyl-1*H-*pyrazol-5-yl)boronic acid were used instead of ((3-bromo-2,2-dimethyl-2*H*-chromen-7-yl)oxy)triisopropylsilane and vinylboronic acid dibutyl ester, respectively, the same synthesis method as in Intermediate 7 (e) above was carried out to obtain 7-(1,3-dimethyl-1*H*-pyrazol-5-yl)-2,2-dimethyl-2H-chromen-3-carbaldehyde (300 mg, 85%).

### (b) Synthesis of 5-(2,2-dimethyl-3-vinyl-2H-chromen-7-yl)-1,3-dimethyl-1H-pyrazole

Except that 7-(1,3-dimethyl-1*H*-pyrazol-5-yl)-2,2-dimethyl-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 5-(2,2-dimethyl-3-vinyl-2*H*-chromen-7-yl)-1,3-dimethyl-1*H*-pyrazole. The obtained compound was used in the next reaction without purification.

### Intermediate 36: Synthesis of 4-(2,2-dimethyl-3-vinyl-2H-chromen-7-yl)-2,5-dimethylthiazole

### (a) Synthesis of 7-(2,5-dimethylthiazol-4-yl)-2,2-dimethyl-2H-chromen-3-carbaldehyde

Except that 7-bromo-2,2-dimethyl-2*H*-chromen-3-carbaldehyde and (1,3-dimethyl-1*H-*pyrazol-5-yl)boronic acid were used instead of ((3-bromo-2,2-dimethyl-2*H*-chromen-7-yl)oxy)triisopropylsilane and vinylboronic acid dibutyl ester, respectively, the same synthesis method as in Intermediate 7 (e) above was carried out to obtain 7-(2,5-dimethylthiazol-4-yl)-2,2-dimethyl-2*H*-chromen-3-carbaldehyde (300 mg, 85%).

### (b) Synthesis of 4-(2,2-dimethyl-3-vinyl-2H-chromen-7-yl)-2,5-dimethylthiazole

Except that 7-(2,5-dimethylthiazol-4-yl)-2,2-dimethyl-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 4-(2,2-dimethyl-3-vinyl-2*H*-chromen-7-yl)-2,5-dimethylthiazole. The obtained compound was used in the next reaction without purification.

### Intermediate 37: Synthesis of 4-(2,2-dimethyl-3-vinyl-2H-chromen-7-yl)-1-ethyl-3,5-dimethyl-1H-pyrazole

### (a) Synthesis of 7-(1-ethyl-3,5-dimethyl-1H-pyrazol-4-yl)-2,2-dimethyl-2H-chromen-3-carbaldehyde

Except that 7-bromo-2,2-dimethyl-2*H*-chromen-3-carbaldehyde and 1-ethyl-3,5-dimethyl-4-(4,4,5,5-tetramethyl-1,3,2-dioxabororane-2-yl)-1*H*-pyrazole were used instead of ((3-bromo-2,2-dimethyl-2*H*-chromen-7-yl)oxy)triisopropylsilane and vinylboronic acid dibutyl ester, respectively, the same synthesis method as in Intermediate 7 (e) above was carried out to obtain 7-(1-ethyl-3,5-dimethyl-1*H*-pyrazol-4-yl)-2,2-dimethyl-2*H*-chromen-3-carbaldehyde (442 mg, 80%).

### (b) Synthesis of 4-(2,2-dimethyl-3-vinyl-2H-chromen-7-yl)-1-ethyl-3,5-dimethyl-1H-pyrazole

Except that 7-(1-ethyl-3,5-dimethyl-1*H*-pyrazol-4-yl)-2,2-dimethyl-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 4-(2,2-dimethyl-3-vinyl-2*H*-chromen-7-yl)-1-ethyl-3,5-dimethyl-1*H*-pyrazole. The obtained compound was used in the next reaction without purification.

### Intermediate 38: Synthesis of 3-(2,2-dimethyl-3-vinyl-2H-chromen-7-yl)pyridine

### (a) Synthesis of 2,2-dimethyl-7-(pyridin-3-yl)-2H-chromen-3-carbaldehyde

Except that 7-bromo-2,2-dimethyl-2*H*-chromen-3-carbaldehyde and 3-pyridyl boronic acid were used instead of ((3-bromo-2,2-dimethyl-2*H*-chromen-7-yl)oxy)triisopropylsilane and vinylboronic acid dibutyl ester, respectively, the same synthesis method as in Intermediate 7 (e) above was carried out to obtain 2,2-dimethyl-7-(pyridin-3-yl)-2*H*-chromen-3-carbaldehyde (300 mg, 85%).

### (b) Synthesis of 3-(2,2-dimethyl-3-vinyl-2H-chromen-7-yl)pyridine

Except that 2,2-dimethyl-7-(pyridin-3-yl)-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 3-(2,2-dimethyl-3-vinyl-2*H-*chromen-7-yl)pyridine. The obtained compound was used in the next reaction without purification.

### Intermediate 39: Synthesis of 3-(2,2-dimethyl-3-vinyl-2H-chromen-7-yl)pyridine

### (a) Synthesis of 2,2-dimethyl-7-(pyridin-4-yl)-2H-chromen-3-carbaldehyde

Except that 7-bromo-2,2-dimethyl-2*H*-chromen-3-carbaldehyde and 4-pyridyl boronic acid were used instead of ((3-bromo-2,2-dimethyl-2*H*-chromen-7-yl)oxy)triisopropylsilane and vinylboronic acid dibutyl ester, respectively, the same synthesis method as in Intermediate 7 (e) above was carried out to obtain 2,2-dimethyl-7-(pyridin-4-yl)-2*H*-chromen-3-carbaldehyde (270 mg, 78%).

### (b) Synthesis of 3-(2,2-dimethyl-3-vinyl-2H-chromen-7-yl)pyridine

Except that 2,2-dimethyl-7-(pyridin-4-yl)-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 3-(2,2-dimethyl-3-vinyl-2*H-*chromen-7-yl)pyridine. The obtained compound was used in the next reaction without purification.

### Intermediate 40: Synthesis of 4-(2,2-dimethyl-3-vinyl-2H-chromen-7-yl)-3-methylisoxazole

### (a) Synthesis of 2,2-dimethyl-7-(3-methylisoxazol-4-yl)-2H-chromen-3-carbaldehyde

Except that 7-bromo-2,2-dimethyl-2*H*-chromen-3-carbaldehyde and 3-methyl-4-(4,4,5,5-tetramethyl-1,3,2-dioxabororan-2-yl)isoxazole were used instead of ((3-bromo-2,2-dimethyl-2*H-*chromen-7-yl)oxy)triisopropylsilane and vinylboronic acid dibutyl ester, respectively, the same synthesis method as in Intermediate 7 (e) above was carried out to obtain 2,2-dimethyl-7-(3-methylisoxazol-4-yl)-2*H*-chromen-3-carbaldehyde (335 mg, 83%).

### (b) Synthesis of 4-(2,2-dimethyl-3-vinyl-2H-chromen-7-yl)-3-methylisoxazole

Except that 2,2-dimethyl-7-(3-methylisoxazol-4-yl)-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 4-(2,2-dimethyl-3-vinyl-2*H*-chromen-7-yl)-3-methylisoxazole. The obtained compound was used in the next reaction without purification.

### Intermediate 41: Synthesis of 2,2-dimethyl-7-((tetrahydro-2H-pyran-2-yl)oxy)-3-vinyl-2H-chromene

### (a) Synthesis of 2,2-dimethyl-7-((tetrahydro-2H-pyran-2-yl)oxy)-2H-chromen-3-carbaldehyde

1-(2-Hydroxy-4-((tetrahydro-2*H*-pyran-2-yl)oxy)phenyl)ethan-1-one (0.64 g, 2.9 mmol) obtained in Intermediate 31 (a) above and potassium carbonate (0.6 g, 4.3 mmol) were dissolved in a mixed solvent of 1,4-dioxane/water (3:1, 8 mL), and 3-methylbut-2-enal (0.49 g, 5.8 mmol) was added dropwise thereto, and heated and stirred at 80°C for 72 hours. When the reaction was completed, the reaction mixture was diluted in ethyl acetate and washed with aqueous sodium chloride solution. The separated organic layer was dried over sodium sulfate, filtered, and concentrated by distillation under reduced pressure to quantitatively obtain 2,2-dimethyl-7-((tetrahydro-2*H*-pyran-2-yl)oxy)-2*H*-chromen-3-carbaldehyde. The obtained compound was used in the next reaction without purification.

### (b) Synthesis of 2,2-dimethyl-7-((tetrahydro-2H-pyran-2-yl)oxy)-3-vinyl-2H-chromene

Except that 2,2-dimethyl-7-((tetrahydro-2H-pyran-2-yl)oxy)-2*H*-chromen-3-carbaldehyde was used instead of tert-butyl ethyl(3-formyl-2,2-dimethylchromen-7-yl)carbamate, the same synthesis method as in Intermediate 17 (b) above was carried out to quantitatively obtain 2,2-dimethyl-7-((tetrahydro-2H-pyran-2-yl)oxy)-3-vinyl-2*H*-chromene. The obtained compound was used in the next reaction without purification.

### Example 1: Synthesis of 7,7-bis(fluoromethyl)-10-hydroxy-2-phenyl-5,12b-dihydro-1H,7H-chromeno[4,3-c][1,2,4]triazolo[1,2-a]pyridazine-1,3(2H)-dione

### (a) Synthesis of 7,7-bis(fluoromethyl)-2-phenyl-10-((triisopropylsilyl)oxy)-5,12b-dihydro-1H,7H-chromeno[4,3-c][1,2,4]triazolo[1,2-a]pyridazine-1,3(2H)-dione

4-Phenyl-1,2,4-triazolidine-3,5-dione (0.3 g, 1.7 mmol) was suspended in tetrahydrofuran (3 mL), and iodobenzene diacetate (0.55 g, 1.7 mmol) was added thereto and stirred for 30 minutes under light blocking. ((2,2-Bis(fluoromethyl)-3-vinyl-2*H*-chromen-7-yl)oxy)triisopropylsilane ( 0.67 g, 1.7 mmol) dissolved in tetrahydrofuran (3 mL) was added to the reaction mixture and stirred for additional 30 minutes. When the reaction was completed, the reaction mixture was diluted in ethyl acetate and washed with water. The separated organic layer was dried over sodium sulfate, filtered, concentrated by distillation under reduced pressure, and separated by MPLC (hexane : ethyl acetate = 5 : 1, v/v) to obtain 7,7-bis(fluoromethyl)-2-phenyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (0.61 g, 67%).

### (b) Synthesis of 7,7-bis(fluoromethyl)-10-hydroxy-2-phenyl-5,12b-dihydro-1H,7H-chromeno[4,3-c][1,2,4]triazolo[1,2-a]pyridazine-1,3(2H)-dione

After dissolving 7,7-bis(fluoromethyl)-2-phenyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (0.41 g, 0.72 mmol) obtained in Example 1 (a) above in tetrahydrofuran (5 mL), it was added to a conical centrifuge tube containing hydrogen fluoride-pyridine (3.85 mmol, 5 mL, 7:3, v/v) and stirred for 20 hours. When the reaction was completed, 2-(trimethylsilyl)ethanol (5 mL) was added dropwise to the reaction mixture and concentrated by distillation under reduced pressure. The obtained residue was separated by MPLC (hexane : ethyl acetate = 5 : 1, v/v) to obtain 7,7-bis(fluoromethyl)-10-hydroxy-2-phenyl-5,12b-dihydro-1*H*,7*H*-chromeno[4,3-*c*][1,2,4]triazolo[1,2-*a*]pyridazine-1,3(2*H*)-dione (296 mg, 54%).
¹H NMR (400 MHz, acetone) δ 8.56 (s, 1H), 7.69 - 7.62 (m, 2H), 7.54 (dd, J = 8.5, 7.1 Hz, 2H), 7.48 - 7.39 (m, 1H), 6.95 - 6.89 (m, 1H), 6.59 - 6.51 (m, 2H), 6.21 - 6.14 (m, 1H), 5.61 (d, J = 1.4 Hz, 1H), 4.93 (t, J = 2.1 Hz, 1H), 4.90 - 4.75 (m, 2H), 4.75 - 4.64 (m, 1H), 4.56 (dd, J = 10.3, 2.7 Hz, 1H), 4.34 - 4.18 (m, 2H), 2.91 - 2.84 (m, 1H)

### Example 2: Synthesis of 2-(4-acetylphenyl)-7,7-bis(fluoromethyl)-10-hydroxy-5,12b-dihydro-1H,7H-chromeno[4,3-c][1,2,4]triazolo[1,2-a]pyridazine-1,3(2H)-dione

### (a) Synthesis of 2-(4-acetylphenyl)-7,7-bis(fluoromethyl)-10-((triisopropylsilyl)oxy)-5,12b-dihydro-1H,7H-chromeno[4,3-c][1,2,4]triazolo[1,2-a]pyridazine-1,3(2H)-dione

4-(4-Acetylphenyl)-1,2,4-triazolidine-3,5-dione (0.3 g, 1.37 mmol) was suspended in tetrahydrofuran (3 mL), and iodobenzene diacetate (0.45 g), 1.37 mmol) was added thereto and stirred for 30 minutes under light blocking. ((2,2-Bis(fluoromethyl)-3-vinyl-2*H*-chromen-7-yl)oxy)triisopropylsilane (0.54 g, 1.37 mmol) dissolved in tetrahydrofuran (3 mL) was added to the reaction mixture and stirred for additional 30 minutes. When the reaction was completed, the reaction mixture was diluted in ethyl acetate and washed with water. The separated organic layer was dried over sodium sulfate, filtered, concentrated by distillation under reduced pressure, and separated by MPLC (hexane : ethyl acetate = 5 : 1, v/v) to obtain 2-(4-acetylphenyl)-7,7-bis(fluoromethyl)-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (0.42 g, 53%).

### (b) Synthesis of 2-(4-acetylphenyl)-7,7-bis(fluoromethyl)-10-hydroxy-5,12b-dihydro-1H,7H-chromeno[4,3-c][1,2,4]triazolo[1,2-a]pyridazine-1,3(2H)-dione

Except that 2-(4-acetylphenyl)-7,7-bis(fluoromethyl)-10-((triisopropylsilyl)oxy)-5,12b-dihydro-1*H*,7*H*-chromeno[4,3-*c*][1,2,4]triazolo[1,2-*a*]pyridazine-1,3(2*H*)-dione was used instead of 7,7-bis(fluoromethyl)-2-phenyl-10-((triisopropylsilyl)oxy)-5,12*b*-dihydro-1*H*,7*H-*chromeno[4,3-*c*][1,2,4]triazolo[1,2-*a*]pyridazine-1,3(2*H*)-dione, the same synthesis method as in Example 1 (b) above was carried out to obtain 2-(4-acetylphenyl)-7,7-bis(fluoromethyl)-10-hydroxy-5,12*b*-dihydro-1*H*,7*H*-chromeno[4,3-*c*][1,2,4]triazolo[1,2-*a*]pyridazine-1,3(2*H*)-dione (132 mg , 60%).
¹H NMR (400 MHz, acetone) δ 9.62 (s, 1H), 8.14-8.12 (m, 1H), 7.80 - 7.78 (m, 1H), 6.81 - 6.79 (m, 1H), 6.45 - 6.11 (m, 1H), 5.63 (s, 1H), 4.88 - 4.53 (m, 4H), 4.24 (s, 1H), 2.63 (s, 3H), 2.07 (s, 1H)

### Example 3: Synthesis of N-(2-(4-acetylphenyl)-7,7-dimethyl-1,3-dioxo-2,3,5,12b-tetrahydroxy-1H,7H-chromeno[4,3-c][1,2,4]triazoto[1,2-a]pyridazin-10-yl)acetamide

Except that *N-*(2,2-dimethyl-3-vinyl-2*H*-chromen-7-yl)acetamide was used instead of ((2,2-bis(fluoromethyl)-3-vinyl-2*H*-chromen-7-yl)oxy)triisopropylsilane, the same synthesis method as in Example 2 (a) above was carried out to obtain *N-*(2-(4-acetylphenyl)-7,7-dimethyl-1,3-dioxo-2,3,5,12*b*-tetrahydroxy-1*H*,7*H*-chromeno[4,3-*c*][1,2,4]triazolo[1,2-*a*]pyridazin-10-yl)acetamide (28 mg, 36%).
¹H NMR (400 MHz, DMSO-D₆) δ 9.95 (s, 1H), 8.17 - 8.10 (m, J = 8.6 Hz, 2H), 7.85 - 7.78 (m, J = 8.6 Hz, 1H), 7.33 (s, 1H), 7.06 - 6.97 (m, 2H), 5.97 (br s, 1H), 5.66 (s, 1H), 4.20 (br s, 2H), 2.64 (s, 3H), 2.02 (s, 3H), 1.57 (s, 3H), 1.52 (s, 3H)

### Example 4: Synthesis of 2-(4-acetylphenyl)-7,7-dimethyl-10-(methylsulfonyl)-5,12b-dihydro-1H,7H-chromeno[4,3-c][1,2,4]triazolo[1,2-a]pyridazine-1,3(2H)-dione

Except that 2,2-dimethyl-7-(methylsulfonyl)-3-vinyl-2*H*-chromene was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-(methylsulfonyl)-5,12*b*-dihydro-1*H*,7*H*-chromeno[4,3-*c*][1,2,4]triazolo[1,2-*a*]pyridazine-1,3(2*H*)-dione (58 mg, 39%).
¹H NMR (400 MHz, DMSO-D₆) δ 8.17 - 8.12 (m, J = 8.4 Hz, 2H), 7.84 - 7.80 (m, J = 8.4 Hz, 2H), 7.54 (d, J = 7.81 Hz, 1H), 7.39 (s, 1H), 7.38 (d, J = 8.1 Hz, 1H), 6.07 (br s, 1H), 5.84 (s, 1H), 4.24 (br s, 2H), 3.21 (s, 3H), 2.67 - 2.63 (s, 3H), 1.65 (s, 3H), 1.55 (s, 3H)

### Example 5: Synthesis of N-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)methanesulfonamide

Except that *N*-(2,2-dimethyl-3-vinyl-2*H*-chromen-7-yl)methanesulfonamide was used instead of ((2,2-bis(fluoromethyl)-3-vinyl-2*H*-chromen-7-yl)oxy)triisopropylsilane, the same synthesis method as in Example 2 (a) above was carried out to obtain *N*-(2-(4-acetylphenyl)-7,7-dimethyl-1,3-dioxo-2,3,5,12*b*-tetrahydro-1*H*,7*H*-chromeno[4,3-*c*][1,2,4]triazolo[1,2-*a*]pyridazin-10-yl)methanesulfonamide (78 mg, 46%).
¹H NMR (400 MHz, DMSO-D₆) δ 9.88 - 9.71 (m, 1H), 8.14 (d, J = 8.6 Hz, 2H), 7.81 (d, J = 8.4 Hz, 2H), 7.06 (d, J = 8.3 Hz, 1H), 6.82 (br d, J = 8.3 Hz, 1H), 6.72 (d, J = 1.6 Hz, 1H), 6.01 (br s, 1H), 5.69 (s, 1H), 4.21 (br s, 2H), 2.98 (s, 3H), 2.65 (s, 3H), 1.56 (d, J = 15.8 Hz, 6h)

### Example 6: Synthesis of tert-butyl (2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)carbamate

Except that tert-butyl (2,2-dimethyl-3-vinyl-2*H*-chromen-7-yl)carbamate was used instead of ((2,2-bis(fluoromethyl)-3-vinyl-2*H*-chromen-7-yl)oxy)triisopropylsilane, the same synthesis method as in Example 2 (a) above was carried out to obtain tert-butyl (2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)carbamate (67 mg, 36%).
¹H NMR (400 MHz, DMSO-D₆) δ 9.36 (s, 1H), 8.16 - 8.11 (m, J = 8.6 Hz, 2H), 7.82 - 7.78 (m, J = 8.6 Hz, 2H), 7.10 (s, 1H), 7.01 - 6.94 (m, 2H), 5.97 (br s, 1H), 5.65 (s, 1H), 4.20 (br s, 2H), 2.64 (s, 3H), 1.56 (s, 3H), 1.52 (s, 3H), 1.46 (s, 9h)

### Example 7: Synthesis of 2-(4-acetylphenyl)-10-amino-7,7-dimethyl-5,12b-dihydro-1H,7H-chromeno[4,3-c][1,2,4]triazolo[1,2-a]pyridazine-1,3(2H)-dione

Tert-butyl (2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)carbamate (40 mg, 0.077 mmol) obtained in Example 6 above was dissolved in dichloromethane (1.5 mL), and trifluoroacetic acid (8.8 mg, 0.077 mmol) was added dropwise thereto and stirred at room temperature for 30 minutes. When the reaction was completed, the reaction mixture was concentrated by distillation under reduced pressure, and the obtained residue was separated by reverse phase chromatography (acetonitrile containing 0.1% formic acid : water containing 0.1% formic acid = 5 : 95-100 : 0) to obtain 2-(4-acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione (22 mg, 68%).
¹H NMR (400 MHz, DMSO-D₆) δ 8.16 - 8.11 (m, J = 8.6 Hz, 2H), 7.82 - 7.76 (m, J = 8.6 Hz, 2H), 6.72 (d, J = 8.3 Hz, 1H), 6.16 (dd, J = 1.8, 8.3 Hz, 1H), 6.08 - 6.04 (m, 1H), 5.92 (br s, 1H), 5.56 (s, 1H), 5.09 (br s, 2H), 4.24 - 4.12 (m, 2H), 2.64 (s, 3H), 1.51 (d, J = 7.8 Hz, 6h)

### Example 8: Synthesis of 2-(4-acetylphenyl)-7,7-dimethyl-10-morpholino-5,12b-dihydro-1H,7H-chromeno[4,3-c][1,2,4]triazolo[1,2-a]pyridazine-1,3(2H)-dione

Except that 4-(2,2-dimethyl-3-vinyl-2*H*-chromen-7-yl)morpholine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-morpholino-5,12b-dihydro-1*H*,7*H*-chromeno[4,3-*c*][1,2,4]triazolo[1,2-*a*]pyridazine-1,3(2*H*)-dione (80 mg, 46%).
¹H NMR (400 MHz, DMSO-D₆) δ 8.09 - 8.03 (m, J = 8.4 Hz, 2H), 7.74 - 7.69 (m, J = 8.4 Hz, 2H), 6.87 (d, J = 8.6 Hz, 1H), 6.48 (dd, J = 2.0, 8.6 Hz, 1H), 6.33 (d, J = 2.0 Hz, 1H), 5.91 (br s, 1H), 5.59 (s, 1H), 4.12 (br s, 2H), 3.62 (br t, J = 4.5 Hz, 4H), 3.02 - 2.93 (m, 4H), 2.57 (s, 3H), 1.47 (d, J = 6.8 Hz, 6h)

### Example 9: Synthesis of N-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)sulfuric diamide

2-(4-Acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione (18 mg, 0.043 mmol) obtained in Example 7 above was dissolved in *N*,*N*-dimethylacetamide (0.5 mL), and sulfamoyl chloride (9.9 mg, 0.086 mmol) was added dropwise thereto and stirred at room temperature for 18 hours. When the reaction was completed, the reaction mixture was concentrated by distillation under reduced pressure, and the obtained residue was separated by reverse-phase chromatography (acetonitrile containing 0.1% formic acid : water containing 0.1% formic acid = 5 : 95-100 : 0) to obtain N-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)sulfuric diamide (4 mg, 19%).
¹H NMR (400 MHz, DMSO-D₆) δ 9.44 (br s, 1H), 8.09 - 8.03 (m, J = 8.6 Hz, 2H), 7.76 - 7.70 (m, J = 8.6 Hz, 2H), 7.03 (s, 2H), 6.90 (d, J = 8.3 Hz, 1H), 6.67 (d, J = 8.7 Hz, 1H), 6.64 (d, J = 1.8 Hz, 1H), 5.92 (br s, 1H), 5.59 (s, 1H) 4.13 (br s, 2H), 2.57 (s, 3H), 1.50 (s, 3H), 1.46 (s, 3H)

### Example 10: Synthesis of 2-(4-acetylphenyl)-9,11-dibromo-10-hydroxy-7,7-dimethyl-5,12b-dihydro-1H,7H-chromeno[4,3-c][1,2,4]triazolo[1,2-a]pyridazine-1,3(2H)-dione

### (a) Synthesis of 2-(4-acetylphenyl)-7,7-dimethyl-10-((triisopropylsilyl)oxy)-5,12b-dihydro-1H,7H-chromeno[4,3-c][1,2,4]triazolo[1,2-a]pyridazine-1,3(2H)-dione

Except that ((3-bromo-2,2-dimethyl-2*H*-chromen7-yl)oxy)triisopropylsilane was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (120 mg, 42%).

### (b) Synthesis of 2-(4-acetylphenyl)-10-hydroxy-7,7-dimethyl-5,12b-dihydro-1H,7H-chromeno[4,3-c][1,2,4]triazolo[1,2-a]pyridazine-1,3(2H)-dione

Except that 2-(4-acetylphenyl)-7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione was used instead of 7,7-bis(fluoromethyl)-2-phenyl-10-((triisopropylsilyl)oxy)-5,12b-dihydro-1*H*,7*H*-chromeno[4,3-c][1,2,4]triazolo[1,2-*a*]pyridazine-1,3(2*H*)-dione, the same synthesis method as in Example 1 (b) above was carried out to obtain 2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (57 mg, 62%).

### (c) Synthesis of 2-(4-acetylphenyl)-9,11-dibromo-10-hydroxy-7,7-dimethyl-5,12b-dihydro-1H,7H-chromeno[4,3-c][1,2,4]triazolo[1,2-a]pyridazine-1,3(2H)-dione

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (30 mg, 0.072 mmol) obtained in Example 10 (b) above was suspended in acetonitrile (2 mL), and *N*-bromosuccinimide (15 mg, 0.086 mmol) was added thereto and stirred at room temperature for 2 hours. 1-Bromopyrrolidine-2,5-dione (3.82 mg, 0.021 mmol) was added thereto and stirred for additional 2 hours. When the reaction was completed, the reaction mixture was diluted in dichloromethane and washed with water. The separated organic layer was dried over sodium sulfate, filtered, concentrated by distillation under reduced pressure, and separated by reverse-phase chromatography (acetonitrile containing 0.1% formic acid : water containing 0.1% formic acid = 5 : 95-100 : 0) to obtain 2-(4-acetylphenyl)-9,11-dibromo-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (17 mg, 41%).
¹H NMR (400 MHz, DMSO-D₆) δ 9.97 (s, 1H), 8.15 - 8.13 (m, 2H), 7.78 - 7.76 (m, 2H), 7.23 (s, 1H), 6.02 - 6.01 (m, 1H), 5.66 (s, 1H), 4.26 - 4.15 (m, 2H), 2.63 (s, 3H), 1.61 (s, 3H), 1.52 (s, 3H)

### Example 11: Synthesis of 2-(4-acetylphenyl)-9,11-dichloro-10-hydroxy-7,7-dimethyl-5,12b-dihydro-1H,7H-chromeno[4,3-c][1,2,4]triazolo[1,2-a]pyridazine-1,3(2H)-dione

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (71 mg, 0.17 mmol) obtained in Example 10 (b) above was suspended in acetonitrile (2 mL), and *N*-chlorosuccinimide (27 mg, 0.203 mmol) was added thereto, and heated and stirred at 70°C for 3 hours. 1-Chloropyrrolidine-2,5-dione (18 mg, 0.136 mmol) was added thereto, and heated and stirred at 70°C for additional 4 hours. When the reaction was completed, the reaction mixture was diluted in dichloromethane and washed with water. The separated organic layer was dried over sodium sulfate, filtered, concentrated by distillation under reduced pressure, and separated by reverse-phase chromatography (acetonitrile containing 0.1% formic acid : water containing 0.1% formic acid = 5 : 95-100 : 0) to obtain 2-(4-acetylphenyl)-9,11-dichloro-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (9 mg, 11%).
¹H NMR (400 MHz, DMSO-D₆) δ 10.20 (s, 1H), 8.15 - 8.12 (m, 2H), 7.79 - 7.76 (m, 2H), 7.09 (s, 1H), 6.04 - 6.02 (m, 1H), 5.64 (s, 1H), 4.26 - 4.18 (m, 2H), 2.63 (s, 3H), 1.61 (s, 3H), 1.53 (s, 3H)

### Example 12: Synthesis of 2-(6-fluoropyridin-2-yl)-10-hydroxy-7,7-dimethyl-5,12b-dihydro-1H,7H-chromeno[4,3-c][1,2,4]triazolo[1,2-a]pyridazine-1,3(2H)-dione

### (a) Synthesis of 7,7-dimethyl-10-((triisopropylsilyl)oxy)-5,12b-dihydro-1H,7H-chromeno[4,3-c][1,2,4]triazolo[1,2-a]pyridazine-1,3(2H)-dione

Except that ((3-bromo-2,2-dimethyl-2*H*-chromen-7-yl)oxy)triisopropylsilane and 1,2,4-triazolidine-3,5-dione were used instead of ((2,2-bis(fluoromethyl)-3-vinyl-2*H*-chromen-7-yl)oxy)triisopropylsilane and 4-(4-acetylphenyl)-1,2,4-triazolidine-3,5-dione, respectively, the same synthesis method as in Example 2 (a) above was carried out to obtain 7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (50 mg, 48%).

### (b) Synthesis of 2-(6-fluoropyridin-2-yl)-10-hydroxy-7,7-dimethyl-5,12b-dihydro-1H,7H-chromeno[4,3-c][1,2,4]triazolo[1,2-a]pyridazine-1,3(2H)-dione

7,7-Dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (35 mg, 0.076 mmol) obtained in Example 12 (a) above was dissolved in *N*,*N-*dimethylformamide (3 mL), and 2, 6-difluoropyridine (14 mg, 0.12 mmol) and cesium carbonate (50 mg, 0.15 mmol) were added thereto, and heated and stirred at 80°C for 2 hours. When the reaction was completed, the reaction mixture was diluted in dichloromethane and washed with water and aqueous sodium chloride solution. The separated organic layer was dried over sodium sulfate, filtered, concentrated by distillation under reduced pressure, and separated by reverse-phase chromatography (acetonitrile containing 0.1% formic acid : water containing 0.1% formic acid = 5 : 95-80 : 20) to obtain 2-(6-fluoropyridin-2-yl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (7 mg, 23%).
¹H NMR (400 MHz, DMSO-D₆) δ 11.71 - 11.57 (m, 1H), 8.09 - 7.78 (m, 1H), 7.01 - 6.97 (m, 1H), 6.90 (ddd, J = 2.0, 8.1, 11.2 Hz, 2H), 6.77 (dd, J = 2.4, 8.4 Hz, 1H), 6.68 (d, J = 2.3 Hz, 1H), 6.08 - 5.86 (m, 1H), 5.59 (s, 1H), 4.16 - 4.05 (m, 1H), 4.05 - 3.94 (m, 1H), 1.55 (d, J = 19.6 Hz, 6h)

### Example 13: Synthesis of 2-(4-acetylphenyl)-7,7-dimethyl-10-nitro-5,12b-dihydro-1H,7H-chromeno[4,3-c][1,2,4]triazolo [1,2-a]pyridazine-1,3(2H)-dione

Except that 2,2-dimethyl-7-nitro-3-vinyl-2*H*-chromene was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-nitro-5,12b-dihydro-1*H*,7*H*-chromeno[4,3-*c*][1,2,4]triazolo [1,2-*a*]pyridazine-1,3(2*H*)-dione (54 mg, 68%).
¹H NMR (400 MHz, DMSO-D₆) δ 8.16 - 8.13 (m, 2H), 7.88 - 7.82 (m, 3H), 7.68 - 7.67 (m, 1H), 7.41 - 7.38 (m, 1H), 6.08 - 6.08 (m, 1H), 5.87 (m, 1H), 4.24 (s, 2H), 2.64 (s, 3H), 1.65 (s, 3H), 1.55 (s, 3H)

### Example 14: Synthesis of 2-(4-acetylphenyl)-10-(hydroxymethyl)-7,7-dimethyl-5,12b-dihydro-1H,7H-chromeno[4,3-c][1,2,4]triazolo[1,2-a]pyridazine-1,3(2H)-dione

### (a) Synthesis of 2-(4-acetylphenyl)-10-(((4-methoxyphenyl)diphenylmethoxy)methyl)-7,7-dimethyl-5,12b-dihydro-1H,7H-chromeno[4,3-c][1,2,4]triazolo[1,2-a]pyridazine-1,3(2H)-dione

Except that 7-(((4-methoxyphenyl)diphenylmethoxy)methyl)-2,2-dimethyl-3-vinyl-2*H-*chromene was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(((4-methoxyphenyl)diphenylmethoxy)methyl)-7,7-dimethyl-5,12b-dihydro-1*H*,7*H*-chromeno[4,3-*c*][1,2,4]triazolo[1,2-*a*]pyridazine-1,3(2*H*)-dione (142 mg, 62%).

### (b) Synthesis of 2-(4-acetylphenyl)-10-(hydroxymethyl)-7,7-dimethyl-5,12b-dihydro-1H,7H-chromeno[4,3-c][1,2,4]triazolo[1,2-a]pyridazine-1,3(2H)-dione

2-(4-Acetylphenyl)-10-(((4-methoxyphenyl)diphenylmethoxy)methyl)-7,7-dimethyl-5,12b-dihydro-1*H*,7*H*-chromeno[4,3-*c*][1,2,4]triazolo[1,2-*a*]pyridazine-1,3(2*H*)-dione (100 mg, 0.14 mmol) obtained in Example 14 (a) above was dissolved in dichloromethane (2 mL), and trifluoroacetic acid (16 mg, 0.14 mmol) was added dropwise thereto and stirred at room temperature for 20 minutes. When the reaction was completed, the reaction mixture was concentrated by distillation under reduced pressure, and the obtained residue was separated by reverse phase chromatography (acetonitrile containing 0.1% formic acid : water containing 0.1% formic acid = 5 : 95-100 : 0) to obtain 2-(4-acetylphenyl)-10-(hydroxymethyl)-7,7-dimethyl-5,12b-dihydro-1*H*,7*H*-chromeno[4,3-*c*][1,2,4]triazolo[1,2-*a*]pyridazine-1,3(2*H*)-dione (43 mg, 70%).
¹H NMR (400 MHz, DMSO-D₆) δ 8.07 (br d, J = 8.2 Hz, 2H), 7.73 (br d, J = 7.8 Hz, 2H), 6.94 (br d, J = 7.7 Hz, 1H), 6.84 (br d, J = 7.6 Hz, 1H), 6.76 (br s, 1H), 5.90 (br s, 1H), 5.61 (br s, 1H), 5.13 - 5.09 (m, 1H), 4.39 - 4.32 (m, 2H), 4.13 (br s, 2H), 2.59 - 2.55 (m, 3H), 1.51 (br s, 3H), 1.43 (br s, 3H)

### Example 15: Synthesis of 1-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)urea

2-(4-Acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione (18 mg, 0.043 mmol) obtained in Example 7 above was dissolved in a mixed solvent of acetic acid/water (2:1, 1 mL), and sodium cyanate (5.6 mg, 0.086 mmol) was added thereto and stirred at room temperature for 30 minutes. When the reaction was completed, the reaction mixture was concentrated by distillation under reduced pressure, and the obtained residue was separated by reverse-phase chromatography (acetonitrile containing 0.1% formic acid : water containing 0.1% formic acid = 5 : 95-100 : 0) to obtain 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)urea (11 mg, 55%).
¹H NMR (400 MHz, DMSO-D₆) δ 8.07 (br d, J = 8.2 Hz, 2H), 7.73 (br d, J = 7.8 Hz, 2H), 6.94 (br d, J = 7.7 Hz, 1H), 6.84 (br d, J = 7.6 Hz, 1H), 6.76 (br s, 1H), 5.90 (br s, 1H), 5.61 (br s, 1H), 5.13 - 5.09 (m, 1H), 4.39 - 4.32 (m, 2H), 4.13 (br s, 2H), 2.59 - 2.55 (m, 3H), 1.51 (br s, 3H), 1.43 (br s, 3H)

### Example 16: Synthesis of 2-(4-acetylphenyl)-10-(ethylamino)-7,7-dimethyl-5,12b-dihydro-1H,7H-chromeno[4,3-c][1,2,4]triazolo[1,2-a]pyridazine-1,3(2H)-dione

### (a) Synthesis of tert-butyl (2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)(ethyl)carbamate

Except that tert-butyl (2,2-dimethyl-3-vinylchromen-7-yl)(ethyl)carbamate was used instead of ((2,2-bis(fluoromethyl)-3-vinyl-2*H*-chromen-7-yl)oxy)triisopropylsilane, the same synthesis method as in Example 2 (a) above was carried out to obtain tert-butyl (2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)(ethyl)carbamate (54 mg, 52%).

### (b) Synthesis of 2-(4-acetylphenyl)-10-(ethylamino)-7,7-dimethyl-5,12b-dihydro-1H,7H-chromeno[4,3-c][1,2,4]triazolo[1,2-a]pyridazine-1,3(2H)-dione

Except that tert-butyl (2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)(ethyl)carbamate was used instead of tert-butyl (2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)carbamate, the same synthesis method as in Example 7 above was carried out to obtain 2-(4-acetylphenyl)-10-(ethylamino)-7,7-dimethyl-5,12b-dihydro-1*H*,7*H*-chromeno[4,3-*c*][1,2,4]triazolo[1,2-*a*]pyridazine-1,3(2*H*)-dione (26 mg, 72%).
¹H NMR(400 MHz, CDCl₃) δ 8.14 - 8.07 (m, J = 8.7 Hz, 2H), 7.88 - 7.80 (m, J = 8.6 Hz, 2H), 6.91 (d, J = 8.4 Hz, 1H), 6.19 (dd, J = 2.2 Hz, 8.4 Hz, 1H), 6.11 (d J = 2.2 Hz, 1H), 5.87 - 5.80 (m, 1H), 5.73 (s, 1H), 4.31 (dd, J = 4.8, 16.0 Hz, 1H), 4.09 (td, J = 2.3, 16.4 Hz, 1H), 3.60 (br,,s, 1H), 3.09 (q, J = 7.1 Hz, 2H), 2.65 (s, 3H), 1.60 (d, J = 9.5 Hz, 6H), 1.30 - 1.14 (m, 3H)

### Example 17: Synthesis of 2-(4-acetylphenyl)-7,7-dimethyl-10-((2,2,2-trifluoroethyl)amino)-5,12b-dihydro-1H,7H-chromeno[4,3-c][1,2,4]triazolo[1,2-a]pyridazine-1,3(2H)-dione

Except that 2,2-dimethyl-*N-*(2,2,2-trifluoroethyl)-3-vinyl-2*H*-chromen-7-amine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-((2,2,2-trifluoroethyl)amino)-5,12b-dihydro-1*H*,7*H*-chromeno[4,3-*c*][1,2,4]triazolo[1,2-*a*]pyridazine-1,3(2*H*)-dione (58 mg, 60%).
¹H NMR(400 MHz, CDCl₃) δ 8.14 - 8.08 (m, J = 8.7 Hz, 2H), 7.86 - 7.81 (m, J = 8.1 Hz, 2H), 6.91 (d, J = 8.4 Hz, 1H), 6.19 (br d, J = 8.6 Hz, 1H), 6.21 (s,1H), 5.86 (br s,1H), 5.73 (br s,1H), 4.36 - 4.27 (m, 1H), 4.11 (br d, J = 17.4 Hz, 1H), 4.01 - 3.92 (m, 1H), 3.77 - 3.66 (m, 2H), 2.65 (s, 3H), 1.60 (br d, J = 9.7 Hz, 6H)

### Example 18: Synthesis of 2-(4-acetylphenyl)-7,7-dimethyl-10-(piperazin-1-yl)-5,12b-dihydro-1H,7H-chromeno[4,3-c][1,2,4]triazolo[1,2-a]pyridazine-1,3(2H)-dione hydrochloride

### (a) Synthesis of tert-butyl 4-(2-(4-acetylphenyl)-7,7-dimethyl-1,3-dioxo-2,3,5,12b-tetrahydro-1H,7H-chromeno[4,3-c][1,2,4]triazolo[1,2-a]pyridazine-10-yl)piperazine-1-carboxylate

Except that tert-butyl 4-(2,2-dimethyl-3-vinyl-2*H*-chromen-7-yl)piperazine-1-carboxylate was used instead of ((2,2-bis(fluoromethyl)-3-vinyl-2*H*-chromen-7-yl)oxy)triisopropylsilane, the same synthesis method as in Example 2 (a) above was carried out to obtain tert-butyl 4-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazine-10-yl)piperazine-1-carboxylate (100 mg, 50%).

### (b) Synthesis of 2-(4-acetylphenyl)-7,7-dimethyl-10-(piperazin-1-yl)-5,12b-dihydro-1H,7H-chromeno[4,3-c][1,2,4]triazolo[1,2-a]pyridazine-1,3(2H)-dione hydrochloride

Tert-butyl 4-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazine-10-yl)piperazine-1-carboxylate (55 mg, 0.09 mmol) obtained in Example 18 (a) above was dissolved in dichloromethane (1 mL), and 4M hydrochloric acid solution (94 µL, 0.374 mmol, 4M HCl/dioxane) was added dropwise thereto and stirred at room temperature for 1 hour. When the reaction was completed, the reaction mixture was concentrated by distillation under reduced pressure, triturated with ethyl acetate, and the obtained solid was filtered to obtain 2-(4-acetylphenyl)-7,7-dimethyl-10-(piperazin-1-yl)-5,12b-dihydro-1*H*,7*H*-chromeno[4,3-*c*][1,2,4]triazolo[1,2-*a*]pyridazine-1,3(2*H*)-dione hydrochloride (31 mg, 63%).
¹H NMR (400 MHz, DMSO-D₆) δ 8.07 (br s, 2H), 8.15 - 8.13 (m, 2H), 7.78 - 7.77 (m, 2H), 6.98-6.96 (m, 1H), 6.61 - 6.60 (m, 1H), 6.58 (s, 1H), 6.49 ( s, 1H), 5.67 (s, 1H), 4.20 (s, 1H), 3.30 (s, 4H) 3.18 (s, 4H), 2.59 (s, 3H), 1.56 (br s, 3H), 1.53 (br s, 3H)

### Example 19: Synthesis of 2-(4-acetylphenyl)-10-(4-acetylpiperazin-1-yl)-7,7-dimethyl-5,12b-dihydro-1H,7H-chromeno[4,3-c][1,2,4]triazolo[1,2-a]pyridazine-1,3(2H)-dione

2-(4-Acetylphenyl)-7,7-dimethyl-10-(piperazin-1-yl)-5,12b-dihydro-1*H*,7*H-*chromeno[4,3-*c*][1,2,4]triazolo[1,2-*a*]pyridazine-1,3(2*H*)-dione hydrochloride (20 mg, 0.038 mmol) obtained in Example 18 (b) above was dissolved in dimethyl sulfoxide (0.5 mL), acetic anhydride (4.32 µL, 0.046 mmol) and triethylamine (5.32 µL, 0.038 mmol) were added thereto and stirred at room temperature for 1 hour. When the reaction was completed, the reaction mixture was concentrated by distillation under reduced pressure, and the obtained residue was separated by reverse phase chromatography (acetonitrile containing 0.1% formic acid : water containing 0.1% formic acid = 5 : 95-100 : 0) to obtain 2-(4-acetylphenyl)-10-(4-acetylpiperazin-1-yl)-7,7-dimethyl-5,12b-dihydro-1*H*,7*H*-chromeno[4,3-*c*][1,2,4]triazolo[1,2-*a*]pyridazine-1,3(2*H*)-dione (18 mg, 89%).
¹H NMR (400 MHz, DMSO-D₆) δ 8.14 - 8.12 (m, 2H), 7.79 - 7.77 (m, 2H), 6.98 - 6.96 (m, 1H), 6.61 - 6.60 (m, 1H), 6.58 (s, 1H), 6.49 ( s, 1H), 5.67 (s, 1H), 4.20 (s, 1H), 3.04 (s, 4H) 3.03 (s, 4H), 2.66 (s, 1H), 2.01 (s, 3H), 1.54 (br s, 3H), 1.53 (br s, 3H)

### Example 20: Synthesis of 2-(5-acetylpyridin-2-yl)-10-hydroxy-7,7-dimethyl-5,12b-dihydro-1H,7H-chromeno[4,3-c][1,2,4]triazolo[1,2-a]pyridazine-1,3(2H)-dione

Except that 1-(6-fluoropyridin-3-yl)ethan-1-one was used instead of 2,6-difluoropyridine, the same synthesis method as in Example 12 (b) above was carried out to obtain 2-(5-acetylpyridin-2-yl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (46 mg, 34%).
¹H NMR (400 MHz, DMSO-D₆) δ 11.65 (br s, 1H), 8.75 (d, J = 2.3 Hz, 1H), 8.30 dd, J = 2.4, 8.7 Hz, 1H), 7.12 (d, J = 8.6 Hz), 6.99 (d, J= 8.3 Hz, 1H), 6.77 (dd, J = 2.3, 8.4 Hz, 1H), 6.69 (d, J = 2.3 Hz 1H), 6.05 - 5.91 (m, 1H), 4.14 - 4.06 (m, 1H), 2.55 (s, 1H), 2.56 - 2.54 (m, 1H), 2.56-2.54 (m, 1H), 2.61 - 2.53 (m, 1H), 2.61 - 2.53 (m, 1H), 1.57 (s, 3H)

### Example 21: Synthesis of 10-acetyl-2-(4-acetylphenyl)-7,7-dimethyl-5,12b-dihydro-1H,7H-chromeno[4,3-c][1,2,4]triazolo[1,2-a]pyridazine-1,3(2H)-dione

Except that 1-(2,2-dimethyl-3-vinyl-2*H*-chromen-7-yl)ethan-1-one was used instead of ((2,2-bis(fluoromethyl)-3-vinyl-2*H*-chromen-7-yl)oxy)triisopropylsilane, the same synthesis method as in Example 2 (a) above was carried out to obtain 10-acetyl-2-(4-acetylphenyl)-7,7-dimethyl-5,12b-dihydro-1*H*,7*H*-chromeno[4,3-*c*][1,2,4]triazolo[1,2-*a*]pyridazine-1,3(2*H*)-dione (37 mg, 46%).
¹H NMR (400 MHz, CDCl₃) δ = 8.12 (d, J = 8.8 Hz, 2H), 7.85 (d, J = 8.7 Hz, 2H), 7.57 (dd, J = 1.5, 8.1 Hz, 1H), 7.49 (d, J = 1.5 Hz, 1H), 7.14 (d, J = 7.9 Hz, 1H), 5.88 - 5.84 (m, 1H), 5.72 (s, 1H), 4.31 (dd, J = 3.7, 16.7 Hz, 1H),. 4.17 (td, J = 2.6, 16.6 Hz, 1H), 2.66 (s, 3H), 2.56 (s, 3H), 1.65 (s, 3H), 1.60 - 1.58 (m, 3H)

### Example 22: Synthesis of 11-acetyl-2-(4-acetylphenyl)-10-hydroxy-7,7-dimethyl-5,12b-dihydro-1H,7H-chromeno[4,3-c][1,2,4]triazolo[1,2-a]pyridazine-1,3(2H)-dione

### (a) Synthesis of 11-acetyl-2-(4-acetylphenyl)-7,7-dimethyl-10-((triisopropylsilyl)oxy)-5,12b-dihydro-1H,7H-chromeno[4,3-c][1,2,4]triazolo[1,2-a]pyridazine-1,3(2H)-dione

Except that 1-(3-bromo-2,2-dimethyl-7-((triisopropylsilyl)oxy)-2*H*-chromen-6-yl)ethan-1-one was used instead of ((2,2-bis(fluoromethyl)-3-vinyl-2*H*-chromen-7-yl)oxy)triisopropylsilane, the same synthesis method as in Example 2 (a) above was carried out to obtain 11-acetyl-2-(4-acetylphenyl)-7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (45 mg, 50%).

### (b) Synthesis of 11-acetyl-2-(4-acetylphenyl)-10-hydroxy-7,7-dimethyl-5,12b-dihydro-1H,7H-chromeno[4,3-c][1,2,4]triazolo[1,2-a]pyridazine-1,3(2H)-dione

Except that 1-(3-bromo-2,2-dimethyl-7-((triisopropylsilyl)oxy)-2H-chromen-6-yl)ethan-1-one was used instead of 7,7-bis(fluoromethyl)-2-phenyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione, the same synthesis method as in Example 1 (b) above was carried out to obtain 11-acetyl-2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (20 mg, 57%).
¹H NMR (400 MHz, DMSO-D₆) δ 12.20 (s, 1H), 8.17 - 8.13 (m, J = 8.6 Hz, 2H), 7.81 - 7.76 (m, J = 8.6 Hz, 2H), 7.69 (s, 1H), 6.36 (s, 1H), 6.13 (br s, 1H), 5.80 (s, 1H), 4.25 (br s, 2H), 2.64 (s, 1H), 2.49 - 2.48 (m, 3H), 1.61 (d, J = 5.6 Hz, 6H)

### Example 23: Synthesis of 2-(6-acetylpyridin-3-yl)-10-hydroxy-7,7-dimethyl-5,12b-dihydro-1H,7H-chromeno[4,3-c][1,2,4]triazolo[1,2-a]pyridazine-1,3(2H)-dione

Except that 1-(5-fluoropyridin-2-yl)ethan-1-one was used instead of 2,6-difluoropyridine, the same synthesis method as in Example 12 (b) above was carried out to obtain 2-(6-acetylpyridin-3-yl)-10-hydroxy-7,7-dimethyl-5,12b-dihydro-1*H*,7*H*-chromeno[4,3-*c*][1,2,4]triazolo[1,2-a]pyridazine-1,3(2*H*)-dione (32 mg, 38%).
¹H NMR (400 MHz, DMSO-D₆) δ = 8.48 - 8.48 (m, 1H), 7.97 - 7.95 (m, 1H), 7.46 - 7.44 (m, 1H), 7.05 - 7.03 (m, 1H), 6.76 -6.73 (m, 1H), 6.64 (s, 1H), 5.95 - 5.94 (m, 1H), 5.49 - 5.49 (m, 1H), 4.04 - 4.00 (m, 1H), 3.85 - 3.81 (m, 1H), 2.60 (s, 3H), 1.55 - 1.51 (m, 6H)

### Example 24: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazine-10-carbonitrile

Except that 2,2-dimethyl-3-vinyl-2*H*-chromene-7-carbonitrile was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazine-10-carbonitrile (34 mg, 46%).
¹H NMR (400 MHz, DMSO-D₆) δ 8.14 - 8.13 (m, 2H), 7.81 - 7.79 (m, 2H), 7.46 - 7.40 (m, 2H), 7.31 - 7.29 (m, 1H), 6.06 - 6.05 (m, 1H), 5.81 (s, 1H), 4.22 (s, 1H), 2.64 (s, 3H), 1.63 (s, 3H), 1.52 (s, 3H)

### Example 25: Synthesis of 2-(4-acetylphenyl)-10-hydroxy-7,7-dimethyl-11-(trifluoromethoxy)-5,12b-dihydro-1H,7H-chromeno[4,3-c][1,2,4]triazolo[1,2-a]pyridazine-1,3(2H)-dione

### (a) Synthesis of 2-(4-acetylphenyl)-7,7-dimethyl-11-(trifluoromethoxy)-10-((triisopropylsilyl)oxy)-5,12b-dihydro-1H,7H-chromeno[4,3-c][1,2,4]triazolo[1,2-a]pyridazine-1,3(2H)-dione

Except that ((2,2-dimethyl-6-(trifluoromethoxy)-3-vinyl-2H-chromen-7-yl)oxy)triisopropylsilane was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-11-(trifluoromethoxy)-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (50 mg, 42%).

### (b) Synthesis of 2-(4-acetylphenyl)-10-hydroxy-7,7-dimethyl-11-(trifluoromethoxy)-5,12b-dihydro-1H,7H-chromeno[4,3-c][1,2,4]triazolo[1,2-a]pyridazine-1,3(2H)-dione

Except that 2-(4-acetylphenyl)-7,7-dimethyl-11-(trifluoromethoxy)-10-((triisopropylsilyl)oxy)-5,12b-dihydro-1*H*,7*H*-chromeno[4,3-*c*][1,2,4]triazolo[1,2-*a*]pyridazine-1,3(2*H*)-dione was used instead of 7,7-bis(fluoromethyl)-2-phenyl-10-((triisopropylsilyl)oxy)-5,12b-dihydro-1*H*,7*H*-chromeno[4,3-c][1,2,4]triazolo[1,2-*a*]pyridazine-1,3(2*H*)-dione, the same synthesis method as in Example 1 (b) above was carried out to obtain 2-(4-acetylphenyl)-10-hydroxy-7,7-dimethyl-11-(trifluoromethoxy)-5,12b-dihydro-1*H*,7*H*-chromeno[4,3-*c*][1,2,4]triazolo[1,2-*a*]pyridazine-1,3(2*H*)-dione (20 mg, 60%).
¹H NMR (400 MHz, DMSO-D₆) δ = 10.30 (s, 1H), 8.17 - 8.11 (m, J = 8.6 Hz, 2H), 7.76 - 7.70 (m, J = 8.4 Hz, 2H), 7.02 (s, 1H), 6.47 (s, 1H), 6.07 (m, 1H), 5.69 (s, 1H), 4.21 (br s, 2H), 2.63 (s, 3H), 1.54 (d, J = 3.1 Hz, 6H)

### Example 26: Synthesis of 2-(4-acetylphenyl)-11-cyclopropyl-10-hydroxy-7,7-dimethyl-5,12b-dihydro-1H,7H-chromeno[4,3-c][1,2,4]triazolo[1,2-a]pyridazine-1,3(2H)-dione

### (a) Synthesis of 2-(4-acetylphenyl)-11-cyclopropyl-7,7-dimethyl-10-((triisopropylsilyl)oxy)-5,12b-dihydro-1H,7H-chromeno[4,3-c][1,2,4]triazolo[1,2-a]pyridazine-1,3(2H)-dione

Except that ((6-cyclopropyl-2,2-dimethyl-3-vinyl-2*H*-chromen-7-yl)oxy)triisopropylsilane was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-11-cyclopropyl-7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (54 mg, 50%).

### (b) Synthesis of 2-(4-acetylphenyl)-11-cyclopropyl-10-hydroxy-7,7-dimethyl-5,12b-dihydro-1H,7H-chromeno[4,3-c][1,2,4]triazolo[1,2-a]pyridazine-1,3(2H)-dione

Except that 2-(4-acetylphenyl)-11-cyclopropyl-7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione was used instead of 7,7-bis(fluoromethyl)-2-phenyl-10-((triisopropylsilyl)oxy)-5,12b-dihydro-1*H*,7*H-*chromeno[4,3-c][1,2,4]triazolo[1,2-*a*]pyridazine-1,3(2*H*)-dione, the same synthesis method as in Example 1 (b) above was carried out to obtain 2-(4-acetylphenyl)-11-cyclopropyl-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (18 mg, 48%).
¹H NMR (400 MHz, DMSO-D₆) δ 9.37 (s, 1H), 8.12 - 8.06 (m, J = 8.6 Hz, 2H), 7.70 - 7.65 (m, J = 8.6 Hz, 2H), 6.38 (s, 1H), 6.22 (s, 1H), 5.88 (br s, 1H), 5.51 (s, 1H), 4.17 - 4.06 (m, 2H), 2.57 (s, 3H), 1.87 - 1.79 (m, 1H), 1.44 (d, J = 2.2, 8.3 Hz, 2H), 0.28 (br t, J = 5.9 Hz, 2H)

### Example 27: Synthesis of 2-(4-acetylphenyl)-9,11-difluoro-10-hydroxy-7,7-dimethyl-5,12b-dihydro-1H,7H-chromeno[4,3-c][1,2,4]triazolo[1,2-a]pyridazine-1,3(2H)-dione

### (a) Synthesis of 2-(4-acetylphenyl)-9,11-difluoro-7,7-dimethyl-10-((triisopropylsilyl)oxy)-5,12b-dihydro-1H,7H-chromeno[4,3-c][1,2,4]triazolo[1,2-a]pyridazine-1,3(2H)-dione

Except that ((6,8-difluoro-2,2-dimethyl-3-vinyl-2H-chromen-7-yl)oxy)triisopropylsilane was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-9,11-difluoro-7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione (54 mg, 52%).

### (b) Synthesis of 2-(4-acetylphenyl)-9,11-difluoro-10-hydroxy-7,7-dimethyl-5,12b-dihydro-1H,7H-chromeno[4,3-c][1,2,4]triazolo[1,2-a]pyridazine-1,3(2H)-dione

Except that 2-(4-acetylphenyl)-9,11-difluoro-7,7-dimethyl-10-((triisopropylsilyl)oxy)-5,12b-dihydro-1*H*,7*H*-chromeno[4,3-*c*][1,2,4]triazolo[1,2-*a*]pyridazine-1,3(2*H*)-dione was used instead of 7,7-bis(fluoromethyl)-2-phenyl-10-((triisopropylsilyl)oxy)-5,12b-dihydro-1*H*,7*H-*chromeno[4,3-*c*][1,2,4]triazolo[1,2-*a*]pyridazine-1,3(2*H*)-dione, the same synthesis method as in Example 1 (b) above was carried out to obtain 2-(4-acetylphenyl)-9,11-difluoro-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (15 mg, 58%).
¹H NMR (400 MHz, DMSO-D₆) δ 10.31 (s, 1H), 8.13 (d, J = 8.6 Hz, 2H), 7.79 (d, J = 8.6 Hz, 2H), 6.83 - 6.80 (m, 2H), 6.02 (s, 1H), 5.67 (s, 1H), 4.25 - 4.15 (m, 2H), 2.63 (s, 3H), 1.59 (s, 3H), 1.53 (s, 3H)

### Example 28: Synthesis of 2-(4-acetylphenyl)-10-amino-9,11-dibromo-7,7-dimethyl-5,12b-dihydro-1H,7H-chromeno[4,3-c][1,2,4]triazolo[1,2-a]pyridazine-1,3(2H)-dione

Except that 2-(4-acetylamine)-7,7-dimethyl-10-morpholino-5,12b-dihydro-1*H*,7*H-*chromeno[4,3-*c*][1,2,4]triazolo[1,2-*a*]pyridazine-1,3(2*H*)-dione was used instead of 2-(4-acetylphenyl)-10-hydroxy-7,7-dimethyl-5,12b-dihydro-1*H*,7*H*-chromeno[4,3-*c*][1,2,4]triazolo[ 1,2-*a*]pyridazine-1,3(2*H*)-dione, the same synthesis method as in Example 10 (c) above was carried out to obtain 2-(4-acetylphenyl)-10-amino-9,11-dibromo-7,7-dimethyl-5,12b-dihydro-1*H*,7*H*-chromeno[4,3-*c*][1,2,4]triazolo[1,2-*a*]pyridazine-1,3(2*H*)-dione (20 mg, 32%).
¹H NMR (400 MHz, DMSO-D₆) δ 8.17-8.11 (m, 2H), 7.80 - 7.75 (m, 2H), 7.15 (d, J = 1.1 Hz, 1H), 6.01 (br s, 1H), 5.64 (s, 1H), 5.39 (s, 2H), 4.26 - 4.12 (m, 2H), 2.64 (s, 3H), 1.60 (s, 3H), 1.53 (s, 3H)

### Example 29: Synthesis of 2-(4-acetylphenyl)-10-(isopropylamino)-7,7-dimethyl-5,12b-dihydro-1H,7H-chromeno[4,3-c][1,2,4]triazolo[1,2-a]pyridazine-1,3(2H)-dione

2-(4-Acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione (713 mg, 1.7 mmol) obtained in Example 7 above was dissolved in 1,2-dichloroethane (7 mL), and 2-methoxyprop-1-ene (253 mg, 3.41 mmol), sodium triacetoxyborohydride (533 mg, 3.41 mmol) and acetic acid (368 mg, 5.94 mmol) were added thereto and stirred at room temperature for 2 hours. When the reaction was completed, the reaction mixture was diluted in dichloromethane and washed with aqueous sodium bicarbonate solution. The separated organic layer was dried over sodium sulfate, filtered, concentrated by distillation under reduced pressure, and separated by reverse-phase chromatography (acetonitrile containing 0.1% formic acid : water containing 0.1% formic acid = 5 : 95-100 : 0) to obtain 2-(4-acetylphenyl)-10-(isopropylamino)-7,7-dimethyl-5,12b-dihydro-1*H*,7*H*-chromeno[4,3-*c*][1,2,4]triazolo[1,2-*a*]pyridazine-1,3(2*H*)-dione (526 mg, 66%).
¹H NMR (400 MHz, DMSO-D₆) δ 8.16 - 8.11 (m, 2H), 7.81 - 7.77 (m, J = 8.7 Hz, 2H), 6.77 (d, J = 8.4 Hz, 1H), 6.16 (dd, J = 2.2, 8.4 Hz, 1H), 6.01 (d, J = 2.1 Hz, 1H), 5.94 (br d, J = 1.8 Hz, 1H), 5.58 (s, 1H), 5.43 (d, J = 8.1 Hz, 1H), 4.25 - 4.13 (m, 2H), 3.50 - 3.36 (m, 1H), 2.64 (s, 3H), 1.52 (d, J = 3.3 Hz, 5H), 1.48 - 1.47 (m, 1H), 1.08 (d, J = 6.4 Hz, 6H)

### Example 30: Synthesis of 2-(4-acetylphenyl)-10-(tert-butylamino)-7,7-dimethyl-5,12b-dihydro-1H,7H-chromeno[4,3-c][1,2,4]triazolo[1,2-a]pyridazine-1,3(2H)-dione

2-(4-Acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione (100 mg, 0.24 mmol) obtained in Example 7 above was dissolved in nitromethane (1 mL), and copper(II) trifluoromethanesulfonate (4.3 mg, 0.012 mmol) and tert-butyl trichloroacetimidate (107 µL, 0.6 mmol) were added thereto and stirred at room temperature for 12 hours. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (ethyl acetate : hexane = 1 : 1, v/v) to obtain 2-(4-acetylphenyl)-10-(tert-butylamino)-7,7-dimethyl-5,12b-dihydro-1*H*,7*H*-chromeno[4,3-*c*][1,2,4]triazolo[1,2-*a*]pyridazine-1,3(2*H*)-dione (5 mg, 4.4%).
¹H NMR (400 MHz, DMSO-D₆) δ 8.09 - 8.03 (m, 2H), 7.75 - 7.69 (m, 2H), 6.72 - 6.65 (m, 1H), 6.28 - 6.21 (m, 1H), 6.12 - 6.07 (m, 1H), 5.90 - 5.83 (m, 1H), 5.54 - 5.48 (m, 1H), 5.18 - 5.12 (m, 1H), 4.19 - 4.03 (m, 2H), 2.58 - 2.56 (m, 3H), 1.47 - 1.43 (m, 6H), 1.19 - 1.17 (m, 9H)

### Example 31: Synthesis of 2-(4-acetylphenyl)-10-amino-9,11-dichloro-7,7-dimethyl-5,12b-dihydro-1H,7H-chromeno[4,3-c][1,2,4]triazolo[1,2-a]pyridazine-1,3(2H)-dione

Except that 2-(4-acetylamine)-7,7-dimethyl-10-morpholino-5,12b-dihydro-1*H*,7*H-*chromeno[4,3-*c*][1,2,4]triazolo[1,2-*a*]pyridazine-1,3(2*H*)-dione was used instead of 2-(4-acetylphenyl)-10-hydroxy-7,7-dimethyl-5,12b-dihydro-1*H*,7*H*-chromeno[4,3-*c*][1,2,4]triazolo[ 1,2-*a*]pyridazine-1,3(2*H*)-dione, the same synthesis method as in Example 11 above was carried out to obtain 2-(4-acetylphenyl)-10-amino-9,11-dichloro-7,7-dimethyl-5,12b-dihydro-1*H*,7*H-*chromeno[4,3-*c*][1,2,4]triazolo[1,2-*a*]pyridazine-1,3(2*H*)-dione (53 mg, 38%).
¹H NMR (400 MHz, CDCl₃) δ = 8.16 - 8.09 (m, J = 8.4 Hz, 2H), 7.86 - 7.82 (m, J = 8.6 Hz, 2H), 6.94 (s, 1H), 5.85 (br s, 1H), 5.64 (br s, 1H), 4.50 (s, 2H), 4.29 (dd, J = 4.4,16.6 Hz, 1H), 4.16 (br d, J = 16.5 Hz, 1H), 2.66 (s, 3H), 2.03 - 2.00 (m, 1H), 1.64 (d, J = 6.5Hz, 6H)

### Example 32: Synthesis of 2-(4-acetylphenyl)-7,7-dimethyl-10-(methylamino)-5,12b-dihydro-1H,7H-chromeno[4,3-c][1,2,4]triazolo[1,2-a]pyridazine-1,3(2H)-dione

### (a) Synthesis of tert-butyl (2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)(methyl)carbamate

Except that tert-butyl (2,2-dimethyl-3-vinyl-2*H*-chromen-7-yl)(methyl)carbamate was used instead of ((2,2-bis(fluoromethyl)-3-vinyl-2*H*-chromen-7-yl)oxy)triisopropylsilane, the same synthesis method as in Example 2 (a) above was carried out to obtain tert-butyl (2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)(methyl)carbamate (54 mg, 36%).

### (b) Synthesis of 2-(4-acetylphenyl)-7,7-dimethyl-10-(methylamino)-5,12b-dihydro-1H,7H-chromeno[4,3-c][1,2,4]triazolo[1,2-a]pyridazine-1,3(2H)-dione

Except that tert-butyl (2-(4-acetylphenyl)-7,7-dimethyl-1,3- dioxo-2,3,5,12b-tetrahydro-1*H*,7*H*-chromeno[4,3-*c*][1,2,4]triazolo[1,2-*a*]pyridazin-10-yl)(methyl)carbamate was used instead of tert-butyl (2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)carbamate, the same synthesis method as in Example 7 above was carried out to obtain 2-(4-acetylphenyl)-7,7-dimethyl-10-(methylamino)-5,12b-dihydro-1*H*,7*H*-chromeno[4,3-*c*][1,2,4]triazolo[1,2-*a*]pyridazine-1,3(2*H*)-dione (23 mg, 58%).
¹H NMR (400 MHz, CDCl₃) δ = 8.15 - 8.10 (m, J = 8.4 Hz, 2H), 7.89 - 7.83 (m, J = 8.4 Hz, 2H), 6.95 (d, J = 8.3 Hz, 1H), 6.22 (dd, J = 2.0, 8.4 Hz, 1H), 6.14 (s, 1H), 5.89 - 5.54 (m, 1H), 5.76 (br s, 1H), 4.33 (dd, J = 4.9,16.5 Hz, 1H), 4.12 (br d, J = 16.4 Hz, 1H), 3.76 (br s, 1H), 2.80 (S, 3H), 2.67 (s, 3H), 2.03 (s, 1H), 1.65 - 1.60 (m, 6H)

### Example 33: Synthesis of 2-(4-acetylphenyl)-10-(diethylamino)-7,7-dimethyl-5,12b-dihydro-1H,7H-chromeno[4,3-c][1,2,4]triazolo[1,2-a]pyridazine-1,3(2H)-dione

Except that *N*,*N-*diethyl-2,2-dimethyl-3-vinyl-2*H*-chromen-7-amine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(diethylamino)-7,7-dimethyl-5,12b-dihydro-1*H*,7*H*-chromeno[4,3-*c*][1,2,4]triazolo[1,2-*a*]pyridazine-1,3(2*H*)-dione (30 mg, 51%).
¹H NMR (400 MHz, DMSO-D₆) δ = 8.14 - 8.12 (m, 2H) 7.79 - 7.78 (m, 2H), 6.87 - 6.85 (m, 1H), 6.27 - 6.24 (m, 1H), 6.07 (s, 1H), 5.98 (s, 1H), 5.62 (s, 1H), 4.24 - 4.14 (m, 2H), 3.29 - 3.23 (m, 4H), 2.64 (s, 3H), 1.53 (s, 6H), 1.05 - 1.02 (m, 6H)

### Example 34: Synthesis of 2-(4-acetylphenyl)-7,7-dimethyl-10-(pyrrolidin-1-yl)-5,12b-dihydro-1H,7H-chromeno[4,3-c][1,2,4]triazolo[1,2-a]pyridazine-1,3(2H)-dione

Except that 1-(2,2-dimethyl-3-vinyl-2*H*-chromen-7-yl)pyrrolidine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-(pyrrolidin-1-yl)-5,12b-dihydro-1*H*,7*H*-chromeno[4,3-*c*][1,2,4]triazolo[1,2-*a*]pyridazine-1,3(2*H*)-dione (100 mg, 42%).
¹H NMR (400 MHz, DMSO-D₆) δ = 8.14 - 8.12 (m, 2H) 7.79 - 7.77 (m, 2H), 6.88 - 6.86 (m, 1H), 6.16 (m, 1H), 5.98 (s, 1H), 5.95 - 5.95 (m, 1H), 5.63 (s, 1H), 4.24 - 4.14 (m, 2H), 3.14 (s, 4H), 2.64 (s, 3H), 1.91 (s, 4H), 1.53 (s, 6H)

### Example 35: Synthesis of 2-(4-fluorophenyl)-7,7-dimethyl-10-(oxetan-3-ylamino)-5,12b-dihydro-1H,7H-chromeno[4,3-c][1,2,4]triazolo[1,2-a]pyridazine-1,3(2H)-dione

### (a) Synthesis of tert-butyl (2-(4-fluorophenyl)-7,7-dimethyl-1,3-dioxo-2,3,5,12b-tetrahydro-1H,7H-chromeno[4,3-c][1,2,4]triazolo[1,2-a]pyridazin-10-yl)carbamate

Except that 4-(4-fluorophenyl)-1,2,4-triazolidine-3,5-dione was used instead of 4-(4-acetylphenyl)-1,2,4-triazolidine-3,5-dione, the same synthesis method as in Example 6 above was carried out to obtain tert-butyl (2-(4-fluorophenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)carbamate (82 mg, 43%).

### (b) Synthesis of 10-amino-2-(4-fluorophenyl)-7,7-dimethyl-5,12b-dihydro-1H,7H-chromeno[4,3-c][1,2,4]triazolo[1,2-a]pyridazine-1,3(2H)-dione

Except that tert-butyl (2-(4-fluorophenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)carbamate was used instead of tert-butyl (2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)carbamate, the same synthesis method as in Example 7 above was carried out to obtain 10-amino-2-(4-fluorophenyl)-7,7-dimethyl-5,12b-dihydro-1*H*,7*H-*chromeno[4,3-*c*][1,2,4]triazolo[1,2-*a*]pyridazine-1,3(2*H*)-dione (56 mg, 78%).

### (c) Synthesis of 2-(4-fluorophenyl)-7,7-dimethyl-10-(oxetan-3-ylamino)-5,12b-dihydro-1H,7H-chromeno[4,3-c][1,2,4]triazolo[1,2-a]pyridazine-1,3(2H)-dione

10-Amino-2-(4-fluorophenyl)-7,7-dimethyl-5,12b-dihydro-1*H*,7*H*-chromeno[4,3-*c*][1,2,4]triazolo[1,2-*a*]pyridazine-1,3(2*H*)-dione (17 mg, 0.043 mmol) obtained in Example 35 (b) above was dissolved in dichloromethane (1 mL) and cooled to 0°C, and 3-oxetanone (4.15 µL, 0.065 mmol), acetic acid (2.468 µL, 0.043 mmol), sodium triacetoxyborohydride (73 mg, 0.35 mmol) and titanium(IV) propane-2-oleate (12.8 µL, 0.043 mmol) were added thereto and stirred at room temperature for 15 hours. When the reaction was completed, the reaction mixture was diluted in dichloromethane and washed with aqueous sodium bicarbonate solution. The separated organic layer was dried over sodium sulfate, filtered, concentrated by distillation under reduced pressure, and separated by MPLC (ethyl acetate : hexane = 1 : 1, v/v) to obtain 2-(4-fluorophenyl)-7,7-dimethyl-10-(oxetan-3-ylamino)-5,12b-dihydro-1*H*,7*H*-chromeno[4,3-*c*][1,2,4]triazolo[1,2-*a*]pyridazine-1,3(2*H*)-dione (8 mg, 41%).
¹H NMR (400 MHz, CDCl₃) δ = 7.63 (br dd, J = 4.6, 8.8 Hz, 2H), 7.23 (br t, J = 8.5 Hz, 2H), 6.96 (d, J = 8.3 Hz, 1H), 6.17 - 6.11 (m, 1H), 5.98 (s, 1H), 5.90 - 5.84 (m, 1H), 5.73 (br s, 1H), 4.99 (br t, J = 6.4 Hz, 2H), 4.58 (br d, J = 5.6 Hz, 1H), 4.55 - 4.48 (m, 2H), 4.32 (dd, J = 4.9, 16.4 Hz, 1H), 4.10 (br d, J= 16.3 Hz, 1H), 1.60 (s, 6H)

### Example 36: Synthesis of 2-(4-acetylphenyl)-9,11-dichloro-7,7-dimethyl-10-(methylamino)-5,12b-dihydro-1H,7H-chromeno[4,3-c][1,2,4]triazolo[1,2-a]pyridazine-1,3(2H)-dione

Except that 2-(4-acetylphenyl)-7,7-dimethyl-10-(methylamino)-5,12b-dihydro-1*H*,7*H-*chromeno[4,3-*c*][1,2,4]triazolo[1,2-*a*]pyridazine-1,3(2*H*)-dione was used instead of 2-(4-acetylphenyl)-10-hydroxy-7,7-dimethyl-5,12b-dihydro-1*H*,7*H*-chromeno[4,3-*c*][1,2,4]triazolo[ 1,2-a]pyridazine-1,3(2*H*)-dione, the same synthesis method as in Example 11 above was carried out to obtain 2-(4-acetylphenyl)-9,11-dichloro-7,7-dimethyl-10-(methylamino)-5,12b-dihydro-1*H*,7*H*-chromeno[4,3-*c*][1,2,4]triazolo[1,2-*a*]pyridazine-1,3(2*H*)-dione (32 mg, 28%).
¹H NMR (400 MHz, CDCl₃) δ 8.15 - 8.08 (m, 2H), 7.87 - 7.80 (m, 2H), 6.97 - 6.92 (m, 1H), 5.83 (t, J = 3.5 Hz, 1H), 5.61 (s, 1H), 4.34 - 4.24 (m, 1H), 4.17 (dt, J = 16.5, 2.8 Hz, 1H), 4.00 (p, J = 6.4 Hz, 1H), 2.65 (d, J = 0.6 Hz, 3H), 1.65 (s, 3H), 1.60 (s, 3H), 1.16 (dd, J = 6.4, 4.5 Hz, 6H)

### Example 37: Synthesis of 2-(4-acetylphenyl)-9,11-dichloro-10-(isopropylamino)-7,7-dimethyl-5,12b-dihydro-1H,7H-chromeno[4,3-c][1,2,4]triazolo[1,2-a]pyridazine-1,3(2H)-dione

Except that 2-(4-acetylphenyl)-10-(isopropylamino)-7,7-dimethyl-5,12b-dihydro-1*H*,7*H-*chromeno[4,3-*c*][1,2,4]triazolo[1,2-*a*]pyridazine-1,3(2*H*)-dione was used instead of 2-(4-acetylphenyl)-10-hydroxy-7,7-dimethyl-5,12b-dihydro-1*H*,7*H*-chromeno[4,3-*c*][1,2,4]triazolo[ 1,2-a]pyridazine-1,3(2*H*)-dione, the same synthesis method as in Example 11 above was carried out to obtain 2-(4-acetylphenyl)-9,11-dichloro-10-(isopropylamino)-7,7-dimethyl-5,12b-dihydro-1*H*,7*H*-chromeno[4,3-*c*][1,2,4]triazolo[1,2-*a*]pyridazine-1,3(2*H*)-dione (22 mg, 30%).
¹H NMR (400 MHz, CDCl₃) δ 8.16 - 8.08 (m, 2H), 7.87 - 7.79 (m, 2H), 6.95 (d, J = 1.2 Hz, 1H), 5.88 - 5.81 (m, 1H), 5.61 (d, J = 2.7 Hz, 1H), 4.28 (ddd, J = 16.6, 4.5, 1.4 Hz, 1H), 4.17 (dt, J = 16.5, 2.8 Hz, 1H), 3.01 (s, 3H), 2.65 (s, 3H), 1.63 (d, J = 15.3 Hz, 7H)

### Example 38: Synthesis of 2-(4-acetylphenyl)-10-(1,3-dimethyl-1H-pyrazol-5-yl)-7,7-dimethyl-5,12b-dihydro-1H,7H-chromeno[4,3-c][1,2,4]triazolo[1,2-a]pyridazine-1,3(2H)-dione

Except that 5-(2,2-dimethyl-3-vinyl-2*H*-chromen-7-yl)-1,3-dimethyl-1*H*-pyrazole was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(1,3-dimethyl-1*H*-pyrazol-5-yl)-7,7-dimethyl-5,12b-dihydro-1H,7H-chromeno[4,3-*c*][1,2,4]triazolo[1,2-*a*]pyridazine-1,3(2*H*)-dione (42 mg, 50%).
¹H NMR (400 MHz, DMSO-D₆) δ 8.18 - 8.10 (m, 2H), 7.85 - 7.77 (m, 2H), 7.18 (dd, J = 8.0, 1.0 Hz, 1H), 7.10 (dd, J = 7.9, 1.7 Hz, 1H), 6.99 (d, J = 1.7 Hz, 1H), 6.15 (s, 1H), 6.03 (td, J = 3.7, 1.8 Hz, 1H), 5.77 (d, J = 1.7 Hz, 1H), 4.23 (dd, J = 4.0, 2.0 Hz, 2H), 3.74 (s, 3H), 2.64 (s, 3H), 2.14 (s, 3H), 1.62 (s, 3H), 1.55 (s, 3H)

### Example 39: Synthesis of 2-(4-acetylphenyl)-10-(2,5-dimethylthiazol-4-yl)-7,7-dimethyl-5,12b-dihydro-1H,7H-chromeno[4,3-c][1,2,4]triazolo[1,2-a]pyridazine-1,3(2H)-dione

Except that 4-(2,2-dimethyl-3-vinyl-2*H*-chromen-7-yl)-2,5-dimethylthiazole was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(2,5-dimethylthiazol-4-yl)-7,7-dimethyl-5,12b-dihydro-1*H*,7*H*-chromeno[4,3-*c*][1,2,4]triazolo[1,2-*a*]pyridazine-1,3(2*H*)-dione (28 mg, 48%).
¹H NMR (400 MHz, DMSO-D₆) δ 8.18 - 8.10 (m, 2H), 7.85 - 7.78 (m, 2H), 7.26 (dd, J = 8.0, 1.7 Hz, 1H), 7.18 - 7.11 (m, 2H), 6.00 (td, J = 3.7, 1.8 Hz, 1H), 5.75 (d, J = 2.3 Hz, 1H), 4.23 (q, J = 2.0 Hz, 2H), 2.64 (s, 3H), 2.60 (s, 3H), 2.48 (s, 3H), 1.62 (s, 3H), 1.53 (s, 3H)

### Example 40: Synthesis of 2-(4-acetylphenyl)-10-(1-ethyl-3,5-dimethyl-1H-pyrazol-4-yl)-7,7-dimethyl-5,12b-dihydro-1H,7H-chromeno[4,3-c][1,2,4]triazolo[1,2-a]pyridazine-1,3(2H)-dione

Except that 4-(2,2-dimethyl-3-vinyl-2*H*-chromen-7-yl)-1-ethyl-3,5-dimethyl-1*H-*pyrazole was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(1-ethyl-3,5-dimethyl-1*H*-pyrazol-4-yl)-7,7-dimethyl-5,12b-dihydro-1*H*,7*H*-chromeno[4,3-*c*][1,2,4]triazolo[1,2-*a*]pyridazine-1,3(2*H*)-dione (38 mg, 51%).
¹H NMR (400 MHz, DMSO-D₆) δ 8.18 - 8.10 (m, 2H), 7.85 - 7.77 (m, 2H), 7.09 (dd, J = 7.9, 1.1 Hz, 1H), 6.87 (dd, J = 7.9, 1.7 Hz, 1H), 6.74 (d, J = 1.6 Hz, 1H), 6.00 (h, J = 1.8 Hz, 1H), 5.73 (s, 1H), 4.23 (q, J = 2.6 Hz, 2H), 4.00 (q, J = 7.2 Hz, 2H), 2.64 (s, 3H), 2.20 (s, 3H), 2.11 (s, 3H), 1.61 (s, 3H), 1.53 (s, 3H), 1.29 (t, J = 7.2 Hz, 3H)

### Example 41: Synthesis of 2-(4-acetylphenyl)-7,7-dimethyl-10-(3-methylisoxazol-4-yl)-5,12b-dihydro-1H,7H-chromeno[4,3-c][1,2,4]triazolo[1,2-a]pyridazine-1,3(2H)-dione

Except that 4-(2,2-dimethyl-3-vinyl-2*H*-chromen-7-yl)-3-methylisoxazole was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-(3-methylisoxazol-4-yl)-5,12b-dihydro-1*H*,7*H*-chromeno[4,3-*c*][1,2,4]triazolo[1,2-*a*]pyridazine-1,3(2*H*)-dione (33 mg, 54%).
¹H NMR (499 MHz, CDCl₃) δ 8.41 (s, 1H), 8.12 (d, J = 8.7 Hz, 2H), 7.88 - 7.84 (m, 2H), 7.15 - 7.11 (m, 1H), 6.98 (dd, J = 8.0, 1.7 Hz, 1H), 6.92 (d, J = 1.7 Hz, 1H), 5.91 - 5.86 (m, 1H), 5.77 (s,1H), 4.33 (dd, J = 16.5, 4.8 Hz, 1H), 4.16 (dt, J = 16.5, 2.6 Hz, 1H), 2.65 (s, 3H), 2.40 (s, 3H), 1.64 (d, J = 11.2 Hz, 6H)

### Example 42: Synthesis of 2-(4-acetylphenyl)-7,7-dimethyl-10-(pyridin-3-yl)-5,12b-dihydro-1H,7H-chromeno[4,3-c][1,2,4]triazolo[1,2-a]pyridazine-1,3(2H)-dione

Except that 3-(2,2-dimethyl-3-vinyl-2*H*-chromen-7-yl)pyridine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-(pyridin-3-yl)-5,12b-dihydro-1*H*,7*H*-chromeno[4,3-*c*][1,2,4]triazolo[1,2-*a*]pyridazine-1,3(2*H*)-dione(25mg, 60%).
¹H NMR (400 MHz, CDCl₃) δ 8.80 (dd, J = 2.2, 0.8 Hz, 1H), 8.59 (dd, J = 4.8, 1.6 Hz, 1H), 8.15 - 8.10 (m, 2H), 7.89 - 7.84 (m, 2H), 7.82 (dt, J = 8.0, 2.1 Hz, 1H), 7.37 - 7.32 (m, 1H), 7.19 (d, J= 1.3 Hz, 2H), 7.15 - 7.11 (m, 1H), 5.91 - 5.86 (m, 1H), 5.79 (s, 1H), 4.37 - 4.29 (m, 1H), 4.17 (dt, J = 16.4, 2.6 Hz, 1H), 2.65 (s, 3H), 1.65 (d, J = 10.6 Hz, 7H)

### Example 43: Synthesis of 2-(4-acetylphenyl)-7,7-dimethyl-10-(pyridin-4-yl)-5,12b-dihydro-1H,7H-chromeno[4,3-c][1,2,4]triazolo[1,2-a]pyridazine-1,3(2H)-dione

Except that 3-(2,2-dimethyl-3-vinyl-2*H*-chromen-7-yl)pyridine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-7,7-dimethyl-10-(pyridin-4-yl)-5,12b-dihydro-1*H*,7*H*-chromeno[4,3-*c*][1,2,4]triazolo[1,2-*a*]pyridazine-1,3(2*H*)-dione(42mg, 50%).
¹H NMR (400 MHz, ) δ 8.66 - 8.61 (m, 2H), 8.15 - 8.09 (m, 2H), 7.89 - 7.83 (m, 2H), 7.47 - 7.42 (m, 2H), 7.24 (dd, J = 8.1, 1.7 Hz, 1H), 7.22 - 7.16 (m, 2H), 5.89 (dt, J = 4.7, 2.3 Hz, 1H),5.78 (q, J = 1.6 Hz, 1H), 4.33 (ddd, J= 16.5, 4.8, 1.4 Hz, 1H), 4.17 (dt, J = 16.5, 2.7 Hz, 1H), 2.65 (s, 3H), 1.65 (d, J= 12.1 Hz, 6H)

### Example 44: Synthesis of 4-(7,7-dimethyl-1,3-dioxo-10-(pyrrolidin-1-yl)-5,12b-dihydro-1H,7H-chromeno[4,3-c][1,2,4]triazolo[1,2-a]pyridazin-2(3H)-yl)benzoic acid

Except that 1-(2,2-dimethyl-3-vinyl-2*H*-chromen-7-yl)pyrrolidine was used instead of ((2,2-bis(fluoromethyl)-3-vinyl-2*H*-chromen-7-yl)oxy)triisopropylsilane, the same synthesis method as in Example 2 (a) above was carried out to obtain 4-(7,7-dimethyl-1,3-dioxo-10-(pyrrolidin-1-yl)-5,12b-dihydro-1*H*,7*H*-chromeno[4,3-*c*][1,2,4]triazolo[1,2-*a*]pyridazin-2(3*H*)-yl)benzoic acid (34 mg, 45%).
¹H NMR (500 MHz, ) δ 8.24 (d, J = 8.4 Hz, 2H), 7.85 (d, J = 8.6 Hz, 2H), 6.99 (dd, J = 8.6, 1.0 Hz, 1H), 6.17 (dd, J = 8.6, 2.4 Hz, 1H), 6.06 (d, J = 2.3 Hz, 1H), 5.84 (dd, J = 4.9, 2.2 Hz, 1H), 5.77 (s, 1H), 4.35 - 4.27 (m, 1H), 4.10 (dt, J = 16.3, 2.5 Hz, 1H), 3.25 - 3.17 (m, 4H), 2.03 - 1.91 (m, 4H), 1.61 (d, J = 12.6 Hz, 6H)

### Example 45: Synthesis of 2-(4-acetylphenyl)-10-(isopropyl(methyl)amino)-7,7-dimethyl-5,12b-dihydro-1H,7H-chromeno[4,3-c][1,2,4]triazolo[1,2-a]pyridazine-1,3(2H)-dione

Except that *N*-isopropyl-*N*,2,2-trimethyl-3-vinyl-2*H*-chromen-7-amine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(isopropyl(methyl)amino)-7,7-dimethyl-5,12b-dihydro-1*H*,7*H*-chromeno[4,3-*c*][1,2,4]triazolo[1,2-*a*]pyridazine-1,3(2*H*)-dione (20 mg, 38%).
¹H NMR (400 MHz, CDCl₃) δ 8.14 - 8.06 (m, 2H), 7.87 - 7.82 (m, 2H), 6.98 (d, *J* = 8.7 Hz, 1H), 6.37 (dd, *J* = 8.8, 2.6 Hz, 1H), 6.26 (d, *J* = 2.5 Hz, 1H), 5.87 - 5.81 (m, 1H), 5.75 (s, 1H), 4.34 - 4.27 (m, 1H), 4.09 (d, *J* = 16.4 Hz, 1H), 4.02 (p, *J* = 6.6 Hz, 1H), 2.68 (s, 3H), 2.64 (s, 3H), 1.60 (d, *J* = 11.9 Hz, 6H), 1.12 (dd, *J* = 6.6, 1.2 Hz, 6H)

### Example 46: Synthesis of 2-(4-acetylphenyl)-10-(ethyl(isopropyl)amino)-7,7-dimethyl-5,12b-dihydro-1H,7H-chromeno[4,3-c][1,2,4]triazolo[1,2-a]pyridazine-1,3(2H)-dione

Except that 2-(4-acetylphenyl)-10-(ethylamino)-7,7-dimethyl-5,12b-dihydro-1*H*,7*H-*chromeno[4,3-*c*][1,2,4]triazolo[1,2-*a*]pyridazine-1,3(2*H*)-dione was used instead of 2-(4-acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione, the same synthesis method as in Example 29 above was carried out to obtain 2-(4-acetylphenyl)-10-(ethyl(isopropyl)amino)-7,7-dimethyl-5,12b-dihydro-1*H*,7*H-*chromeno[4,3-*c*][1,2,4]triazolo[1,2-*a*]pyridazine-1,3(2*H*)-dione (15 mg, 39%).
¹H NMR (400 MHz, DMSO-D₆) δ 8.17 - 8.09 (m, 2H), 7.82 - 7.74 (m, 2H), 6.87 (dd, J = 8.7, 1.0 Hz, 1H), 6.32 (dd, J = 8.8, 2.5 Hz, 1H), 6.13 (d, J = 2.5 Hz, 1H), 5.96 (q, J = 2.5 Hz, 1H), 5.62 (s, 1H), 4.25 - 4.13 (m, 2H), 3.95 (p, J = 6.7 Hz, 1H), 3.16 (q, J = 6.9 Hz, 2H), 2.64 (s, 3H), 1.53 (s, 6H), 1.09 (d, J = 6.6 Hz, 6H), 1.05 (t, J = 6.9 Hz, 3H)

### Example 47: Synthesis of 2-(4-acetylphenyl)-10-(diisopropylamino)-7,7-dimethyl-5,12b-dihydro-1H,7H-chromeno[4,3-c][1,2,4]triazolo[1,2-a]pyridazine-1,3(2H)-dione

Except that *N*,*N*-diisopropyl-2,2-dimethyl-3-vinyl-2*H*-chromen-7-amine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(diisopropylamino)-7,7-dimethyl-5,12b-dihydro-1*H*,7*H*-chromeno[4,3-*c*][1,2,4]triazolo[1,2-*a*]pyridazine-1,3(2*H*)-dione (30 mg, 40%).
¹H NMR (400 MHz, DMSO-D₆) δ 8.17 - 8.09 (m, 2H), 7.83 - 7.75 (m, 2H), 6.85 (dd, J = 8.7, 1.0 Hz, 1H), 6.43 (dd, J = 8.7, 2.5 Hz, 1H), 6.21 (d, J = 2.4 Hz, 1H), 5.96 (dt, J = 4.7, 2.3 Hz, 1H), 5.62 (s, 1H), 4.27 - 4.12 (m, 2H), 3.74 (hept, J = 6.6 Hz, 2H), 2.64 (s, 3H), 1.53 (d, J = 3.5 Hz, 6H), 1.15 (dd, J = 6.7, 1.4 Hz, 12H)

### Example 48: Synthesis of 2-(4-(tert-butyl)phenyl)-7,7-dimethyl-10-(4-methylpiperazin-1-yl)-5,12b-dihydro-1H,7H-chromeno[4,3-c][1,2,4]triazolo[1,2-a]pyridazine-1,3(2H)-dione

Except that 1-(2,2-dimethyl-3-vinyl-2*H*-chromen-7-yl)-4-methylpiperazine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-(tert-butyl)phenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione (22 mg, 47%).
¹H NMR (400 MHz, CDCl₃) δ 7.52 (s, 3H), 7.03 (d, J = 8.7 Hz, 1H), 6.50 (dd, J = 8.7, 2.5 Hz, 1H), 6.41 (d, J = 2.4 Hz, 1H), 5.86 - 5.82 (m, 1H), 5.73 (s, 1H), 4.29 (dd, J = 16.3, 5.0 Hz, 1H), 4.07 (d, J = 16.2 Hz, 1H), 3.21 - 3.13 (m, 4H), 2.54 (s, 4H), 2.33 (s, 3H), 1.61 (s, 3H), 1.59 (s, 3H), 1.35 (s, 9H)

### Example 49: Synthesis of 7,7-dimethyl-10-(4-methylpyrerazin-1-yl)-2-(4-(trifluoromethyl)phenyl)-5,12b-dihydro-1H,7H-chromeno[4,3-c][1,2,4]triazolo[1,2-a]pyridazine-1,3(2H)-dione

Except that 4-(4-(trifluoromethyl)phenyl)-1,2,4-triazolidine-3,5-dione was used instead of 4-(4-(tert-butyl)phenyl)-1,2,4-triazolidine-3,5-dione, the same synthesis method as in Example 48 above was carried out to obtain 7,7-dimethyl-10-(4-methylpyrerazin-1-yl)-2-(4-(trifluoromethyl)phenyl)-5,12b-dihydro-1*H*,7*H*-chromeno[4,3-*c*][1,2,4]triazolo[1,2-*a*]pyridazine-1,3(2*H*)-dione (30 mg, 60%).
¹H NMR (400 MHz, CDCl₃) δ 7.86 (d, J = 8.4 Hz, 2H), 7.78 (d, J = 8.6 Hz, 2H), 7.00 (d, J = 8.5 Hz, 1H), 6.51 (dd, J = 8.6, 2.5 Hz, 1H), 6.42 (d, J = 2.4 Hz, 1H), 5.87 - 5.83 (m, 1H), 5.73 (s, 1H), 4.31 (dd, J = 16.3, 5.0 Hz, 1H), 4.15 - 4.07 (m, 1H), 3.19 (t, J = 4.8 Hz, 4H), 2.58 (s, 4H), 2.36 (s, 3H), 1.60 (d, J = 6.8 Hz, 6H)

### Example 50: Synthesis of 2-(4-fluorophenyl)-7,7-dimethyl-10-(4-methylpiperazin-1-yl)-5,12b-dihydro-1H,7H-chromeno[4,3-c][1,2,4]triazolo[1,2-a]pyridazine-1,3(2H)-dione

Except that 4-(4-fluorophenyl)-1,2,4-triazolidine-3,5-dione was used instead of 4-(4-(tert-butyl)phenyl)-1,2,4-triazolidine-3,5-dione, the same synthesis method as in Example 48 above was carried out to obtain 2-(4-fluorophenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione (30 mg, 60%).
¹H NMR (400 MHz, CDCl₃) δ 7.63 - 7.58 (m, 2H), 7.20 (dd, J = 9.0, 8.2 Hz, 2H), 7.01 (dd, J = 8.8, 1.1 Hz, 1H), 6.50 (dd, J = 8.7, 2.5 Hz, 1H), 6.41 (d, J = 2.4 Hz, 1H), 5.88 - 5.82 (m, 1H), 5.72 (s, 1H), 4.29 (dd, J = 16.6, 5.2 Hz, 1H), 4.13 - 4.05 (m, 1H), 3.26 - 3.16 (m, 4H), 2.66 (t, J = 4.9 Hz, 4H), 2.40 (s, 3H), 1.59 (d, J = 6.9 Hz, 6H)

### Example 51: Synthesis of 2-(4-acetylphenyl)-7,7-dimethyl-10-(4-methylpiperazin-1-yl)-5,12b-dihydro-1H,7H-chromeno[4,3-c][1,2,4]triazolo[1,2-a]pyridazine-1,3(2H)-dione

Except that 4-(4-acetylphenyl)-1,2,4-triazolidine-3,5-dione was used instead of 4-(4-(tert-butyl)phenyl)-1,2,4-triazolidine-3,5-dione, the same synthesis method as in Example 48 above was carried out to obtain 2-(4-acetylphenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione (32 mg, 48%).
¹H NMR (400 MHz, CDCl₃) δ 8.10 (d, J = 8.7 Hz, 2H), 7.84 (d, J = 8.6 Hz, 2H), 7.01 (dd, J = 8.4, 0.9 Hz, 1H), 6.50 (dd, J = 8.7, 2.5 Hz, 1H), 6.41 (d, J = 2.4 Hz, 1H), 5.88 - 5.82 (m, 1H), 5.73 (s, 1H), 4.30 (dd, J = 16.5, 5.0 Hz, 1H), 4.15 - 4.07 (m, 1H), 3.24 (t, J = 5.1 Hz, 4H), 2.70 (s, 4H), 2.64 (s, 3H), 2.43 (s, 3H), 1.60 (d, J = 5.9 Hz, 6H)

### Example 52: Synthesis of 2-(4-acetylphenyl)-7,7-dimethyl-10-(4-(methylsulfonyl)piperazin-1-yl)-5,12b-dihydro-1H,7H-chromeno[4,3-c][1,2,4]triazolo[1,2-a]pyridazine-1,3(2H)-dione

Except that 2-(4-acetylphenyl)-7,7-dimethyl-10-(piperazin-1-yl)-5,12b-dihydro-1*H*,7*H-*chromeno[4,3-*c*][1,2,4]triazolo[1,2-*a*]pyridazine-1,3(2*H*)-dione hydrochloride was used instead of 7-amino-3-bromo-2,2-dimethylchroman-4-one, the same synthesis method as in Intermediate 11 (a) above was carried out to obtain 2-(4-acetylphenyl)-7,7-dimethyl-10-(4-(methylsulfonyl)piperazin-1-yl)-5,12b-dihydro-1*H*,7*H*-chromeno[4,3-*c*][1,2,4]triazolo[1,2-*a*]pyridazine-1,3(2*H*)-dione (34 mg, 70%).
¹H NMR (400 MHz, CDCl₃) δ 8.11 (d, J = 8.7 Hz, 2H), 7.84 (d, J = 8.6 Hz, 2H), 7.03 (d, J = 8.5 Hz, 1H), 6.51 (dd, J = 8.6, 2.5 Hz, 1H), 6.42 (d, J = 2.4 Hz, 1H), 4.31 (dd, J = 16.4, 4.9 Hz, 1H), 4.14 - 4.09 (m, 1H), 3.36 - 3.31 (m, 4H), 3.27 - 3.23 (m, 4H), 2.81 (s, 3H), 2.65 (s, 3H), 1.6 (d, J= 4.0 Hz, 6H)

### Example 53: Synthesis of 10-hydroxy-7,7-dimethyl-2-(4-(morpholine-4-carbonyl)phenyl)-5,12b-dihydro-1H,7H-chromeno[4,3-c][1,2,4]triazolo[1,2-a]pyridazine-1,3(2H)-dione

### (a) Synthesis of 4-(7,7-dimethyl-1,3-dioxo-10-((tetrahydro-2H-pyran-2-yl)oxy)-5,12b-dihydro-1H,7H-chromeno[4,3-c][1,2,4]triazolo[1,2-a]pyridazin-2(3H)-yl)benzoic acid

Except that 2,2-dimethyl-7-((tetrahydro-2*H*-pyran-2-yl)oxy)-3-vinyl-2*H*-chromene and 4-(3,5-dioxo-1,2,4-triazolidin-4-yl)benzoic acid were used instead of ((2,2-bis(fluoromethyl)-3-vinyl-2*H*-chromen-7-yl)oxy)triisopropylsilane and 4-(4-acetylphenyl)-1,2,4-triazolidine-3,5-dione, respectively, the same synthesis method as in Example 2 (a) above was carried out to obtain 4-(7,7-dimethyl-1,3-dioxo-10-((tetrahydro-2*H*-pyran-2-yl)oxy)-5,12b-dihydro-1*H*,7*H-*chromeno[4,3-*c*][1,2,4]triazolo[1,2-*a*]pyridazin-2(3*H*)-yl)benzoic acid (100 mg, 30%).

### (b) Synthesis of 7,7-dimethyl-2-(4-(morpholine-4-carbonyl)phenyl)-10-((tetrahydro-2H-pyran-2-yl)oxy)-5,12b-dihydro-1H,7H-chromeno[4,3-c][1,2,4]triazolo[1,2-a]pyridazine-1,3(2H)-dione

4-(7,7-Dimethyl-1,3-dioxo-10-((tetrahydro-2*H*-pyran-2-yl)oxy)-5,12b-dihydro-1*H*,7*H-*chromeno[4,3-*c*][1,2,4]triazolo[1,2-*a*]pyridazin-2(3*H*-yl)benzoic acid (230 mg, 0.46 mmol) obtained in Example 53 (a) above was dissolved in *N*,*N*-dimethylformamide (3 mL), and triethylamine (92 mg, 0.91 mmol), 1-(3-dimethylaminopropyl)-3-ethylcarbodiimide hydrochloride (131 mg, 0.68 mmol) and 1-hydroxybenzotriazole (92 mg, 0.68 mmol) were added thereto and stirred at room temperature for 10 minutes. Morpholine (51.4 mg, 0.59 mmol) was added thereto and stirred for additional 3 hours at room temperature. When the reaction was completed, the reaction mixture was diluted in ethyl acetate and washed with aqueous sodium chloride solution. The separated organic layer was dried over sodium sulfate, filtered, concentrated by distillation under reduced pressure, and separated by MPLC (dichloromethane : methanol = 30 : 1, v/v) to obtain 7,7-dimethyl-2-(4-(morpholine-4-carbonyl)phenyl)-10-((tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione (140 mg, 54%).

### (c) Synthesis of 10-hydroxy-7,7-dimethyl-2-(4-(morpholine-4-carbonyl)phenyl)-5,12b-dihydro-1H,7H-chromeno[4,3-c][1,2,4]triazolo[1,2-a]pyridazine-1,3(2H)-dione

Except that 7,7-dimethyl-2-(4-(morpholine-4-carbonyl)phenyl)-10-((tetrahydro-2*H-*pyran-2-yl)oxy)-5,12b-dihydro-1*H*,7*H*-chromeno[4,3-*c*][1,2,4]triazolo[1,2-*a*]pyridazine-1,3(2*H*)-dione was used instead of 2,2-bis(fluoromethyl)-7-((tetrahydro-2*H*-pyran-2-yl)oxy)chroman-4-one, the same synthesis method as in Intermediate 31 (d) above was carried out to obtain 10-hydroxy-7,7-dimethyl-2-(4-(morpholine-4-carbonyl)phenyl)-5,12b-dihydro-1*H*,7*H-*chromeno[4,3-*c*][1,2,4]triazolo[1,2-*a*]pyridazine-1,3(2*H*)-dione (50 mg, 70%).
¹H NMR (400 MHz, DMSO-D₆) δ 9.47 (s, 1H), 7.68 (d, J = 8.5 Hz, 2H), 7.59 (d, J = 8.5 Hz, 2H), 6.85 (dd, J = 8.3, 1.1 Hz, 1H), 6.40 - 6.36 (m, 1H), 6.25 (d, J = 2.3 Hz, 1H), 5.95 (dt, J = 4.8, 2.3 Hz, 1H), 5.60 (s, 1H), 4.25 - 4.18 (m, 1H), 4.18 - 4.11 (m, 1H), 3.63 (s, 8H), 1.54 (s, 3H), 1.51 (s, 3H)

### Example 54: Synthesis of tert-butyl 4-(4-(10-hydroxy-7,7-dimethyl-1,3-dioxo-5,12b-dihydro-1H,7H-chromeno[4,3-c][1,2,4]triazolo[1,2-a]pyridazin-2(3H)-yl)benzoyl)piperazine-1-carboxylate

### (a) Synthesis of tert-butyl 4-(4-(7,7-dimethyl-1,3-dioxo-10-((tetrahydro-2H-pyran-2-yl)oxy)-5,12b-dihydro-1H,7H-chromeno[4,3-c][1,2,4]triazolo[1,2-a]pyridazin-2(3H)-yl)benzoyl)piperazine-1-carboxylate

Except that 1-(tert-butoxycarbonyl)piperazine was used instead of morpholine, the same synthesis method as in Example 53 (b) above was carried out to obtain tert-butyl 4-(4-(7,7-dimethyl-1,3-dioxo-10-((tetrahydro-2*H*-pyran-2-yl)oxy)-5,12b-dihydro-1*H*,7*H*-chromeno[4,3-*c*][1,2,4]triazolo[1,2-*a*]pyridazin-2(3*H*)-yl)benzoyl)piperazine-1-carboxylate (100 mg, 70%).

### (b) Synthesis of tert-butyl 4-(4-(10-hydroxy-7,7-dimethyl-1,3-dioxo-5,12b-dihydro-1H,7H-chromeno[4,3-c][1,2,4]triazolo[1,2-a]pyridazin-2(3H)-yl)benzoyl)piperazine-1-carboxylate

Except that tert-butyl 4-(4-(7,7-dimethyl-1,3-dioxo-10-((tetrahydro-2*H*-pyran-2-yl)oxy)-5,12b-dihydro-1*H*,7*H*-chromeno[4,3-*c*][1,2,4]triazolo[1,2-*a*]pyridazin-2(3*H*)-yl)benzoyl)piperazine-1-carboxylate was used instead of 2,2-bis(fluoromethyl)-7-((tetrahydro-2*H*-pyran-2-yl)oxy)chroman-4-one, the same synthesis method as in Intermediate 31 (d) above was carried out to obtain tert-butyl 4-(4-(10-hydroxy-7,7-dimethyl-1,3-dioxo-5,12b-dihydro-1*H*,7*H*-chromeno[4,3-*c*][1,2,4]triazolo[1,2-*a*]pyridazin-2(3*H*)-yl)benzoyl)piperazine-1-carboxylate (38 mg, 51%).
⁺¹H NMR (400 MHz, CDCl₃) δ 7.76 (d, J = 8.5 Hz, 2H), 7.56 (d, J = 8.4 Hz, 2H), 7.29 (s, 1H), 6.95 (d, J = 8.4 Hz, 1H), 6.41 (dd, J = 8.4, 2.5 Hz, 1H), 6.38 (d, J = 2.4 Hz, 1H), 5.84 (d, J = 3.0 Hz, 1H), 5.70 (s, 1H), 4.92 (s, 1H), 4.36 - 4.21 (m, 1H), 4.11 (d, J = 16.4 Hz, 1H), 3.49 (s, 8H), 1.60 (s, 6H), 1.48 (s, 9H)

### Example 55: Synthesis of 10-hydroxy-7,7-dimethyl-2-(4-(piperazine-1-carbonyl)phenyl)-5,12b-dihydro-1H,7H-chromeno[4,3-c][1,2,4]triazolo[1,2-a]pyridazine-1,3(2H)-dione

Except that tert-butyl 4-(4-(10-hydroxy-7,7-dimethyl-1,3-dioxo-5,12b-dihydro-1*H*,7*H-*chromeno[4,3-*c*][1,2,4]triazolo[1,2-*a*]pyridazin-2(3*H*)-yl)benzoyl)piperazine-1-carboxylate was used instead of tert-butyl (2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)carbamate, the same synthesis method as in Example 7 above was carried out to obtain 10-hydroxy-7,7-dimethyl-2-(4-(piperazine-1-carbonyl)phenyl)-5,12b-dihydro-1*H*,7*H*-chromeno[4,3-*c*][1,2,4]triazolo[1,2-*a*]pyridazine-1,3(2*H*)-dione (12 mg, 70%).
¹H NMR (400 MHz, cd3od) δ 8.31 (s, 1H), 7.79 (d, J = 8.5 Hz, 2H), 7.66 (d, J = 8.5 Hz, 2H), 6.92 - 6.84 (m, 1H), 6.39 (dd, J = 8.5, 2.5 Hz, 1H), 6.31 (d, J = 2.4 Hz, 1H), 5.96 (d, J = 2.1 Hz, 1H), 5.67 (s, 1H), 4.31 - 4.22 (m, 1H), 4.20 - 4.12 (m, 1H), 3.83 (s, 4H), 3.25 (s, 4H), 1.58 (d, J = 10.7 Hz, 6H)

### Example 56: Synthesis of 10-hydroxy-7,7-dimethyl-2-(4-(4-methylpiperazine-1-carbonyl)phenyl)-5,12b-dihydro-1H,7H-chromeno[4,3-c][1,2,4]triazolo[1,2-a]pyridazine-1,3(2H)-dione

### (a) Synthesis of 7,7-dimethyl-2-(4-(4-methylpiperazine-1-carbonyl)phenyl)-10-((tetrahydro-2H-pyran-2-yl)oxy)-5,12b-dihydro-1H,7H-chromeno[4,3-c][1,2,4]triazolo[1,2-a]pyridazine-1,3(2H)-dione

Except that *N*-methyl piperazine was used instead of morpholine, the same synthesis method as in Example 53 (b) above was carried out to obtain 7,7-dimethyl-2-(4-(4-methylpiperazine-1-carbonyl)phenyl)-10-((tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione (80 mg, 56%).

### (b) Synthesis of 10-hydroxy-7,7-dimethyl-2-(4-(4-methylpiperazine-1-carbonyl)phenyl)-5,12b-dihydro-1H,7H-chromeno[4,3-c][1,2,4]triazolo[1,2-a]pyridazine-1,3(2H)-dione

Except that 7,7-dimethyl-2-(4-(4-methylpiperazine-1-carbonyl)phenyl)-10-((tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione was used instead of 2,2-bis(fluoromethyl)-7-((tetrahydro-2*H*-pyran-2-yl)oxy)chroman-4-one, the same synthesis method as in Intermediate 31 (d) above was carried out to obtain 10-hydroxy-7,7-dimethyl-2-(4-(4-methylpiperazine-1-carbonyl)phenyl)-5,12b-dihydro-1*H*,7*H*-chromeno[4,3-*c*][1,2,4]triazolo[1,2-*a*]pyridazine-1,3(2*H*)-dione (42 mg, 72%).
¹H NMR (400 MHz, CDCl₃) δ 8.14 (s, 1H), 7.75 (d, J = 8.5 Hz, 2H), 7.55 (d, J = 8.6 Hz, 2H), 6.94 (d, J = 8.1 Hz, 1H), 6.43 - 6.36 (m, 2H), 5.87 - 5.81 (m, 1H), 5.70 (s, 1H), 4.30 (dd, J = 16.3, 4.9 Hz, 1H), 4.15 - 4.06 (m, 1H), 3.89 (s, 2H), 3.59 (s, 2H), 2.62 (d, J = 39.6 Hz, 4H), 2.43 (s, 3H), 1.59 (s, 6H)

### Example 57: Synthesis of 7,7-dimethyl-2-(4-(morpholine-4-carbonyl)phenyl)-10-(pyrrolidin-1-yl)-5,12b-dihydro-1H,7H-chromeno[4,3-c][1,2,4]triazolo[1,2-a]pyridazine-1,3(2H)-dione

Except that 4-(7,7-dimethyl-1,3-dioxo-10-(pyrrolidin-1-yl)-5,12b-dihydro-1*H*,7*H-*chromeno[4,3-*c*][1,2,4]triazolo[1,2-*a*]pyridazin-2(3*H*)-yl)benzoic acid was used instead of 4-(7,7-dimethyl-1,3-dioxo-10-((tetrahydro-2*H*-pyran-2-yl)oxy)-5,12b-dihydro-1*H*,7*H*-chromeno[4,3-*c*][1,2,4]triazolo[1,2-*a*]pyridazin-2(3*H*)-yl)benzoic acid, the same synthesis method as in Example 53 (b) above was carried out to obtain 7,7-dimethyl-2-(4-(morpholine-4-carbonyl)phenyl)-10-(pyrrolidin-1-yl)-5,12b-dihydro-1*H*,7*H*-chromeno[4,3-*c*][1,2,4]triazolo[1,2-*a*]pyridazine-1,3(2*H*)-dione (40 mg, 62%).
¹H NMR (400 MHz, CDCl₃) δ 7.76 (d, J = 8.4 Hz, 2H), 7.56 (d, J = 8.4 Hz, 2H), 6.97 (d, J = 8.6 Hz, 1H), 6.19 - 6.12 (m, 1H), 6.06 (d, J = 2.3 Hz, 1H), 5.83 (d, J = 5.1 Hz, 1H), 5.75 (s, 1H), 4.30 (dd, J = 16.3, 5.0 Hz, 1H), 4.08 (d, J = 16.4 Hz, 1H), 3.73 (s, 7H), 3.51 (s, 1H), 3.21 (d, J = 6.6 Hz, 4H), 1.99 - 1.92 (m, 4H), 1.60 (d, J = 10.3 Hz, 6H)

### Example 58: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl pivalate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (300 mg, 0.72 mmol) obtained in Example 10 (b) above was dissolved in dichloromethane (7 mL), and triethylamine (220 mg, 2.15 mmol) was added thereto and stirred at room temperature for 10 minutes. Trimethylacetyl chloride (136 mg, 1.12 mmol) was added dropwise to the reaction mixture and stirred at room temperature for 15 hours. When the reaction was completed, the reaction mixture was diluted in dichloromethane and washed with water. The separated organic layer was dried over sodium sulfate, filtered, concentrated by distillation under reduced pressure, and separated by MPLC (ethyl acetate : hexane = 1 : 1, v/v) to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl pivalate (331 mg, 91%).
¹H NMR (400 MHz, DMSO-D₆) δ 8.17 - 8.09 (m, 2H), 7.84 - 7.76 (m, 2H), 7.12 (dd, J = 8.4, 1.1 Hz, 1H), 6.70 (dd, J = 8.4, 2.3 Hz, 1H), 6.66 (d, J = 2.3 Hz, 1H), 6.02 (h, J = 1.8 Hz, 1H), 5.75 - 5.70 (m, 1H), 4.22 (dd, J = 3.7, 2.0 Hz, 2H), 2.64 (s, 3H), 1.60 (s, 3H), 1.53 (s, 3H), 1.27 (s, 9H).

### Example 59: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 3-methylbutanoate

Except that 3-methylbutanoyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 3-methylbutanoate (100 mg, 90%).
¹H NMR (400 MHz, DMSO-D₆) δ 8.17 - 8.09 (m, 2H), 7.84 - 7.76 (m, 2H), 7.12 (dd, J = 8.4, 1.1 Hz, 1H), 6.71 (dd, J = 8.4, 2.3 Hz, 1H), 6.65 (d, J = 2.3 Hz, 1H), 6.02 (q, J = 3.1 Hz, 1H), 5.73 (d, J = 2.3 Hz, 1H), 4.22 (dd, J = 3.7, 2.0 Hz, 2H), 2.64 (s, 3H), 2.43 (d, J = 7.1 Hz, 2H), 2.14 - 2.02 (m, 1H), 1.60 (s, 3H), 1.53 (s, 3H), 0.98 (d, J = 6.7 Hz, 6H).

### Example 60: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl acetate

Except that acetyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl acetate (102 mg, 98%).
¹H NMR (400 MHz, DMSO-D₆) δ 8.17 - 8.10 (m, 2H), 7.84 - 7.77 (m, 2H), 7.11 (dd, J = 8.4, 1.1 Hz, 1H), 6.73 (dd, J = 8.4, 2.3 Hz, 1H), 6.68 (d, J = 2.3 Hz, 1H), 6.02 (d, J = 1.9 Hz, 1H), 5.72 (d, J = 2.4 Hz, 1H), 4.22 (dd, J = 3.9, 2.0 Hz, 2H), 2.64 (s, 3H), 2.23 (s, 3H), 1.60 (s, 3H), 1.53 (s, 3H).

### Example 61: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl ethyl carbonate

Except that ethyl chloroformate was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl ethyl carbonate (120 mg, 94%).
¹H NMR (400 MHz, CDCl₃) δ 8.11 (d, J = 8.7 Hz, 2H), 7.84 (d, J = 8.7 Hz, 2H), 7.09 (dd, J = 8.4, 1.1 Hz, 1H), 6.80 - 6.72 (m, 2H), 5.87 (dt, J = 4.7, 2.3 Hz, 1H), 5.72 (q, J = 1.6 Hz, 1H), 4.33 - 4.25 (m, 3H), 4.13 (dt, J = 16.5, 2.7 Hz, 1H), 2.64 (s, 3H), 1.61 (d, J = 4.5 Hz, 6H), 1.37 (t, J = 7.1 Hz, 3H)

### Example 62: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl dimethylcarbamate

Except that dimethylcarbamoyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dimethylcarbamate (400 mg, 99%).
¹H NMR (400 MHz, DMSO-D₆) δ 8.13 (d, J = 8.6 Hz, 2H), 7.80 (d, J = 8.6 Hz, 2H), 7.08 (dd, J = 8.4, 1.1 Hz, 1H), 6.72 (dd, J = 8.4, 2.3 Hz, 1H), 6.65 (d, J = 2.3 Hz, 1H), 6.01 (td, J = 3.7, 1.8 Hz,1H), 5.74 - 5.66 (m, 1H), 4.21 (dd, J = 3.8, 2.0 Hz, 2H), 3.01 (s, 3H), 2.88 (s, 3H), 2.64 (s, 3H), 1.59 (s, 3H), 1.53 (s, 3H).

### Example 63: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl morpholine-4-carboxylate

Except that 4-morpholinecarbonyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl morpholine-4-carboxylate (210 mg, 96%).
¹H NMR (400 MHz, DMSO-D₆) δ 8.17 - 8.09 (m, 2H), 7.84 - 7.76 (m, 2H), 7.09 (dd, J = 8.4, 1.1 Hz, 1H), 6.75 (dd, J = 8.4, 2.3 Hz, 1H), 6.69 (d, J = 2.3 Hz, 1H), 6.01 (q, J = 3.1 Hz, 1H), 5.71 (s, 1H), 4.21 (dd, J = 3.8, 2.0 Hz, 2H), 3.62 (dd, J = 5.7, 4.0 Hz, 4H), 3.47 (d, J = 58.3 Hz, 4H), 2.64 (s, 3H), 1.59 (s, 3H), 1.52 (s, 3H).

### Example 64: Synthesis of (2S,3S,4S,5R,6S)-6-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)-3,4,5-trihydroxytetrahydro-2H-pyran-2-carboxylic acid

### (a) Synthesis of (2S,3R,4S,5S,6S)-2-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)-6-(methoxycarbonyl)tetrahydro-2H-pyran-3,4,5-triyl triacetate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (1g, 2.38 mmol) obtained in Example 10 (b) above, methyl (2*S*,3*S*,4*S*,5*R*,6*R*)-3,4,5-triacetoxy-6-bromo-tetrahydropyran-2-carboxylate (1.42 g, 3.58 mmol) and 4Å molecular sieve (3 g) were suspended in dichloromethane (10 mL) and stirred for 1 hour under nitrogen atmosphere. Silver carbonate (1.31 g, 4.77 mmol) was added to the reaction mixture, stirred for 5 minutes and then cooled to 0°C. Trifluoromethanesulfonic acid (358 mg, 2.38 mmol) was added thereto and stirred for additional 15 hours at room temperature. When the reaction was completed, a few drops of triethylamine were added to the reaction mixture, and the insoluble solid was filtered through Celite and washed with dichloromethane. The filtrate was washed with water, dried over sodium sulfate, filtered, and concentrated by distillation under reduced pressure. The obtained residue was separated by reverse-phase chromatography (acetonitrile containing 0.1% formic acid : water containing 0.1% formic acid = 5 : 95-100 : 0) to obtain (2*S*,3*R*,4*S*,5*S*,6*S*)-2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-6-(methoxycarbonyl)tetrahydro-2*H*-pyran-3,4,5-triyl triacetate (178 mg, 10%).

### (b) Synthesis of (2S,3S,4S,5R,6S)-6-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)-3,4,5-trihydroxytetrahydro-2H-pyran-2-carboxylic acid

(2*S*,3*R*,4*S*,5*S*,6*S*)-2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-6-(methoxycarbonyl)tetrahydro-2*H*-pyran-3,4,5-triyl triacetate (178 mg, 0.24 mmol) obtained in Example 64 (a) above was dissolved in a mixed solvent of methanol/triethylamine (1:1, 10 mL), and water (9 mL) was added dropwise thereto and stirred at room temperature for 2 hours. When the reaction was completed, the reaction mixture was concentrated by distillation under reduced pressure, and the obtained residue was separated by reverse-phase chromatography (acetonitrile containing 0.1% formic acid : water containing 0.1% formic acid = 5 : 95-100 : 0) to obtain (2*S*,3*S*,4*S*,5*R*,6*S*)-6-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)-3,4,5-trihydroxytetrahydro-2*H-*pyran-2-carboxylic acid (90 mg, 62%).
¹H NMR (500 MHz, MeOD) δ 8.18 - 8.13 (m, 2H), 7.86 - 7.81 (m, 2H), 7.03 (dd, J = 8.6, 1.1 Hz, 1H), 6.71 (ddd, J = 8.6, 4.2, 2.4 Hz, 1H), 6.62 (dd, J = 4.8, 2.4 Hz, 1H), 6.01 - 5.94 (m, 1H), 5.71 (t, J = 2.0 Hz, 1H), 4.97 - 4.91 (m, 1H), 4.31 - 4.22 (m, 1H), 4.23 - 4.13 (m, 1H), 3.96 (d, J = 9.8 Hz, 1H), 3.59 (ddt, J = 9.5, 7.4, 1.8 Hz, 1H), 3.48 - 3.44 (m, 2H), 2.66 (s, 3H), 1.61 (s, 3H), 1.57 (d, J = 4.3 Hz, 3H).

### Example 65: Synthesis of sodium (2S,3S,4S,5R,6S)-6-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)-3,4,5-trihydroxy tetrahydro-2H-pyran-2-carboxylate

(2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxytetrahydro-2*H*-pyran-2-carboxylic acid (100 mg, 0.17 mmol) obtained in Example 64 (b) was suspended in water (1 mL), and sodium bicarbonate (14.11 mg, 0.17 mmol) was added thereto and stirred at room temperature for 2 hours. When the reaction was completed, the reaction mixture was concentrated by distillation under reduced pressure, and the obtained residue was triturated with ethyl acetate to obtain sodium (2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxy tetrahydro-2*H*-pyran-2-carboxylate (97 mg, 94%).
¹H NMR (500 MHz, D₂O) δ 7.98 - 7.90 (m, 2H), 7.59 - 7.50 (m, 2H), 6.94 (ddd, J = 24.0, 8.7, 1.0 Hz, 1H), 6.73 (ddd, J = 9.4, 8.5, 2.5 Hz, 1H), 6.66 (dd, J = 4.1, 2.4 Hz, 1H), 5.94 (dd, J = 3.9, 1.9 Hz, 1H), 5.64 (d, J = 7.8 Hz, 1H), 5.08 - 4.97 (m, 1H), 4.20 - 4.09 (m, 2H), 3.91 - 3.83 (m, 1H), 3.64 - 3.55 (m, 3H), 2.62 (s, 3H), 1.57 - 1.50 (m, 6H).

### Example 66: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-methylpiperazine-1-carboxylate hydrochloride

Except that 4-methylpiperazine-1-carbonyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-methylpiperazine-1-carboxylate hydrochloride (400 mg, 87%).
¹H NMR (400 MHz, D₂O) δ 8.13 (d, J = 8.3 Hz, 2H), 7.70 (d, J = 8.2 Hz, 2H), 7.15 (d, J = 8.3 Hz, 1H), 6.84 (d, J = 11.9 Hz, 2H), 6.03 (s, 1H), 5.79 (s, 1H), 4.28 (s, 3H), 3.63 (d, J = 12.5 Hz, 2H), 3.53 (s, 1H), 3.37 (s, 1H), 3.24 (s, 2H), 3.00 (s, 3H), 2.71 (s, 3H), 1.62 (d, J = 20.3 Hz, 6H)

### Example 67: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl piperazine-1-carboxylate hydrochloride

### (a) Synthesis of 1-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)4-(tert-butyl)piperazine-1,4-dicarboxylate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (4 g, 9.54 mmol) obtained in Example 10 (b) above was dissolved in dichloromethane (40 mL), and triethylamine (2.9 g, 11.44 mmol) was added thereto and stirred for 10 minutes. Tert-butyl 4-chlorocarbonylpiperazine-1-carboxylate (2.85 g, 11.44 mmol) and 4-dimethylaminopyridine (350 mg, 2.86 mmol) were added thereto and stirred for 15 hours. When the reaction was completed, the reaction mixture was diluted in dichloromethane and washed with water and aqueous ammonium chloride solution. The separated organic layer was dried over sodium sulfate, filtered, concentrated by distillation under reduced pressure, and separated by MPLC (ethyl acetate : hexane = 1 : 1, v/v) to obtain 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)4-(tert-butyl)piperazine-1,4-dicarboxylate (6 g, 99%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl piperazine-1-carboxylate hydrochloride

1-(2-(4-Acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)4-(tert-butyl)piperazine-1,4-dicarboxylate (6 g, 9.5 mmol) obtained in Example 67 (a) above was dissolved in 1,4-dioxane (30 mL), and 4M hydrochloric acid solution (10 mL, 4M/dioxane) was added dropwise thereto and stirred at room temperature for 15 hours. When the reaction was completed, the resulting solid was filtered and washed with ethyl acetate to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperazine-1-carboxylate hydrochloride (5.2 g, 96%).
¹H NMR (400 MHz, DMSO-D₆) δ 8.13 (d, J = 8.2 Hz, 2H), 7.80 (d, J = 8.6 Hz, 2H), 7.10 (d, J = 8.3 Hz, 1H), 6.79 - 6.72 (m, 2H), 6.02 (s, 1H), 5.71 (s, 1H), 4.22 (s, 2H), 3.75 (s, 2H), 3.63 (s, 2H), 3.18 (s, 4H), 2.64 (s, 3H), 1.60 (s, 3H), 1.52 (s, 3H).

### Example 68: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(2-oxopropanoyl)piperazine-1-carboxylate

Except that pyruvic acid and 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperazine-1-carboxylate hydrochloride were used instead of 4-(7,7-dimethyl-1,3-dioxo-10-((tetrahydro-2*H-*pyran-2-yl)oxy)-5,12b-dihydro-1*H*,7*H*-chromeno[4,3-*c*][1,2,4]triazolo[1,2-*a*]pyridazin-2(3*H*)-yl)benzoic acid and morpholine, respectively, the same synthesis method as in Example 53 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(2-oxopropanoyl)piperazine-1-carboxylate (100 mg, 53%).
¹H NMR (400 MHz, CDCl₃) δ 8.11 (d, J = 8.7 Hz, 2H), 7.83 (d, J = 8.7 Hz, 2H), 7.08 (dd, J = 8.4, 1.2 Hz, 1H), 6.73 - 6.65 (m, 2H), 5.89 - 5.81 (m, 1H), 5.72 (s, 1H), 4.30 (dd, J = 16.4, 4.8 Hz, 1H), 4.16 - 4.10 (m, 1H), 3.70 (s, 4H), 3.61 (d, J = 17.3 Hz, 4H), 2.64 (s, 3H), 2.47 (s, 3H), 1.60 (d, J = 5.7 Hz, 6H).

### Example 69: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl dihydrogen phosphate

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl diethyl phosphate

Except that diethyl chlorophosphate was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diethyl phosphate (3 g, 95%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl dihydrogen phosphate

2-(4-Acetylphenyl)-7,7-dimethyl-1,3-dioxo-2,3,5,12b-tetrahydro-1*H*,7*H*-chromeno[4,3-c][1,2,4]triazolo[1,2-a]pyridazin-10-yl diethyl phosphate (1.5 g, 2.7 mmol) obtained in Example 69 (a) above was dissolved in dichloromethane (15 mL), and bromotrimethylsilane (1.65 g, 10.8 mmol) was added thereto and stirred at room temperature for 15 hours. When the reaction was completed, the reaction mixture was concentrated by distillation under reduced pressure, and methanol (30 mL) was added to the resulting residue and stirred at room temperature for 1 hour. The reaction mixture was concentrated by distillation under reduced pressure and recrystallized with ethanol to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate (1.3 g, 96%).
¹H NMR (500 MHz, D₂O) δ 8.22 (dt, J = 8.4, 0.5 Hz, 2H), 7.80 - 7.74 (m, 2H), 7.09 - 7.05 (m, 1H), 6.93 (ddd, J = 8.5, 2.3, 1.0 Hz, 1H), 6.86 (dd, J = 2.3, 1.0 Hz, 1H), 6.02 (td, J = 4.2, 3.8, 1.9Hz, 1H), 5.81 (s, 1H), 4.36 - 4.28 (m, 2H), 2.76 (s, 3H), 1.67 (s, 3H), 1.62 (s, 3H).

### Example 70: Synthesis of disodium 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl phosphate

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dihydrogen phosphate (100 mg, 0.2 mmol) obtained in Example 69 (b) above was suspended in water (3 mL), and sodium bicarbonate (33.78 mg, 0.4 mmol) was added thereto and stirred at room temperature for 1 hour. When the reaction was completed, the reaction mixture was added dropwise to acetone (10 mL), and the precipitated solid was filtered and washed with acetone to obtain disodium 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl phosphate (80 mg , 80%).
¹H NMR (400 MHz, D₂O) δ 8.30 - 8.22 (m, 2H), 7.89 - 7.78 (m, 2H), 7.13 (d, J = 8.5 Hz, 1H), 7.05 - 6.96 (m, 1H), 6.93 (d, J = 2.3 Hz, 1H), 6.08 (d, J = 3.5 Hz, 1H), 5.87 (s, 1H), 4.36 (t, J = 3.1 Hz, 2H), 2.82 (s, 3H), 1.71 (d, J = 19.9 Hz, 6H).

### Example 71: Synthesis of 1,3-dihydroxy-2-(hydroxymethyl)propane-2-aminium 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl phosphate

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dihydrogen phosphate (100 mg, 0.2 mmol) obtained in Example 69 (b) above was suspended in water (3 mL), and tromethamine (24 mg, 0.4 mmol) was added thereto and stirred at room temperature for 1 hour. When the reaction was completed, the reaction mixture was added dropwise to acetone (10 mL), and the precipitated solid is filtered and washed with acetone to obtain 1,3-dihydroxy-2-(hydroxymethyl)propane-2-aminium 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl phosphate (78 mg, 53%).
¹H NMR (400 MHz, D₂O) δ 8.22 (d, J = 8.7 Hz, 2H), 7.86 - 7.73 (m, 2H), 7.16 (d, J = 8.5 Hz, 1H), 7.00 - 6.95 (m, 1H), 6.92 (dd, J = 2.4, 1.2 Hz, 1H), 6.07 (d, J = 2.2 Hz, 1H), 5.84 (s, 1H), 4.35 (d, J = 2.9 Hz, 2H), 3.83 (s, 12H), 2.80 (s, 3H), 2.32 (s, 2H), 1.70 (d, J = 18.5 Hz, 6H).

### Example 72: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl dimethylglycinate hydrochloride

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (2 g, 4.77 mmol) obtained in Example 10 (b) above was dissolved in dichloromethane (10 mL), and triethylamine (1.45 g, 14.31 mmol) and 2-(dimethylamino)acetyl chloride (0.87 g, 7.15 mmol) were added thereto and stirred at room temperature for 15 hours. When the reaction was completed, the reaction mixture was diluted in dichloromethane and washed with water. The separated organic layer was dried over sodium sulfate, filtered, and concentrated by distillation under reduced pressure. The obtained residue was dissolved in acetone (20 mL), and 4M hydrochloric acid solution (10 mL, 4M/dioxane) was added dropwise thereto and stirred at room temperature for 30 minutes. The obtained solid precipitate was filtered, washed with acetone to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dimethylglycinate hydrochloride (900 mg, 35%).
¹H NMR (500 MHz, D₂O) δ 8.00 - 7.89 (m, 2H), 7.60 - 7.52 (m, 2H), 7.07 (dd, J = 8.6, 1.2 Hz, 1H), 6.84 - 6.77 (m, 2H), 5.96 - 5.91 (m, 1H), 5.65 (s, 1H), 4.42 (s, 2H), 4.16 (dt, J = 3.9, 2.3 Hz, 2H), 3.03 (d, J = 0.8 Hz, 6H), 2.61 (s, 3H), 1.53 (d, J = 9.7 Hz, 6H).

### Example 73: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (2-methoxyethyl) carbonate

Except that methoxyethyl carbonochloridate was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (2-methoxyethyl) carbonate (120 mg, 95%).
¹H NMR (400 MHz, DMSO-D₆) δ 8.17 - 8.07 (m, 2H), 7.85 - 7.74 (m, 2H), 7.14 (dd, J = 8.4, 1.2 Hz, 1H), 6.84 (dd, J = 8.4, 2.4 Hz, 1H), 6.80 (d, J = 2.3 Hz, 1H), 6.03 (td, J = 3.8, 1.8 Hz, 1H), 5.76 - 5.68 (m, 1H), 4.34 - 4.26 (m, 2H), 4.22 (dd, J = 3.9, 2.0 Hz, 2H), 3.62 - 3.55 (m, 2H), 3.28 (s, 3H), 2.64 (s, 3H), 1.60 (s, 3H), 1.54 (s, 3H).

### Example 74: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl pyrrolidine-1-carboxylate

Except that pyrrolidine-1-carbonyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl pyrrolidine-1-carboxylate (98 mg, 96%).
¹H NMR (400 MHz, CDCl₃) δ 8.10 (d, J = 8.7 Hz, 2H), 7.84 (d, J = 8.7 Hz, 2H), 7.04 (dd, J = 8.3, 1.1 Hz, 1H), 6.75 - 6.69 (m, 2H), 5.84 (dt, J = 4.7, 2.3 Hz, 1H), 5.75 - 5.68 (m, 1H), 4.29 (ddd, J = 16.5, 4.8, 1.4 Hz, 1H), 4.12 (dt, J = 16.4, 2.6 Hz, 1H), 3.49 (dt, J = 27.1, 6.4 Hz, 4H), 2.64 (s, 3H), 1.99 - 1.85 (m, 4H), 1.59 (d, J = 6.6 Hz, 6H).

### Example 75: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (2,5,8,11-tetraoxatridecan-13-yl) carbonate

Except that 2-[2-[2-(2-methoxyethoxy)ethoxy]ethoxy]ethyl carbonochloridate was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2,5,8,11-tetraoxatridecan-13-yl) carbonate (60 mg, 90%).
¹H NMR (400 MHz, CDCl₃) δ 8.13 - 8.04 (m, 2H), 7.89 - 7.78 (m, 2H), 7.08 (d, J = 8.4 Hz, 1H), 6.81 - 6.71 (m, 2H), 5.86 (dt, J = 4.7, 2.2 Hz, 1H), 5.72 (s, 1H), 4.40 - 4.34 (m, 2H), 4.30 (dd, J = 16.5, 4.8 Hz, 1H), 4.13 (dt, J = 16.5, 2.6 Hz, 1H), 3.80 - 3.75 (m, 2H), 3.65 (dd, J = 13.9, 5.4 Hz, 10H), 3.54 (dd, J = 5.9, 3.4 Hz, 2H), 3.37 (s, 3H), 2.64 (s, 3H), 1.60 (d, J = 3.9 Hz, 6H).

### Example 76: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl diethylcarbamate

Except that diethylcarbamic chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diethylcarbamate (120 mg, 94%).
¹H NMR (400 MHz, CDCl₃) δ 8.11 (d, J = 8.7 Hz, 2H), 7.83 (d, J = 8.7 Hz, 2H), 7.05 (d, J = 8.2 Hz, 1H), 6.74 - 6.68 (m, 2H), 5.84 (dt, J = 4.7, 2.2 Hz, 1H), 5.73 (s, 1H), 4.29 (ddd, J = 16.6, 4.8, 1.3 Hz, 1H), 4.12 (dt, J = 16.4, 2.6 Hz, 1H), 3.38 (s, 4H), 2.64 (s, 3H), 1.60 (d, J = 4.1 Hz, 6H), 1.26 - 1.15 (m, 6H).

### Example 77: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-glutaminate 2,2,2-trifluoroacetic acid

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (tert-butoxycarbonyl)-L-glutaminate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (350 mg, 0.84 mmol) obtained in Example 10 (b) above, (2*S*)-5-amino-2-(tert-butoxycarbonylamino)-5-oxo-pentanoic acid (247 mg, 1.0 mmol), 1-(3-dimethylaminopropyl)-3-ethylcarbodiimide hydrochloride (320 mg, 1.67 mmol) and 4-dimethylaminopyridine (20 mg, 0.17 mmol) were dissolved in dichloromethane (5 mL) and stirred at room temperature for 15 hours. When the reaction was completed, the reaction mixture was diluted in dichloromethane and washed with water and aqueous sodium chloride solution. The separated organic layer was dried over sodium sulfate, filtered, concentrated by distillation under reduced pressure, and separated by MPLC (dichloromethane : methanol = 95 : 5, v/v) to obtain 2-(4-acetylphenyl)-7,7-dimethyl-1,3-dioxo-2,3,5,12b-tetrahydro-1*H*,7*H*-chromeno[4,3-c][1,2,4]triazolo[1,2-a]pyridazin-10-yl (tert-butoxycarbonyl)-*L*-glutaminate (250 mg, 46%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-glutaminate

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-glutaminate (300 mg, 0.46 mmol) obtained in Example 77 (a) above was dissolved in dichloromethane (2 mL), and trifluoroacetic acid (2 mL) was added dropwise thereto and stirred at room temperature for 5 hours. When the reaction was completed, the reaction mixture was diluted in water and washed with ethyl acetate. The separated aqueous layer was freeze-dried to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl *L-*glutaminate (80 mg, 24%).
¹H NMR (400 MHz, D₂O) δ 8.10 (d, J = 8.3 Hz, 2H), 7.68 (d, J = 8.3 Hz, 2H), 7.18 (d, J = 8.5 Hz, 1H), 6.89 (d, J = 8.4 Hz, 2H), 6.04 (s, 1H), 5.78 (s, 1H), 4.50 (t, J = 6.6 Hz, 1H), 4.28 (s, 2H), 2.71 (s, 3H), 2.66 (s, 2H), 2.42 (dd, J = 17.8, 7.3 Hz, 2H), 1.93 (s, 1H), 1.63 (d, J = 16.6 Hz, 6H), 1.32 (s, 3H).

### Example 78: Synthesis of (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-L-arginine 2,2,2-trifluoroacetic acid

### (a) Synthesis of tert-butyl (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-L-argininate

Tert-butyl (2*S*)-2-amino-5-guanidino-pentanoate (790 mg, 2.48 mmol, 2HCl) and 4-dimethylaminopyridine (611 mg, 4.95 mmol) were dissolved in dichloromethane (10 mL), and a solution of bis-(4-nitrophenyl) carbonate (768 mg, 2.48 mmol) dissolved in 10 mL of dichloromethane was added dropwise to the reaction mixture. The reaction mixture was cooled to -10°C and stirred for 5 hours, and a solution of 2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione(1.25 g, 2.97 mmol) and 4-dimethylaminopyridine (305 mg, 2.48 mmol) dissolved in dichloromethane (10 mL) was added dropwise to the reaction mixture, stirred at -10°C for 2 hours, raised to room temperature, and stirred for additional 72 hours. When the reaction was completed, the reaction mixture was diluted in dichloromethane and washed with 1N aqueous hydrochloric acid solution. The separated organic layer was dried over sodium sulfate, filtered, concentrated by distillation under reduced pressure, and separated by MPLC (dichloromethane : methanol = 9 : 1, v/v) to obtain tert-butyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-argininate (800 mg, 47%).

### (b) Synthesis of (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-L-arginine 2,2,2-trifluoroacetic acid

Tert-butyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-argininate (600 mg, 0.89 mmol) obtained in Example 78 (a) above was dissolved in dichloromethane (3 mL), and the reaction mixture was cooled to 0°C. Trifluoroacetic acid (2.96 g, 26 mmol) was added dropwise thereto and stirred at 0°C for 3 hours. When the reaction was completed, the reaction mixture was concentrated by distillation under reduced pressure, and the obtained residue is triturated with diethyl ether to obtain (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-arginine 2,2,2-trifluoroacetic acid (611 mg, 91%).
¹H NMR (400 MHz, DMSO-D₆) δ 8.17 - 8.09 (m, 3H), 7.84 - 7.76 (m, 2H), 7.53 (s, 1H), 7.09 (d, J = 8.4 Hz, 1H), 6.71 (dt, J = 8.5, 2.0 Hz, 1H), 6.62 (t, J = 2.0 Hz, 1H), 6.02 (q, J = 3.2 Hz, 1H), 5.71 (s, 1H), 4.25 - 4.19 (m, 2H), 3.99 (td, J = 8.1, 4.4 Hz, 1H), 3.11 (q, J = 6.5 Hz, 2H), 2.64 (s, 3H), 1.86 - 1.72 (m, 1H), 1.72 - 1.40 (m, 10H).

### Example 79: Synthesis of 1-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl) 2-methyl (2S)-pyrrolidine-1,2-dicarboxylate

Except that 1-(chlorocarbonyl)proline methyl ester was used instead of trimethylacetyl chloride, the same synthesis method as in Example 58 above was carried out to obtain 1-(2-(4-acetylphenyl)-7,7-dimethyl-1,3-dioxo-2,3,5,12b-tetrahydro-1*H*,7*H*-chromeno[4,3-c][1,2,4]triazolo[1,2-a]pyridazin-10-yl) 2-methyl (2*S*)-pyrrolidine-1,2-dicarboxylate (100 mg, 85%).
¹H NMR (400 MHz, CDCl₃) δ 8.11 (d, J = 8.6 Hz, 2H), 7.83 (d, J = 8.6 Hz, 2H), 7.04 (d, J = 8.4 Hz, 1H), 6.77 - 6.72 (m, 1H), 6.70 - 6.62 (m, 1H), 5.84 (s, 1H), 5.71 (s, 1H), 4.29 (d, J = 16.5 Hz, 1H), 4.12 (d, J = 16.2 Hz, 1H), 3.73 (s, 3H), 3.70 - 3.53 (m, 2H), 2.64 (s, 3H), 2.37 - 2.17 (m, 1H), 2.14 - 1.89 (m, 4H), 1.59 (d, J = 4.4 Hz, 6H).

### Example 80: Synthesis of methyl (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-L-leucinate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (189 mg, 0.45 mmol) obtained in Example 10 (b) above and triethylamine (69 mg, 0.68 mmol) were dissolved in tetrahydrofuran (2 mL), and a solution of methyl 2-isocyanato-4-methyl-pentanoate (100 mg, 0.58 mmol) dissolved in tetrahydrofuran (1 mL) and 4-dimethylaminopyridine (56 mg, 0.45 mmol) were added thereto and stirred at 100°C for 20 hours. When the reaction was completed, the reaction mixture was diluted in dichloromethane and washed with water and aqueous sodium chloride solution. The separated organic layer was dried over sodium sulfate, filtered, concentrated by distillation under reduced pressure, and separated by reverse-phase chromatography (acetonitrile containing 0.1% formic acid : water containing 0.1% formic acid = 5 : 95-100 : 0) to obtain methyl (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*L*-leucinate (141 mg, 53%).
¹H NMR (400 MHz, DMSO-D₆) δ 8.24 (d, J = 7.9 Hz, 1H), 8.17 - 8.09 (m, 2H), 7.83 - 7.76 (m, 2H), 7.09 (d, J = 8.4 Hz, 1H), 6.74 - 6.67 (m, 1H), 6.62 (dd, J = 2.4, 1.0 Hz, 1H), 6.04 - 5.99 (m, 1H), 5.71 (s, 1H), 4.21 (t, J = 2.7 Hz, 2H), 4.09 (dq, J = 5.0, 2.9 Hz, 1H), 3.65 (s, 3H), 2.64 (s, 3H), 1.66 (dd, J= 14.8, 7.1 Hz, 1H), 1.63 - 1.45 (m, 8H), 0.89 (dd, J = 12.4, 6.5 Hz, 6H).

### Example 81: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-leucinate 2,2,2-trifluoroacetic acid

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (tert-butoxycarbonyl)-L-leucinate

Except that (2*S*)-2-(tert-butoxycarbonylamino)-4-methyl-pentanoic acid was used instead of (2*S*)-5-amino-2-(tert-butoxycarbonylamino)-5-oxo-pentanoic acid, the same synthesis method as in Example 77 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-leucinate (200 mg, 78%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-leucinate 2,2,2-trifluoroacetic acid

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (tert-butoxycarbonyl)-*L*-leucinate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-glutaminate, the same synthesis method as in Example 77 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl *L-*leucinate 2,2,2-trifluoroacetic acid (39 mg, 35%).
¹H NMR (400 MHz, ) δ 8.64 - 8.33 (m, 3H), 8.17 - 8.09 (m, 2H), 7.85 - 7.74 (m, 2H), 7.19 (ddd, J = 8.4, 3.4, 1.1 Hz, 1H), 6.81 (dd, J = 8.4, 2.4 Hz, 1H), 6.77 (dd, J = 2.4, 1.3 Hz, 1H), 6.04 (dt, J = 5.9, 3.5 Hz, 1H), 5.75 (t, J = 2.7 Hz, 1H), 4.27 (s, 1H), 4.25 - 4.18 (m, 2H), 2.64 (s, 3H), 1.83 (td, J = 13.0, 12.0, 6.4 Hz, 2H), 1.74 (q, J = 7.3, 6.7 Hz, 1H), 1.57 (d, J = 28.9 Hz, 6H), 0.96 (dd, J = 6.1, 3.3 Hz, 6H).

### Example 82: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(L-prolyl)piperazine-1-carboxylate hydrochloride

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-((tert-butoxycarbonyl)-L-prolyl)piperazine-1-carboxylate

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperazine-1-carboxylate hydrochloride (200 mg, 0.35 mmol) obtained in Example 67 (b) above, *N*-(tert-butoxycarbonyl)-*L*-proline (91 mg, 0.42 mmol), 1-(3-dimethylaminopropyl)-3-ethylcarbodiimide hydrochloride (135 mg, 0.7 mmol) and 4-dimethylaminopyridine (8.6 mg, 0.07 mmol) were dissolved in dichloromethane (4 mL), and the reaction mixture was stirred at room temperature for 3 hours. When the reaction was completed, the reaction mixture was diluted in dichloromethane and washed with water. The separated organic layer was dried over sodium sulfate, filtered, concentrated by distillation under reduced pressure, and separated by MPLC (ethyl acetate : hexane = 1 : 1, v/v) to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-((tert-butoxycarbonyl)-*L*-prolyl)piperazine-1-carboxylate (160 mg, 62%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(L-prolyl)piperazine-1-carboxylate hydrochloride

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-((tert-butoxycarbonyl)-*L-*prolyl)piperazine-1-carboxylate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl) piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-prolyl)piperazine-1-carboxylate hydrochloride (50 mg, 78%).
¹H NMR (400 MHz, D₂O) δ 7.86 (d, J = 8.2 Hz, 2H), 7.51(d, J = 8.2 Hz, 2H), 7.01 (d, J = 8.4 Hz, 1H), 6.73 - 6.60(m, 2H), 5.87 (s, 1H), 5.58 (s, 1H), 4.73 (s, 1H), 4.03 (q, J= 16.4 Hz, 2H), 3.58 (d, J = 20.7 Hz, 8H), 3.49 - 3.38 (m,3H), 2.58 (s, 3H), 2.49 (s, 1H), 2.00 (ddd, J = 50.6, 13.5,6.9 Hz, 4H), 1.50 (d, J = 6.6 Hz, 7H).

### Example 83: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl methyl(2-(methylamino)ethyl)carbamate 2,2,2-trifluoroacetic acid

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl tert-butyl ethane-1,2-diylbis(methylcarbamate)

Triphosgene (611 mg, 2.02 mmol) was dissolved in dichloromethane (20 mL) and cooled to 0°C. Tert-butyl *N*-methyl-*N*-[2-(methylamino)ethyl]carbamate (400 mg, 2.02 mmol) was added dropwise thereto, and triethylamine (619 mg, 6.06 mmol) was subsequently added dropwise. The reaction mixture was stirred at 0°C for 5 minutes, raised to room temperature and stirred for 1 hour. After adding water, the layer separation was carried out to extract the organic layer. The separated organic layer was dried over sodium sulfate, filtered, and concentrated by distillation under reduced pressure. The obtained residue was dissolved in dichloromethane (10 mL), and triethylamine (1.86 g, 18.17 mmol) and a solution of 2-(4-acetylphenyl)-10-hydroxy-7,7-dimethyl-5,12b-dihydro-1*H*,7*H*-chromeno[4,3-*c*][1,2,4]triazolo[1,2-*a*] pyridazine-1,3(2*H*)-dione (847 mg, 2.02 mmol) dissolved in dichloromethane (10 mL) were added dropwise to the reaction mixture and stirred at room temperature for 30 minutes. When the reaction was completed, the reaction mixture was diluted in dichloromethane and washed with water. The separated organic layer was dried over sodium sulfate, filtered, and concentrated by distillation under reduced pressure. The obtained residue was separated by MPLC (ethyl acetate : hexane = 1 : 9, v/v) to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl tert-butyl ethane-1,2-diylbis(methylcarbamate) (1.01 g, 79%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl methyl(2-(methylamino)ethyl)carbamate 2,2,2-trifluoroacetic acid

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl tert-butyl ethane-1,2-diylbis(methylcarbamate) was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-glutaminate, the same synthesis method as in Example 77 (b) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(2-(methylamino)ethyl)carbamate 2,2,2-trifluoroacetic acid (230 mg, 42%).
¹H NMR (400 MHz, DMSO-D₆) δ 8.54 (d, J = 34.8 Hz, 1H), 8.18 - 8.10 (m, 2H), 7.84 - 7.76 (m, 2H), 7.09 (dd, J = 9.0, 1.1 Hz, 1H), 6.82 - 6.75 (m, 2H), 6.02 (td, J = 3.7, 1.8 Hz, 1H), 5.71 (q, J = 1.9 Hz, 1H), 4.22 (dd, J = 4.1, 2.0 Hz, 2H), 3.71 - 3.60 (m, 1H), 3.57 - 3.50 (m, 1H), 3.21 - 3.11 (m, 2H), 2.96 (d, J = 42.0 Hz, 3H), 2.64 (s, 3H), 2.63 - 2.56 (m, 3H), 1.59 (s, 3H), 1.52 (s, 3H).

### Example 84: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (2-aminoethyl)(ethyl)carbamate hydrochloride

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (2-((tert-butoxycarbonyl)amino)ethyl)(ethyl)carbamate

Except that tert-butyl *N*-[2-[chlorocarbonyl(ethyl)amino]ethyl]carbamate was used instead of trimethylacetyl chloride, the same synthesis method as in Example 58 above was carried out to quantitatively obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (2-((tert-butoxycarbonyl)amino)ethyl)(ethyl)carbamate. The obtained compound was used in the next reaction without purification.

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (2-aminoethyl)(ethyl)carbamate hydrochloride

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-((tert-butoxycarbonyl)amino)ethyl)(ethyl)carbamate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl) piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (2-aminoethyl)(ethyl)carbamate hydrochloride (80 mg, 54%).
¹H NMR (500 MHz, D₂O) δ 8.09 (dd, J = 13.4, 8.3 Hz, 2H), 7.71 - 7.61 (m, 2H), 7.11 (dd, J = 16.6, 8.5 Hz, 1H), 6.87 - 6.75 (m, 2H), 6.00 (s, 1H), 5.76 (s, 1H), 4.26 (s, 2H), 3.80 (s, 1H), 3.66 (s, 1H), 3.51 (d, J = 7.3 Hz, 1H), 3.39 (d, J = 7.2 Hz, 1H), 3.28 (dt, J = 28.2, 6.1 Hz, 2H), 2.69 (d, J = 2.7 Hz, 3H), 1.62 (d, J = 2.3 Hz, 3H), 1.57 (s, 3H), 1.22 (dt, J = 37.3, 7.1 Hz, 3H).

### Example 85: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl methyl(pyrrolidin-3-yl)carbamate hydrochloride

### (a) Synthesis of tert-butyl 3-((((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)(methyl)amino)pyrrolidine-1-carboxylate

Except that *N*-methyl-*N*-pyrrolidin-3-yl-carbamoyl chloride was used instead of trimethylacetyl chloride, the same synthesis method as in Example 58 above was carried out to obtain tert-butyl 3-((((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)(methyl)amino)pyrrolidine-1-carboxylate (220 mg, 86%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl methyl(pyrrolidin-3-yl)carbamate hydrochloride

Except that tert-butyl 3-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)(methyl)amino)pyrrolidine-1-carboxylate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl) piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl methyl(pyrrolidin-3-yl)carbamate hydrochloride (80 mg, 54%).
¹H NMR (500 MHz, D₂O) δ 7.94 (d, J = 8.6 Hz, 2H), 7.56 (d, J = 8.5 Hz, 2H), 7.03 (d, J = 8.4 Hz, 1H), 6.74 (s, 1H), 6.69 (s, 1H), 5.93 (s, 1H), 5.64 (s, 1H), 4.67 - 4.57 (m, 1H), 4.16 - 4.10 (m, 2H), 3.60 - 3.50 (m, 2H), 3.43 (s, 1H), 3.32 (d, J = 21.5 Hz, 1H), 3.07 (s, 2H), 2.94 (s, 1H), 2.62 (s, 3H), 2.38 (dtd, J = 12.8, 7.9, 4.5 Hz, 1H), 2.22 (s, 1H), 1.54 (d, J = 4.1 Hz, 6H).

### Example 86: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl bis(3-(dimethylamino)propyl)carbamate dihydrochloride

Triphosgene (772 mg, 2.6 mmol) was dissolved in dichloromethane (15 mL) and cooled to 0°C. Pyridine (3.1 g, 39 mmol) was added dropwise to the reaction mixture and stirred at 0°C for 15 minutes. While maintaining the reaction mixture at 0°C, *N*-[3-(dimethylamino)propyl]-*N'*,*N'*-dimethyl-propane-1,3-diamine (974 mg, 5.2 mmol) was added dropwise, raised to room temperature and stirred for 6 hours. 4-Dimethylaminopyridine (974 mg, 5.2 mmol) was added to the reaction mixture, and a solution of 2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2H)-dione (545 mg, 1.3 mmol) dissolved in dichloromethane (10 mL) was added dropwise thereto and stirred at room temperature for 15 hours. When the reaction was completed, the reaction mixture was diluted in dichloromethane and washed with water. The separated organic layer was dried over sodium sulfate, filtered, concentrated by distillation under reduced pressure, separated by column chromatography (dichloromethane : methanol = 9 : 1, v/v), and concentrated under reduced pressure. The obtained residue was purified with ethyl acetate (0.5 mL), and the solid was precipitated by adding 4M hydrochloric acid solution (1 mL, 4M/diethyl ether). The precipitated solid was filtered to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl bis(3-(dimethylamino)propyl)carbamate dihydrochloride (88 mg, 9.6%).
¹H NMR (400 MHz, DMSO-D₆) δ 10.33 (d, J = 40.8 Hz, 2H), 8.25 - 8.03 (m, 2H), 7.93 - 7.72 (m, 2H),7.10 (dd, J = 8.4, 1.1 Hz, 1H), 6.88 - 6.74 (m, 2H), 6.04 (td, J = 3.8, 1.8 Hz, 1H), 5.72 (d, J = 2.5 Hz,1H), 4.23 (t, J = 2.8 Hz, 2H), 3.35 (d, J = 6.8 Hz, 4H), 3.15 - 3.00 (m, 4H), 2.76 (d, J = 4.0 Hz, 12H),2.65 (s, 3H), 2.07 - 1.92 (m, 4H), 1.57 (d, J = 24.2 Hz, 6H)

### Example 87: Synthesis of N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methyl-L-alanine

### (a) Synthesis of tert-butyl N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methyl-L-alaninate

Except that tert-butyl (2*S*)-2-(methylamino)propanoate was used instead of tert-butyl *N-*methyl-*N*-[2-(methylamino)ethyl]carbamate, the same synthesis method as in Example 83 (a) above was carried out to obtain tert-butyl *N-*(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N*-methyl-*L*-alaninate (120 mg, 40%).

### (b) Synthesis of N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methyl-L-alanine

Except that tert-butyl *N*-(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N-*methyl-*L*-alaninate was used instead of tert-butyl (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*L*-argininate, the same synthesis method as in Example 78 (b) above was carried out to obtain *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N*-methyl-*L*-alanine (600 mg, 52%).
¹H NMR (500 MHz, DMSO-D₆) δ 9.48 (s, 1H), 8.16 - 8.09 (m, 2H), 7.82 - 7.72 (m, 2H), 6.87 (dd, J = 8.4, 1.1 Hz, 1H), 6.37 (dd, J = 8.4, 2.4 Hz, 1H), 6.25 (d, J = 2.4 Hz, 1H), 5.98 - 5.92 (m, 1H),5.63 - 5.58 (m, 1H), 4.39 (q, J = 7.1 Hz, 1H), 4.25 - 4.12 (m, 2H), 2.84 (s, 3H), 2.63 (s, 3H), 1.54 (s, 3H), 1.51 (s, 3H), 1.38 (d, J = 7.1 Hz, 3H)

### Example 88: Synthesis of sodium N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methyl-L-alaninate

*N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N*-methyl-*L*-alanine (530 mg, 0.97 mmol) obtained in Example 87 (b) above was suspended in water (20 mL), and sodium bicarbonate (82 mg, 0.98 mmol) was added thereto and stirred at room temperature for 3 hours. When the reaction was completed, the reaction mixture was diluted in water and washed with ethyl acetate. The separated aqueous layer was freeze-dried to obtain sodium *N*-(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N*-methyl-*L*-alaninate (510 mg , 88%).
¹H NMR (500 MHz, D₂O) δ 8.06 (d, J = 8.3 Hz, 2H), 7.65 (d, J = 8.3 Hz, 2H), 7.09 (t, J = 9.4 Hz, 1H), 6.88 - 6.71 (m, 2H), 5.97 (s, 1H), 5.72 (s, 1H), 4.28 - 4.15 (m, 2H), 3.45 (q, J = 6.9 Hz, 1H), 2.99 (d, J = 43.2 Hz, 3H), 2.67 (s, 3H), 2.58 (s, 2H), 1.61 (s, 3H), 1.57 (s, 3H), 1.40 (d, J = 7.0 Hz, 3H).

### Example 89: Synthesis of N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methylglycine

### (a) Synthesis of N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methylglycinate

Except that tert-butyl 2-(methylamino)acetate was used instead of tert-butyl *N*-methyl-*N-*[2-(methylamino)ethyl]carbamate, the same synthesis method as in Example 83 (a) above was carried out to obtain *N*-(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N-*methylglycinate (620 mg, 42%).

### (b) Synthesis of N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methylglycine

Except that *N-*(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N*-methylglycinate was used instead of tert-butyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-argininate, the same synthesis method as in Example 78 (b) above was carried out to obtain *N*-(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N*-methylglycine (220 mg, 58%).
¹H NMR (400 MHz, DMSO-D₆) δ 12.83 (s, 1H), 8.13 (d, J = 8.6 Hz, 2H), 7.84 - 7.77 (m, 2H), 7.13 - 7.05 (m, 1H), 6.78 - 6.66 (m, 1H), 6.66 - 6.54 (m, 1H), 6.01 (dt, J = 3.8, 2.4 Hz, 1H), 5.71 (s,1H), 4.21 (t, J = 2.5 Hz, 2H), 4.10 (s, 1H), 3.98 (s, 1H), 3.05 - 2.89 (amide tautomer, m, 3H), 2.64 (s, 3H), 1.59 (d, J = 1.8 Hz, 3H), 1.53 (s, 3H)

### Example 90: Synthesis of sodium N-(((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-N-methylglycinate

*N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N-*methylglycine (155 mg, 0.29 mmol) obtained in Example 89 (b) above was suspended in water (4 mL), and sodium bicarbonate (25 mg, 0.29 mmol) was added thereto and stirred at room temperature for 3 hours. When the reaction was completed, the reaction mixture was diluted in water and washed with ethyl acetate. The separated aqueous layer was freeze-dried to obtain sodium *N-*(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N*-methylglycinate (139 mg, 83%).
¹H NMR (500 MHz, D₂O) δ 7.95 - 7.88 (m, 2H), 7.58 - 7.52 (m, 2H), 7.00 (ddd, J = 23.8, 8.4, 1.1 Hz, 1H), 6.83 - 6.73 (m, 1H), 6.73 - 6.66 (m, 1H), 5.91 - 5.87 (m, 1H), 5.64 - 5.58 (m, 1H), 4.15 - 4.06 (m, 2H), 4.00 (s, 1H), 3.87 (d, J = 2.7 Hz, 1H), 3.05 (d, J= 59.3 Hz, 3H), 2.61 (s, 3H), 1.57 - 1.50 (m, 6H).

### Example 91: Synthesis of 3-((((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)(methyl)amino)propanoic acid

### (a) Synthesis of tert-butyl 3-((((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)(methyl)amino)propanoate

Except that tert-butyl 3-(methylamino)propanoate was used instead of tert-butyl *N-*methyl-*N*-[2-(methylamino)ethyl]carbamate, the same synthesis method as in Example 83 (a) above was carried out to obtain tert-butyl 3-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)(methyl)amino)propanoate (230 mg, 38%).

### (b) Synthesis of 3-((((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)(methyl)amino)propanoic acid

Except that tert-butyl 3-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)(methyl)amino)propanoate was used instead of tert-butyl (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*L*-argininate, the same synthesis method as in Example 78 (b) above was carried out to obtain 3-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)(methyl)amino)propanoic acid (90 mg, 50%).
¹H NMR (400 MHz, DMSO-D₆) δ 12.28 (s, 1H), 8.13 (d, J = 8.6 Hz, 2H), 7.84 - 7.77 (m, 2H), 7.08 (d, J = 8.4 Hz, 1H), 6.72 (dd, J = 8.4, 2.3 Hz, 1H), 6.67 (d, J = 2.7 Hz, 1H), 6.01 (s, 1H), 5.71 (s,1H), 4.24 - 4.19 (m, 2H), 3.59 (t, J = 7.2 Hz, 1H), 3.47 (t, J = 7.3 Hz, 1H), 3.02 - 2.87 (amide tautomer, m, 3H), 2.64 (s, 3H), 1.59 (s, 3H), 1.53 (s, 3H)

### Example 92: Synthesis of sodium 3-((((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)(methyl)amino)propanoate

Except that 3-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)(methyl)amino)propanoic acid was used instead of *N*-(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N*-methylglycine, the same synthesis method as in Example 90 above was carried out to obtain sodium 3-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)(methyl)amino)propanoate (92 mg, 87%).
¹H NMR (500 MHz, D₂O) δ 7.98 (dd, J = 8.5, 4.1 Hz, 2H), 7.59 (dd, J= 8.4, 4.5 Hz, 2H), 7.04 (d, J = 8.3 Hz, 1H), 6.78 - 6.69 (m, 2H), 5.93 (s, 1H), 5.66 (s, 1H), 4.19 - 4.12 (m, 2H), 3.70 (t, J = 7.2 Hz, 1H), 3.55 (t, J = 7.3 Hz, 1H), 3.03 (d, J = 59.9 Hz, 3H), 2.64 (s, 3H), 2.54 (t, J = 7.1 Hz, 1H), 2.45 (t, J = 7.3 Hz, 1H), 1.58 - 1.53 (m, 6H).

### Example 93: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(aminomethyl)piperidine-1-carboxylate trifluoroacetate

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(((tert-butoxycarbonyl)amino)methyl)piperidine-1-carboxylate

Except that tert-butyl *N-*(4-piperidylmethyl)carbamate was used instead of tert-butyl *N-*methyl-*N*-[2-(methylamino)ethyl]carbamate, the same synthesis method as in Example 83 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(((tert-butoxycarbonyl)amino)methyl)piperidine-1-carboxylate (250 mg, 50%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(aminomethyl)piperidine-1-carboxylate trifluoroacetate

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(((tert-butoxycarbonyl)amino)methyl)piperidine-1-carboxylate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-glutaminate, the same synthesis method as in Example 77 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(aminomethyl)piperidine-1-carboxylate trifluoroacetate (50 mg, 31%).
¹H NMR (400 MHz, DMSO-D₆) δ 8.17 - 8.10 (m, 2H), 7.86 - 7.76 (m, 4H), 7.08 (dt, J = 8.4, 1.2 Hz, 1H), 6.73 (dd, J = 8.5, 2.3 Hz, 1H), 6.67 (d, J = 2.3 Hz, 1H), 6.02 (q, J = 3.2 Hz, 1H), 5.73 - 5.68 (m, 1H), 4.22 (dd, J = 4.0, 2.0 Hz, 2H), 4.13 - 3.95 (m, 2H), 2.94 - 2.81 (m, 1H), 2.80 - 2.71 (m, 2H), 2.64 (s, 3H), 1.88 - 1.76 (m, 2H), 1.74 - 1.67 (m, 1H), 1.59 (s, 3H), 1.52 (s, 3H), 1.47 - 1.37 (m, 1H), 1.25 - 119 (m, 1H).

### Example 94: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(piperidin-4-yl)piperazine-1-carboxylate dihydrochloride

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(1-(tert-butoxycarbonyl)piperidin-4-yl)piperazine-1-carboxylate

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperazine-1-carboxylate hydrochloride and tert-butyl 4-oxopiperidine-1-carboxylate were used instead of 2-(4-acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione and 2-methoxyprop-1-ene, respectively, the same synthesis method as in Example 29 above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(1-(tert-butoxycarbonyl)piperidin-4-yl)piperazine-1-carboxylate (200 mg, 40%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(piperidin-4-yl)piperazine-1-carboxylate dihydrochloride

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(1-(tert-butoxycarbonyl)piperidin-4-yl)piperazine-1-carboxylate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl)piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(piperidin-4-yl)piperazine-1-carboxylate dihydrochloride (200 mg, 89%).
¹H NMR (400 MHz, DMSO-D₆) δ 11.70 (s, 1H), 9.07 (s, 1H), 8.85 (s, 1H), 8.18 - 8.09 (m, 2H), 7.87 - 7.72 (m, 2H), 7.10 (d, J = 8.2 Hz, 1H), 6.83 - 6.66 (m, 2H), 6.02 (q, J = 3.1 Hz, 1H), 5.71 (s, 1H), 4.27 - 4.10 (m, 3H), 3.69 - 3.42 (m, 7H), 3.22 - 3.11 (m, 2H), 2.95 - 2.85 (m, 2H), 2.64 (s, 3H), 2.34 - 2.23 (m, 2H), 2.04 - 1.85 (m, 2H), 1.60 (s, 3H), 1.53 (s, 3H).

### Example 95: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(pyridin-2-ylmethyl)piperazine-1-carboxylate dihydrochloride

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperazine-1-carboxylate hydrochloride (200 mg, 0.35 mmol) obtained in Example 67 (b) was dissolved in tetrahydrofuran, and sodium hydride (25 mg, 1.06 mmol, 60%) was added thereto and stirred at room temperature for 30 minutes. 2-(Chloromethyl)pyridine (87 mg, 0.53 mmol, HCl) was added thereto and stirred at room temperature for 12 hours. When the reaction was completed, the reaction mixture was quenched by adding water (5 mL) and extracted with dichloromethane. The separated organic layer was washed with aqueous sodium chloride solution, dried over sodium sulfate, filtered, and concentrated by distillation under reduced pressure. The obtained residue was separated by MPLC (ethyl acetate : hexane = 3 : 7, v/v) and concentrated by distillation under reduced pressure. The obtained residue was dissolved in ethyl acetate, and 4M hydrochloric acid solution (1 mL, 4M/dioxane) was added thereto and stirred for 1 hour. The obtained precipitate was filtered and washed with ethyl acetate to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(pyridin-2-ylmethyl)piperazine-1-carboxylate dihydrochloride (115 mg, 47%).
¹H NMR (400 MHz, DMSO-D₆) δ 8.72 - 8.68 (m, 1H), 8.16 - 8.11 (m, 2H), 7.96 (td, J = 7.7, 1.9 Hz, 1H), 7.82 - 7.77 (m, 2H), 7.62 (d, J = 7.7 Hz, 1H), 7.51 (dd, J = 7.7, 5.0 Hz, 1H), 7.10 (d, J = 8.4 Hz, 1H), 6.79 - 6.69 (m, 2H), 6.02 (q, J = 2.5, 1.8 Hz, 1H), 5.71 (d, J = 2.4 Hz, 1H), 4.52 (s, 2H), 4.21 (d, J = 3.3 Hz, 2H), 3.80 (d, J = 56.2 Hz, 8H), 2.64 (s, 3H), 1.59 (s, 3H), 1.52 (s, 3H).

### Example 96: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(2-(2-(2-methoxyethoxy)ethoxy)acetyl)piperazine-1-carboxylate

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl piperazine-1-carboxylate hydrochloride and 2-[2-(2-methoxyethoxy)ethoxy]acetic acid were used instead of 2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione and (2*S*)-5-amino-2-(tert-butoxycarbonylamino)-5-oxo-pentanoic acid, respectively, the same synthesis method as in Example 77 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(2-(2-(2-methoxyethoxy)ethoxy)acetyl)piperazine-1-carboxylate (200 mg, 78%).
¹H NMR (400 MHz, CDCl₃) δ 8.11 (d, J = 8.6 Hz, 2H), 7.88 -7.80 (m, 2H), 7.09 - 7.04 (m, 1H), 6.76 - 6.64 (m, 2H), 5.85(dt, J = 4.7, 2.2 Hz, 1H), 5.72 (d, J = 2.6 Hz, 1H), 4.33 - 4.27(m, 1H), 4.23 (s, 2H), 4.13 (dt, J = 16.7, 2.7 Hz, 1H), 3.72 -3.52 (m, 17H), 2.64 (s, 3H), 1.60 (d, J = 5.7 Hz, 6H).

### Example 97: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(L-valyl)piperazine-1-carboxylate formate

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-((tert-butoxycarbonyl)-L-valyl)piperazine-1-carboxylate

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperazine-1-carboxylate hydrochloride (200 mg, 0.38 mmol) obtained in Example 67 (b), (2*S*)-2-(tert-butoxycarbonylamino)-3-methyl-butanoic acid (91 mg, 0.41 mmol) and 2-(1*H*-benzotriazol-1-yl)-1,1,3,3-tetramethyluronium hexafluorophosphate (189 mg, 0.49 mmol) were dissolved in acetonitrile (5 mL), and triethylamine (156 mg, 1.52 mmol) was added dropwise thereto and stirred at room temperature for 2 hours. When the reaction was completed, the reaction mixture was concentrated by distillation under reduced pressure, and the obtained residue was separated by MPLC (ethyl acetate : hexane = 3 : 7, v/v) to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-((tert-butoxycarbonyl)-*L-*valyl)piperazine-1-carboxylate (200 mg, 73%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(L-valyl)piperazine-1-carboxylate formate

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl4-((tert-butoxycarbonyl)-*L*-valyl)piperazine-1-carboxylate (200 mg, 0.27 mmol) obtained in Example 97 (a) above was dissolved in dichloromethane (1 mL), and 4M hydrochloric acid solution (0.6 mL, 4M/dioxane) was added dropwise thereto and stirred at room temperature for 3 hours. When the reaction was completed, the reaction mixture was concentrated by reduced pressure distillation, and the obtained residue was separated by reverse-phase chromatography (acetonitrile containing 0.1% formic acid : water containing 0.1% formic acid = 5 : 95-100 : 0) and freeze-dried to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(*L-*valyl)piperazine-1-carboxylate formate (179 mg, 94%).
¹H NMR (400 MHz, DMSO-D₆) δ 8.24 (amine, d, J = 1.5 Hz, 2H), 8.17 - 8.09 (m, 2H), 7.84 - 7.76 (m, 2H), 7.10 (dd, J = 8.3, 1.1 Hz, 1H), 6.76 (dd, J = 8.4, 2.3 Hz, 1H), 6.71 (d, J = 2.3 Hz, 1H), 6.02 (td, J = 3.7, 1.8 Hz, 1H), 5.71 (d, J = 2.5 Hz, 1H), 4.22 (dd, J = 3.8, 2.0 Hz, 2H), 3.73 (d, J = 5.2 Hz, 1H), 3.69 - 3.36 (m, 8H), 2.69 (s, 1H), 2.54 (s, 1H), 1.81 (dt, J = 13.1, 6.7 Hz, 1H), 1.59 (s, 3H), 1.53 (s, 3H), 0.92 (d, J = 6.8 Hz, 3H), 0.85 (d, J = 6.7 Hz, 3H).

### Example 98: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(L-leucyl)piperazine-1-carboxylate hydrochloride

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-((tert-butoxycarbonyl)-L-leucyl)piperazine-1-carboxylate

Except that (2*S*)-2-(tert-butoxycarbonylamino)-4-methyl-pentanoic acid was used instead of (2*S*)-2-(tert-butoxycarbonylamino)-3-methyl-butanoic acid, the same synthesis method as in Example 97 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-((tert-butoxycarbonyl)-*L*-leucyl)piperazine-1-carboxylate (200 mg, 76%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(L-leucyl)piperazine-1-carboxylate hydrochloride

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-((tert-butoxycarbonyl)-*L-*leucyl)piperazine-1-carboxylate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl) piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-leucyl)piperazine-1-carboxylate hydrochloride (120 mg, 88%).
¹H NMR (400 MHz, DMSO-D₆) δ 8.20 (s, 3H), 8.16 - 8.12 (m, 2H), 7.84 - 7.76 (m, 2H), 7.10 (dd, J = 8.5, 1.1 Hz, 1H), 6.76 (dd, J = 8.4, 2.4 Hz, 1H), 6.72 (d, J = 2.3 Hz, 1H), 6.02 (q, J = 3.4 Hz, 1H), 5.72 (d, J = 2.3 Hz, 1H), 4.39 (s, 1H), 4.25 - 4.20 (m, 2H), 3.81 - 3.71 (m, 2H), 3.65 - 3.58 (m, 2H), 3.43 - 3.24 (m, 4H), 2.64 (s, 3H), 1.77 - 1.71 (m, 1H), 1.60 (s, 3H), 1.53 (s, 3H), 1.50 - 1.42 (m, 2H), 0.94 (d, J = 6.5 Hz, 3H), 0.90 (d, J = 6.5 Hz, 3H).

### Example 99: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(dimethylglycyl)piperazine-1-carboxylate hydrochloride

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl piperazine-1-carboxylate hydrochloride was used instead of 2-(4-acetylphenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione, the same synthesis method as in Example 72 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(dimethylglycyl)piperazine-1-carboxylate hydrochloride (320 mg, 67%).
¹H NMR (400 MHz, D₂O) δ 8.19 (d, J = 8.4 Hz, 2H), 7.77 (d, J = 8.5 Hz, 2H), 7.21 (d, J = 8.4 Hz, 1H), 6.89 (d, J = 8.2 Hz, 2H), 6.10 (s, 1H), 5.86 (s, 1H), 4.43 (s, 2H), 4.34 (s, 2H), 3.88 - 3.63 (m, 8H), 3.08 (s, 6H), 2.79 (s, 3H), 1.70 (d, J = 18.4 Hz, 6H).

### Example 100: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2,6-dimethylpiperazine-1-carboxylate hydrochloride

### (a) Synthesis of 1-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl) 4-(tert-butyl) 2,6-dimethylpiperazine-1,4-dicarboxylate

Except that tert-butyl 4-chlorocarbonyl-3,5-dimethyl-piperazine-1-carboxylate was used instead of trimethylacetyl chloride, the same synthesis method as in Example 58 above was carried out to obtain 1-(2-(4-acetylphenyl)-7,7-dimethyl-1,3-dioxo-2,3,5,12b-tetrahydro-1*H*,7*H-*chromeno[4,3-c][1,2,4]triazolo[1,2-*a*]pyridazin-10-yl) 4-(tert-butyl) 2,6-dimethylpiperazine-1,4-dicarboxylate (700 mg, 88%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2,6-dimethylpiperazine-I-carboxylate hydrochloride

Except that 1-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl) 4-(tert-butyl) 2,6-dimethylpiperazine-1,4-dicarboxylate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl) piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethylpiperazine-I-carboxylate hydrochloride (180 mg, 88%).
¹H NMR (500 MHz, DMSO-D₆) δ 9.31 (s, 1H), 8.13 (d, J = 8.6 Hz, 2H), 7.80 (d, J = 8.7 Hz, 2H), 7.10 (dd, J = 8.3, 1.1 Hz, 1H), 6.75 (dd, J = 8.4, 2.3 Hz, 1H), 6.71 (d, J = 2.3 Hz, 1H), 6.02 (td, J = 3.7, 1.8 Hz, 1H), 5.75 - 5.69 (m, 1H), 4.53 - 4.45 (m, 1H), 4.40 (t, J = 6.5 Hz, 2H), 4.22 (dd, J = 3.9, 2.0 Hz, 2H), 3.46 - 3.42 (m, 2H), 3.04 (t, J = 12.6 Hz, 2H), 2.63 (s, 3H), 1.59 (s, 3H), 1.53 (s, 3H), 1.30 (d, J = 6.6 Hz, 6H).

### Example 101: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2,6-dimethyl-4-prolylpiperazine-1-carboxylate hydrochloride

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-((tert-butoxycarbonyl)prolyl)-2,6-dimethylpiperazine-1-carboxylate

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethylpiperazine-I-carboxylate hydrochloride and 1-tert-butoxycarbonylpyrrolidine-2-carboxylic acid were used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperazine-1-carboxylate hydrochloride and (2*S*)-2-(tert-butoxycarbonylamino)-3-methyl-butanoic acid, respectively, the same synthesis method as in Example 97 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-((tert-butoxycarbonyl)prolyl)-2,6-dimethylpiperazine-1-carboxylate (100 mg, 70%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2,6-dimethyl-4-prolylpiperazine-1-carboxylate hydrochloride

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-((tert-butoxycarbonyl)prolyl)-2,6-dimethylpiperazine-1-carboxylate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl) piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethyl-4-prolylpiperazine-1-carboxylate hydrochloride (78 mg, 88%).
¹H NMR NMR (500 MHz, D₂O) δ 7.99 (d, J = 8.2 Hz, 2H), 7.59 (d, J = 8.3 Hz, 2H), 7.06 (d, J = 8.4 Hz, 1H), 6.75 (s, 1H), 6.71 (s, 1H), 5.96 (s, 1H), 5.69 (s, 1H), 4.22 (s, 1H), 4.17 (s, 2H), 3.76 (dd, J = 24.6, 12.7 Hz, 1H), 3.53 - 3.37 (m, 4H), 3.09 - 3.01 (m, 1H), 2.65 (s, 3H), 2.54 (dd, J = 13.8, 7.0 Hz, 1H), 2.24 - 1.92 (m, 5H), 1.56 (d, J = 9.4 Hz, 6H), 1.34 (s, 3H), 1.28 - 1.24 (m, 3H).

### Example 102: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-ylheptanoate

Except that heptanoyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-ylheptanoate (100 mg, 92%).
¹H NMR (400 MHz, CDCl₃) δ 8.15 - 8.07 (m, 2H), 7.88 - 7.80 (m, 2H),7.08 (dd, J = 8.3, 1.1 Hz, 1H), 6.73 - 6.60 (m, 2H), 5.86 (dt, J = 4.7, 2.3Hz, 1H), 5.73 (d, J = 2.5 Hz, 1H), 4.30 (ddd, J = 16.5, 4.8, 1.3 Hz, 1H),4.13 (dt, J = 16.6, 2.7 Hz, 1H), 2.64 (s, 3H), 2.52 (t, J = 7.5 Hz, 2H), 1.72(p, J = 7.4 Hz, 2H), 1.60 (d, J = 4.5 Hz, 6H), 1.42 - 1.29 (m, 6H), 0.93 -0.86 (m, 3H).

### Example 103: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2,2-dimethylbutanoate

Except that 2,2-dimethylbutanoyl chloride was used instead of trimethylacetyl chloride, the same synthesis method as in Example 58 above was carried out to obtain 2-(4-acetylphenyl)-7,7-dimethyl-1,3-dioxo-2,3, 5,12b-tetrahydro-1*H*,7*H*-chromeno[4,3-*c*][1,2,4]triazolo[1,2-*a*]pyridazin-10-yl 2,2-dimethylbutanoate (115 mg, 95%).
¹H NMR (400 MHz, CDCl₃) δ 8.14 - 8.07 (m, 2H), 7.86 - 7.80 (m, 2H), 7.11 - 7.04 (m, 1H), 6.66 - 6.59 (m, 2H), 5.88 - 5.82 (m, 1H), 5.73 (s, 1H), 4.34 - 4.26 (m, 1H), 4.13 (dt, J = 16.4, 2.6 Hz, 1H), 2.65 (d, J = 0.6 Hz, 3H), 1.69 (q, J = 7.5 Hz, 2H), 1.60 (d, J = 3.6 Hz, 6H), 1.55 (s, 3H), 1.29 - 1.25 (m, 6H), 0.98 - 0.90 (m, 3H).

### Example 104: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl ethyl succinate

Except that ethyl 4-chloro-4-oxo-butanoate was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl ethyl succinate (120 mg, 92%).
¹H NMR (400 MHz, DMSO-D₆) δ 8.17 - 8.09 (m, 2H), 7.84 - 7.77 (m, 2H), 7.13 (dd, J = 8.4, 1.1 Hz, 1H), 6.71 (dd, J = 8.4, 2.3 Hz, 1H), 6.64 (d, J = 2.2 Hz, 1H), 6.02 (td, J = 3.7, 1.8 Hz, 1H), 5.75- 5.69 (m, 1H), 4.22 (dd, J = 3.8, 2.0 Hz, 2H), 4.08 (q, J = 7.1 Hz, 2H), 2.80 (dd, J = 7.6, 5.4 Hz, 2H), 2.67 - 2.62 (m, 5H), 1.60 (s, 3H), 1.53 (s, 3H), 1.18 (t, J = 7.1 Hz, 3H).

### Example 105: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl diisopropylcarbamate

Except that *N*,*N*-diisopropylcarbamoyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diisopropylcarbamate (80 mg, 95%).
¹H NMR (400 MHz, DMSO-D₆) δ 8.16 - 8.10 (m, 2H), 7.82 - 7.77 (m, 2H), 7.12 - 7.03 (m,1H), 6.70 (dd, J = 8.4, 2.3 Hz, 1H), 6.62 (d, J = 2.3 Hz, 1H), 6.02 (q, J = 3.2 Hz, 1H), 5.71(s, 1H), 4.21 (t, J = 2.8 Hz, 2H), 4.02 - 3.89 (m, 2H), 2.63 (s, 3H), 1.56 (d, J = 21.8 Hz,6H), 1.20 (d, J = 19.9 Hz, 14H).

### Example 106: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl isopropyl carbonate

Except that isopropyl chloroformate 1M solution (1M/toluene) was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl isopropyl carbonate (120 mg, 93%).
¹H NMR (400 MHz, DMSO-D₆) δ 8.17 - 8.09 (m, 2H), 7.83 -7.76 (m, 2H), 7.12 (dd, J = 8.4, 1.1 Hz, 1H), 6.91 - 6.72 (m,2H), 6.02 (td, J = 3.7, 1.8 Hz, 1H), 5.73 (d, J = 1.9 Hz, 1H),4.84 (p, J = 6.2 Hz, 1H), 4.21 (dd, J = 3.8, 2.0 Hz, 2H), 2.63(s, 3H), 1.59 (s, 3H), 1.53 (s, 3H), 1.29 (d, J = 6.2 Hz, 6H).

### Example 107: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (2-((2-hydroxyethyl)disulfanyl)ethyl) carbonate

Except that 2-hydroxyethyl disulfide was used instead of tert-butyl *N-*methyl-*N-*[2-(methylamino)ethyl]carbamate, the same synthesis method as in Example 83 (a) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-((2-hydroxyethyl)disulfanyl)ethyl) carbonate (830 mg, 58%).
¹H NMR (400 MHz, CDCl₃) δ 8.14 - 7.99 (m, 2H), 7.94 - 7.70 (m, 2H), 7.09 (dd, J = 8.5, 1.1 Hz, 1H), 6.88 - 6.66 (m, 2H), 5.87 (dt, J = 4.9, 2.3 Hz, 1H), 5.73 (d, J = 2.3 Hz, 1H), 4.48 (td, J = 6.7, 1.1 Hz, 2H), 4.37 (t, J = 6.5 Hz, 2H), 4.31 (ddd, J = 16.1, 4.7, 1.2 Hz, 1H), 4.14 (dt, J = 16.3, 2.7 Hz, 1H), 3.03 - 2.93 (m, 4H), 2.64 (d, J = 2.7 Hz, 6H), 1.61 (d, J = 6.3 Hz, 6H).

### Example 108: Synthesis of 4-(2-((2-((((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)oxy)ethyl)disulfanyl)ethoxy)-4-oxobutanoic acid

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (2-((2-hydroxyethyl)disulfanyl)ethyl) carbonate (700 mg, 1.17 mmol) obtained in Example 107 above and 4-dimethylaminopyridine (71 mg, 0.58 mmol) were dissolved in dichloromethane (30 mL), and tetrahydrofuran-2,5-dione (467 mg, 4.67 mmol) was added thereto, and heated and stirred at 35°C for 1 hour. When the reaction was completed, the reaction mixture was diluted in dichloromethane and washed with aqueous ammonium chloride solution. The separated organic layer was dried over sodium sulfate, filtered, concentrated by distillation under reduced pressure, and separated by MPLC (ethyl acetate : hexane = 3 : 1, v/v) to obtain 4-(2-((2-((((2-(4-acetylphenyl)-7,7-dimethyl-1,3-dioxo-2,3,5,12b-tetrahydro-1H, *7H-*chromeno[4,3-c][1,2,4]triazolo[1,2-*a*]pyridazin-10-yl)oxy)carbonyl)oxy)ethyl)disulfanyl)ethoxy)-4-oxobutanoic acid (0.81 g, 99%).
¹H NMR (400 MHz, DMSO-D₆) δ 8.13 (d, J = 8.6 Hz, 2H), 7.80 (d, J = 8.1 Hz, 2H), 7.14 (d, J = 8.4 Hz, 1H), 6.85 (dd, J = 8.3, 2.4 Hz, 1H), 6.81 (d, J = 2.4 Hz, 1H), 6.05 - 5.99 (m, 1H), 5.73 (s, 1H), 4.43 (t, J = 6.2 Hz, 2H), 4.21 (d, J = 6.7 Hz, 4H), 3.08 (t, J = 6.2 Hz, 2H), 2.98 (t, J = 6.5 Hz, 2H), 2.63 (s, 3H), 2.38 (d, J = 6.4 Hz, 2H), 2.13 (t, J = 7.8 Hz, 2H), 1.60 (s, 3H), 1.54 (s, 3H).

### Example 109: Synthesis of sodium 4-(2-((2-((((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)oxy)ethyl)disulfanyl)ethoxy )-4-oxobutanoate

Except that 4-(2-((2-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)oxy)ethyl)disulfanyl)ethoxy)-4-oxobutanoic acid was used instead of *N-*(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N-*methylglycine, the same synthesis method as in Example 90 above was carried out to obtain sodium 4-(2-((2-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)oxy)ethyl)disulfanyl)ethoxy )-4-oxobutanoate (100 mg, 90%).
¹H NMR (400 MHz, DMSO-D₆) δ 8.13 (d, J = 8.6 Hz, 2H), 7.80 (d, J = 8.1 Hz, 2H), 7.14 (d, J = 8.4 Hz, 1H), 6.85 (dd, J = 8.3, 2.4 Hz, 1H), 6.81 (d, J = 2.4 Hz, 1H), 6.05 - 5.99 (m, 1H), 5.73 (s, 1H), 4.43 (t, J = 6.2 Hz, 2H), 4.21 (d, J = 6.7 Hz, 4H), 3.08 (t, J = 6.2 Hz, 2H), 2.98 (t, J = 6.5 Hz, 2H), 2.63 (s, 3H), 2.38 (d, J = 6.4 Hz, 2H), 2.13 (t, J = 7.8 Hz, 2H), 1.60 (s, 3H), 1.54 (s, 3H)

### Example 110: Synthesis of 2-(4-acetylphenyl)-10-(2-hydroxyethoxy)-7,7-dimethyl-5,12b-dihydro-1H,7H-chromeno[4,3-c][1.2.4]triazolo[1.2-a]pyridazine-1.3(2H)-dione

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (500 mg, 1.19 mmol) obtained in Example 10 (b) above and potassium carbonate (752 mg, 3.58 mmol) were suspended in acetone, and 2-bromoethanol (298 mg, 2.38 mmol) was added to the reaction mixture, and heated and stirred at 60°C for 24 hours. When the reaction was completed, acetone was removed by distillation under reduced pressure. The obtained residue was diluted in ethyl acetate and washed with water. The separated organic layer was dried over sodium sulfate, filtered, concentrated by distillation under reduced pressure, and separated by MPLC (ethyl acetate : hexane = 1 : 1, v/v) to obtain 2-(4-acetylphenyl)-10-(2-hydroxyethoxy)-7,7-dimethyl-5,12b-dihydro-1*H*,7*H*-chromeno[4,3-*c*][1,2,4]triazolo[1,2-*a*]pyridazine-1,3(2*H*)-dione (478 mg, 87%).
¹H NMR (500 MHz, DMSO-D₆) δ 8.13 (d, J = 8.0 Hz, 2H), 7.79 (d, J = 7.7 Hz, 2H), 6.99 (d, J = 8.9 Hz, 1H), 6.54 (d, J = 8.4 Hz, 1H), 6.44 (d, J = 3.0 Hz, 1H), 5.99 (d, J = 4.1 Hz, 1H), 5.66 (s, 1H), 4.20 (d, J = 3.7 Hz, 2H), 3.92 (q, J = 4.1 Hz, 2H), 3.67 (s, 2H), 2.64 (d, J = 2.5 Hz, 3H), 1.55 (d, J = 16.2 Hz, 6H)

### Example 111: Synthesis of 2-((2-(4-acetylphenyl)-7,7-dimethyl-1,3-dioxo-2,3,5,12b-tetrahydro-1H,7H-chchromeno[4,3-c][1,2,4]triazolo[1,2-a]pyridazin-10-yl)oxy)ethyl L-leucinate hydrochloride

### (a) Synthesis of 2-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)ethyl (tert-butoxycarbonyl)-L-leucinate

Except that 2-(4-acetylphenyl)-10-(2-hydroxyethoxy)-7,7-dimethyl-5,12b-dihydro-1*H*,7*H*-chromeno[4,3-*c*][1,2,4]triazolo[1,2-*a*]pyridazine-1,3(2*H*)-dione and *N*-(tert-butoxycarbonyl)-L-leucine monohydrate were used instead of 2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione and (2*S*)-5-amino-2-(tert-butoxycarbonylamino)-5-oxo-pentanoic acid, respectively, the same synthesis method as in Example 77 (a) above was carried out to obtain 2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)ethyl (tert-butoxycarbonyl)-*L*-leucinate (98 mg, 62%).

### (b) Synthesis of 2-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)ethyl L-leucinate hydrochloride

Except that 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)ethyl (tert-butoxycarbonyl)-L-leucinate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*c*]pyridazin-10-yl) 4-(tert-butyl) piperazine-1,4-dicarboxylate, the same synthesis method as in Example 67 (b) above was carried out to obtain 2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)ethyl *L*-leucinate hydrochloride (70 mg, 80%).
¹H NMR (500 MHz, DMSO-D₆) δ 8.51 (s, 3H), 8.17 - 8.11 (m, 2H), 7.82 - 7.76 (m, 2H), 7.01 (d, J = 8.5 Hz, 1H), 6.55 (dd, J = 8.6, 2.5 Hz, 1H), 6.47 (d, J = 2.4 Hz, 1H), 6.00 (d, J = 2.5 Hz, 1H), 5.67 (s, 1H), 4.54 (dq, J= 13.0, 4.5 Hz, 1H), 4.44 - 4.39 (m, 1H), 4.20 (q, J = 4.1 Hz, 4H), 3.98 (s, 1H), 2.64 (s, 3H), 1.75 (dt, J = 13.4, 6.8 Hz, 1H), 1.62 (td, J = 7.2, 2.6 Hz, 2H), 1.57 (s, 3H), 1.53 (s, 3H), 0.87 - 0.84 (m, 6H).

### Example 112: Synthesis of 2-(4-acetylphenyl)-7,7-dimethyl-10-(2-(piperidin-1-yl)ethoxy)-5,12b-dihydro-1H,7H-chromeno[4,3-c][1,2,4]triazolo[1,2-a]pyridazine-1,3(2H)-dione hydrochloride

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (300 mg, 0.72 mmol) obtained in Example 10 (b) above and potassium carbonate (395 mg, 2.86 mmol) were suspended in acetone, and 1-(2-chloroethyl)piperidine (158 mg, 0.86 mmol, HCl) was added thereto and stirred at 70°C for 5 hours. When the reaction was completed, acetone was removed by distillation under reduced pressure, and the obtained residue was diluted in dichloromethane and washed with water. The separated organic layer was dried over sodium sulfate, filtered, concentrated by distillation under reduced pressure, separated by MPLC (ethyl acetate : hexane = 1 : 1, v/v), and concentrated by distillation under reduced pressure. The obtained residue was dissolved in ethyl acetate (3 mL), and 4M hydrochloric acid solution (2 mL, 4M/dioxane) was added dropwise thereto and stirred at room temperature for 30 minutes. The obtained solid precipitate was filtered and washed with ethyl acetate to obtain 2-(4-acetylphenyl)-7,7-dimethyl-10-(2-(piperidin-1-yl)ethoxy)-5,12b-dihydro-1*H*,7*H*-chromeno[4,3-*c*][1,2,4]triazolo[1,2-*a*]pyridazine-1,3(2*H*)-dione hydrochloride (120 mg, 30%).
¹H NMR (500 MHz, DMSO-D₆) δ 10.14 (s, 1H), 8.11 (d, J = 8.6Hz, 2H), 7.77 (d, J = 8.6 Hz, 2H), 7.02 (d, J = 8.5 Hz, 1H), 6.59(dd, J = 8.6, 2.5 Hz, 1H), 6.53 (d, J = 2.5 Hz, 1H), 5.99 (d, J =2.0 Hz, 1H), 5.66 (s, 1H), 4.33 (t, J = 5.1 Hz, 2H), 4.19 (t, J =2.9 Hz, 2H), 3.49 - 3.38 (m, 6H), 3.02 - 2.85 (m, 2H), 2.62 (s,3H), 1.82 - 1.62 (m, 5H), 1.54 (d, J = 13.7 Hz, 6H).

### Example 113: Synthesis of 2-(4-acetylphenyl)-7,7-dimethyl-10-(2-morpholinoethoxy)-5,12b-dihydro-1H,7H-chromeno[4,3-c][1,2,4]triazolo[1,2-a]pyridazine-1,3(2H)-dione formate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (560 mg, 1.34 mmol) obtained in Example 10 (b) above was dissolved in *N*,*N*-dimethylformamide (10 mL), and sodium hydride (64 mg, 2.67 mmol, 60%) was added thereto and stirred at room temperature for 30 minutes. 4-(2-Chloroethyl)morpholine hydrochloride (298 mg, 1.6 mmol) was added to the reaction mixture and stirred at room temperature for 15 hours. When the reaction was completed, the reaction mixture was quenched by adding water (5 mL) and extracted with dichloromethane. The separated organic layer was washed with aqueous sodium chloride solution, dried over sodium sulfate, filtered, and concentrated by distillation under reduced pressure. The obtained residue was separated by reverse phase chromatography (acetonitrile containing 0.1% formic acid : water containing 0.1% formic acid = 5 : 95-100 : 0) and freeze-dried to obtain 2-(4-acetylphenyl)-7,7-dimethyl-10-(2-morpholinoethoxy)-5,12b-dihydro-1*H*,7*H*-chromeno[4,3-*c*][1,2,4]triazolo[1,2-*a*]pyridazine-1,3(2*H*)-dione formate (60 mg, 70%).
¹H NMR (500 MHz, CDCl₃) δ 8.11 (d, J = 8.7 Hz, 2H), 7.83 (d, J = 8.7 Hz, 2H), 7.09 - 7.05 (m, 1H), 6.72 - 6.67 (m, 2H), 5.86 (dt, J = 4.6, 2.3 Hz, 1H), 5.72 (s, 1H), 4.34 - 4.24 (m, 1H), 4.14 (dt, J = 16.5, 2.6 Hz, 1H), 3.64 (d, J = 60.0 Hz, 9H), 2.65 (s, 3H), 2.47 (s, 3H), 1.61 (s, 3H), 1.59 (s, 3H).

### Example 114: Synthesis of (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)methyl)phosphonic acid

### (a) Synthesis of diethyl (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)methyl)phosphonate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (300 mg, 0.72 mmol) obtained in Example 10 (b) above was dissolved in *N*,*N*-dimethylformamide (8 mL), and sodium hydride (17 mg, 0.72 mmol, 60%) was added thereto and stirred at room temperature for 1 hour. Diethoxyphosphorylmethyl 4-methylbenzenesulfonate (346 mg, 1.07 mmol) was added to the reaction mixture and stirred at room temperature under nitrogen atmosphere for 15 hours. When the reaction was completed, the reaction mixture was diluted in ethyl acetate and washed with aqueous sodium chloride solution. The separated organic layer was dried over sodium sulfate, filtered, and concentrated by distillation under reduced pressure to quantitatively obtain diethyl (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)methyl)phosphonate. The obtained compound was used in the next reaction without purification.

### (b) Synthesis of (((2-(4-acetylphenyl)-7,7-dimethyl-1,3-dioxo-2,3,5,12b-tetrahydro-1H,7H-chromeno[4,3-c][1,2,4]triazolo[1,2-c]pyridazin-10-yl)oxy)methyl)phosphonic acid

Except that diethyl (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)methyl)phosphonate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diethyl phosphate, the same synthesis method as in Example 69 (b) above was carried out to obtain (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)methyl)phosphonic acid (70 mg, 50%).
¹H NMR (500 MHz, DMSO-D₆) δ 8.09 (d, J = 8.4 Hz, 2H), 7.71 (d, J = 8.1 Hz, 2H), 7.23 (d, J = 14.2 Hz, 1H), 7.03 (d, J = 8.7 Hz, 1H), 6.61 (s, 1H), 6.51 (d, J = 8.4 Hz, 1H), 6.42 (s, 1H), 5.87 (d, J = 14.2 Hz, 1H), 4.05 (d, J = 10.1 Hz, 2H), 2.62 (s, 3H), 1.43 (s, 6H)

### Example 115: Synthesis of disodium (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)methyl)phosphonate

Except that (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)methyl)phosphonic acid was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dihydrogen phosphate, the same synthesis method as in Example 70 above was carried out to obtain disodium (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)methyl)phosphonate.
¹H NMR (500 MHz, D₂O) δ 8.17 - 8.10 (m, 2H), 7.60 - 7.54 (m, 2H), 7.30 (d, J = 14.0 Hz, 1H), 7.10 (d, J = 8.4 Hz, 1H), 6.68 (dd, J = 8.4, 2.5 Hz, 1H), 6.61 (s, 1H), 6.56 (d, J = 2.5 Hz, 1H), 6.11 (dd, J = 14.1, 1.0 Hz, 1H), 3.99 (d, J = 9.8 Hz, 3H), 2.71 (s, 3H), 1.53 (s, 7H)

### Example 116: Synthesis of 2-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)ethyl dimethylglycinate hydrochloride

Except that 2-(4-acetylphenyl)-10-(2-hydroxyethoxy)-7,7-dimethyl-5,12b-dihydro-1*H*,7*H*-chromeno[4,3-*c*][1,2,4]triazolo[1,2-*a*]pyridazine-1,3(2*H*)-dione was used instead of 2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione, the same synthesis method as in Example 72 above was carried out to obtain 2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)ethyl dimethylglycinate hydrochloride (98 mg, 62%).
¹H NMR (500 MHz, DMSO-D₆) δ 10.35 (s, 1H), 8.14 (d, J = 7.9Hz, 2H), 7.79 (q, J = 5.9, 4.5 Hz, 2H), 7.05 - 6.96 (m, 1H),6.57 (d, J = 8.9 Hz, 1H), 6.49 (s, 1H), 6.00 (s, 1H), 5.67 (s,1H), 4.48 (s, 2H), 4.32 - 4.12 (m, 6H), 2.83 (t, J = 3.4 Hz,6H), 2.63 (d, J = 4.4 Hz, 3H), 1.61 - 1.46 (m, 6H).

### Example 117: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(1-(2-oxopropanoyl)piperidin-4-yl)piperazine-1-carboxylate hydrochloride

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(piperidin-4-yl)piperazine-1-carboxylate dihydrochloride (201 mg, 0.29 mmol) obtained in Example 94 (b) above, pyruvic acid (32 mg, 0.35 mmol) and 2-(1*H*-benzotriazol-1-yl)-1,1,3,3-tetramethyluronium hexafluorophosphate (225 mg, 0.58 mmol) were dissolved in dichloromethane (4 mL), and this reaction mixture was added dropwise to triethylamine (131 mg, 0.58 mmol) and stirred at room temperature for 19 hours. When the reaction was completed, the reaction mixture was concentrated by distillation under reduced pressure, separated by MPLC (ethyl acetate : hexane = 3 : 7, v/v), and concentrated by distillation under reduced pressure. The obtained residue was dissolved in dichloromethane (1 mL), and 1M hydrochloric acid solution (1 mL, 1M/diethyl ether) was added dropwise thereto and stirred at room temperature for 30 minutes. The obtained solid precipitate was filtered and washed with diethyl ether to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(1-(2-oxopropanoyl)piperidin-4-yl)piperazine-1-carboxylate hydrochloride (51 mg, 0.07 mmol).
¹H NMR (400 MHz, DMSO-D₆) δ 11.18 (s, 1H), 8.17 - 8.10 (m, 2H), 7.83 - 7.76 (m, 2H), 7.10 (d, J = 8.2 Hz, 1H), 6.81 - 6.73 (m, 2H), 6.02 (td, J = 3.8, 1.8 Hz, 1H), 5.71 (d, J = 2.5 Hz, 1H), 4.39 (d, J = 13.2 Hz, 1H), 4.31 - 4.18 (m, 3H), 4.12 (s, 1H), 3.77 (d, J = 13.7 Hz, 1H), 3.54 (s, 2H), 3.48 (d, J = 12.8 Hz, 3H), 3.12 (dd, J = 24.6, 12.6 Hz, 3H), 2.82 - 2.70 (m, 1H), 2.64 (s, 3H), 2.38 (s, 3H), 2.18 (dd, J = 22.4, 12.3 Hz, 2H), 1.64 (s, 2H), 1.60 (s, 3H), 1.52 (s, 3H).

### Example 118: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2,2-dimethylpiperazine-1-carboxylate hydrochloride

### (a) Synthesis of 1-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl) 4-(tert-butyl) 2,2-dimethylpiperazine-1,4-dicarboxylate

Except that tert-butyl 4-(chlorocarbonyl)-3,3-dimethylpiperidine-1-carboxylate was used instead of trimethylacetyl chloride, the same synthesis method as in Example 58 above was carried out to obtain 1-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl) 4-(tert-butyl) 2,2-dimethylpiperazine-1,4-dicarboxylate (200 mg, 78%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2,2-dimethylpiperazine-1-carboxylate hydrochloride

Except that 1-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl) 4-(tert-butyl) 2,2-dimethylpiperazine-1,4-dicarboxylate was used instead of 1-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 4-(tert-butyl) piperazine-1,4-dicarboxylate, the same synthesis method as in Example 67 (b) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,2-dimethylpiperazine-1-carboxylate hydrochloride (100 mg, 80%).
¹H NMR (500 MHz, D₂O) δ 8.08 (d, J = 8.4 Hz, 2H), 7.64 (d, J = 8.3 Hz, 2H), 7.09 (d, J = 8.1 Hz, 1H), 6.76 (d, J = 8.9 Hz, 2H), 5.97 (s, 1H), 5.73 (s, 1H), 3.73 (d, J = 1.4 Hz, 3H), 3.59 - 3.53 (m, 4H), 3.46 (t, J = 5.6 Hz, 4H), 3.43 (s, 2H), 3.37 (s, 3H), 3.28 (s, 2H), 1.55 (s, 6H).

### Example 119: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 4-(dimethylglycyl)-2,2-dimethylpiperazine-1-carboxylate hydrochloride

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,2-dimethylpiperazine-1-carboxylate hydrochloride was used instead of 2-(4-acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione, the same synthesis method as in Example 72 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 4-(dimethylglycyl)-2,2-dimethylpiperazine-1-carboxylate hydrochloride (50 mg, 74%).
¹H NMR (500 MHz, DMSO-D₆) δ 9.70 (d, J = 40.2 Hz, 1H), 8.15 - 8.10 (m, 2H), 7.81 - 7.77 (m, 2H), 7.09 (dd, J = 8.4, 3.2 Hz, 1H), 6.72 (ddd, J = 8.5, 6.5, 2.3 Hz, 1H), 6.66 (dd, J = 11.1, 2.3 Hz, 1H), 6.03 (dt, J = 5.3, 2.4 Hz, 1H), 5.72 (s, 1H), 4.28 (dd, J = 10.4, 4.9 Hz, 2H), 4.22 (t, J = 2.8 Hz, 2H), 3.87 (dt, J = 21.6, 6.0 Hz, 2H), 3.56 (dt, J = 19.4, 5.8 Hz, 3H), 2.83 (d, J = 4.6 Hz, 6H), 2.64 (s, 3H), 1.59 (s, 3H), 1.53 (s, 3H), 1.41 (d, J = 12.6 Hz, 6H).

### Example 120: Synthesis of 2-(4-acetylphenyl)-10-(3-(4-(3-chlorophenyl)piperazin-1-yl)propoxy)-7,7-dimethyl-5,12b-dihydro-1H,7H-chromeno[4,3-c][1,2,4]triazolo[1,2-a]pyridazine-1,3(2H)-dione dihydrochloride

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (500 mg, 1.19 mmol) obtained in Example 10 (b) above was dissolved in N,N-dimethylformamide (10 mL), and potassium tert-butoxide (401 mg, 3.58 mmol) was added thereto and stirred at room temperature for 30 minutes. 1-(3-Chlorophenyl)-4-(3-chloropropyl)piperazine hydrochloride (554 mg, 1.79 mmol) was added to the reaction mixture and stirred at room temperature for additional 15 hours. When the reaction was completed, the reaction mixture was quenched by adding water (5 mL) and extracted with ethyl acetate. The separated organic layer was washed with aqueous sodium chloride solution, dried over sodium sulfate, filtered, concentrated by distillation under reduced pressure, separated by MPLC (ethyl acetate : hexane = 3 : 7, v/v), and concentrated by distillation under reduced pressure. The obtained residue was dissolved in ethyl acetate (5 mL), and 4M hydrochloric acid solution (1 mL, 4M/dioxane) was added dropwise thereto and stirred at room temperature for 30 minutes. The obtained solid precipitate was filtered and washed with ethyl acetate to obtain 2-(4-acetylphenyl)-10-(3-(4-(3-chlorophenyl)piperazin-1-yl)propoxy)-7,7-dimethyl-5,12b-dihydro-1*H*,7*H*-chromeno[4,3-*c*][1,2,4]triazolo[1,2-*a*]pyridazine-1,3(2*H*)-dione dihydrochloride (128 mg, 16%).
¹H NMR (500 MHz, DMSO-D₆) δ 10.69 (s, 1H), 8.11 - 8.06 (m, 2H), 7.73 - 7.68 (m, 2H), 7.29 - 7.19 (m, 2H), 7.08 - 7.02 (m, 2H), 6.97 (dd, J = 8.4, 2.4 Hz, 1H), 6.88 (dd, J = 7.9, 1.9 Hz, 1H), 6.61 (s, 1H), 6.47 (dd, J = 8.3, 2.5 Hz, 1H), 6.40 (d, J = 2.4 Hz, 1H), 5.91 (d, J = 14.2 Hz, 1H), 4.04 (q, J = 7.0, 6.5 Hz, 2H), 3.90 (d, J = 10.7 Hz, 3H), 3.27 (d, J = 10.8 Hz, 3H), 3.21 - 3.06 (m, 5H), 2.62 (s, 3H), 2.19 (dd, J = 10.1, 5.9 Hz, 2H), 1.43 (s, 6H).

### Example 121: Synthesis of 2-(4-acetylphenyl)-7,7-dimethyl-10-(((2S,3R,4S,SS,6R)-3,4,5-trihydroxy-6-(hydroxymethyl)tetrahydro-2H-pyran-2-yl)oxy)-5,12b-dihydro-1H,7H-chromeno [4,3-c] [1,2,4]triazolo[1,2-a]pyridazine-1,3(2H)-dione

### (a) Synthesis of (2R,3R,4S,5R,6S)-2-(acetoxymethyl)-6-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)tetrahydro-2H-pyran-3,4,5-triyl triacetate

2-(4-Acetylphenyl)-10-hydroxy-7,7-dimethyl-5,12b-dihydro-1H,7H-chromeno[4,3-*c*][1,2,4]triazolo[1,2-*a*]pyridazine-1,3(2*H*)-dione (500 mg, 1.19 mmol) obtained in Example 10 (b) above was dissolved in *N*,*N-*dimethylformamide (5 mL), and sodium hydride (71 mg, 1.79 mmol, 60%) was added thereto and stirred at room temperature for 30 minutes. Acetobromo-α-D-glucose (734 mg, 1.79 mmol) was added thereto and stirred at room temperature for 4 hours. When the reaction was completed, the reaction mixture was diluted in dichloromethane and washed with aqueous sodium chloride solution. The separated organic layer was dried over sodium sulfate, filtered, concentrated by distillation under reduced pressure, and separated by MPLC (ethyl acetate : hexane = 1 : 1, v/v) to obtain (2*R*,3*R*,4*S*,5*R*,6*S*)-2-(acetoxymethyl)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)tetrahydro-2*H*-pyran-3,4,5-triyl triacetate (450 mg, 50%).

### (b) Synthesis of 2-(4-acetylphenyl)-7,7-dimethyl-10-(((25,3A,45,55,6A)-3,4,5-trihydroxy-6-(hydroxymethyl)tetrahydro-2H-pyran-2-yl)oxy)-5,12b-dihydro-1H,7H-chromeno[4,3-c][1,2,4]triazolo[1,2-a]pyridazine-1,3(2H)-dione

Except that (2*R*,3*R*,4*S*,5*R*,6*S*)-2-(acetoxymethyl)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)tetrahydro-2H-pyran-3,4,5-triyl triacetate was used instead of (2*S*,3*R*,4*S*,5*S*,6*S*)-2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-6-(methoxycarbonyl)tetrahydro-2*H*-pyran-3,4,5-triyl triacetate, the same synthesis method as in Example 64 (b) above was carried out to obtain 2-(4-acetylphenyl)-7,7-dimethyl-10-(((2*S*,3*R*,4*S*,5*S*,6*R*)-3,4,5-trihydroxy-6-(hydroxymethyl)tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione (200 mg, 52%).
¹H NMR (500 MHz, DMSO-D₆) δ 8.12 - 8.03 (m, 2H), 7.76 - 7.68 (m, 2H), 7.23 (d, J = 14.3 Hz, 1H), 7.02 (d, J = 8.4 Hz, 1H), 6.61 (s, 1H), 6.53 (dd, J = 8.3, 2.4 Hz, 1H), 6.43 (d, J = 2.4 Hz, 1H), 5.87 (dd, J = 14.3, 1.0 Hz, 1H), 5.12 (s, 1H), 4.84 (s, 1H), 4.79 (d, J = 7.6 Hz, 1H), 4.66 (s, 1H), 4.49 (s, 1H), 3.68 (d, J = 3.3 Hz, 1H), 3.61 - 3.43 (m, 6H), 2.62 (s, 3H), 1.43 (d, J = 5.2 Hz, 6H).

### Example 122: Synthesis of 2-(4-acetylphenyl)-7,7-dimethyl-10-(((2S,3R,4S,SR,6R)-3,4,5-trihydroxy-6-(hydroxymethyl)tetrahydro-2H-pyran-2-yl)oxy)-5,12b-dihydro-1H,7H-chromeno[4,3-c] [1,2,4]triazolo[1,2-a]pyridazine-1,3(2H)-dione

### (a) Synthesis of (2R,3S,4S,5R,6S)-2-(acetoxymethyl)-6-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)tetrahydro-2H-pyran-3,4,5-triyl triacetate

Except that acetobromo-α-D-galactose was used instead of acetobromo-α-D-glucose, the same synthesis method as in Example 121 (a) above was carried out to obtain (2*R*,3*S*,4*S*,5*R*,6*S*)-2-(acetoxymethyl)-6-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)tetrahydro-2*H*-pyran-3,4,5-triyl triacetate (500 mg, 54%).

### (b) Synthesis of 2-(4-acetylphenyl)-7,7-dimethyl-10-(((2S,3R,4S,5R,6R)-3,4,5-trihydroxy-6-(hydroxymethyl)tetrahydro-2H-pyran-2-yl)oxy)-5,12b-dihydro-1H,7H-chromeno[4,3-c][1,2,4]triazolo[1,2-a]pyridazine-1,3(2H)-dione

Exceptthat(2*R*,3*S*,4*S*,5*S*,6*S*)-2-(acetoxymethyl)-6-((2-(4-acetylphenyl)-7,7-dimethyl-1,3-dioxo-2,3,5,12b-tetrahydro-1H,7H-chromeno[4,3-*c*][1,2,4]triazolo[1,2-a]pyridazin-10-yl)oxy)tetrahydro-2*H*-pyran-3,4,5-triyl triacetate was used instead of (2*S*,3*R*,4*S*,5*S*,6*S*)-2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-6-(methoxycarbonyl)tetrahydro-2*H*-pyran-3,4,5-triyl triacetate, the same synthesis method as in Example 64 (b) above was carried out to obtain 2-(4-acetylphenyl)-7,7-dimethyl-10-(((2*S*,3*R*,4*S*,5*R*,6*R*)-3,4,5-trihydroxy-6-(hydroxymethyl)tetrahydro-2H-pyran-2-yl)oxy)-5,12b-dihydro-1*H*,7*H*-chromeno[4,3-*c*][1,2,4]triazolo[1,2-*a*]pyridazine-1,3(2*H*)-dione (142 mg, 58%).
¹H NMR (500 MHz, DMSO-D₆) δ 8.12 - 8.05 (m, 2H), 7.74 - 7.67 (m, 2H), 7.24 (d, J = 14.3 Hz, 1H), 7.02 (d, J = 8.4 Hz, 1H), 6.61 (s, 1H), 6.54 (dd, J = 8.3, 2.4 Hz, 1H), 6.43 (d, J = 2.3 Hz, 1H), 5.87 (d, J = 14.2 Hz, 1H), 5.76 (s, 1H), 5.28 (s, 1H), 5.04 (d, J = 29.3 Hz, 2H), 4.83 (d, J = 7.6 Hz, 1H), 4.58 (s, 1H), 3.68 (d, J = 11.4 Hz, 1H), 3.24 (d, J = 8.9 Hz, 3H), 3.16 (dt, J = 26.4, 8.7 Hz, 3H), 2.62 (s, 3H), 1.43 (d, J = 1.9 Hz, 6H).

### Example 123: Synthesis of 2-((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)-N,N,N-trimethyl-2-oxoethan-1-aminium iodide

2-(4-Acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dimethylglycinate hydrochloride (200 mg, 0.37 mmol) obtained in Example 72 above was dissolved in dichloromethane (5 mL), and triethylamine (75 mg, 0.74 mmol) was added thereto and stirred at room temperature for 30 minutes. Water (5 mL) was added to the reaction mixture, and the organic layer was separated by layer separation. The separated organic layer was concentrated by distillation under reduced pressure. The obtained residue was dissolved in acetone (3 mL), and iodomethane (222 mg, 0.79 mmol) was added dropwise thereto and stirred at room temperature for 3 hours. The obtained solid precipitate was filtered and washed with acetone to obtain 2-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-*N*,*N*,*N-*trimethyl-2-oxoethan-1-aminium iodide (220 mg, 46%).
¹H NMR (400 MHz, DMSO-D₆) δ 8.16 - 8.09 (m, 2H), 7.83 - 7.76 (m, 2H), 7.19 (dd, J = 8.3, 1.1 Hz, 1H), 6.89 - 6.84 (m, 2H), 6.05 (td, J = 3.7, 1.7 Hz, 1H), 5.77 - 5.71 (m, 1H), 4.71 (s, 2H), 4.23 (dd, J = 4.2, 2.0 Hz, 2H), 3.29 (s, 9H), 2.64 (s, 3H), 1.61 (s, 3H), 1.54 (s, 3H).

### Example 124: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (4-nitrobenzyl) carbonate

Except that (4-nitrophenyl)methyl carbonochloridate was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (4-nitrobenzyl) carbonate (120 mg, 90%).
¹H NMR (400 MHz, DMSO-D₆) δ 11.18 (s, 1H), 8.17 - 8.10 (m, 2H), 7.83 - 7.76 (m, 2H), 7.10 (d, J = 8.2 Hz, 1H), 6.81 - 6.73 (m, 2H), 6.02 (td, J = 3.8, 1.8 Hz, 1H), 5.71 (d, J = 2.5 Hz, 1H), 4.39 (d, J = 13.2 Hz, 1H), 4.31 - 4.18 (m, 3H), 4.12 (s, 1H), 3.77 (d, J = 13.7 Hz, 1H), 3.54 (s, 2H), 3.48 (d, J = 12.8 Hz, 3H), 3.12 (dd, J = 24.6, 12.6 Hz, 3H), 2.82 - 2.70 (m, 1H), 2.64 (s, 3H), 2.38 (s, 3H), 2.18 (dd, J = 22.4, 12.3 Hz, 2H), 1.64 (s, 2H), 1.60 (s, 3H), 1.52 (s, 3H).

### Example 125: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl piperidine-1-carboxylate

Except that piperidine-1-carbonyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl piperidine-1-carboxylate (140 mg, 92%).
¹H NMR (400 MHz, DMSO-D₆) δ 8.17 - 8.09 (m, 2H), 7.85 - 7.76 (m, 2H),7.08 (dd, J = 8.4, 1.1 Hz, 1H), 6.72 (dd, J = 8.4, 2.3 Hz, 1H), 6.65 (d, J =2.3 Hz, 1H), 6.01 (td, J = 3.7, 1.9 Hz, 1H), 5.73 - 5.67 (m, 1H), 4.21 (dd,J = 3.9, 2.0 Hz, 2H), 3.44 (d, J = 51.2 Hz, 4H), 2.64 (s, 3H), 1.56 (d, J =25.9 Hz, 12H).

### Example 126: Synthesis of O-(2-(4-acetylphenyl)-7,7-dimethyl-1,3-dioxo-2,3,5,12b-tetrahydro-1H,7H chromeno[4,3-c] [1,2,4]triazolo[1,2-a]pyridazin-10-yl) dimethylcarbamothioate

Except that dimethylcarbamothioic chloride was used instead of trimethylacetyl chloride, the same synthesis method as in Example 58 above was carried out to obtain O-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) dimethylcarbamothioate (200 mg, 95%).
¹H NMR (400 MHz, CDCl₃) δ 8.11 (d, J = 8.7 Hz, 2H), 7.84 (d, J = 8.7 Hz, 2H), 7.10 (dd, J = 8.5, 1.1 Hz, 1H), 6.67 (dd, J = 8.4, 2.3 Hz, 1H), 6.63 (d, J = 2.3 Hz, 1H), 5.88 - 5.82 (m, 1H), 5.74 (s, 1H), 4.36 - 4.27 (m, 1H), 4.13 (dt, J = 16.5, 2.6 Hz, 1H), 3.44 (s, 3H), 3.31 (s, 3H), 2.64 (s, 3H), 1.61 (d, J = 4.9 Hz, 6H).

### Example 127: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl 2-oxoimidazolidine-1-carboxylate

Except that 2-oxoimidazolidine-1-carbonyl chloride was used instead of trimethylacetyl chloride, the same synthesis method as in Example 58 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl 2-oxoimidazolidine-1-carboxylate (132 mg, 92%).
¹H NMR (400 MHz, DMSO-D₆) δ 8.13 (d, J = 8.6 Hz, 2H), 7.81 (d, J = 8.6 Hz, 2H), 7.55 (s, 1H), 7.17 - 7.09 (m, 1H), 6.79 (dd, J = 8.4, 2.4 Hz, 1H), 6.73 (d, J = 2.3 Hz, 1H), 6.02 (s, 1H), 5.73 (d, J = 6.1 Hz, 1H), 4.22 (s, 2H), 3.95 - 3.85 (m, 2H), 3.34 (s, 1H), 3.29 (s, 1H), 2.64 (s, 3H), 1.60 (s, 3H), 1.53 (s, 3H).

### Example 128: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (3-(dimethylamino)-2,2-dimethylpropyl)carbamate formate

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (830 mg, 1.98 mmol) obtained in Example 10 (b) above was dissolved in dichloromethane (10 mL), and triphosgene (300 mg, 0.99 mmol) and *N,N-*diisopropylethylamine (307 mg, 2.37 mmol) were added thereto and stirred at room temperature for 10 minutes. *N'*,*N'*-tetramethylpropane-1,3-diamine (503 mg, 3.86 mmol) was added dropwise to the reaction mixture and stirred at room temperature for 30 minutes. When the reaction was completed, the reaction mixture was diluted in dichloromethane and washed with saline. The separated organic layer was dried over sodium sulfate, filtered, concentrated by distillation under reduced pressure, separated by reverse-phase chromatography (acetonitrile containing 0.1% formic acid : water containing 0.1% formic acid = 5 : 95-100 : 0), and freeze-dried to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (3-(dimethylamino)-2,2-dimethylpropyl)carbamate formate (170 mg, 14%).
¹H NMR (500 MHz, DMSO-D₆) δ 8.13 (dd, J = 8.6, 2.1 Hz, 2H),7.83 - 7.77 (m, 2H), 7.69 (t, J = 6.2 Hz, 1H), 7.07 (d, J = 8.6 Hz,1H), 6.69 (dd, J = 8.4, 2.2 Hz, 1H), 6.61 (d, J = 2.3 Hz, 1H), 6.01(s, 1H), 5.71 (s, 1H), 4.21 (d, J = 3.7 Hz, 2H), 2.94 (d, J = 6.2Hz, 2H), 2.63 (d, J = 2.3 Hz, 4H), 2.22 (s, 6H), 2.11 (s, 2H), 1.59(d, J = 2.2 Hz, 3H), 1.53 (s, 3H), 0.83 (d, J = 1.8 Hz, 6H).

### Example 129: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (2-(dimethylamino)ethyl)(methyl)carbamate formate

2-(4-Acetylphenyl)-10-hydroxy-7,7-dimethyl-5,12b-dihydro-1H,7H-chromeno[4,3-*c*][1,2,4]triazolo[1,2-*a*]pyridazine-1,3(2*H*)-dione (664 mg, 1.58 mmol) obtained in Example 10 (b) above was dissolved in dichloromethane (5 mL), and triphosgene (383 mg, 1.27 mmol) and *N,N-*diisopropylethylamine (245 mg, 1.9 mmol) were added thereto and stirred at room temperature for 10 minutes. *N*,*N'*,*N'-*trimethylethane-1,2-diamine (250 mg, 2.37 mmol) was added dropwise to the reaction mixture and stirred at room temperature for 30 minutes. When the reaction was completed, the undissolved solid product was filtered and washed with dichloromethane. The filtered solid product was separated by reverse phase chromatography (acetonitrile containing 0.1% formic acid : water containing 0.1% formic acid = 5 : 95-100 : 0) and freeze-dried to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (2-(dimethylamino)ethyl)(methyl)carbamate formate (330 mg, 35%).
¹H NMR (500 MHz, DMSO-D₆) δ 11.00 (s, 1H), 10.53 (s, 1H), 9.41 (s, 1H), 8.11 (d, J =8.6 Hz, 2H), 7.78 (d, J = 8.6 Hz, 2H), 7.10 - 7.02 (m, 1H), 6.83 - 6.74 (m, 2H), 6.00(s, 1H), 5.69 (s, 1H), 4.20 (dd, J = 3.8, 2.0 Hz, 2H), 3.19 (s, 3H), 3.15 (s, 1H), 3.01(s, 2H), 2.94 (s, 2H), 2.91 (s, 1H), 2.80 (s, 4H), 2.77 (d, J = 4.6 Hz, 6H), 2.62 (s,3H), 2.57 (d, J = 5.9 Hz, 2H), 1.57 (s, 3H), 1.51 (s, 3H).

### Example 130: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl methyl(piperidin-3-yl)carbamate hydrochloride

Except that *N*-methyl-*N*-(3-piperidyl)carbamoyl chloride was used instead of 2-(dimethylamino)acetyl chloride, the same synthesis method as in Example 72 above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(piperidin-3-yl)carbamate hydrochloride (120 mg, 74%).
¹HNMR (500 MHz, D₂O) δ 8.16 (d, J = 8.3 Hz, 2H), 7.71 (d, J = 8.4 Hz, 2H), 7.15 (s, 1H), 6.84 (dd, J = 28.7, 10.7 Hz, 3H), 6.02 (s, 1H), 5.80 (s, 1H), 4.30 (s, 2H), 4.13 (q, J = 7.1 Hz, 2H), 3.40 (s, 2H), 3.20 (s, 2H), 3.04 (s, 1H), 2.92 (s, 1H), 2.71 (s, 2H), 2.07 (s, 3H), 1.65 (s, 3H), 1.58 (s, 3H).

### Example 131: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl methyl(methylsulfonyl)carbamate

Except that *N*-methylmethanesulfonamide was used instead of tert-butyl *N-*methyl-*N-*[2-(methylamino)ethyl]carbamate, the same synthesis method as in Example 83 (a) above was carried out to obtain 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(methylsulfonyl)carbamate (58 mg, 46%).
¹HNMR (400 MHz, DMSO-D₆) δ 8.17 - 8.09 (m, 2H), 7.86 - 7.76 (m, 2H), 7.16 (dd, J = 8.3, 1.1 Hz, 1H), 6.93 - 6.78 (m, 2H), 6.03 (td, J = 3.8, 1.8 Hz, 1H), 5.74 (d, J = 1.7 Hz, 1H), 4.22 (dd, J = 4.0, 2.0 Hz, 2H), 3.45 (s, 3H), 3.27 (s, 3H), 2.64 (s, 3H), 1.61 (s, 3H), 1.54 (s, 3H).

### Example 132: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-valinate 2,2,2-trifluoroacetic acid

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (tert-butoxycarbonyl)-L-valinate

Except that (2*S*)-2-(tert-butoxycarbonylamino)-3-methyl-butanoic acid was used instead of (2*S*)-5-amino-2-(tert-butoxycarbonylamino)-5-oxo-pentanoic acid, the same synthesis method as in Example 77 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-valinate (214 mg, 50%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-valinate 2,2,2-trifluoroacetic acid

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (tert-butoxycarbonyl)-*L*-valinate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-glutaminate, the same synthesis method as in Example 77 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl *L-*valinate 2,2,2-trifluoroacetic acid (70 mg, 48%).
¹H NMR (400 MHz, DMSO-D₆) δ 8.16 - 8.10 (m, 2H), 7.82 - 7.78 (m, 2H), 7.13 (dt, J = 8.4, 1.4 Hz, 1H), 6.72 (dt, J = 8.4, 2.1 Hz, 1H), 6.64 (d, J = 2.3 Hz, 1H), 6.06 - 5.99 (m, 1H), 5.73 (d, J = 2.6 Hz, 1H), 4.22 (t, J = 2.9 Hz, 2H), 3.34 - 3.32 (m, 1H), 2.64 (s, 3H), 2.04 - 1.94 (m, 1H), 1.84 (s, 2H), 1.57 (d, J = 25.1 Hz, 7H), 0.95 (dd, J = 17.6, 6.8 Hz, 6H).

### Example 133: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-prolinate 2,2,2-trifluoroacetic acid

### (a) Synthesis of 2-(2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl) 1-(tert-butyl) (2S)-pyrrolidine-1,2-dicarboxylate

Except that 1-tert-butoxycarbonylpyrrolidine-2-carboxylic acid was used instead of (2*S*)-5-amino-2-(tert-butoxycarbonylamino)-5-oxo-pentanoic acid, the same synthesis method as in Example 77 (a) above was carried out to obtain 2-(2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl) 1-(tert-butyl) (2*S*)-pyrrolidine-1,2-dicarboxylate (120 mg, 48%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-prolinate 2,2,2-trifluoroacetic acid

Except that 2-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl) 1-(tert-butyl) (2*S*)-pyrrolidine-1,2-dicarboxylate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L-*glutaminate, the same synthesis method as in Example 77 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl *L*-prolinate 2,2,2-trifluoroacetic acid (48 mg, 55%).
¹H NMR (500 MHz, DMSO-D₆) δ 8.16 - 8.09 (m, 2H), 7.83 - 7.77 (m, 2H), 7.18 (dt, J = 9.0, 1.4 Hz, 1H), 6.89 - 6.80 (m, 2H), 6.04 (q, J = 2.9, 2.3 Hz, 1H), 5.74 (s, 1H), 4.67 (t, J = 8.1 Hz, 1H), 4.23 (dd, J = 4.3, 2.0 Hz, 2H), 3.41 - 3.34 (m, 1H), 3.29 (td, J = 16.0, 13.5, 8.7 Hz, 2H), 2.64 (s, 3H), 2.43 - 2.34 (m, 2H), 2.23 (dq, J = 13.0, 7.6 Hz, 1H), 2.02 - 1.93 (m, 2H), 1.61 (s, 3H), 1.53 (s, 3H).

### Example 134: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl acetyl-L-glutaminate

Except that (2*S*)-2-acetamido-5-amino-5-oxo-pentanoic acid was used instead of (2*S*)-5-amino-2-(tert-butoxycarbonylamino)-5-oxo-pentanoic acid, the same synthesis method as in Example 77 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl acetyl-L-glutaminate (90 mg, 70%).
¹H NMR (400 MHz, DMSO-D₆) δ 8.45 (d, J = 6.7 Hz, 1H), 8.16 - 8.09 (m, 2H), 7.82 - 7.77 (m, 2H), 7.36 - 7.30 (m, 1H), 7.13 (ddd, J = 8.4, 2.2, 1.1 Hz, 1H), 6.82 (s, 1H), 6.72 (dd, J = 8.4, 2.3 Hz, 1H), 6.65 (d, J = 2.3 Hz, 1H), 6.03 (tt, J = 3.9, 1.8 Hz, 1H), 5.73 (q, J = 1.7 Hz, 1H), 4.33 (ddt, J = 8.9, 6.1, 3.0 Hz, 1H), 4.22 (dd, J = 3.9, 2.0 Hz, 2H), 2.64 (s, 3H), 2.22 (t, J = 7.5 Hz, 2H), 2.04 (td, J = 6.9, 5.9, 2.1 Hz, 1H), 1.97 - 1.89 (m, 1H), 1.88 (s, 3H), 1.60 (s, 3H), 1.54 (s, 3H).

### Example 135: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-argininate 2,2,2-trifluoroacetic acid

### (a) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl (tert-butoxycarbonyl)-L-argininate

Except that (2*S*)-2-(tert-butoxycarbonylamino)-5-guanido-pentanoic acid was used instead of (2*S*)-5-amino-2-(tert-butoxycarbonylamino)-5-oxo-pentanoic acid, the same synthesis method as in Example 77 (a) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl (tert-butoxycarbonyl)-*L*-argininate (320 mg, 59%).

### (b) Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl L-argininate 2,2,2-trifluoroacetic acid

Except that 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (tert-butoxycarbonyl)-L-argininate was used instead of 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (tert-butoxycarbonyl)-Z-glutaminate, the same synthesis method as in Example 77 (b) above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl *L*-argininate 2,2,2-trifluoroacetic acid (60mg, 38%).
¹H NMR (400 MHz, D₂O) δ 8.13 (d, J = 8.5 Hz, 2H), 7.71 (d, J = 8.6 Hz, 2H), 7.20 (d, J = 8.4 Hz, 1H), 6.94 - 6.84 (m, 2H), 6.06 (s, 1H), 5.81 (s, 1H), 4.55 - 4.49 (m, 1H), 4.30 (s, 2H), 3.35 (t, J = 6.8 Hz, 2H), 3.29 (d, J = 6.9 Hz, 1H), 2.29 - 2.13 (m, 2H), 2.01 - 1.88 (m, 2H), 1.86 - 1.73 (m, 2H), 1.67 (s, 3H), 1.62 (s, 3H).

### Example 136: Synthesis of methyl (((2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl)oxy)carbonyl)-L-valinate

Except that methyl 2-isocyanato-methyl-butanoate was used instead of methyl 2-isocyanato-4-methyl-pentanoate, the same synthesis method as in Example 80 above was carried out to obtain methyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-valinate (67 mg, 58%).
¹H NMR (500 MHz, DMSO-D₆) δ 8.22 (d, J = 8.1 Hz, 1H), 8.13 (dd, J = 8.6, 2.4 Hz, 2H), 7.80 (dd, J = 8.8, 2.5 Hz, 2H), 7.09 (d, J = 8.3 Hz, 1H), 6.71 (dt, J = 8.5, 1.9 Hz, 1H), 6.62 (d, J = 2.3 Hz, 1H), 6.04 - 5.98 (m, 1H), 5.71 (s, 1H), 4.21 (t, J = 3.6 Hz, 2H), 4.09 - 3.90 (m, 1H), 3.65 (d, J = 4.2 Hz, 3H), 2.63 (d, J = 2.0 Hz, 3H), 2.08 (dt, J = 13.5, 6.8 Hz, 1H), 1.60 - 1.50 (m, 7H), 0.96 -0.88 (m, 7H).

### Example 137: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl sulfamate

2-(4-Acetylphenyl)-10-hydroxy-7,7-dimethyl-5,12b-dihydro-1*H*,*7*H-chromeno[4,3-*c*][1,2,4]triazolo[1,2-*a*]pyridazine-1,3(2*H*)-dione (200 mg, 0.48 mmol) obtained in Example 10 (b) above was dissolved in tetrahydrofuran (5 mL), and sodium hydride (23 mg, 0.95 mmol, 60%) was added thereto and stirred at room temperature for 30 minutes. Sulfamoyl chloride (83 mg, 0.72 mmol) was added thereto and stirred at 70°C for 12 hours. When the reaction was completed, the reaction mixture was quenched by adding water (5 mL) and extracted with dichloromethane. The separated organic layer was dried over sodium sulfate, filtered, concentrated by distillation under reduced pressure, and separated by MPLC (dichloromethane : methanol = 9 : 1, v/v) to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl sulfamate (102 mg, 41%).
¹H NMR (500 MHz, CDCl₃) δ 9.28 (s, 2H), 8.10 (d, J = 8.7 Hz, 2H), 7.84 - 7.79 (m, 2H), 6.99 (dd, J = 9.1, 1.0 Hz, 1H), 6.97 - 6.94 (m, 2H), 5.82 (dt, J = 4.5, 2.1 Hz, 1H), 5.67 (d, J = 2.5 Hz, 1H), 4.28 (ddd, J = 16.4, 4.9, 1.3 Hz, 1H), 4.12 (dt, J = 16.4, 2.7 Hz, 1H), 2.64 (s, 3H), 1.57 (d, J = 8.9 Hz, 6H).

### Example 138: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl picolinate hydrochloride

Except that picolinoyl chloride was used instead of 2-(dimethylamino)acetyl chloride, the same synthesis method as in Example 72 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl picolinate hydrochloride (500 mg, 89%).
¹H NMR (400 MHz, DMSO-D₆) δ 8.80 (ddd, J = 4.7, 1.7, 0.9 Hz, 1H), 8.22 (dt, J = 7.9, 1.1 Hz, 1H), 8.17 - 8.10 (m, 2H), 8.08 (td, J = 7.7, 1.8 Hz, 1H), 7.86 - 7.78 (m, 2H), 7.74 (ddd, J = 7.7, 4.7, 1.2 Hz, 1H), 7.19 (dd, J = 8.4, 1.1 Hz, 1H), 6.95 - 6.85 (m, 2H), 6.38 (s, 1H), 6.05 (td, J = 3.7, 1.8 Hz, 1H), 5.79 - 5.74 (m, 1H), 4.24 (dd, J = 4.0, 2.0 Hz, 2H), 2.64 (s, 3H), 1.62 (s, 3H), 1.56 (s, 3H).

### Example 139: Synthesis of 2-(4-acetylphenyl)-7,7-dimethyl-1,3-dioxo-2,3,5,12b-tetrahydro-1H,7H-chromeno[4,3-c][1,2,4]triazolo[1,2-a]pyridazin-10-yl isonicotinate hydrochloride

Except that isonicotinoyl chloride was used instead of 2-(dimethylamino)acetyl chloride, the same synthesis method as in Example 72 above was carried out to obtain 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl isonicotinate hydrochloride (420 mg, 87%).
¹H NMR (400 MHz, DMSO-D₆) δ 9.00 - 8.87 (m, 2H), 8.18 - 8.04 (m, 4H), 7.81 (d, J = 8.4 Hz, 2H), 7.51 (s, 1H), 7.21 (d, J = 8.3 Hz, 1H), 7.00 - 6.86 (m, 2H), 6.05 (q, J = 3.2 Hz, 1H), 5.76 (d, J = 3.3 Hz, 1H), 4.24 (t, J = 2.8 Hz, 2H), 1.62 (s, 3H), 1.55 (s, 3H).

### Example 140: Synthesis of 10-hydroxy-2-(4-(1-hydroxyethyl)phenyl)-7,7-dimethyl-5,12b-dihydro-1H,7H-chronieno[4,3-c][1,2,4]triazolo[1,2-a]pyridazine-1,3(2H)-dione

2-(4-Acetylphenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (100 mg, 0.24 mmol) obtained in Example 10 (b) above was suspended in methanol, and sodium borohydride (11 mg, 0.29 mmol). mmol) was added thereto and stirred at room temperature for 15 hours. When the reaction was completed, the reaction mixture was concentrated by distillation under reduced pressure, diluted in dichloromethane, and washed with aqueous sodium chloride solution. The separated organic layer was dried over sodium sulfate, filtered, concentrated by distillation under reduced pressure, and separated by MPLC (dichloromethane : methanol = 20 : 1, v/v) to obtain 10-hydroxy-2-(4-(1-hydroxyethyl)phenyl)-7,7-dimethyl-5,12b-dihydro-1*H*,7*H*-chromeno[4,3-*c*][1,2,4]triazolo[1,2-*a*]pyridazine-1,3(2*H*)-dione (50 mg, 48%).
¹H NMR (400 MHz, DMSO-D₆) δ 9.50 (s, 1H), 7.50 (d, J = 2.6 Hz, 4H), 6.83 (d, J = 8.5 Hz, 1H), 6.38 (dd, J = 8.3, 2.6 Hz, 1H), 6.24 (d, J = 2.5 Hz, 1H), 5.94 (d, J = 4.3 Hz, 1H), 5.58 (s, 1H), 5.27 (d, J = 4.0 Hz, 1H), 4.79 (s, 1H), 4.23 - 4.09 (m, 2H), 1.52 (d, J = 11.7 Hz, 6H), 1.36 (d, J = 6.4 Hz, 3H).

### Example 141: Synthesis of 2-(4-acetylphenyl)-10-(azetidin-1-yl)-7,7-dimethyl-5,12b-dihydro-1H,7H-chromeno[4,3-c][1,2,4]triazolo[1,2-a]pyridazine-1,3(2H)-dione

Except that 1-(2,2-dimethyl-3-vinyl-2H-chromen-7-yl)azetidine was used instead of ((2,2-bis(fluoromethyl)-3-vinyl-2*H*-chromen-7-yl)oxy)triisopropylsilane, the same synthesis method as in Example 2 (a) above was carried out to obtain 2-(4-acetylphenyl)-10-(azetidin-1-yl)-7,7-dimethyl-5,12b-dihydro-1*H*,7*H*-chromeno[4,3-*c*][1,2,4]triazolo[1,2-*a*]pyridazine-1,3(2*H*)-dione (25 mg, 60%).
¹H NMR (400 MHz, DMSO-D₆) δ 8.13 - 8.12 (m, 2H), 7.79 - 7.77 (m, 2H), 6.87 - 6.85 (m, 1H), 6.02 - 5.99 (m, 1H), 5.95 - 5.95 (m, 1H), 5.87 (s, 1H) 5.62 (s, 1H), 4.24 - 4.13 (m, 2H), 3.74 - 3.71 (m, 4H), 2.64 (s, 3H), 2.28 - 2.21 (m, 2H), 1.53 - 1.52 (m, 6H)

### Example 142: Synthesis of 7,7-dimethyl-1,3-dioxo-2-(4-(trifluoromethyl)phenyl)-2,3,5,12b-tetrahydro-1H,7H-chromeno[4,3-c][1,2,4]triazolo[1,2-a]pyridazin-10-yl dihydrogen phosphate

### (a) Synthesis of 7,7-dimethyl-10-((tetrahydro-2H-pyran-2-yl)oxy)-2-(4-(trifluoromethyl)phenyl)-5,12b-dihydro-1H,7H-chromeno[4,3-c][1,2,4]triazolo[1,2-a]pyridazine-1,3(2H)-dione

Except that 2,2-dimethyl-7-((tetrahydro-2*H*-pyran-2-yl)oxy)-3-vinyl-2*H*-chromene and 4-(4-(trifluoromethyl)phenyl)-1,2,4-triazolidine-3,5-dione were used instead of 2,2-bis(fluoromethyl)-7-((tetrahydro-2*H*-pyran-2-yl)oxy)chroman-4-one and 4-(4-acetylphenyl)-1,2,4-triazolidine-3,5-dione, respectively, the same synthesis method as in Example 2 (a) above was carried out to obtain 7,7-dimethyl-10-((tetrahydro-2*H*-pyran-2-yl)oxy)-2-(4-(trifluoromethyl)phenyl)-5,12b-dihydro-1*H*,7*H*-chromeno[4,3-*c*][1,2,4]triazolo[1,2-*a*]pyridazine-1,3(2*H*)-dione (0.97 g, 15%).

### (b) Synthesis of 10-hydroxy-7,7-dimethyl-2-(4-(trifluoromethyl)phenyl)-5,12b-dihydro-1H,7H-chromeno[4,3-c][1,2,4]triazolo[1,2-a]pyridazine-1,3(2H)-dione

Except that 7,7-dimethyl-10-((tetrahydro-2*H*-pyran-2-yl)oxy)-2-(4-(trifluoromethyl)phenyl)-5,12b-dihydro-1*H*,7*H*-chromeno[4,3-*c*][1,2,4]triazolo[1,2-*a*]pyridazine-1,3(2*H*)-dione was used instead of 2,2-bis(fluoromethyl)-7-((tetrahydro-2*H*-pyran-2-yl)oxy)chroman-4-one, the same synthesis method as in Intermediate 31 (d) above was carried out to obtain 10-hydroxy-7,7-dimethyl-2-(4-(trifluoromethyl)phenyl)-5,12b-dihydro-1*H*,7*H-*chromeno[4,3-*c*][1,2,4]triazolo[1,2-*a*]pyridazine-1,3(2*H*)-dione (0.41 g, 50%).

### (c) Synthesis of 7,7-dimethyl-1,3-dioxo-2-(4-(trifluoromethyl)phenyl)-2,3,5,12b-tetrahydro-1H,7H-chromeno[4,3-c][1,2,4]triazolo[1,2-a]pyridazin-10-yl diethyl phosphate

Except that diethyl chlorophosphate was used instead of trimethylacetyl chloride, the same synthesis method as in Example 58 above was carried out to obtain 7,7-dimethyl-1,3-dioxo-2-(4-(trifluoromethyl)phenyl)-2,3,5,12b-tetrahydro-1*H*,7*H*-chromeno[4,3-*c*][1,2,4]triazolo[1,2-*a*]pyridazin-10-yl diethyl phosphate (230 mg, 90%).

### (d) Synthesis of 7,7-dimethyl-1,3-dioxo-2-(4-(trifluoromethyl)phenyl)-2,3,5,12b-tetrahydro-1H,7H-chromeno[4,3-c][1,2,4]triazolo[1,2-a]pyridazin-10-yl dihydrogen phosphate

Except that 7,7-dimethyl-1,3-dioxo-2-(4-(trifluoromethyl)phenyl)-2,3,5,12b-tetrahydro-1*H*,7*H*-chromeno[4,3-*c*][1,2,4]triazolo[1,2-*a*]pyridazin-10-yl diethyl phosphate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diethyl phosphate, the same synthesis method as in Example 69 (b) above was carried out to obtain 7,7-dimethyl-1,3-dioxo-2-(4-(trifluoromethyl)phenyl)-2,3,5,12b-tetrahydro-1*H*,7*H*-chromeno[4,3-*c*][1,2,4]triazolo[1,2-*a*]pyridazin-10-yl dihydrogen phosphate (43 mg, 50%).
¹H NMR (400 MHz, DMSO-D₆) δ 7.95 (d, J = 8.5 Hz, 2H), 7.90 (d, J = 8.5 Hz, 2H), 7.06 (d, J = 8.5 Hz, 1H), 6.74 (dd, J = 8.6, 2.3 Hz, 1H), 6.70 (d, J = 2.4 Hz, 1H), 6.04 - 5.97 (m, 1H), 5.70 (s, 1H), 4.27 - 4.15 (m, 2H), 1.58 (s, 3H), 1.54 (s, 3H).

### Example 143: Synthesis of 2-(4-fluorophenyl)-7,7-dimethyl-1,3-dioxo-2,3,5,12b-tetrahydro-1H,7H-chromeno[4,3-c][1,2,4]triazolo[1,2-a]pyridazin-10-yl dihydrogen phosphate

### (a) Synthesis of 2-(4-fluorophenyl)-7,7-dimethyl-10-((tetrahydro-2H-pyran-2-yl)oxy)-5,12b-dihydro-1H,7H-chromeno[4,3-c][1,2,4]triazolo[1,2-a]pyridazine-1,3(2H)-dione

Except that 2,2-dimethyl-7-((tetrahydro-2*H*-pyran-2-yl)oxy)-3-vinyl-2*H*-chromene and 4-(4-fluorophenyl)-1,2,4-triazolidine-3,5-dione were used instead of ((2,2-bis(fluoromethyl)-3-vinyl-2*H*-chromen-7-yl)oxy)triisopropylsilane and 4-(4-acetylphenyl)-1,2,4-triazolidine-3,5-dione, respectively, the same synthesis method as in Example 2 (a) above was carried out to obtain 2-(4-fluorophenyl)-7,7-dimethyl-10-((tetrahydro-2H-pyran-2-yl)oxy)-5,12b-dihydro-1*H*,7*H-*chromeno[4,3-*c*][1,2,4]triazolo[1,2-*a*]pyridazine-1,3(2*H*)-dione (0.6 g, 10%).

### (b) Synthesis of 2-(4-fluorophenyl)-10-hydroxy-7,7-dimethyl-5,12b-dihydro-1H,7H-chromeno[4,3-c][1,2,4]triazolo[1,2-a]pyridazine-1,3(2H)-dione

Except that 2-(4-fluorophenyl)-7,7-dimethyl-10-((tetrahydro-2H-pyran-2-yl)oxy)-5,12b-dihydro-1*H*,7*H*-chromeno[4,3-*c*][1,2,4]triazolo[1,2-*a*]pyridazine-1,3(2*H*)-dione was used instead of 2,2-bis(fluoromethyl)-7-((tetrahydro-2*H*-pyran-2-yl)oxy)chroman-4-one, the same synthesis method as in Intermediate 31 (d) above was carried out to obtain 2-(4-fluorophenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (0.37 g, 74%).

### (c) Synthesis of diethyl (2-(4-fluorophenyl)-7,7-dimethyl-1,3-dioxo-2,3,5,12b-tetrahydro-1H,7H-chromeno[4,3-c][1,2,4]triazolo[1,2-a]pyridazine-10-yl) phosphate

Except that diethyl chlorophosphate was used instead of trimethylacetyl chloride, the same synthesis method as in Example 58 above was carried out to obtain diethyl (2-(4-fluorophenyl)-7,7-dimethyl-1,3-dioxo-2,3,5,12b-tetrahydro-1*H*,7*H*-chromeno[4,3-*c*][1,2,4]triazolo[1,2-*a*]pyridazine-10-yl) phosphate (230 mg, 89%).

### (d) Synthesis of 2-(4-fluorophenyl)-7,7-dimethyl-1,3-dioxo-2,3,5,12b-tetrahydro-1H,7H-chromeno[4,3-c][1,2,4]triazolo[1,2-a]pyridazin-10-yl dihydrogen phosphate

Except that diethyl (2-(4-fluorophenyl)-7,7-dimethyl-1,3-dioxo-2,3,5,12b-tetrahydro-1*H*,7*H*-chromeno[4,3-*c*][1,2,4]triazolo[1,2-*a*]pyridazine-10-yl) phosphate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diethyl phosphate, the same synthesis method as in Example 69 (b) above was carried out to obtain 2-(4-fluorophenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dihydrogen phosphate (50 mg, 44%).
¹H NMR (400 MHz, DMSO-D₆) δ 7.66 (dd, J = 8.8, 5.0 Hz, 2H), 7.40 (t, J = 8.8 Hz, 2H), 7.04 (d, J = 8.5 Hz, 1H), 6.75 (dd, J = 8.5, 2.3 Hz, 1H), 6.69 (d, J = 2.4 Hz, 1H), 6.06 - 5.93 (m, 1H), 5.68 (s, 1H), 4.26 - 4.12 (m, 2H), 1.58 (s, 3H), 1.54 (s, 3H).

### Example 144: Synthesis of 2-(4-(tert-butyl)phenyl)-7,7-dimethyl-1,3-dioxo-2,3,5,12b-tetrahydro-1H,7H-chromeno[4,3-c][1.2.4]triazolo[1.2-a]pyridazin-10-yl dihydrogen phosphate

### (a) Synthesis of 2-(4-(tert-butyl)phenyl)-7,7-dimethyl-10-((tetrahydro-2H-pyran-2-yl)oxy)-5,12b-dihydro-1H,7H-chromeno[4,3-c][1,2,4]triazolo[1,2-a]pyridazine-1,3(2H)-dione

Except that 2,2-dimethyl-7-((tetrahydro-2*H*-pyran-2-yl)oxy)-3-vinyl-2*H*-chromene and 4-(4-(tert-butyl)phenyl)-1,2,4-triazolidine-3,5-dione were used instead of ((2,2-bis(fluoromethyl)-3-vinyl-2*H*-chromen-7-yl)oxy)triisopropylsilane and 4-(4-acetylphenyl)-1,2,4-triazolidine-3,5-dione, respectively, the same synthesis method as in Example 2 (a) above was carried out to obtain 2-(4-(tert-butyl)phenyl)-7,7-dimethyl-10-((tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione (0.46 g, 7%).

### (b) Synthesis of 2-(4-(tert-butyl)phenyl)-10-hydroxy-7,7-dimethyl-5,12b-dihydro-1H,7H-chromeno[4,3-c][1,2,4]triazolo[1,2-a]pyridazine-1,3(2H-dione

Except that 2-(4-(tert-butyl)phenyl)-7,7-dimethyl-10-((tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione was used instead of 2,2-bis(fluoromethyl)-7-((tetrahydro-2H-pyran-2-yl)oxy)chroman-4-one, the same synthesis method as in Intermediate 31 (d) above was carried out to obtain 2-(4-(tert-butyl)phenyl)-10-hydroxy-7,7-dimethyl-5,12b-dihydro-1*H*,7*H*-chromeno[4,3-*c*][1,2,4]triazolo[1,2-*a*]pyridazine-1,3(2*H*)-dione (100 mg, 28%).

### (c) Synthesis of 2-(4-(tert-butyl)phenyl)-7,7-dimethyl-1,3-dioxo-2,3,5,12b-tetrahydro-1H,7H-chromeno[4,3-c][1,2,4]triazolo[1,2-a]pyridazin-10-yl diethyl phosphate

Except that diethyl chlorophosphate was used instead of trimethylacetyl chloride, the same synthesis method as in Example 58 above was carried out to obtain 2-(4-(tert-butyl)phenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diethyl phosphate (80 mg, 90%).

### (d) Synthesis of 2-(4-(tert-butyl)phenyl)-7,7-dimethyl-1,3-dioxo-2,3,5,12b-tetrahydro-1H,7H-chromeno[4,3-c][1,2,4]triazolo[1,2-a]pyridazin-10-yl dihydrogen phosphate

Except that 2-(4-(tert-butyl)phenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl diethyl phosphate was used instead of 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl diethyl phosphate, the same synthesis method as in Example 69 (b) above was carried out to obtain 2-(4-(tert-butyl)phenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dihydrogen phosphate (25 mg, 40%).
¹H NMR (400 MHz, DMSO-D₆) δ 7.57 (d, J = 8.5 Hz, 2H), 7.50 (d, J = 8.4 Hz, 2H), 7.00 (d, J = 8.5 Hz, 1H), 6.79 - 6.72 (m, 1H), 6.70 (d, J = 2.4 Hz, 1H), 6.00 (s, 1H), 5.68 (s, 1H), 4.24 - 4.11 (m, 2H), 1.58 (s, 3H), 1.54 (s, 3H), 1.33 (s, 9H).

### Example 145: Synthesis of 7,7-dimethyl-1,3-dioxo-2-(4-(trifluoromethyl)phenyl)-2,3,5,12b-tetrahydro-1H,7H-chromeno[4,3-c][1,2,4]triazolo[1,2-a]pyridazin-10-yl dimethylcarbamate

Except that 10-hydroxy-7,7-dimethyl-2-(4-(trifluoromethyl)phenyl)-5,12b-dihydro-1*H*,7*H*-chromeno[4,3-*c*][1,2,4]triazolo[1,2-*a*]pyridazine-1,3(2*H*)-dione and dimethylcarbamoyl chloride were used instead of 2-(4-acetylphenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione and trimethylacetyl chloride, respectively, the same synthesis method as in Example 58 above was carried out to obtain 7,7-dimethyl-1,3-dioxo-2-(4-(trifluoromethyl)phenyl)-2,3,5,12b-tetrahydro-1H,7H-chromeno[4,3-*c*][1,2,4]triazolo[1,2-*a*]pyridazin-10-yl dimethylcarbamate (32 mg, 99%).
¹H NMR (400 MHz, DMSO-D₆) δ 7.96 (d, J = 8.6 Hz, 2H), 7.90 (d, J = 8.4 Hz, 2H), 7.09 (dd, J = 8.4, 1.3 Hz, 1H), 6.75 - 6.68 (m, 1H), 6.65 (d, J = 2.1 Hz, 1H), 6.05 - 5.98 (m, 1H), 5.74 - 5.68 (m, 1H), 4.25 - 4.19 (m, 2H), 3.00 (s, 3H), 2.88 (s, 3H), 1.59 (s, 3H), 1.53 (s, 3H).

### Example 146: Synthesis of 2-(4-fluorophenyl)-7,7-dimethyl-1,3-dioxo-2,3,5,12b-tetrahydro-1H,7H-chromeno[4,3-c][1,2,4]triazolo[1,2-a]pyridazin-10-yl dimethylcarbamate

Except that 2-(4-fluorophenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione and dimethylcarbamoyl chloride were used instead of 2-(4-acetylphenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione and trimethylacetyl chloride, respectively, the same synthesis method as in Example 58 above was carried out to obtain 2-(4-fluorophenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dimethylcarbamate (40 mg, 96%).
¹H NMR (400 MHz, DMSO-D₆) δ 7.70 - 7.60 (m, 2H), 7.46 - 7.35 (m, 2H), 7.06 (d, J = 8.3 Hz, 1H), 6.72 (dd, J = 8.4, 2.4 Hz, 1H), 6.65 (d, J = 2.3 Hz, 1H), 6.04 - 5.97 (m, 1H), 5.69 (s, 1H), 4.22 - 4.16 (m, 2H), 3.01 (s, 3H), 2.88 (s, 3H), 1.59 (s, 3H), 1.52 (s, 3H).

### Example 147: Synthesis of 2-(4-(tert-butyl)phenyl)-7,7-dimethyl-1,3-dioxo-2,3,5,12b-tetrahydro-1H,7H-chromeno[4,3-c][1,2,4]triazolo[1,2-a]pyridazin-10-yl dimethylcarbamate

Except that 2-(4-(tert-butyl)phenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione and dimethylcarbamoyl chloride were used instead of 2-(4-acetylphenyl)-10-hydroxy-7,7-dimethyl-5,12b-dihydro-1*H,*7*H-*chromeno[4,3-*c*][1,2,4]triazolo[1,2-*a*]pyridazine-1,3(2*H*)-dione and trimethylacetyl chloride, respectively, the same synthesis method as in Example 58 above was carried out to obtain 2-(4-(tert-butyl)phenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl dimethylcarbamate (44 mg, 99%).
¹H NMR (400 MHz, DMSO-D₆) δ 7.57 (d, J = 8.7 Hz, 2H), 7.53 - 7.46 (m, 2H), 7.02 (d, J = 8.4 Hz, 1H), 6.72 (dd, J = 8.4, 2.3 Hz, 1H), 6.65 (d, J = 2.3 Hz, 1H), 6.01 (s, 1H), 5.69 (s, 1H), 4.25 - 4.12 (m, 2H), 3.01 (s, 3H), 2.88 (s, 3H), 1.59 (s, 3H), 1.53 (s, 3H), 1.33 (s, 9H)

| **Compound No.** | **Structure** | **Compound No.** | **Structure** |
|---|---|---|---|
| 1 | 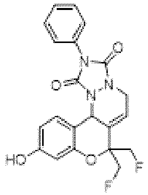 | 7 | 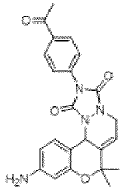 |
| 2 | 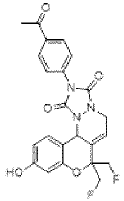 | 8 | 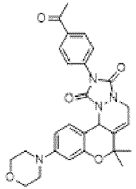 |
| 3 | 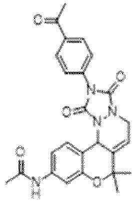 | 9 | 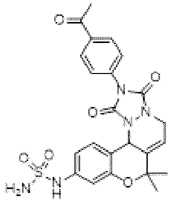 |
| 4 | 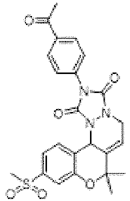 | 10 | 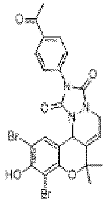 |
| 5 | 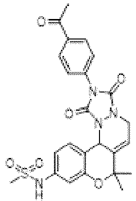 | 11 | 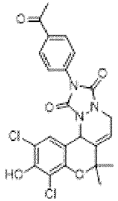 |
| 6 | 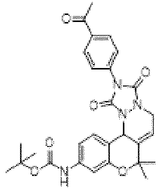 | 12 | 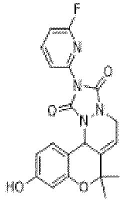 |
| 13 | 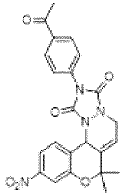 | 19 | 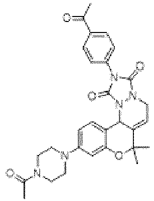 |
| 14 | 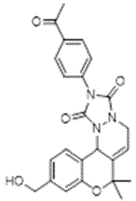 | 20 | 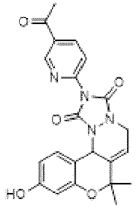 |
| 15 | 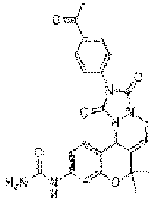 | 21 | 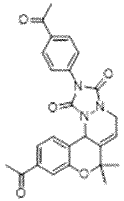 |
| 16 | 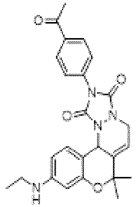 | 22 | 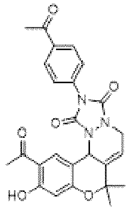 |
| 17 | 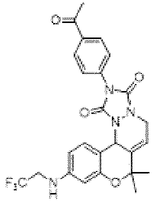 | 23 | 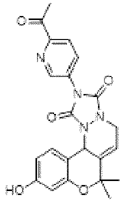 |
| 18 | 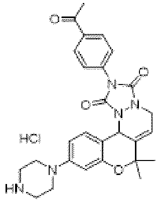 | 24 | 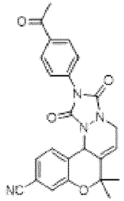 |
| 25 | 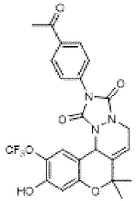 | 31 | 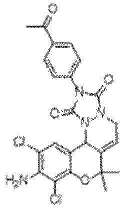 |
| 26 | 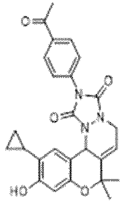 | 32 | 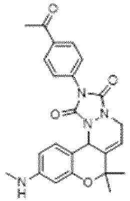 |
| 27 | 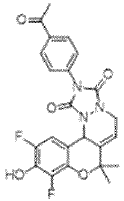 | 33 | 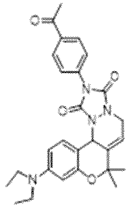 |
| 28 | 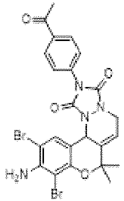 | 34 | 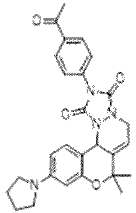 |
| 29 | 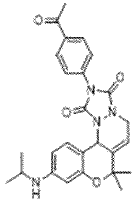 | 35 | 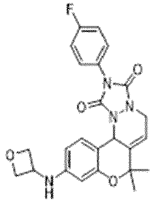 |
| 30 | 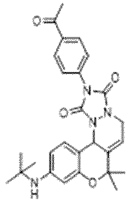 | 36 | 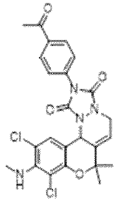 |
| 37 | 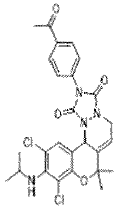 | 43 | 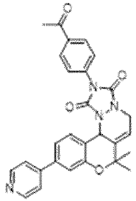 |
| 38 | 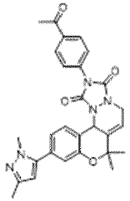 | 44 | 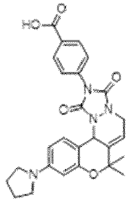 |
| 39 | 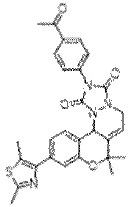 | 45 | 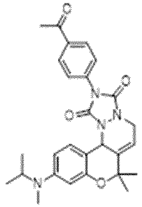 |
| 40 | 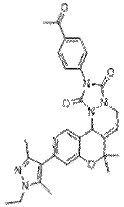 | 46 | 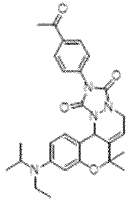 |
| 41 | 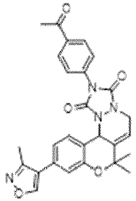 | 47 | 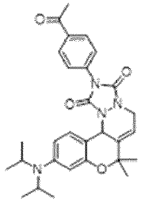 |
| 42 | 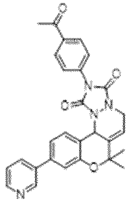 | 48 | 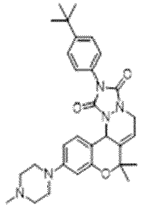 |
| 49 | 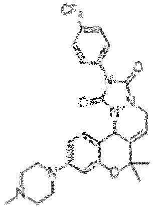 | 55 | 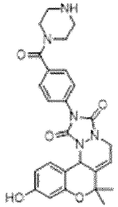 |
| 50 | 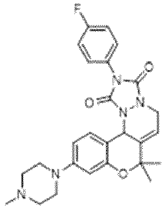 | 56 | 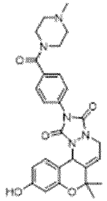 |
| 51 | 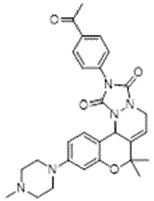 | 57 | 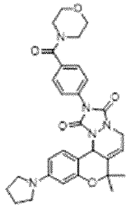 |
| 52 | 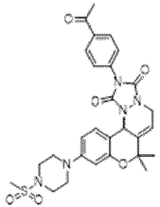 | 58 | 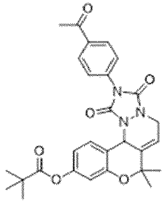 |
| 53 | 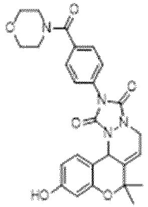 | 59 | 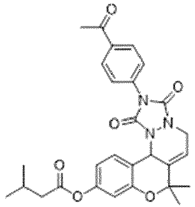 |
| 54 | 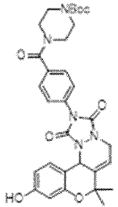 | 60 | 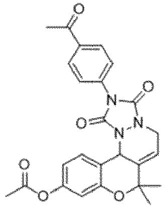 |
| 61 | 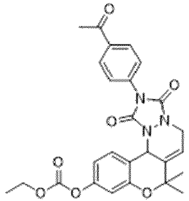 | 67 | 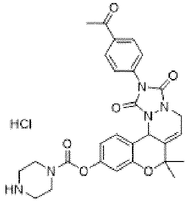 |
| 62 | 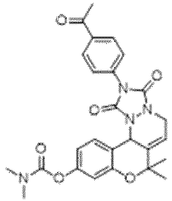 | 68 | 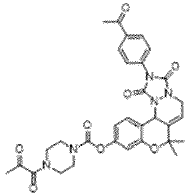 |
| 63 | 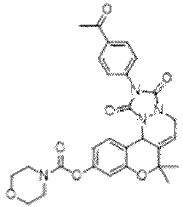 | 69 | 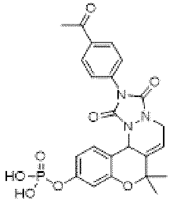 |
| 64 | 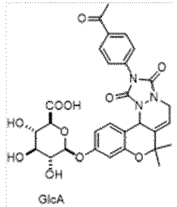 | 70 | 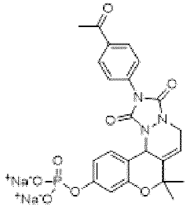 |
| 65 | 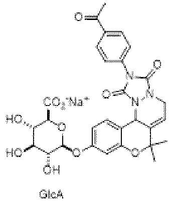 | 71 | 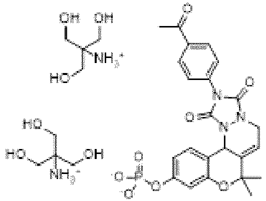 |
| 66 | 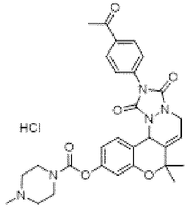 | 72 | 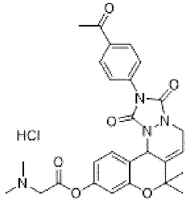 |
| 73 | 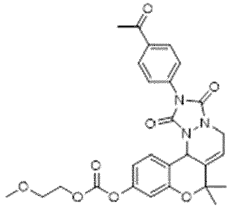 | 79 | 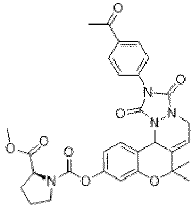 |
| 74 | 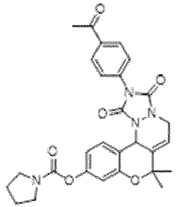 | 80 | 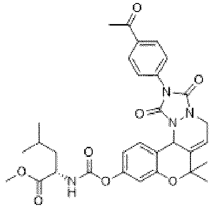 |
| 75 | 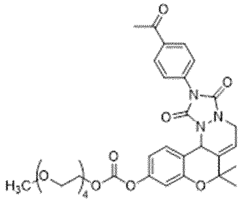 | 81 | 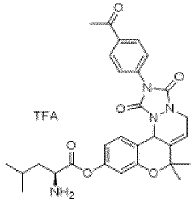 |
| 76 | 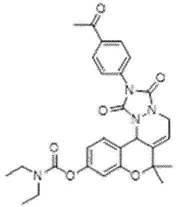 | 82 | 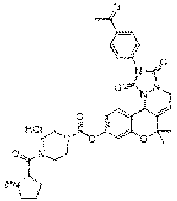 |
| 77 | 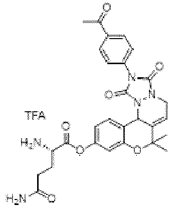 | 83 | 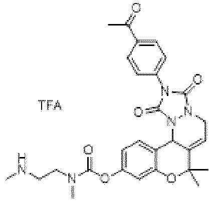 |
| 78 | 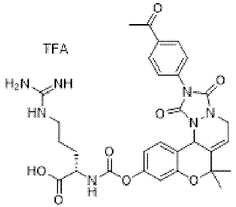 | 84 | 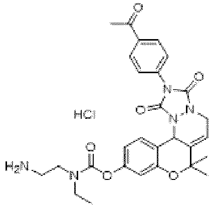 |
| 85 | 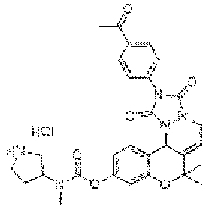 | 91 | 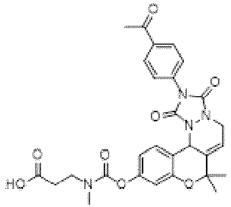 |
| 86 | 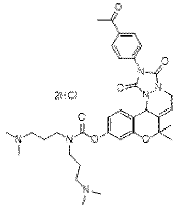 | 92 | 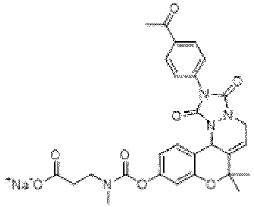 |
| 87 | 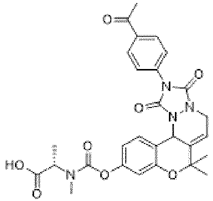 | 93 | 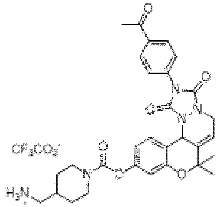 |
| 88 | 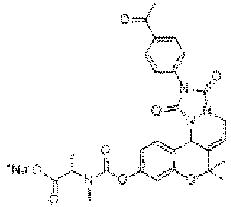 | 94 | 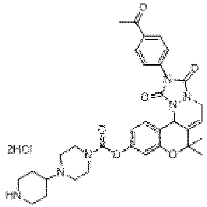 |
| 89 | 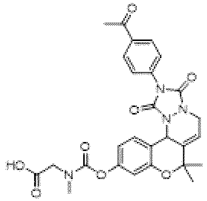 | 95 | 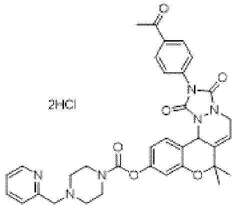 |
| 90 | 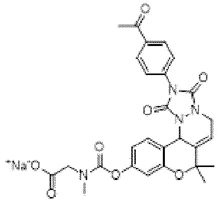 | 96 | 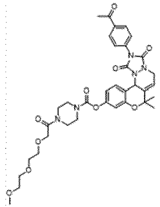 |
| 97 | 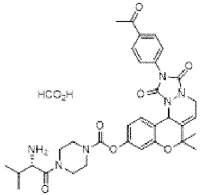 | 103 | 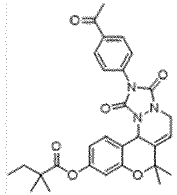 |
| 98 | 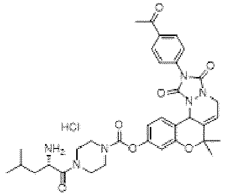 | 104 | 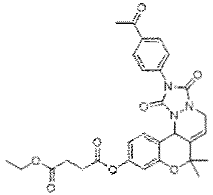 |
| 99 | 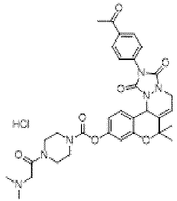 | 105 | 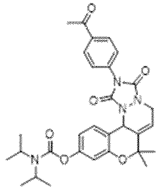 |
| 100 | 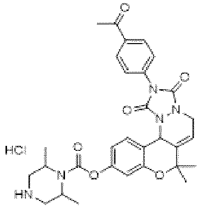 | 106 | 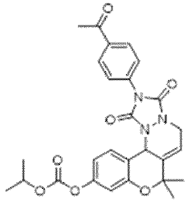 |
| 101 | 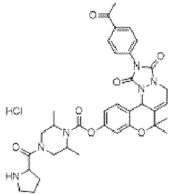 | 107 | 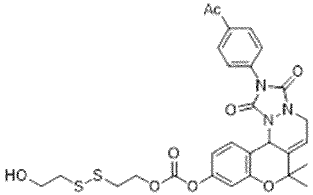 |
| 102 | 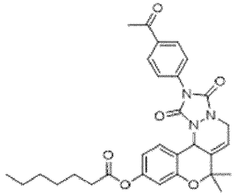 | 108 | 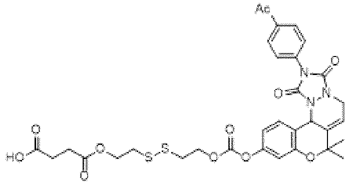 |
| 109 | 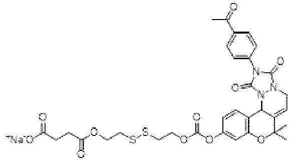 | 115 | 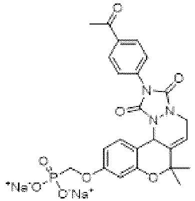 |
| 110 | 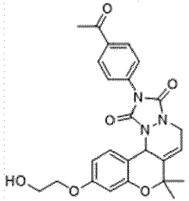 | 116 | 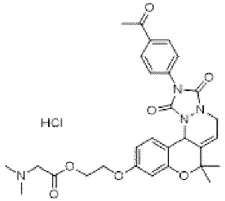 |
| 111 | 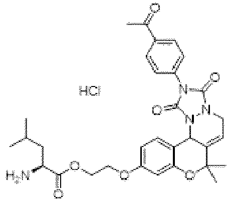 | 117 | 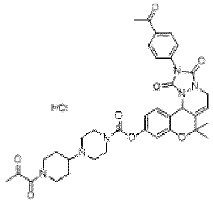 |
| 112 | 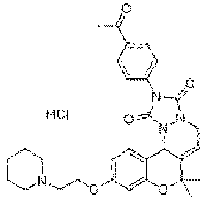 | 118 | 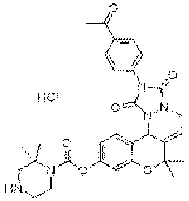 |
| 113 | 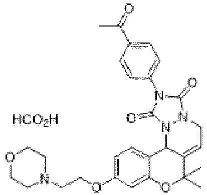 | 119 | 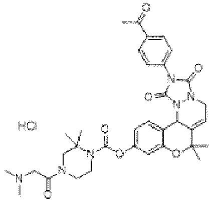 |
| 114 | 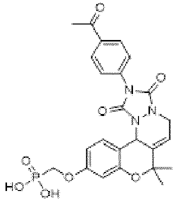 | 120 | 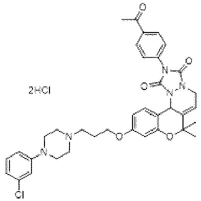 |
| 121 | 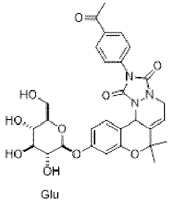 | 127 | 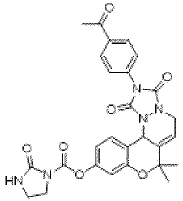 |
| 122 | 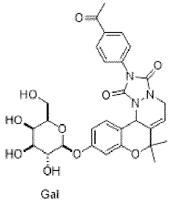 | 128 | 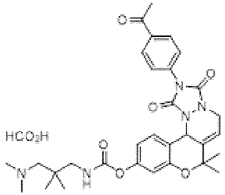 |
| 123 | 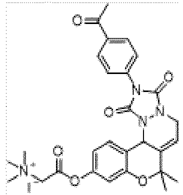 | 129 | 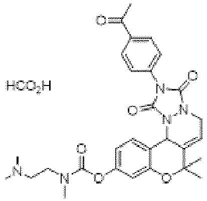 |
| 124 | 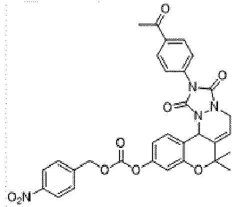 | 130 | 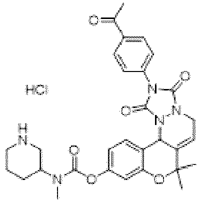 |
| 125 | 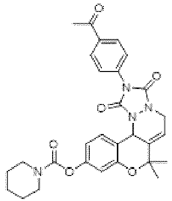 | 131 | 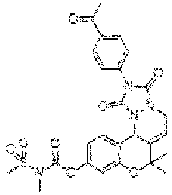 |
| 126 | 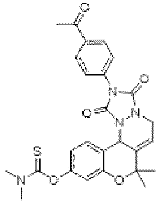 | 132 | 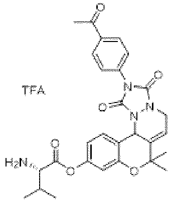 |
| 133 | 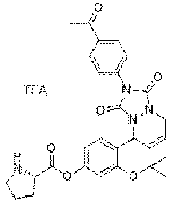 | 139 | 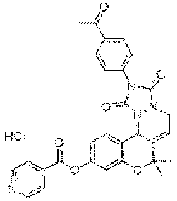 |
| 134 | 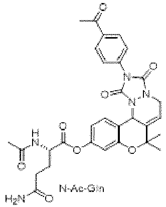 | 140 | 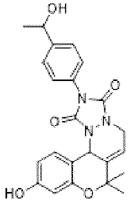 |
| 135 | 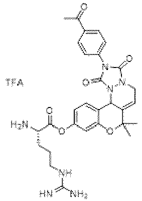 | 141 | 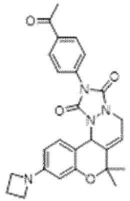 |
| 136 | 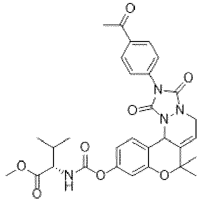 | 142 | 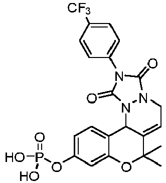 |
| 137 | 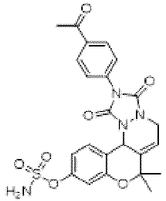 | 143 | 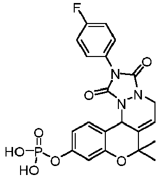 |
| 138 | 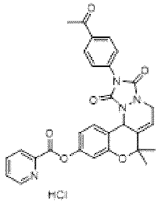 | 144 | 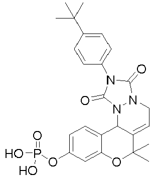 |
| 145 | 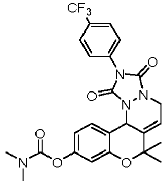 | 147 | 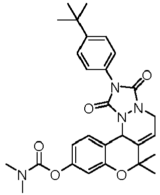 |
| 146 | 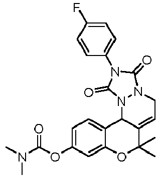 | | |

### Experimental Example 1: Confirmation of Tumor Growth Inhibition Effect

The tumor growth inhibition effect of benzopyrene derivatives was evaluated in a C57BL/6 mouse tumor model (syngeneic mouse model) in which melanoma cells (B16F10) derived from C57BL/6 mice are implanted.

After the quarantine and acclimation period was over, a cell suspension prepared with 5×10⁵ cells/0.1 mL PBS was filled into a disposable syringe and administered in an amount of 0.1 mL PBS/head subcutaneously to the right back of healthy test animals (female C57BL/6, 7 weeks old) for implantation. After cell line implantation, general symptoms were observed once daily during the engraftment and growth period. Animals with no abnormalities in their health and whose tumor volume reached 50 to 100 mm³ when measured with a digital caliper were selected. Then, based on tumor volume and body weight, the groups were separated so that the average tumor volume per group was 70 to 100 mm³. Ten (10) animals were separated per group, and 20 mg/kg of benzopyran derivatives were administered orally once a day for 9 or 10 days. Tumor volume was measured once every 2 or 3 days.

The measured tumor volume of the test compound administered groups was compared with that of the control group and is represented in Table 2 as the tumor growth inhibition (TGI, %).

As a result, as can be seen from Table 2, it was confirmed that the compounds of Example 44, Example 46, Example 68, Example 69, Example 71, Example 72, Example 74, Example 81 and Example 91 show 25 to 66% tumor growth inhibition effect.

**[Table 2]**

| **Example** | **TGI (%)*** | **Example** | **TGI (%)** |
|---|---|---|---|
| **60** | 56.5 | **73** | 31.0 |
| **62** | 32.3 | **75** | 25.5 |
| **68** | 33.7 | **82** | 66.3 |
| **70** | 26.2 | **92** | 25.5 |
| **72** | 29.6 | | |

| | | | |
|---|---|---|---|
| * TGI(%) = (Central group TV - Test compound administered group TV) / Control group TV × 100 | | | |

### Experimental Example 2: Confirmation of Inhibitory Effect against the Activity of Human M2 Macrophages

To obtain human M2 macrophages, human monocytes (THP-1 monocyte, CD4 expression) were stabilized in a 96-well plate for 24 hours and then incubated in culture media containing 100 ng/mL phorbal 12-myristate-13-acetate (PMA; Sigma, P8139) for 24 hours to differentiate into M0 macrophages (macrophage-like phenotype). It was confirmed that differentiated M0 macrophages had reduced CD4 expression and expressed CD11b. M0 macrophages in the plate well were washed once with culture media and incubated in culture media containing 25 ng/mL hIL-4 (Peprotech, 200-04-50) and 25 ng/mL hIL-13 (Peprotech, 200-13-50) for 72 hours to differentiate into M2-like macrophages. Test compounds were diluted by concentration in M2 differentiation media and treated simultaneously during a differentiation period of 72 hours. The inhibitory effect of the test compouns on M2 macrophages was determined by measuring hCCL22 in the medium culture supernatant cultured for 72 hours using an ELISA assay (R&D system, DMD00). The results of the activity measurements are represented in Table 3 (EC₅₀), Table 4 (hCCL22 inhibitory activity at 5 µM) and Table 5 (hCCL22 inhibitory activity at 10 µM).

As a result, in Tables 3 to 5 it was confirmed that the secretion of hCCL22 was suppressed in differentiated M2 macrophages by the Example compounds at 100 µM or less.

**[Table 3]**

| **Example** | **EC₅₀ (µM)** | **Example** | **EC₅₀ (µM)** | **Example** | **EC₅₀ (µM)** |
|---|---|---|---|---|---|
| **7** | 4.5 | **44** | 9.5 | **78** | 5.2 |
| **16** | 4.0 | **45** | 9.1 | **82** | 68.6 |
| **17** | 8.2 | **46** | 7.0 | **99** | 32.0 |
| **29** | 3.3 | **47** | 8.1 | **100** | 15.9 |
| **30** | 4.3 | **48** | 9.7 | **101** | 17.9 |
| **31** | 2.0 | **49** | 12.1 | **115** | >80.0 |
| **32** | 6.1 | **50** | 25.6 | **118** | 5.3 |
| **33** | 5.8 | **51** | 33.5 | **119** | 9.5 |
| **34** | 7.7 | **52** | 26.4 | **141** | 7.2 |
| **35** | 4.8 | **53** | 4.5 | **142** | 6.3 |
| **36** | 10.8 | **54** | 1.5 | **143** | 9.4 |
| **37** | 14.7 | **58** | 58.7 | **144** | 5.4 |
| **38** | 31.6 | **62** | 41.3 | **145** | 77.3 |
| **39** | 73.1 | **67** | 17.5 | **146** | >80 |
| **40** | 53.8 | **68** | 74.7 | **147** | >80 |
| **42** | 31.2 | **70** | 9.4 | | |
| **43** | 19.2 | **72** | 4.7 | | |

**[Table 4]**

| **Example** | **Activity (%)** | **Example** | **Activity (%)** | **Example** | **Activity (%)** |
|---|---|---|---|---|---|
| **25** | 42.00 | **27** | 29.86 | **28** | 71.22 |

**[Table 5]**

| **Example** | **Activity (%)** | **Example** | **Activity (%)** | **Example** | **Activity (%)** |
|---|---|---|---|---|---|
| **1** | 80.36 | **9** | 74.81 | **30** | 97.33 |
| **2** | 64.37 | **10** | 98.43 | **31** | 97.23 |
| **3** | 16.93 | **11** | 78.07 | **32** | 84.37 |
| **4** | 51.86 | **12** | 16.76 | **33** | 96.47 |
| **5** | 42.26 | **16** | 79.87 | **34** | 73.70 |
| **7** | 83.43 | **19** | 18.75 | **35** | 96.67 |
| **8** | 60.70 | **29** | 95.23 | **141** | 94.23 |

## Claims

1. A compound of the following Formula 1, or a pharmaceutically acceptable salt or isomer thereof: 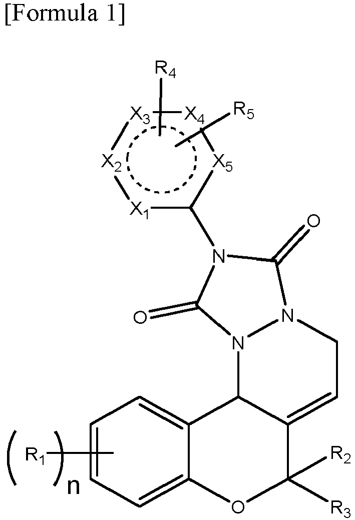 wherein
X₁, X₂, X₃, X₄ and X₅ are each independently carbon or nitrogen;
R₁ is halogen, hydroxy, nitro, amino, cyano, alkyl, cycloalkyl, alkylamino, dialkylamino, alkylcarbonyl, haloalkyl, haloalkoxy, hydroxyalkyl, hydroxyalkoxy, alkylsulfonyl, alkylsulfonylamino, aminosulfonyloxy, 3- to 10-membered heterocycloalkyl, 5- to 10-membered heteroaryl, 3- to 10-membered heterocycloalkyl-oxy, 3- to 10-membered heterocyclyl-alkoxy, or any one of the following formulas (i) to (vii), wherein the heterocycloalkyl or heteroaryl may be substituted with one or more substituents selected from the group consisting of hydroxy, carboxy, alkyl, alkylsulfonyl, alkylcarbonyl, oxo, hydroxyalkyl and halophenyl; 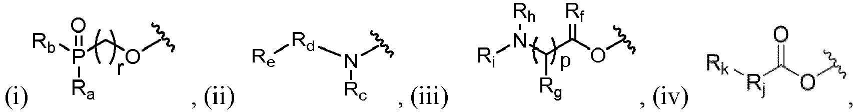 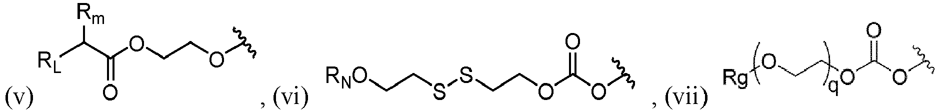
Ra and Rb are each independently hydroxy or alkoxy;
Rc is hydrogen or alkyl;
Rd is a direct bond, carbonyl or sulfonyl;
Re is hydrogen, alkyl, haloalkyl, alkoxy, amino or 3- to 10-membered heterocycloalkyl;
Rf is O or S;
Rg is hydrogen, alkyl, aminocarbonylalkyl or guanidinoalkyl;
Rh is hydrogen, alkyl, dialkylaminoalkyl, or may be fused with Ri to form 3- to 12-membered heterocycloalkyl, wherein the heterocycloalkyl may be substituted with one or more substituents selected from the group consisting of alkyl, oxo, aminoalkyl, dialkylaminoalkylcarbonyl, alkoxycarbonyl, acetylcarbonyl, 3- to 10-membered heterocycloalkyl, 5- to 10-membered heteroaryl, 3- to 10-membered heterocycloalkylcarbonyl, 3- to 10-membered heterocycloalkyl-alkyl, 5- to 10-membered heteroarylalkyl, acetylcarbonylheterocycloalkyl, alkoxyalkoxyalkoxyalkylcarbonyl and alkylcarbonyl substituted with amino;
Ri is hydrogen, alkyl, aminoalkyl, carboxyalkyl, alkylaminoalkyl, dialkylaminoalkyl, alkylsulfonyl, alkylcarbonyl, 3- to 10-membered heterocycloalkyl, alkyl substituted with alkoxycarbonyl, guanidinoalkyl substituted with carboxy, acetylamino or alkoxycarbonylalkyl;
Rj is a direct bond, alkylene, arylene-alkyleneoxy or alkylene substituted with alkylcarbonylamino;
Rk is alkyl, alkoxycarbonylalkyl, alkoxy, trialkylamino, nitro, alkyl substituted with amino, aminocarbonylalkyl, 3- to 10-membered heterocycloalkyl or 5- to 10-membered heteroaryl;
RL is amino or dialkylamino;
Rm is hydrogen or alkyl;
RN is hydrogen or carboxyalkylcarbonyl;
R₂ and R₃ are each independently alkyl or haloalkyl;
R₄ is hydrogen, halogen, alkyl, alkylcarbonyl, haloalkyl, carboxy, hydroxyalkyl, 3- to 10-membered heterocycloalkylcarbonyl, or 3- to 10-membered heterocycloalkylcarbonyl substituted with alkyl or alkoxycarbonyl;
R₅ is hydrogen, halogen or alkyl;
n is an integer of 1 to 3;
r and p are an integer of 0 to 3; and
q is an integer of 1 to 4;
provided that when R₁ is hydroxy and n is 1, i) R₂ and R₃ are not alkyl at the same time, ii) at least one of X₁, X₂, X₃, X₄ and X₅ is nitrogen, or iii) R₄ is hydroxyalkyl, 3- to 10-membered heterocycloalkylcarbonyl, or 3- to 10-membered heterocycloalkylcarbonyl substituted with alkyl or alkoxycarbonyl; and
when R₁ is halogen or alkylamino, R₄ and R₅ are not hydrogen at the same time; and
wherein the heterocycloalkyl and heteroaryl have one or more heteroatoms selected from the group consisting of N, O and S.

2. The compound, or a pharmaceutically acceptable salt or isomer thereof according to Claim 1, wherein
X₁, X₂, X₃, X₄ and X₅ are each independently -CH=, =CH-, -N= or =N-; or -C= or =C-when it is substituted with R₄ or R₅;
R₁ is halogen, hydroxy, nitro, amino, cyano, C₁-C₇ alkyl, C₃-C₁₀ cycloalkyl, C₁-C₇ alkylamino, di(C₁-C₇ alkyl)amino, C₁-C₇ alkylcarbonyl, halo-C₁-C₇ alkyl, halo-C₁-C₇ alkoxy, hydroxy-C₁-C₇ alkyl, hydroxy-C₁-C₇ alkoxy, C₁-C₇ alkylsulfonyl, C₁-C₇ alkylsulfonylamino, aminosulfonyloxy, 3- to 10-membered heterocycloalkyl, 5- to 10-membered heteroaryl, 3- to 10-membered heterocycloalkyl-oxy, 3- to 10-membered heterocyclyl-alkoxy, or any one of the following formulas (i) to (vii), wherein the heterocycloalkyl or heteroaryl may be substituted with 1 to 4 substituents selected from the group consisting of hydroxy, carboxy, C₁-C₇ alkyl, C₁-C₇ alkylsulfonyl, C₁-C₇ alkylcarbonyl, oxo, hydroxy-C₁-C₇ alkyl and halophenyl; 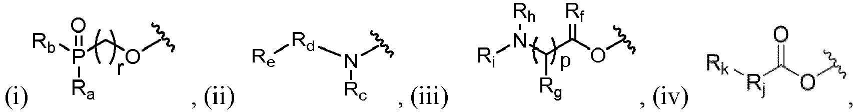 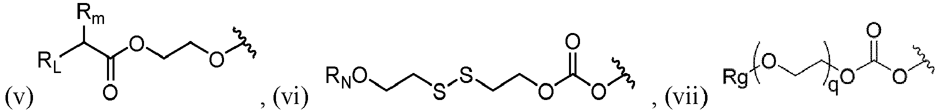
Ra and Rb are each independently hydroxy or C₁-C₇ alkoxy;
Rc is hydrogen or C₁-C₇ alkyl;
Rd is a direct bond, carbonyl or sulfonyl;
Re is hydrogen, C₁-C₇ alkyl, halo-C₁-C₇ alkyl, C₁-C₇ alkoxy, amino or 3- to 10-membered heterocycloalkyl;
Rf is O or S;
Rg is hydrogen, C₁-C₇ alkyl, aminocarbonyl-C₁-C₇ alkyl or guanidino-C₁-C₇ alkyl;
Rh is hydrogen, C₁-C₇ alkyl, di(C₁-C₇ alkyl)amino-C₁-C₇ alkyl, or may be fused with Ri to form 3- to 12-membered heterocycloalkyl, wherein the heterocycloalkyl may be substituted with 1 to 4 substituents selected from the group consisting of C₁-C₇ alkyl, oxo, amino-C₁-C₇ alkyl, di(C₁-C₇ alkyl)amino-C₁-C₇ alkylcarbonyl, C₁-C₇ alkoxycarbonyl, acetylcarbonyl, 3- to 10-membered heterocycloalkyl, 5- to 10-membered heteroaryl, 3- to 10-membered heterocycloalkylcarbonyl, 3- to 10-membered heterocycloalkyl-C₁-C₇ alkyl, 5- to 10-membered heteroaryl-C₁-C₇ alkyl, acetylcarbonyl-3- to 10-membered heterocycloalkyl, C₁-C₇ alkoxy-C₁-C₇ alkoxy-C₁-C₇ alkoxy-C₁-C₇ alkylcarbonyl and C₁-C₇ alkylcarbonyl substituted with amino;
Ri is hydrogen, C₁-C₇ alkyl, amino-C₁-C₇ alkyl, carboxy-C₁-C₇ alkyl, C₁-C₇ alkylamino-C₁-C₇ alkyl, di(C₁-C₇ alkyl)amino-C₁-C₇ alkyl, C₁-C₇ alkylsulfonyl, C₁-C₇ alkylcarbonyl, 3- to 10-membered heterocycloalkyl, C₁-C₇ alkyl substituted with C₁-C₇ alkoxycarbonyl, guanidino-C₁-C₇ alkyl substituted with carboxy, acetylamino or C₁-C₇ alkoxycarbonyl-C₁-C₇ alkyl;
Rj is a direct bond, C₁-C₇ alkylene, C₆-C₁₀ arylene-C₁-C₇ alkyleneoxy or C₁-C₇ alkylene substituted with C₁-C₇ alkylcarbonylamino;
Rk is C₁-C₇ alkyl, C₁-C₇ alkoxycarbonyl-C₁-C₇ alkyl, C₁-C₇ alkoxy, tri(C₁-C₇ alkyl)amino, nitro, C₁-C₇ alkyl substituted with amino, aminocarbonyl-C₁-C₇ alkyl, 3- to 10-membered heterocycloalkyl or 5- to 10-membered heteroaryl;
RL is amino or di(C₁-C₇ alkyl)amino;
Rm is hydrogen or C₁-C₇ alkyl;
RN is hydrogen or carboxy-C₁-C₇ alkylcarbonyl;
R₂ and R₃ are each independently C₁-C₇ alkyl or halo-C₁-C₇ alkyl;
R₄ is hydrogen, halogen, C₁-C₇ alkyl, C₁-C₇ alkylcarbonyl, halo-C₁-C₇ alkyl, carboxy, hydroxy-C₁-C₇ alkyl, 3- to 10-membered heterocycloalkylcarbonyl, or 3- to 10-membered heterocycloalkylcarbonyl substituted with C₁-C₇ alkyl or C₁-C₇ alkoxycarbonyl;
R₅ is hydrogen, halogen or C₁-C₇ alkyl;
n is an integer of 1 to 3;
r and p are an integer of 0 to 3; and
q is an integer of 1 to 4;
provided that when R₁ is hydroxy and n is 1, i) R₂ and R₃ are not C₁-C₇ alkyl at the same time, ii) at least one of X₁, X₂, X₃, X₄ and X₅ is -N= or =N-, or iii) R₄ is hydroxy-C₁-C₇ alkyl, 3-to 10-membered heterocycloalkylcarbonyl, or 3- to 10-membered heterocycloalkylcarbonyl substituted with C₁-C₇ alkyl or C₁-C₇ alkoxycarbonyl; and
when R₁ is halogen or C₁-C₇ alkylamino, R₄ and R₅ are not hydrogen at the same time; and
wherein the heterocycloalkyl and heteroaryl have 1 to 4 heteroatoms selected from the group consisting of N, O and S.

3. The compound, or a pharmaceutically acceptable salt or isomer thereof according to Claim 1, wherein the compound of Formula 1 is a compound of the following Formula 2: 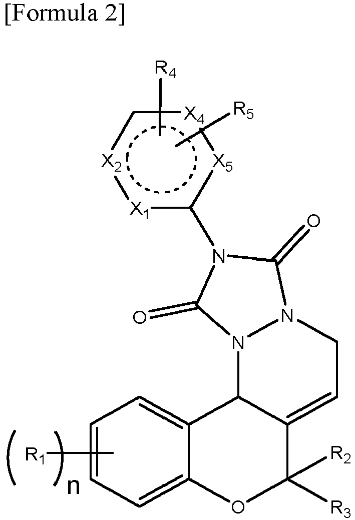 wherein X₁, X₂, X₄, X₅, R₁, R₂, R₃, R₄, R₅ and n are the same as defined in Claim 1.

4. The compound, or a pharmaceutically acceptable salt or isomer thereof according to Claim 1, wherein the compound of Formula 1 is a compound of the following Formula 3: 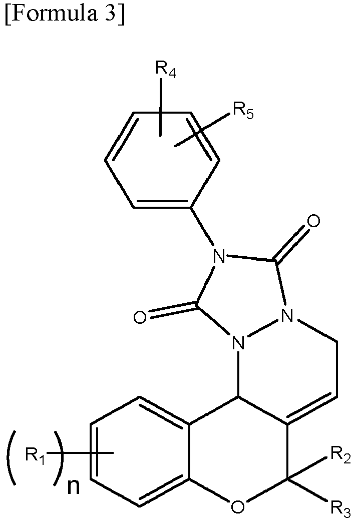 wherein R₁, R₂, R₃, R₄, R₅ and n are the same as defined in Claim 1.

5. The compound, or a pharmaceutically acceptable salt or isomer thereof according to Claim 1, wherein
R₁ is halogen, hydroxy, nitro, amino, cyano, C₁-C₅ alkyl, C₃-C₈ cycloalkyl, C₁-C₅ alkylamino, di(C₁-C₅ alkyl)amino, C₁-C₅ alkylcarbonyl, halo-C₁-C₅ alkyl, halo-C₁-C₅ alkoxy, hydroxy-C₁-C₅ alkyl, hydroxy-C₁-C₅ alkoxy, C₁-C₅ alkylsulfonyl, C₁-C₅ alkylsulfonylamino, aminosulfonyloxy, 4- to 8-membered heterocycloalkyl, 5- to 8-membered heteroaryl, 4- to 8-membered heterocycloalkyl-oxy, 4-to 8-membered heterocyclyl-alkoxy, or any one of the following formulas (i) to (vii); wherein the heterocycloalkyl may be substituted with 1 to 4 substituents selected from the group consisting of hydroxy, carboxy, C₁-C₅ alkyl, C₁-C₅ alkylsulfonyl, C₁-C₅ alkylcarbonyl, oxo, hydroxy-C₁-C₅ alkyl and halophenyl; and the heteroaryl may be substituted with 1 to 3 substituents selected from the group consisting of hydroxy, C₁-C₅ alkyl, C₁-C₅ alkylsulfonyl, C₁-C₅ alkylcarbonyl and hydroxy-C₁-C₅ alkyl; 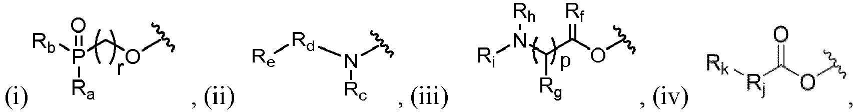 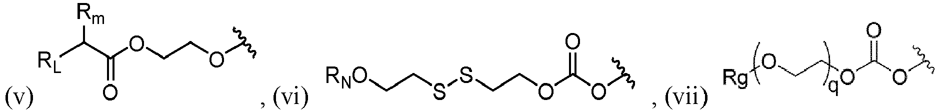
Ra and Rb are each independently hydroxy or C₁-C₅ alkoxy;
Rc is hydrogen or C₁-C₅ alkyl;
Rd is a direct bond, carbonyl or sulfonyl;
Re is hydrogen, C₁-C₅ alkyl, halo-C₁-C₅ alkyl, C₁-C₅ alkoxy, amino or 3- to 10-membered heterocycloalkyl;
Rf is O or S;
Rg is hydrogen, C₁-C₅ alkyl, aminocarbonyl-C₁-C₅ alkyl or guanidino-C₁-C₅ alkyl;
Rh is hydrogen, C₁-C₅ alkyl, di(C₁-C₅ alkyl)amino-C₁-C₅ alkyl, or may be fused with Ri to form 4- to 8-membered heterocycloalkyl, wherein the heterocycloalkyl may be substituted with one or more substituents selected from the group consisting of C₁-C₅ alkyl, oxo, amino-C₁-C₅ alkyl, di(C₁-C₅ alkyl)amino-C₁-C₅ alkylcarbonyl, C₁-C₅ alkoxycarbonyl, acetylcarbonyl, 4- to 8-membered heterocycloalkyl, 5- to 8-membered heteroaryl, 4-8 membered heterocycloalkylcarbonyl, 4- to 8-membered heterocycloalkyl-Ci-Cs alkyl, 5- to 8-membered heteroaryl-C₁-C₅ alkyl, acetylcarbonyl-4- to 8-membered heterocycloalkyl, C₁-C₅ alkoxy-C₁-C₅ alkoxy-C₁-C₅ alkoxy-C₁-C₅ alkylcarbonyl and C₁-C₅ alkylcarbonyl substituted with amino;
Ri is hydrogen, C₁-C₅ alkyl, amino-C₁-C₅ alkyl, carboxy-C₁-C₅ alkyl, C₁-C₅ alkylamino-C₁-C₅ alkyl, di(C₁-C₅ alkyl)amino-C₁-C₅ alkyl, C₁-C₅ alkylsulfonyl, C₁-C₅ alkylcarbonyl, 3- to 10-membered heterocycloalkyl, C₁-C₅ alkyl substituted with C₁-C₅ alkoxycarbonyl, guanidino-C₁-C₅ alkyl substituted with carboxy, acetylamino or C₁-C₅ alkoxycarbonyl-C₁-C₅ alkyl;
Rj is a direct bond, C₁-C₅ alkylene, C₆-C₁₀ arylene-C₁-C₅ alkyleneoxy, C₁-C₅ alkylene substituted with C₁-C₅ alkylcarbonylamino;
Rk is C₁-C₅ alkyl, C₁-C₅ alkoxycarbonyl-C₁-C₅ alkyl, C₁-C₅ alkoxy, tri(C₁-C₅ alkyl)amino, nitro, C₁-C₅ alkyl substituted with amino, aminocarbonyl-C₁-C₅ alkyl, 4- to 8-membered heterocycloalkyl or 5- to 8-membered heteroaryl;
RL is amino or di(C₁-C₅ alkyl)amino;
Rm is hydrogen or C₁-C₅ alkyl; and
RN is hydrogen or carboxy-C₁-C₅ alkylcarbonyl.

6. The compound, or a pharmaceutically acceptable salt or isomer thereof according to Claim 1, wherein R₂ and R₃ are each independently C₁-C₅ alkyl or halo-C₁-C₅ alkyl.

7. The compound, or a pharmaceutically acceptable salt or isomer thereof according to Claim 1, wherein
R₄ is hydrogen, halogen, C₁-C₅ alkyl, C₁-C₅ alkylcarbonyl, halo-C₁-C₅ alkyl, carboxy, hydroxy-C₁-C₅ alkyl, 4- to 8-membered heterocycloalkylcarbonyl, or 4- to 8-membered heterocycloalkylcarbonyl substituted with C₁-C₅ alkyl or C₁-C₅ alkoxycarbonyl; and
R₅ is hydrogen, halogen or C₁-C₅ alkyl.

8. The compound, or a pharmaceutically acceptable salt or isomer thereof according to Claim 1, wherein wherein the compound of Formula 1 is selected from the group consisting of:
1) 7,7-bis(fluoromethyl)-10-hydroxy-2-phenyl-5,12b-dihydro-1*H*,7*H*-chromeno[4,3-*c*][1,2,4]triazolo[1,2-*a*]pyridazine-1,3(2*H*)-dione;
2) 2-(4-acetylphenyl)-7,7-bis(fluoromethyl)-10-hydroxy-5,12b-dihydro-1*H*,7*H-*chromeno[4,3-*c*][1,2,4]triazolo[1,2-*a*]pyridazine-1,3(2*H*)-dione;
3) *N*-(2-(4-acetylphenyl)-7,7-dimethyl-1,3-dioxo-2,3,5,12b-tetrahydroxy-1*H*,7*H-*chromeno[4,3-*c*][1,2,4]triazolo[1,2-*a*]pyridazin-10-yl)acetamide;
4) 2-(4-acetylphenyl)-7,7-dimethyl-10-(methylsulfonyl)-5,12b-dihydro-1*H*,7*H-*chromeno[4,3-*c*][1,2,4]triazolo[1,2-*a*]pyridazine-1,3(2*H*)-dione;
5) *N*-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)methanesulfonamide;
6) tert-butyl (2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)carbamate;
7) 2-(4-acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione;
8) 2-(4-acetylphenyl)-7,7-dimethyl-10-morpholino-5,12b-dihydro-1*H*,7*H*-chromeno[4,3-*c*][1,2,4]triazolo[1,2-*a*]pyridazine-1,3(2*H*)-dione;
9) *N-*(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)sulfuric diamide;
10) 2-(4-acetylphenyl)-9,11-dibromo-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
11) 2-(4-acetylphenyl)-9,11-dichloro-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
12) 2-(6-fluoropyridin-2-yl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
13) 2-(4-acetylphenyl)-7,7-dimethyl-10-nitro-5,12b-dihydro-1*H*,7*H*-chromeno[4,3-*c*][1,2,4]triazolo[1,2-*a*]pyridazine-1,3(2*H*)-dione;
14) 2-(4-acetylphenyl)-10-(hydroxymethyl)-7,7-dimethyl-5,12b-dihydro-1*H*,7*H-*chromeno[4,3-*c*][1,2,4]triazolo[1,2-*a*]pyridazine-1,3(2*H*)-dione;
15) 1-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)urea;
16) 2-(4-acetylphenyl)-10-(ethylamino)-7,7-dimethyl-5,12b-dihydro-1*H*,7*H-*chromeno[4,3-*c*][1,2,4]triazolo[1,2-*a*]pyridazine-1,3(2*H*)-dione;
17) 2-(4-acetylphenyl)-7,7-dimethyl-10-((2,2,2-trifluoroethyl)amino)-5,12b-dihydro-1*H*,7*H*-chromeno[4,3-*c*][1,2,4]triazolo[1,2-*a*]pyridazine-1,3(2*H*)-dione;
18) 2-*(*4-acetylphenyl)-7,7-dimethyl-10-(piperazin-1-yl)-5,12b-dihydro-1*H*,7*H-*chromeno[4,3-*c*][1,2,4]triazolo[1,2-*a*]pyridazine-1,3(2*H*)-dione hydrochloride;
19) 2-(4-acetylphenyl)-10-(4-acetylpiperazin-1-yl)-7,7-dimethyl-5,12b-dihydro-1*H*,7*H-*chromeno[4,3-*c*][1,2,4]triazolo[1,2-*a*]pyridazine-1,3(2*H*)-dione;
20) 2-(5-acetylpyridin-2-yl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
21) 10-acetyl-2-(4-acetylphenyl)-7,7-dimethyl-5,12b-dihydro-1*H*,7*H*-chromeno[4,3-*c*][1,2,4]triazolo[1,2-*a*]pyridazine-1,3(2*H*)-dione;
22) 11-acetyl-2-(4-acetylphenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
23) 2-(6-acetylpyridin-3-yl)-10-hydroxy-7,7-dimethyl-5,12b-dihydro-1*H*,7*H-*chromeno[4,3-c][1,2,4]triazolo[1,2-*a*]pyiidazine-1,3(2*H*)-dione;
24) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazine-10-carbonitrile;
25) 2-(4-acetylphenyl)-10-hydroxy-7,7-dimethyl-11-(trifluoromethoxy)-5,12b-dihydro-1*H*,7*H*-chromeno[4,3-*c*][1,2,4]triazolo[1,2-*a*]pyridazine-1,3(2*H*)-dione;
26) 2-(4-acetylphenyl)-11-cyclopropyl-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
27) 2-(4-acetylphenyl)-9,11-difluoro-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
28) 2-(4-acetylphenyl)-10-amino-9,11-dibromo-7,7-dimethyl-5,12b-dihydro-1*H*,7*H-*chromeno[4,3-*c*][1,2,4]triazolo[1,2-*a*]pyridazine-1,3(2*H*)-dione;
29) 2-(4-acetylphenyl)-10-(isopropylamino)-7,7-dimethyl-5,12b-dihydro-1*H*,7*H-*chromeno[4,3-*c*][1,2,4]triazolo[1,2-*a*]pyridazine-1,3(2*H*)-dione;
30) 2-(4-acetylphenyl)-10-(tert-butylamino)-7,7-dimethyl-5,12b-dihydro-1*H*,7*H-*chromeno[4,3-*c*][1,2,4]triazolo[1,2-*a*]pyridazine-1,3(2*H*)-dione;
31) 2-(4-acetylphenyl)-10-amino-9,11-dichloro-7,7-dimethyl-5,12b-dihydro-1*H*,7*H-*chromeno[4,3-*c*][1,2,4]triazolo[1,2-*a*]pyridazine-1,3(2*H*)-dione;
32) 2-(4-acetylphenyl)-7,7-dimethyl-10-(methylamino)-5,12b-dihydro-1*H*,7*H-*chromeno[4,3-*c*][1,2,4]triazolo[1,2-*a*]pyridazine-1,3(2*H*)-dione;
33) 2-(4-acetylphenyl)-10-(diethylamino)-7,7-dimethyl-5,12b-dihydro-1*H*,7*H-*chromeno[4,3-*c*][1,2,4]triazolo[1,2-*a*]pyridazine-1,3(2*H*)-dione;
34) 2-(4-acetylphenyl)-7,7-dimethyl-10-(pyrrolidin-1-yl)-5,12b-dihydro-1*H*,7*H-*chromeno[4,3-*c*][1,2,4]triazolo[1,2-*a*]pyridazine-1,3(2*H*)-dione;
35) 2-(4-fluorophenyl)-7,7-dimethyl-10-(oxetan-3-ylamino)-5,12b-dihydro-1*H*,7*H-*chromeno[4,3-*c*][1,2,4]triazolo[1,2-*a*]pyridazine-1,3(2*H*)-dione;
36) 2-(4-acetylphenyl)-9,11-dichloro-7,7-dimethyl-10-(methylamino)-5,12b-dihydro-1*H*,7*H*-chromeno[4,3-*c*][1,2,4]triazolo[1,2-*a*]pyridazine-1,3(2*H*)-dione;
37) 2-(4-acetylphenyl)-9,11-dichloro-10-(isopropylamino)-7,7-dimethyl-5,12b-dihydro-1*H*,7*H*-chromeno[4,3-*c*][1,2,4]triazolo[1,2-*a*]pyridazine-1,3(2*H*)-dione;
38) 2-(4-acetylphenyl)-10-(1,3-dimethyl-1*H*-pyrazol-5-yl)-7,7-dimethyl-5,12b-dihydro-1*H*,7*H*-chromeno[4,3-*c*][1,2,4]triazolo[1,2-*a*]pyridazine-1,3(2*H*)-dione;
39) 2-(4-acetylphenyl)-10-(2,5-dimethylthiazol-4-yl)-7,7-dimethyl-5,12b-dihydro-1*H*,7*H-*chromeno[4,3-*c*][1,2,4]triazolo[1,2-*a*]pyridazine-1,3(2*H*)-dione;
40) 2-(4-acetylphenyl)-10-(1-ethyl-3,5-dimethyl-1*H*-pyrazol-4-yl)-7,7-dimethyl-5,12b-dihydro-1*H*,7*H*-chromeno[4,3-*c*][1,2,4]triazolo[1,2-*a*]pyridazine-1,3(2*H*)-dione;
41) 2-(4-acetylphenyl)-7,7-dimethyl-10-(3-methylisoxazol-4-yl)-5,12b-dihydro-1*H*,7*H-*chromeno[4,3-*c*][1,2,4]triazolo[1,2-*a*]pyridazine-1,3(2*H*)-dione;
42) 2-(4-acetylphenyl)-7,7-dimethyl-10-(pyridin-3-yl)-5,12b-dihydro-1*H*,7*H-*chromeno[4,3-*c*][1,2,4]triazolo[1,2-*a*]pyridazine-1,3(2*H*)-dione;
43) 2-(4-acetylphenyl)-7,7-dimethyl-10-(pyridin-4-yl)-5,12b-dihydro-1*H*,7*H-*chromeno[4,3-*c*][1,2,4]triazolo[1,2-*a*]pyridazine-1,3(2*H*)-dione;
44) 4-(7,7-dimethyl-1,3-dioxo-10-(pyrrolidin-1-yl)-5,12b-dihydro-1*H*,7*H*-chromeno[4,3-*c*][1,2,4]triazolo[1,2-*a*]pyridazin-2(3*H*)-yl)benzoic acid;
45) 2-(4-acetylphenyl)-10-(isopropyl(methyl)amino)-7,7-dimethyl-5,12b-dihydro-1*H*,7*H-*chromeno[4,3-c][1,2,4]triazolo[1,2-*a*]pyridazine-1,3(2*H*)-dione;
46) 2-(4-acetylphenyl)-10-(ethyl(isopropyl)amino)-7,7-dimethyl-5,12b-dihydro-1*H*,7*H-*chromeno[4,3-*c*][1,2,4]triazolo[1,2-*a*]pyridazine-1,3(2*H*)-dione;
47) 2-(4-acetylphenyl)-10-(diisopropylamino)-7,7-dimethyl-5,12b-dihydro-1*H*,7*H-*chromeno[4,3-*c*][1,2,4]triazolo[1,2-*a*]pyridazine-1,3(2*H*)-dione;
48) 2-(4-(tert-butyl)phenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione;
49) 7,7-dimethyl-10-(4-methylpyrerazin-1-yl)-2-(4-(trifluoromethyl)phenyl)-5,12b-dihydro-1*H*,7*H*-chromeno[4,3-*c*][1,2,4]triazolo[1,2-*a*]pyridazine-1,3(2*H*)-dione;
50) 2-(4-fluorophenyl)-7,7-dimethyl-10-(4-methylpiperazin-1-yl)-5,12b-dihydro-1*H*,7*H-*chromeno[4,3-*c*][1,2,4]triazolo[1,2-*a*]pyridazine-1,3(2*H*)-dione;
51) 2-(4-acetylphenyl)-7,7-dimethyl-10-(4-methylpiperazin-1-yl)-5,12b-dihydro-1*H*,7*H-*chromeno[4,3-*c*][1,2,4]triazolo[1,2-*a*]pyridazine-1,3(2*H*)-dione;
52) 2-(4-acetylphenyl)-7,7-dimethyl-10-(4-(methylsulfonyl)piperazin-1-yl)-5,12b-dihydro-1*H*,7*H*-chromeno[4,3-*c*][1,2,4]triazolo[1,2-*a*]pyridazine-1,3(2*H*)-dione;
53) 10-hydroxy-7,7-dimethyl-2-(4-(morpholine-4-carbonyl)phenyl)-5,12b-dihydro-1*H*,7*H*-chromeno[4,3-*c*][1,2,4]triazolo[1,2-*a*]pyridazine-1,3(2*H*)-dione;
54) tert-butyl 4-(4-(10-hydroxy-7,7-dimethyl-1,3-dioxo-5,12b-dihydro-1*H*,7*H-*chromeno[4,3-*c*][1,2,4]triazolo[1,2-*a*]pyridazin-2(3*H*)-yl)benzoyl)piperazine-1-carboxylate;
55) 10-hydroxy-7,7-dimethyl-2-(4-(piperazine-1-carbonyl)phenyl)-5,12b-dihydro-1*H*,7*H-*chromeno[4,3-*c*][1,2,4]triazolo[1,2-*a*]pyridazine-1,3(2*H*)-dione;
56) 10-hydroxy-7,7-dimethyl-2-(4-(4-methylpiperazine-1-carbonyl)phenyl)-5,12b-dihydro-1*H*,7*H*-chromeno[4,3-*c*][1,2,4]triazolo[1,2-*a*]pyridazine-1,3(2*H*)-dione;
57) 7,7-dimethyl-2-(4-(morpholine-4-carbonyl)phenyl)-10-(pyrrolidin-1-yl)-5,12b-dihydro-1*H*,7*H*-chromeno[4,3-*c*][1,2,4]triazolo[1,2-*a*]pyridazine-1,3(2*H*)-dione;
58) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl pivalate;
59) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 3-methylbutanoate;
60) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl acetate;
61) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl ethyl carbonate;
62) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate;
63) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl morpholine-4-carboxylate;
64) (2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxytetrahydro-2*H*-pyran-2-carboxylic acid;
65) sodium (2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxy tetrahydro-2*H*-pyran-2-carboxylate;
66) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-methylpiperazine-1-carboxylate hydrochloride;
67) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl piperazine-1-carboxylate hydrochloride;
68) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(2-oxopropanoyl)piperazine-1-carboxylate;
69) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
70) disodium 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl phosphate;
71) 1,3-dihydroxy-2-(hydroxymethyl)propane-2-aminium 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl phosphate;
72) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylglycinate hydrochloride;
73) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-methoxyethyl) carbonate;
74) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl pyrrolidine-1-carboxylate;
75) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2,5,8,11-tetraoxatridecan-13-yl) carbonate;
76) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl diethylcarbamate;
77) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-glutaminate 2,2,2-trifluoroacetic acid;
78) (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-arginine 2,2,2-trifluoroacetic acid;
79) 1-(2-(4-acetylphenyl)-7,7-dimethyl-1,3-dioxo-2,3,5,12b-tetrahydro-1*H*,7*H-*chromeno[4,3-*c*][1,2,4]triazolo[1,2-a]pyridazin-10-yl) 2-methyl (2*S*)-pyrrolidine-1,2-dicarboxylate;
80) methyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-leucinate;
81) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-leucinate 2,2,2-trifluoroacetic acid;
82) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-prolyl)piperazine-1-carboxylate hydrochloride;
83) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(2-(methylamino)ethyl)carbamate 2,2,2-trifluoroacetic acid;
84) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-aminoethyl)(ethyl)carbamate hydrochloride;
85) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(pyrrolidin-3-yl)carbamate hydrochloride;
86) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl bis(3-(dimethylamino)propyl)carbamate dihydrochloride;
87) *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N-*methyl-*L*-alanine;
88) sodium *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N-*methyl-*L*-alaninate;
89) *N-*(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N-*methylglycine;
90) sodium *N-*(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N-*methylglycinate;
91) 3-((((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)(methyl)amino)propanoic acid;
92) sodium 3-((((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)(methyl)amino)propanoate;
93) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(aminomethyl)piperidine-1-carboxylate trifluoroacetate;
94) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(piperidin-4-yl)piperazine-1-carboxylate dihydrochloride;
95) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(pyridin-2-ylmethyl)piperazine-1-carboxylate dihydrochloride;
96) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(2-(2-(2-methoxyethoxy)ethoxy)acetyl)piperazine-1-carboxylate;
97) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-valyl)piperazine-1-carboxylate formate;
98) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-leucyl)piperazine-1-carboxylate hydrochloride;
99) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(dimethylglycyl)piperazine-1-carboxylate hydrochloride;
100) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethylpiperazine-1-carboxylate hydrochloride;
101) 2-(4-acetylphenyl)-7,7-dimethyl-1,3-dioxo-2,3,5,12b-tetrahydro-1*H*,7*H-*chromeno[4,3*-c*][1,2,4]triazolo[1,2-*a*]pyridazin-10-yl 2,6-dimethyl-4-prolylpiperazine-1-carboxylate hydrochloride;
102) 2-(4-acetylphenyl)-7,7-dimethyl-1,3-dioxo-2,3,5,12b-tetrahydro-1*H*,7*H-*chromeno[4,3-*c*][1,2,4]triazolo[1,2-a]pyridazin-10-ylheptanoate;
103) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,2-dimethylbutanoate;
104) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl ethyl succinate;
105) 2-(4-acetylphenyl)-7,7-dimethyl-1,3-dioxo-2,3,5,l2b-tetrahydro-1*H*,7*H-*chromeno[4,3-*c*][1,2,4]triazolo[1,2-*a*]pyridazin-10-yl diisopropylcarbamate;
106) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl isopropyl carbonate;
107) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-((2-hydroxyethyl)disulfanyl)ethyl) carbonate;
108) 4-(2-((2-((((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)oxy)ethyl)disulfanyl)ethoxy)-4-oxobutanoic acid;
109) sodium 4-(2-((2-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)oxy)ethyl)disulfanyl)ethoxy )-4-oxobutanoate;
110) 2-(4-acetylphenyl)-10-(2-hydroxyethoxy)-7,7-dimethyl-5,12b-dihydro-1*H*,7*H-*chromeno[4,3-*c*][1,2,4]triazolo[1,2-*a*]pyridazine-1,3(2*H*)-dione;
111) 2-((2-(4-acetylphenyl)-7,7-dimethyl-1,3-dioxo-2,3,5,12b-tetrahydro-1*H*,7*H-*chromeno[4,3-c][1,2,4]triazolo[1,2-*a*]pyridazin-10-yl)oxy)ethyl *L*-leucinate hydrochloride;
112) 2-(4-acetylphenyl)-7,7-dimethyl-10-(2-(piperidin-1-yl)ethoxy)-5,12b-dihydro-1*H*,7*H*-chromeno[4,3-*c*][1,2,4]triazolo[1,2-*a*]pyridazine-1,3(2*H*)-dione hydrochloride;
113) 2-(4-acetylphenyl)-7,7-dimethyl-10-(2-morpholinoethoxy)-5,12b-dihydro-1*H*,7*H-*chromeno[4,3-*c*][1,2,4]triazolo[1,2-*a*]pyridazine-1,3(2*H*)-dione formate;
114) (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)methyl)phosphonic acid;
115) disodium (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)methyl)phosphonate;
116) 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)ethyl dimethylglycinate hydrochloride;
117) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(1-(2-oxopropanoyl)piperidin-4-yl)piperazine-1-carboxylate hydrochloride;
118) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,2-dimethylpiperazine-1-carboxylate hydrochloride;
119) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(dimethylglycyl)-2,2-dimethylpiperazine-1-carboxylate hydrochloride;
120) 2-(4-acetylphenyl)-10-(3-(4-(3-chlorophenyl)piperazin-1-yl)propoxy)-7,7-dimethyl-5,12b-dihydro-1*H*,7*H*-chromeno[4,3-*c*][1,2,4]triazolo[1,2-*a*]pyridazine-1,3(2*H*)-dione dihydrochloride;
121) 2-(4-acetylphenyl)-7,7-dimethyl-10-(((2*S*,3*R*,4*S*,5*S*,6*R*)-3,4,5-trihydroxy-6-(hydroxymethyl)tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione;
122) 2-(4-acetylphenyl)-7,7-dimethyl-10-(((2*S*,3*R*,4*S*,5*R*,6*R*)-3,4,5-trihydroxy-6-(hydroxymethyl)tetrahydro-2H-pyran-2-yl)oxy)-5,12b-dihydro-1*H*,7*H*-chromeno[4,3-*c*][1,2,4]triazolo[1,2-*a*]pyridazine-1,3(2*H*)-dione;
123) 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)-*N*,*N*,*N*-trimethyl-2-oxoethan-1-aminium iodide;
124) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (4-nitrobenzyl) carbonate;
125) 2-(4-acetylphenyl)-7,7-dimethyl-l,3-dioxo-2,3,5,12b-tetrahydro-1*H*,7*H-*chromeno[4,3-*c*][1,2,4]triazolo[1,2-*a*]pyridazin-10-yl piperidine-1-carboxylate;
126) *O*-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl) dimethylcarbamothioate;
127) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2-oxoimidazolidine-1-carboxylate;
128) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (3-(dimethylamino)-2,2-dimethylpropyl)carbamate formate;
129) 2-(4-acetylphenyl)-7,7-dimethyl-1,3-dioxo-2,3,5,12b-tetrahydro-1*H*,7*H-*chromeno[4,3-c][1,2,4]triazolo[1,2-*a*]pyridazin-10-yl (2-(dimethylamino)ethyl)(methyl)carbamate formate;
130) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(piperidin-3-yl)carbamate hydrochloride;
131) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(methylsulfonyl)carbamate;
132) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-valinate 2,2,2-trifluoroacetic acid;
133) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-prolinate 2,2,2-trifluoroacetic acid;
134) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl acetyl-*L*-glutaminate;
135) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-argininate 2,2,2-trifluoroacetic acid;
136) methyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-valinate;
137) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl sulfamate;
138) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl picolinate hydrochloride;
139) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl isonicotinate hydrochloride;
140) 10-hydroxy-2-(4-(1-hydroxyethyl)phenyl)-7,7-dimethyl-5,12b-dihydro-1*H*,7*H-*chromeno[4,3-*c*][1,2,4]triazolo[1,2-*a*]pyridazine-1,3(2*H*)-dione;
141) 2-(4-acetylphenyl)-10-(azetidin-1-yl)-7,7-dimethyl-5,12b-dihydro-1*H*,7*H-*chromeno[4,3-*c*][1,2,4]triazolo[1,2-*a*]pyridazine-1,3(2*H*)-dione;
142) 7,7-dimethyl-1,3-dioxo-2-(4-(trifluoromethyl)phenyl)-2,3,5,12b-tetrahydro-1*H*,7*H-*chromeno[4,3-*c*][1,2,4]triazolo[1,2-*a*]pyridazin-10-yl dihydrogen phosphate;
143) 2-(4-fluorophenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
144) 2-(4-(tert-butyl)phenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
145) 7,7-dimethyl-1,3-dioxo-2-(4-(trifluoromethyl)phenyl)-2,3,5,12b-tetrahydro-1*H*,7*H-*chromeno[4,3-*c*][1,2,4]triazolo[1,2-*a*]pyridazin-10-yl dimethylcarbamate;
146) 2-(4-fluorophenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate; and
147) 2-(4-(tert-butyl)phenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate.

9. A pharmaceutical composition comprising a therapeutically effective amount of the compound, or a pharmaceutically acceptable salt or isomer thereof as defined in any one of Claims 1 to 8 as an active ingredient, together with a pharmaceutically acceptable carrier.

10. The pharmaceutical composition according to Claim 9, which is for the prevention or treatment of a cancer disease.

11. The pharmaceutical composition according to Claim 10, wherein the cancer disease is selected from the group consisting of colon cancer, skin cancer, melanoma, glioblastoma, bone cancer, liver cancer, stomach cancer, pancreas cancer, colon cancer, rectal cancer, blood cancer, bladder cancer, kidney cancer, biliary tract cancer, cervical cancer, uterine cancer, ovarian cancer, breast cancer, lung cancer, non-small cell lung cancer, esophageal cancer, small intestine cancer, endocrine cancer, thyroid cancer and parathyroid cancer.

## Amended claims

### Amended claims under Art. 19.1 PCT

1. A compound of the following Formula 1, or a pharmaceutically acceptable salt or isomer thereof: 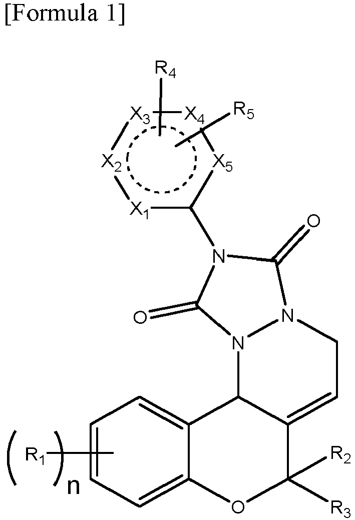 wherein
X₁, X₂, X₃, X₄ and X₅ are each independently carbon or nitrogen;
R₁ is halogen, hydroxy, nitro, amino, cyano, alkyl, cycloalkyl, alkylamino, dialkylamino, alkylcarbonyl, haloalkyl, haloalkoxy, hydroxyalkyl, hydroxyalkoxy, alkylsulfonyl, alkylsulfonylamino, aminosulfonyloxy, 3- to 10-membered heterocycloalkyl, 5-to 10-membered heteroaryl, 3- to 10-membered heterocycloalkyl-oxy, 3- to 10-membered heterocyclyl-alkoxy, or any one of the following formulas (i) to (vii), wherein the heterocycloalkyl or heteroaryl may be substituted with one or more substituents selected from the group consisting of hydroxy, carboxy, alkyl, alkylsulfonyl, alkylcarbonyl, oxo, hydroxyalkyl and halophenyl; 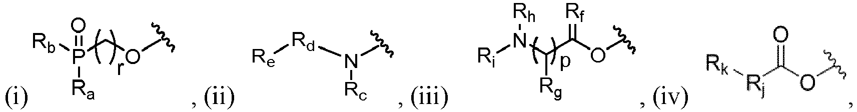 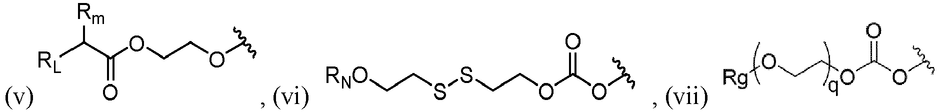
Ra and Rb are each independently hydroxy or alkoxy;
Rc is hydrogen;
Rd is a direct bond, carbonyl or sulfonyl;
Re is hydrogen, alkyl, haloalkyl, alkoxy, amino or 3- to 10-membered heterocycloalkyl;
Rf is O or S;
Rg is hydrogen, alkyl, aminocarbonylalkyl or guanidinoalkyl;
Rh is hydrogen, alkyl, dialkylaminoalkyl, or may be fused with Ri to form 3- to 12-membered heterocycloalkyl, wherein the heterocycloalkyl may be substituted with one or more substituents selected from the group consisting of alkyl, oxo, aminoalkyl, dialkylaminoalkylcarbonyl, alkoxycarbonyl, acetylcarbonyl, 3- to 10-membered heterocycloalkyl, 5- to 10-membered heteroaryl, 3- to 10-membered heterocycloalkylcarbonyl, 3- to 10-membered heterocycloalkyl-alkyl, 5- to 10-membered heteroarylalkyl, acetylcarbonylheterocycloalkyl, alkoxyalkoxyalkoxyalkylcarbonyl and alkylcarbonyl substituted with amino;
Ri is hydrogen, alkyl, aminoalkyl, carboxyalkyl, alkylaminoalkyl, dialkylaminoalkyl, alkylsulfonyl, alkylcarbonyl, 3- to 10-membered heterocycloalkyl, alkyl substituted with alkoxycarbonyl, guanidinoalkyl substituted with carboxy, acetylamino or alkoxycarbonylalkyl;
Rj is a direct bond, alkylene, arylene-alkyleneoxy or alkylene substituted with alkylcarbonylamino;
Rk is alkyl, alkoxycarbonylalkyl, alkoxy, trialkylamino, nitro, alkyl substituted with amino, aminocarbonylalkyl, 3- to 10-membered heterocycloalkyl or 5- to 10-membered heteroaryl;
RL is amino or dialkylamino;
Rm is hydrogen or alkyl;
RN is hydrogen or carboxyalkylcarbonyl;
R₂ and R₃ are each independently alkyl or haloalkyl;
R₄ is hydrogen, halogen, alkyl, alkylcarbonyl, haloalkyl, carboxy, hydroxyalkyl, 3- to 10-membered heterocycloalkylcarbonyl, or 3- to 10-membered heterocycloalkylcarbonyl substituted with alkyl or alkoxycarbonyl;
R₅ is hydrogen, halogen or alkyl;
n is an integer of 1 to 3;
r and p are an integer of 0 to 3; and
q is an integer of 1 to 4;
provided that when R₁ is hydroxy and n is 1, i) R₂ and R₃ are not alkyl at the same time, ii) at least one of X₁, X₂, X₃, X₄ and X₅ is nitrogen, or iii) R₄ is hydroxyalkyl, 3- to 10-membered heterocycloalkylcarbonyl, or 3- to 10-membered heterocycloalkylcarbonyl substituted with alkyl or alkoxycarbonyl; and
when R₁ is halogen or alkylamino, R₄ and R₅ are not hydrogen at the same time; and
wherein the heterocycloalkyl and heteroaryl have one or more heteroatoms selected from the group consisting of N, O and S.

2. The compound, or a pharmaceutically acceptable salt or isomer thereof according to Claim 1, wherein
X₁, X₂, X₃, X₄ and X₅ are each independently -CH=, =CH-, -N= or =N-; or -C= or =C-when it is substituted with R₄ or R₅;
R₁ is halogen, hydroxy, nitro, amino, cyano, C₁-C₇ alkyl, C₃-C₁₀ cycloalkyl, C₁-C₇ alkylamino, di(C₁-C₇ alkyl)amino, C₁-C₇ alkylcarbonyl, halo-C₁-C₇ alkyl, halo-C₁-C₇ alkoxy, hydroxy-C₁-C₇ alkyl, hydroxy-C₁-C₇ alkoxy, C₁-C₇ alkylsulfonyl, C₁-C₇ alkylsulfonylamino, aminosulfonyloxy, 3- to 10-membered heterocycloalkyl, 5- to 10-membered heteroaryl, 3- to 10-membered heterocycloalkyl-oxy, 3- to 10-membered heterocyclyl-alkoxy, or any one of the following formulas (i) to (vii), wherein the heterocycloalkyl or heteroaryl may be substituted with 1 to 4 substituents selected from the group consisting of hydroxy, carboxy, C₁-C₇ alkyl, C₁-C₇ alkylsulfonyl, C₁-C₇ alkylcarbonyl, oxo, hydroxy-C₁-C₇ alkyl and halophenyl; 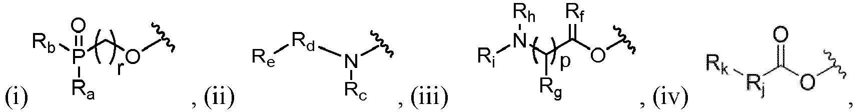 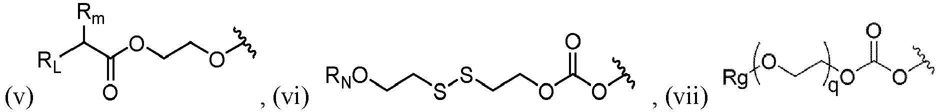
Ra and Rb are each independently hydroxy or C₁-C₇ alkoxy;
Rc is hydrogen;
Rd is a direct bond, carbonyl or sulfonyl;
Re is hydrogen, C₁-C₇ alkyl, halo-C₁-C₇ alkyl, C₁-C₇ alkoxy, amino or 3- to 10-membered heterocycloalkyl;
Rf is O or S;
Rg is hydrogen, C₁-C₇ alkyl, aminocarbonyl-C₁-C₇ alkyl or guanidino-C₁-C₇ alkyl;
Rh is hydrogen, C₁-C₇ alkyl, di(C₁-C₇ alkyl)amino-C₁-C₇ alkyl, or may be fused with Ri to form 3- to 12-membered heterocycloalkyl, wherein the heterocycloalkyl may be substituted with 1 to 4 substituents selected from the group consisting of C₁-C₇ alkyl, oxo, amino-C₁-C₇ alkyl, di(C₁-C₇ alkyl)amino-C₁-C₇ alkylcarbonyl, C₁-C₇ alkoxycarbonyl, acetylcarbonyl, 3- to 10-membered heterocycloalkyl, 5- to 10-membered heteroaryl, 3- to 10-membered heterocycloalkylcarbonyl, 3- to 10-membered heterocycloalkyl-C₁-C₇ alkyl, 5- to 10-membered heteroaryl-C₁-C₇ alkyl, acetylcarbonyl-3- to 10-membered heterocycloalkyl, C₁-C₇ alkoxy-C₁-C₇ alkoxy-C₁-C₇ alkoxy-C₁-C₇ alkylcarbonyl and C₁-C₇ alkylcarbonyl substituted with amino;
Ri is hydrogen, C₁-C₇ alkyl, amino-C₁-C₇ alkyl, carboxy-C₁-C₇ alkyl, C₁-C₇ alkylamino-C₁-C₇ alkyl, di(C₁-C₇ alkyl)amino-C₁-C₇ alkyl, C₁-C₇ alkylsulfonyl, C₁-C₇ alkylcarbonyl, 3- to 10-membered heterocycloalkyl, C₁-C₇ alkyl substituted with C₁-C₇ alkoxycarbonyl, guanidino-C₁-C₇ alkyl substituted with carboxy, acetylamino or C₁-C₇ alkoxycarbonyl-C₁-C₇ alkyl;
Rj is a direct bond, C₁-C₇ alkylene, C₆-C₁₀ arylene-C₁-C₇ alkyleneoxy or C₁-C₇ alkylene substituted with C₁-C₇ alkylcarbonylamino;
Rk is C₁-C₇ alkyl, C₁-C₇ alkoxycarbonyl-C₁-C₇ alkyl, C₁-C₇ alkoxy, tri(C₁-C₇ alkyl)amino, nitro, C₁-C₇ alkyl substituted with amino, aminocarbonyl-C₁-C₇ alkyl, 3- to 10-membered heterocycloalkyl or 5- to 10-membered heteroaryl;
RL is amino or di(C₁-C₇ alkyl)amino;
Rm is hydrogen or C₁-C₇ alkyl;
RN is hydrogen or carboxy-C₁-C₇ alkylcarbonyl;
R₂ and R₃ are each independently C₁-C₇ alkyl or halo-C₁-C₇ alkyl;
R₄ is hydrogen, halogen, C₁-C₇ alkyl, C₁-C₇ alkylcarbonyl, halo-C₁-C₇ alkyl, carboxy, hydroxy-C₁-C₇ alkyl, 3- to 10-membered heterocycloalkylcarbonyl, or 3- to 10-membered heterocycloalkylcarbonyl substituted with C₁-C₇ alkyl or C₁-C₇ alkoxycarbonyl;
R₅ is hydrogen, halogen or C₁-C₇ alkyl;
n is an integer of 1 to 3;
r and p are an integer of 0 to 3; and
q is an integer of 1 to 4;
provided that when R₁ is hydroxy and n is 1, i) R₂ and R₃ are not C₁-C₇ alkyl at the same time, ii) at least one of X₁, X₂, X₃, X₄ and X₅ is -N= or =N-, or iii) R₄ is hydroxy-C₁-C₇ alkyl, 3- to 10-membered heterocycloalkylcarbonyl, or 3- to 10-membered heterocycloalkylcarbonyl substituted with C₁-C₇ alkyl or C₁-C₇ alkoxycarbonyl; and
when R₁ is halogen or C₁-C₇ alkylamino, R₄ and R₅ are not hydrogen at the same time; and
wherein the heterocycloalkyl and heteroaryl have 1 to 4 heteroatoms selected from the group consisting of N, O and S.

3. The compound, or a pharmaceutically acceptable salt or isomer thereof according to Claim 1, wherein the compound of Formula 1 is a compound of the following Formula 2: 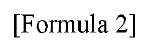 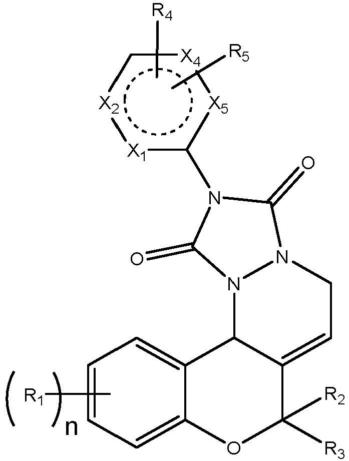 wherein X₁, X₂, X₄, X₅, R₁, R₂, R₃, R₄, R₅ and n are the same as defined in Claim 1.

4. The compound, or a pharmaceutically acceptable salt or isomer thereof according to Claim 1, wherein the compound of Formula 1 is a compound of the following Formula 3: 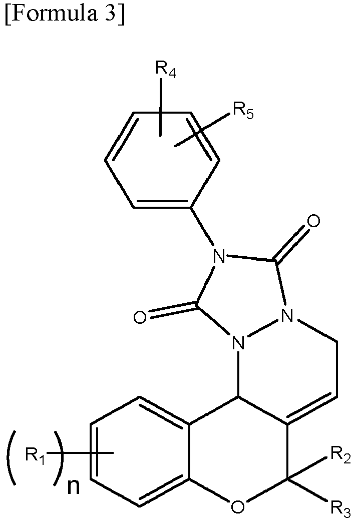 wherein R₁, R₂, R₃, R₄, R₅ and n are the same as defined in Claim 1.

5. The compound, or a pharmaceutically acceptable salt or isomer thereof according to Claim 1, wherein
R₁ is halogen, hydroxy, nitro, amino, cyano, C₁-C₅ alkyl, C₃-C₈ cycloalkyl, C₁-C₅ alkylamino, di(C₁-C₅ alkyl)amino, C₁-C₅ alkylcarbonyl, halo-C₁-C₅ alkyl, halo-C₁-C₅ alkoxy, hydroxy-C₁-C₅ alkyl, hydroxy-C₁-C₅ alkoxy, C₁-C₅ alkylsulfonyl, C₁-C₅ alkylsulfonylamino, aminosulfonyloxy, 4- to 8-membered heterocycloalkyl, 5- to 8-membered heteroaryl, 4- to 8-membered heterocycloalkyl-oxy, 4-to 8-membered heterocyclyl-alkoxy, or any one of the following formulas (i) to (vii); wherein the heterocycloalkyl may be substituted with 1 to 4 substituents selected from the group consisting of hydroxy, carboxy, C₁-C₅ alkyl, C₁-C₅ alkylsulfonyl, C₁-C₅ alkylcarbonyl, oxo, hydroxy-C₁-C₅ alkyl and halophenyl; and the heteroaryl may be substituted with 1 to 3 substituents selected from the group consisting of hydroxy, C₁-C₅ alkyl, C₁-C₅ alkylsulfonyl, C₁-C₅ alkylcarbonyl and hydroxy-C₁-C₅ alkyl; 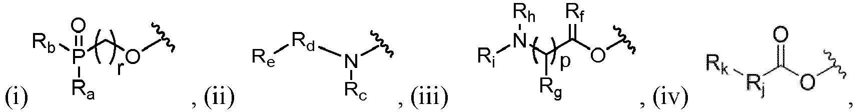 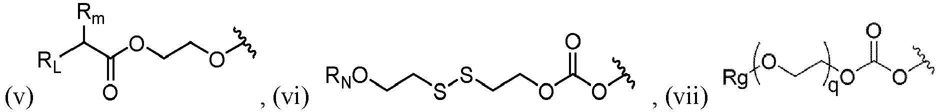
Ra and Rb are each independently hydroxy or C₁-C₅ alkoxy;
Rc is hydrogen;
Rd is a direct bond, carbonyl or sulfonyl;
Re is hydrogen, C₁-C₅ alkyl, halo-C₁-C₅ alkyl, C₁-C₅ alkoxy, amino or 3- to 10-membered heterocycloalkyl;
Rf is O or S;
Rg is hydrogen, C₁-C₅ alkyl, aminocarbonyl-C₁-C₅ alkyl or guanidino-C₁-C₅ alkyl;
Rh is hydrogen, C₁-C₅ alkyl, di(C₁-C₅ alkyl)amino-C₁-C₅ alkyl, or may be fused with Ri to form 4- to 8-membered heterocycloalkyl, wherein the heterocycloalkyl may be substituted with one or more substituents selected from the group consisting of C₁-C₅ alkyl, oxo, amino-C₁-C₅ alkyl, di(C₁-C₅ alkyl)amino-C₁-C₅ alkylcarbonyl, C₁-C₅ alkoxycarbonyl, acetylcarbonyl, 4- to 8-membered heterocycloalkyl, 5- to 8-membered heteroaryl, 4-8 membered heterocycloalkylcarbonyl, 4- to 8-membered heterocycloalkyl-Ci-Cs alkyl, 5- to 8-membered heteroaryl-C₁-C₅ alkyl, acetylcarbonyl-4- to 8-membered heterocycloalkyl, C₁-C₅ alkoxy-C₁-C₅ alkoxy-C₁-C₅ alkoxy-C₁-C₅ alkylcarbonyl and C₁-C₅ alkylcarbonyl substituted with amino;
Ri is hydrogen, C₁-C₅ alkyl, amino-C₁-C₅ alkyl, carboxy-C₁-C₅ alkyl, C₁-C₅ alkylamino-C₁-C₅ alkyl, di(C₁-C₅ alkyl)amino-C₁-C₅ alkyl, C₁-C₅ alkylsulfonyl, C₁-C₅ alkylcarbonyl, 3- to 10-membered heterocycloalkyl, C₁-C₅ alkyl substituted with C₁-C₅ alkoxycarbonyl, guanidino-C₁-C₅ alkyl substituted with carboxy, acetylamino or C₁-C₅ alkoxycarbonyl-C₁-C₅ alkyl;
Rj is a direct bond, C₁-C₅ alkylene, C₆-C₁₀ arylene-C₁-C₅ alkyleneoxy, C₁-C₅ alkylene substituted with C₁-C₅ alkylcarbonylamino;
Rk is C₁-C₅ alkyl, C₁-C₅ alkoxycarbonyl-C₁-C₅ alkyl, C₁-C₅ alkoxy, tri(C₁-C₅ alkyl)amino, nitro, C₁-C₅ alkyl substituted with amino, aminocarbonyl-C₁-C₅ alkyl, 4- to 8-membered heterocycloalkyl or 5- to 8-membered heteroaryl;
RL is amino or di(C₁-C₅ alkyl)amino;
Rm is hydrogen or C₁-C₅ alkyl; and
RN is hydrogen or carboxy-C₁-C₅ alkylcarbonyl.

6. The compound, or a pharmaceutically acceptable salt or isomer thereof according to Claim 1, wherein R₂ and R₃ are each independently C₁-C₅ alkyl or halo-C₁-C₅ alkyl.

7. The compound, or a pharmaceutically acceptable salt or isomer thereof according to Claim 1, wherein
R₄ is hydrogen, halogen, C₁-C₅ alkyl, C₁-C₅ alkylcarbonyl, halo-C₁-C₅ alkyl, carboxy, hydroxy-C₁-C₅ alkyl, 4- to 8-membered heterocycloalkylcarbonyl, or 4- to 8-membered heterocycloalkylcarbonyl substituted with C₁-C₅ alkyl or C₁-C₅ alkoxycarbonyl; and
R₅ is hydrogen, halogen or C₁-C₅ alkyl.

8. The compound, or a pharmaceutically acceptable salt or isomer thereof according to Claim 1, wherein wherein the compound of Formula 1 is selected from the group consisting of:
1) 7,7-bis(fluoromethyl)-10-hydroxy-2-phenyl-5,12b-dihydro-1*H*,7*H*-chromeno[4,3-c][1,2,4]triazolo[1,2-*a*]pyridazine-1,3(2*H*)-dione;
2) 2-(4-acetylphenyl)-7,7-bis(fluoromethyl)-10-hydroxy-5,12b-dihydro-1*H*,7*H-*chromeno[4,3-*c*][1,2,4]triazolo[1,2-*a*]pyridazine-1,3(2*H*)-dione;
3) *N*-(2-(4-acetylphenyl)-7,7-dimethyl-1,3-dioxo-2,3,5,12b-tetrahydroxy-1*H*,7*H-*chromeno[4,3-*c*][1,2,4]triazolo[1,2-*a*]pyridazin-10-yl)acetamide;
4) 2-(4-acetylphenyl)-7,7-dimethyl-10-(methylsulfonyl)-5,12b-dihydro-1*H*,7*H-*chromeno[4,3-*c*][1,2,4]triazolo[1,2-*a*]pyridazine-1,3(2*H*)-dione;
5) *N*-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)methanesulfonamide;
6) tert-butyl (2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)carbamate;
7) 2-(4-acetylphenyl)-10-amino-7,7-dimethyl-5,12b-dihydro-1*H*,7*H*-chromeno[4,3-*c*][1,2,4]triazolo[1,2-*a*]pyridazine-1,3(2*H*)-dione;
8) 2-(4-acetylphenyl)-7,7-dimethyl-10-morpholino-5,12b-dihydro-1*H*,7*H-*chromeno[4,3-*c*][1,2,4]triazolo[1,2-*a*]pyridazine-1,3(2*H*)-dione;
9) *N*-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)sulfuric diamide;
10) 2-(4-acetylphenyl)-9,11-dibromo-10-hydroxy-7,7-dimethyl-5,12b-dihydro-1*H*,7*H*-chromeno[4,3-*c*][1,2,4]triazolo[1,2-*a*]pyridazine-1,3(2*H*)-dione;
11) 2-(4-acetylphenyl)-9,11-dichloro-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
12) 2-(6-fluoropyridin-2-yl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
13) 2-(4-acetylphenyl)-7,7-dimethyl-10-nitro-5,12b-dihydro-1*H*,7*H*-chromeno[4,3-*c*][1,2,4]triazolo[1,2-*a*]pyridazine-1,3(2*H*)-dione;
14) 2-(4-acetylphenyl)-10-(hydroxymethyl)-7,7-dimethyl-5,12b-dihydro-1*H*,7*H-*chromeno[4,3-*c*][1,2,4]triazolo[1,2-*a*]pyridazine-1,3(2*H*)-dione;
15) 1-(2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)urea;
16) 2-(4-acetylphenyl)-10-(ethylamino)-7,7-dimethyl-5,12b-dihydro-1*H*,7*H-*chromeno[4,3-*c*][1,2,4]triazolo[1,2-*a*]pyridazine-1,3(2*H*)-dione;
17) 2-(4-acetylphenyl)-7,7-dimethyl-10-((2,2,2-trifluoroethyl)amino)-5,12b-dihydro-1*H*,7*H*-chromeno[4,3-*c*][1,2,4]triazolo[1,2-*a*]pyridazine-1,3(2*H*)-dione;
18) 2-(4-acetylphenyl)-7,7-dimethyl-10-(piperazin-1-yl)-5,12b-dihydro-1*H*,7*H-*chromeno[4,3-*c*][1,2,4]triazolo[1,2-*a*]pyridazine-1,3(2*H*)-dione hydrochloride;
19) 2-(4-acetylphenyl)-10-(4-acetylpiperazin-1-yl)-7,7-dimethyl-5,12b-dihydro-1*H*,7*H*-chromeno[4,3-*c*][1,2,4]triazolo[1,2-*a*]pyridazine-1,3(2*H*)-dione;
20) 2-(5-acetylpyridin-2-yl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
21) 10-acetyl-2-(4-acetylphenyl)-7,7-dimethyl-5,12b-dihydro-1*H*,7*H-*chromeno[4,3-*c*][1,2,4]triazolo[1,2-*a*]pyridazine-1,3(2*H*)-dione;
22) 11-acetyl-2-(4-acetylphenyl)-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
23) 2-(6-acetylpyridin-3-yl)-10-hydroxy-7,7-dimethyl-5,12b-dihydro-1*H*,7*H-*chromeno[4,3-c][1,2,4]triazolo[1,2-*a*]pyridazine-1,3(2*H*)-dione;
24) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazine-10-carbonitrile;
25) 2-(4-acetylphenyl)-10-hydroxy-7,7-dimethyl-11-(trifluoromethoxy)-5,12b-dihydro-1*H*,7*H*-chromeno[4,3-*c*][1,2,4]triazolo[1,2-*a*]pyridazine-1,3(2*H*)-dione;
26) 2-(4-acetylphenyl)-11-cyclopropyl-10-hydroxy-7,7-dimethyl-5,12b-dihydro-1*H*,7*H*-chromeno[4,3-*c*][1,2,4]triazolo[1,2-*a*]pyridazine-1,3(2*H*)-dione;
27) 2-(4-acetylphenyl)-9,11-difluoro-10-hydroxy-7,7-dimethyl-5,12b-dihydro-1*H*,7*H-*chromeno[4,3-*c*][1,2,4]triazolo[1,2-*a*]pyridazine-1,3(2*H*)-dione;
28) 2-(4-acetylphenyl)-10-amino-9,11-dibromo-7,7-dimethyl-5,12b-dihydro-1*H*,7*H-*chromeno[4,3-*c*][1,2,4]triazolo[1,2-*a*]pyridazine-1,3(2*H*)-dione;
29) 2-(4-acetylphenyl)-10-(isopropylamino)-7,7-dimethyl-5,12b-dihydro-1*H*,7*H-*chromeno[4,3-*c*][1,2,4]triazolo[1,2-*a*]pyridazine-1,3(2*H*)-dione;
30) 2-(4-acetylphenyl)-10-(tert-butylamino)-7,7-dimethyl-5,12b-dihydro-1*H*,7*H-*chromeno[4,3-*c*][1,2,4]triazolo[1,2-*a*]pyridazine-1,3(2*H*)-dione;
31) 2-(4-acetylphenyl)-10-amino-9,11-dichloro-7,7-dimethyl-5,12b-dihydro-1*H*,7*H-*chromeno[4,3-*c*][1,2,4]triazolo[1,2-*a*]pyridazine-1,3(2*H*)-dione;
32) 2-(4-acetylphenyl)-7,7-dimethyl-10-(methylamino)-5,12b-dihydro-1*H*,7*H-*chromeno[4,3-*c*][1,2,4]triazolo[1,2-*a*]pyridazine-1,3(2*H*)-dione;
33) 2-(4-acetylphenyl)-10-(diethylamino)-7,7-dimethyl-5,12b-dihydro-1*H*,7*H-*chromeno[4,3-*c*][1,2,4]triazolo[1,2-*a*]pyridazine-1,3(2*H*)-dione;
34) 2-(4-acetylphenyl)-7,7-dimethyl-10-(pyrrolidin-1-yl)-5,12b-dihydro-1*H*,7*H-*chromeno[4,3-*c*][1,2,4]triazolo[1,2-*a*]pyridazine-1,3(2*H*)-dione;
35) 2-(4-fluorophenyl)-7,7-dimethyl-10-(oxetan-3-ylamino)-5,12b-dihydro-1*H*,7*H-*chromeno[4,3-*c*][1,2,4]triazolo[1,2-*a*]pyridazine-1,3(2*H*)-dione;
36) 2-(4-acetylphenyl)-9,11-dichloro-7,7-dimethyl-10-(methylamino)-5,12b-dihydro-1*H*,7*H*-chromeno[4,3-*c*][1,2,4]triazolo[1,2-*a*]pyridazine-1,3(2*H*)-dione;
37) 2-(4-acetylphenyl)-9,11-dichloro-10-(isopropylamino)-7,7-dimethyl-5,12b-dihydro-1*H*,7*H*-chromeno[4,3-*c*][1,2,4]triazolo[1,2-*a*]pyridazine-1,3(2*H*)-dione;
38) 2-(4-acetylphenyl)-10-(1,3-dimethyl-1*H*-pyrazol-5-yl)-7,7-dimethyl-5,12b-dihydro-1*H*,7*H*-chromeno[4,3-*c*][1,2,4]triazolo[1,2-*a*]pyridazine-1,3(2*H*)-dione;
39) 2-(4-acetylphenyl)-10-(2,5-dimethylthiazol-4-yl)-7,7-dimethyl-5,12b-dihydro-1*H*,7*H*-chromeno[4,3-*c*][1,2,4]triazolo[1,2-*a*]pyridazine-1,3(2*H*)-dione;
40) 2-(4-acetylphenyl)-10-(1-ethyl-3,5-dimethyl-1*H*-pyrazol-4-yl)-7,7-dimethyl-5,12b-dihydro-1*H*,7*H*-chromeno[4,3-*c*][1,2,4]triazolo[1,2-*a*]pyridazine-1,3(2*H*)-dione;
41) 2-(4-acetylphenyl)-7,7-dimethyl-10-(3-methylisoxazol-4-yl)-5,12b-dihydro-1*H*,7*H*-chromeno[4,3-*c*][1,2,4]triazolo[1,2-*a*]pyridazine-1,3(2*H*)-dione;
42) 2-(4-acetylphenyl)-7,7-dimethyl-10-(pyridin-3-yl)-5,12b-dihydro-1*H*,7*H-*chromeno[4,3-*c*][1,2,4]triazolo[1,2-*a*]pyridazine-1,3(2*H*)-dione;
43) 2-(4-acetylphenyl)-7,7-dimethyl-10-(pyridin-4-yl)-5,12b-dihydro-1H,*7H-*chromeno[4,3-*c*][1,2,4]triazolo[1,2-*a*]pyridazine-1,3(2*H*)-dione;
44) 4-(7,7-dimethyl-1,3-dioxo-10-(pyrrolidin-1-yl)-5,12b-dihydro-1*H*,7*H-*chromeno[4,3-*c*][1,2,4]triazolo[1,2-*a*]pyridazin-2(3*H*)-yl)benzoic acid;
45) 2-(4-acetylphenyl)-10-(isopropyl(methyl)amino)-7,7-dimethyl-5,12b-dihydro-1*H*,7*H*-chromeno[4,3-*c*][1,2,4]triazolo[1,2-*a*]pyridazine-1,3(2*H*)-dione;
46) 2-(4-acetylphenyl)-10-(ethyl(isopropyl)amino)-7,7-dimethyl-5,12b-dihydro-1*H*,7*H*-chromeno[4,3-*c*][1,2,4]triazolo[1,2-*a*]pyridazine-1,3(2*H*)-dione;
47) 2-(4-acetylphenyl)-10-(diisopropylamino)-7,7-dimethyl-5,12b-dihydro-1*H*,7*H-*chromeno[4,3-*c*][1,2,4]triazolo[1,2-*a*]pyridazine-1,3(2*H*)-dione;
48) 2-(4-(tert-butyl)phenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione;
49) 7,7-dimethyl-10-(4-methylpyrerazin-1-yl)-2-(4-(trifluoromethyl)phenyl)-5,12b-dihydro-1*H*,7*H*-chromeno[4,3-*c*][1,2,4]triazolo[1,2-*a*]pyridazine-1,3(2*H*)-dione;
50) 2-(4-fluorophenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione;
51) 2-(4-acetylphenyl)-7,7-dimethyl-10-(4-methylpiperazin-1-yl)-5,12b-dihydro-1*H*,7*H*-chromeno[4,3-*c*][1,2,4]triazolo[1,2-*a*]pyridazine-1,3(2*H*)-dione;
52) 2-(4-acetylphenyl)-7,7-dimethyl-10-(4-(methylsulfonyl)piperazin-1-yl)-5,12b-dihydro-1*H*,7*H*-chromeno[4,3-*c*][1,2,4]triazolo[1,2-*a*]pyridazine-1,3(2*H*)-dione;
53) 10-hydroxy-7,7-dimethyl-2-(4-(morpholine-4-carbonyl)phenyl)-5,12b-dihydro-1*H*,7*H*-chromeno[4,3-*c*][1,2,4]triazolo[1,2-*a*]pyridazine-1,3(2*H*)-dione;
54) tert-butyl 4-(4-(10-hydroxy-7,7-dimethyl-1,3-dioxo-5,12b-dihydro-1*H*,7*H-*chromeno[4,3-*c*][1,2,4]triazolo[1,2-*a*]pyridazin-2(3*H*)-yl)benzoyl)piperazine-1-carboxylate;
55) 10-hydroxy-7,7-dimethyl-2-(4-(piperazine-1-carbonyl)phenyl)-5,12b-dihydro-1*H*,7*H*-chromeno[4,3-*c*][1,2,4]triazolo[1,2-*a*]pyridazine-1,3(2*H*)-dione;
56) 10-hydroxy-7,7-dimethyl-2-(4-(4-methylpiperazine-1-carbonyl)phenyl)-5,12b-dihydro-1*H*,7*H*-chromeno[4,3-*c*][1,2,4]triazolo[1,2-*a*]pyridazine-1,3(2*H*)-dione;
57) 7,7-dimethyl-2-(4-(morpholine-4-carbonyl)phenyl)-10-(pyrrolidin-1-yl)-5,12b-dihydro-1*H*,7*H*-chromeno[4,3-*c*][1,2,4]triazolo[1,2-*a*]pyridazine-1,3(2*H*)-dione;
58) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl pivalate;
59) 2-(4-acetylphenyl)-7,7-dimethyl-1,3-dioxo-2,3,5,12b-tetrahydro-1*H*,7*H-*chromeno[4,3-c][1,2,4]triazolo[1,2-*a*]pyridazin-10-yl 3-methylbutanoate;
60) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl acetate;
61) 2-(4-acetylphenyl)-7,7-dimethyl-1,3-dioxo-2,3,5,l2b-tetrahydro-1*H*,7*H-*chromeno[4,3-*c*][1,2,4]triazolo[1,2-*a*]pyridazin-10-yl ethyl carbonate;
62) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate;
63) 2-(4-acetylphenyl)-7,7-dimethyl-l,3-dioxo-2,3,5,12b-tetrahydro-1*H*,7*H-*chromeno[4,3-*c*][1,2,4]triazolo[1,2-*a*]pyridazin-10-yl morpholine-4-carboxylate;
64) (2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxytetrahydro-2*H*-pyran-2-carboxylic acid;
65) sodium (2*S*,3*S*,4*S*,5*R*,6*S*)-6-((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)-3,4,5-trihydroxy tetrahydro-2*H*-pyran-2-carboxylate;
66) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-methylpiperazine-1-carboxylate hydrochloride;
67) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl piperazine-1-carboxylate hydrochloride;
68) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(2-oxopropanoyl)piperazine-1-carboxylate;
69) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
70) disodium 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl phosphate;
71) 1,3-dihydroxy-2-(hydroxymethyl)propane-2-aminium 2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl phosphate;
72) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylglycinate hydrochloride;
73) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-methoxyethyl) carbonate;
74) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl pyrrolidine-1-carboxylate;
75) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2,5,8,11-tetraoxatridecan-13-yl) carbonate;
76) 2-(4-acetylphenyl)-7,7-dimethyl-1,3-dioxo-2,3,5,12b-tetrahydro-1*H*,7*H-*chromeno[4,3-c][1,2,4]triazolo[1,2-a]pyridazin-10-yl diethylcarbamate;
77) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl L-glutaminate 2,2,2-trifluoroacetic acid;
78) (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-L-arginine 2,2,2-trifluoroacetic acid;
79) 1-(2-(4-acetylphenyl)-7,7-dimethyl-1,3-dioxo-2,3,5,12b-tetrahydro-1*H*,7*H-*chromeno[4,3-c][1,2,4]triazolo[1,2-a]pyridazin-10-yl) 2-methyl (2*S*)-pyrrolidine-1,2-dicarboxylate;
80) methyl (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*L*-leucinate;
81) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-leucinate 2,2,2-trifluoroacetic acid;
82) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-prolyl)piperazine-1-carboxylate hydrochloride;
83) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(2-(methylamino)ethyl)carbamate 2,2,2-trifluoroacetic acid;
84) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-aminoethyl)(ethyl)carbamate hydrochloride;
85) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(pyrrolidin-3-yl)carbamate hydrochloride;
86) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl bis(3-(dimethylamino)propyl)carbamate dihydrochloride;
87) *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N-*methyl-*L*-alanine;
88) sodium *N*-(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N-*methyl-*L-*alaninate;
89) *N*-(((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)-*N-*methylglycine;
90) sodium *N*-(((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*N-*methylglycinate;
91) 3-((((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)carbonyl)(methyl)amino)propanoic acid;
92) sodium 3-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)(methyl)amino)propanoate;
93) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(aminomethyl)piperidine-1-carboxylate trifluoroacetate;
94) 2-(4-acetylphenyl)-7,7-dimethyl-1,3-dioxo-2,3,5,12b-tetrahydro-1*H*,7*H-*chromeno[4,3-c][1,2,4]triazolo[1,2-*a*]pyridazin-10-yl 4-(piperidin-4-yl)piperazine-1-carboxylate dihydrochloride;
95) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(pyridin-2-ylmethyl)piperazine-1-carboxylate dihydrochloride;
96) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(2-(2-(2-methoxyethoxy)ethoxy)acetyl)piperazine-1-carboxylate;
97) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-valyl)piperazine-1-carboxylate formate;
98) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(*L*-leucyl)piperazine-1-carboxylate hydrochloride;
99) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(dimethylglycyl)piperazine-1-carboxylate hydrochloride;
100) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethylpiperazine-1-carboxylate hydrochloride;
101) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,6-dimethyl-4-prolylpiperazine-1-carboxylate hydrochloride;
102) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-ylheptanoate;
103) 2-(4-acetylphenyl)-7,7-dimethyl-1,3-dioxo-2,3,5,12b-tetrahydro-1*H*,7*H-*chromeno[4,3-c][1,2,4]triazolo[1,2-*a*]pyridazin-10-yl 2,2-dimethylbutanoate;
104) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl ethyl succinate;
105) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl diisopropylcarbamate;
106) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl isopropyl carbonate;
107) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-((2-hydroxyethyl)disulfanyl)ethyl) carbonate;
108) 4-(2-((2-((((2-(4-acetylphenyl)-7,7-dimethyl-1,3-dioxo-2,3,5,12b-tetrahydro-1*H*, 7*H*-chromeno[4,3-*c*][1,2,4]triazolo[1,2-*a*]pyridazin-10-yl)oxy)carbonyl)oxy)ethyl)disulfanyl)ethoxy)-4-oxobutanoic acid;
109) sodium 4-(2-((2-((((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)oxy)ethyl)disulfanyl)ethoxy )-4-oxobutanoate;
110) 2-(4-acetylphenyl)-10-(2-hydroxyethoxy)-7,7-dimethyl-5,12b-dihydro-1*H*,7*H-*chromeno[4,3-*c*][1,2,4]triazolo[1,2-*a*]pyridazine-1,3(2*H*)-dione;
111) 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)ethyl *L*-leucinate hydrochloride;
112) 2-(4-acetylphenyl)-7,7-dimethyl-10-(2-(piperidin-1-yl)ethoxy)-5,12b-dihydro-1*H*,7*H*-chromeno[4,3-*c*][1,2,4]triazolo[1,2-*a*]pyridazine-1,3(2*H*)-dione hydrochloride;
113) 2-(4-acetylphenyl)-7,7-dimethyl-10-(2-morpholinoethoxy)-5,12b-dihydro-1*H*,7*H*-chromeno[4,3-*c*][1,2,4]triazolo[1,2-*a*]pyiidazine-1,3(2*H*)-dione formate;
114) (((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)methyl)phosphonic acid;
115) disodium (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)methyl)phosphonate;
116) 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)ethyl dimethylglycinate hydrochloride;
117) 2-(4-acetylphenyl)-7,7-dimethyl-1,3-dioxo-2,3,5,12b-tetrahydro-1*H*,7*H-*chromeno[4,3*-c*][1,2,4]triazolo[1,2-*a*]pyridazin-10-yl 4-(1-(2-oxopropanoyl)piperidin-4-yl)piperazine-1-carboxylate hydrochloride;
118) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2,2-dimethylpiperazine-1-carboxylate hydrochloride;
119) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 4-(dimethylglycyl)-2,2-dimethylpiperazine-1-carboxylate hydrochloride;
120) 2-(4-acetylphenyl)-10-(3-(4-(3-chlorophenyl)piperazin-1-yl)propoxy)-7,7-dimethyl-5,12b-dihydro-1*H*,7*H*-chromeno[4,3-*c*][1,2,4]triazolo[1,2-*a*]pyridazine-1,3(2*H*)-dione dihydrochloride;
121) 2-(4-acetylphenyl)-7,7-dimethyl-10-(((2*S*,3*R*,4*S*,5*S*,6*R*)-3,4,5-trihydroxy-6-(hydroxymethyl)tetrahydro-2*H*-pyran-2-yl)oxy)-5,12b-dihydro-1*H*,7*H*-chromeno[4,3-*c*][1,2,4]triazolo[1,2-*a*]pyridazine-1,3(2*H*)-dione;
122) 2-(4-acetylphenyl)-7,7-dimethyl-10-(((2*S*,3*R*,4*S*,5*R*,6*R*)-3,4,5-trihydroxy-6-(hydroxymethyl)tetrahydro-2H-pyran-2-yl)oxy)-5,12b-dihydro-1*H*,7*H*-chromeno[4,3-*c*][1,2,4]triazolo[1,2-*a*]pyridazine-1,3(2*H*)-dione;
123) 2-((2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl)oxy)-*N*,*N*,*N*-trimethyl-2-oxoethan-1-aminium iodide;
124) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (4-nitrobenzyl) carbonate;
125) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl piperidine-1-carboxylate;
126) *O*-(2-(4-acetylphenyl)-7,7-dimethyl-1,3-dioxo-2,3,5,12b-tetrahydro-1H,7H-chromeno[4,3-c ][1,2,4]triazolo[1,2-a]pyridazin-10-yl) dimethylcarbamothioate;
127) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl 2-oxoimidazolidine-1-carboxylate;
128) 2-(4-acetylphenyl)-7,7-dimethyl-l,3-dioxo-2,3,5,12b-tetrahydro-1*H*,7*H-*chromeno[4,3-*c*][1,2,4]triazolo[1,2-*a*]pyridazin-10-yl (3-(dimethylamino)-2,2-dimethylpropyl)carbamate formate;
129) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl (2-(dimethylamino)ethyl)(methyl)carbamate formate;
130) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(piperidin-3-yl)carbamate hydrochloride;
131) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl methyl(methylsulfonyl)carbamate;
132) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-valinate 2,2,2-trifluoroacetic acid;
133) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-prolinate 2,2,2-trifluoroacetic acid;
134) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl acetyl-*L*-glutaminate;
135) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl *L*-argininate 2,2,2-trifluoroacetic acid;
136) methyl (((2-(4-acetylphenyl)-7,7-dimethyl-1,3-dioxo-2,3,5,12b-tetrahydro-1*H*,7*H*-chromeno[4,3-*c*][1,2,4]triazolo[1,2-*a*]pyridazin-10-yl)oxy)carbonyl)-*L*-valinate;
137) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl sulfamate;
138) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl picolinate hydrochloride;
139) 2-(4-acetylphenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl isonicotinate hydrochloride;
140) 10-hydroxy-2-(4-(1-hydroxyethyl)phenyl)-7,7-dimethyl-5,12b-dihydro-1*H*,7*H-*chromeno[4,3-*c*][1,2,4]triazolo[1,2-*a*]pyridazine-1,3(2*H*)-dione;
141) 2-(4-acetylphenyl)-10-(azetidin-1-yl)-7,7-dimethyl-5,12b-dihydro-1*H*,7*H-*chromeno[4,3-*c*][1,2,4]triazolo[1,2-*a*]pyridazine-1,3(2*H*)-dione;
142) 7,7-dimethyl-1,3-dioxo-2-(4-(trifluoromethyl)phenyl)-2,3,5,12b-tetrahydro-1*H*,7*H*-chromeno[4,3-*c*][1,2,4]triazolo[1,2-*a*]pyridazin-10-yl dihydrogen phosphate;
143) 2-(4-fluorophenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
144) 2-(4-(tert-butyl)phenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dihydrogen phosphate;
145) 7,7-dimethyl-1,3-dioxo-2-(4-(trifluoromethyl)phenyl)-2,3,5,12b-tetrahydro-1*H*,7*H*-chromeno[4,3-*c*][1,2,4]triazolo[1,2-*a*]pyridazin-10-yl dimethylcarbamate;
146) 2-(4-fluorophenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate; and
147) 2-(4-(tert-butyl)phenyl)-7,7-dimethyl-1,3-dioxo-2,3,5,12b-tetrahydro-1*H*,7*H-*chromeno[4,3-*c*][1,2,4]triazolo[1,2-*a*]pyridazin-10-yl dimethylcarbamate.

9. A pharmaceutical composition comprising a therapeutically effective amount of the compound, or a pharmaceutically acceptable salt or isomer thereof as defined in any one of Claims 1 to 8 as an active ingredient, together with a pharmaceutically acceptable carrier.

10. The pharmaceutical composition according to Claim 9, which is for the prevention or treatment of a cancer disease.

11. The pharmaceutical composition according to Claim 10, wherein the cancer disease is selected from the group consisting of colon cancer, skin cancer, melanoma, glioblastoma, bone cancer, liver cancer, stomach cancer, pancreas cancer, colon cancer, rectal cancer, blood cancer, bladder cancer, kidney cancer, biliary tract cancer, cervical cancer, uterine cancer, ovarian cancer, breast cancer, lung cancer, non-small cell lung cancer, esophageal cancer, small intestine cancer, endocrine cancer, thyroid cancer and parathyroid cancer.
